# EUROPÄISCHE PATENTSCHRIFT

(11) **EP 2 036 984 B1**
(45) Veröffentlichungstag und Bekanntmachung des Hinweises auf die Patenterteilung: **22.02.2012**
(21) Anmeldenummer: 08159889.8
(22) Anmeldetag: 18.07.2003
(51) Int. Cl.: C12N 15/82, C12N 9/12, C12N 15/54, C12N 15/11, A01H 5/00

(54) **Revertierung der negativ-selektiven Wirkung von negativen Markerproteinen als Selektionsverfahren**
Reversion of the negative selective effect of negative marker proteins as selection procedure
Reversion de l'effet sélectif négatif d'un protéin de marquage comme procédure de sélection

(30) Priorität: 26.07.2002 DE 10234287
(43) Veröffentlichungstag der Anmeldung: 18.03.2009
(62) Teilanmeldung aus: 03766234.3
(73) Patentinhaber: BASF Plant Science GmbH, 67056 Ludwigshafen (DE)
(72) Erfinder: Kock, Michael, 79618 Rheinfelden (DE); Frank, Markus, 67434 Neustadt (DE); Badur, Ralf, 79539 Lörrach (DE)
(74) Vertreter: Popp, Andreas

(56) Entgegenhaltungen:
- ESCOBAR M A ET AL: "RNAi-mediated oncogene silencing confers resistance to crown gall tumorigenesis" PROCEEDINGS OF THE NATIONAL ACADEMY OF SCIENCES OF USA, NATIONAL ACADEMY OF SCIENCE, WASHINGTON, DC.; US, Bd. 98, Nr. 23, 6. November 2001 (2001-11-06), Seiten 13437-13442, XP002998547 ISSN: 0027-8424
- CAPLEN N J ET AL: "dsRNA-mediated gene silencing in cultured Drosophila cells: a tissue culture model for the analysis of RNA interference" GENE, ELSEVIER, AMSTERDAM, NL, Bd. 252, Nr. 1-2, 11. Juli 2000 (2000-07-11), Seiten 95-105, XP004210158 ISSN: 0378-1119
- CHUANG CHIOU-FEN ET AL: "Specific and heritable genetic interference by double-stranded RNA in Arabidopsis thaliana" PROCEEDINGS OF THE NATIONAL ACADEMY OF SCIENCES OF USA, NATIONAL ACADEMY OF SCIENCE. WASHINGTON, US, Bd. 97, Nr. 9, 25. April 2000 (2000-04-25), Seiten 4985-4990, XP002184942 ISSN: 0027-8424
- WESLEY S VARSHA ET AL: "Construct design for efficient, effective and high-throughput gene silencing in plants" PLANT JOURNAL, BLACKWELL SCIENTIFIC PUBLICATIONS, OXFORD, GB, Bd. 27, Nr. 6, 1. September 2001 (2001-09-01), Seiten 581-590, XP002187670 ISSN: 0960-7412
- LEVIN J Z ET AL: "METHODS OF DOUBLE-STRANDED RNA-MEDIATED GENE INACTIVATION IN ARABIDOPSIS AND THEIR USE TO DEFINE AN ESSENTIAL GENE IN METHIONINE BIOSYNTHESIS" PLANT MOLECULAR BIOLOGY, SPRINGER, DORDRECHT, NL, Bd. 44, Nr. 6, 1. Dezember 2000 (2000-12-01), Seiten 759-775, XP001148825 ISSN: 0167-4412
- WANG E ET AL: "Elucidation of the functions of genes central to diterpene metabolism in tobacco trichomes using posttranscriptional gene silencing" PLANTA, SPRINGER VERLAG, DE, Bd. 216, Nr. 4, 1. Februar 2003 (2003-02-01), Seiten 686-691, XP002318258 ISSN: 0032-0935

### SEQUENZPROTOKOLL

<110> BASF Plant Science GmbH
<120> Neue Selektionssysteme
<130> PF53790-AT
<140>
   <141>
<160> 71
<170> PatentIn Ver. 2.1
<210> 1
   <211> 1284
   <212> DNA
   <213> Escherichia coli
<220>
   <221> CDS
   <222> (1)..(1281)
   <223> coding for cytosine deaminase (codA)
<400> 1
   gtg tcg aat aac gct tta caa aca att att aac gcc cgg tta cca ggc     48
   Val Ser Asn Asn Ala Leu Gln Thr Ile Ile Asn Ala Arg Leu Pro Gly
    1               5                  10                  15
   gaa gag ggg ctg tgg cag att cat ctg cag gac gga aaa atc agc gcc     96
   Glu Glu Gly Leu Trp Gln Ile His Leu Gln Asp Gly Lys Ile Ser Ala
               20                  25                  30
   att gat gcg caa tcc ggc gtg atg ccc ata act gaa aac agc ctg gat    144
   Ile Asp Ala Gln Ser Gly Val Met Pro Ile Thr Glu Asn Ser Leu Asp
           35                  40                  45
   gcc gaa caa ggt tta gtt ata ccg ccg ttt gtg gag cca cat att cac    192
   Ala Glu Gln Gly Leu Val Ile Pro Pro Phe Val Glu Pro His Ile His
       50                  55                  60
   ctg gac acc acg caa acc gcc gga caa ccg aac tgg aat cag tcc ggc    240
   Leu Asp Thr Thr Gln Thr Ala Gly Gln Pro Asn Trp Asn Gln Ser Gly
   65                  70                  75                  80
   acg ctg ttt gaa ggc att gaa cgc tgg gcc gag cgc aaa gcg tta tta    288
   Thr Leu Phe Glu Gly Ile Glu Arg Trp Ala Glu Arg Lys Ala Leu Leu
                   85                  90                  95
   acc cat gac gat gtg aaa caa cgc gca tgg caa acg ctg aaa tgg cag    336
   Thr His Asp Asp Val Lys Gln Arg Ala Trp Gln Thr Leu Lys Trp Gln
               100                 105                 110
   att gcc aac ggc att cag cat gtg cgt acc cat gtc gat gtt tcg gat    384
   Ile Ala Asn Gly Ile Gln His Val Arg Thr His Val Asp Val Ser Asp
           115                 120                 125
   gca acg cta act gcg ctg aaa gca atg ctg gaa gtg aag cag gaa gtc    432
   Ala Thr Leu Thr Ala Leu Lys Ala Met Leu Glu Val Lys Gln Glu Val
       130                 135                 140
   gcg ccg tgg att gat ctg caa atc gtc gcc ttc cct cag gaa ggg att    480
   Ala Pro Trp Ile Asp Leu Gln Ile Val Ala Phe Pro Gln Glu Gly Ile
   145                 150                 155                 160
   ttg tcg tat ccc aac ggt gaa gcg ttg ctg gaa gag gcg tta cgc tta    528
   Leu Ser Tyr Pro Asn Gly Glu Ala Leu Leu Glu Glu Ala Leu Arg Leu
                   165                 170                 175
   ggg gca gat gta gtg ggg gcg att ccg cat ttt gaa ttt acc cgt gaa     576
   Gly Ala Asp Val Val Gly Ala Ile Pro His Phe Glu Phe Thr Arg Glu
               180                 185                 190
   tac ggc gtg gag tcg ctg cat aaa acc ttc gcc ctg gcg caa aaa tac     624
   Tyr Gly Val Glu Ser Leu His Lys Thr Phe Ala Leu Ala Gln Lys Tyr
           195                 200                 205
   gac cgt ctc atc gac gtt cac tgt gat gag atc gat gac gag cag tcg     672
   Asp Arg Leu Ile Asp Val His Cys Asp Glu Ile Asp Asp Glu Gln Ser
       210                 215                 220
   cgc ttt gtc gaa acc gtt gct gcc ctg gcg cac cat gaa ggc atg ggc     720
   Arg Phe Val Glu Thr Val Ala Ala Leu Ala His His Glu Gly Met Gly
   225                 230                 235                 240
   gcg cga gtc acc gcc agc cac acc acg gca atg cac tcc tat aac ggg     768
   Ala Arg Val Thr Ala Ser His Thr Thr Ala Met His Ser Tyr Asn Gly
                   245                 250                 255
   gcg tat acc tca cgc ctg ttc cgc ttg ctg aaa atg tcc ggt att aac     816
   Ala Tyr Thr Ser Arg Leu Phe Arg Leu Leu Lys Met Ser Gly Ile Asn
               260                 265                 270
   ttt gtc gcc aac ccg ctg gtc aat att cat ctg caa gga cgt ttc gat     864
   Phe Val Ala Asn Pro Leu Val Asn Ile His Leu Gln Gly Arg Phe Asp
           275                 280                 285
   acg tat cca aaa cgt cgc ggc atc acg cgc gtt aaa gag atg ctg gag     912
   Thr Tyr Pro Lys Arg Arg Gly Ile Thr Arg Val Lys Glu Met Leu Glu
       290                 295                 300
   tcc ggc att aac gtc tgc ttt ggt cac gat gat gtc ttc gat ccg tgg     960
   Ser Gly Ile Asn Val Cys Phe Gly His Asp Asp Val Phe Asp Pro Trp
   305                 310                 315                 320
   tat ccg ctg gga acg gcg aat atg ctg caa gtg ctg cat atg ggg ctg    1008
   Tyr Pro Leu Gly Thr Ala Asn Met Leu Gln Val Leu His Met Gly Leu
                   325                 330                 335
   cat gtt tgc cag ttg atg ggc tac ggg cag att aac gat ggc ctg aat    1056
   His Val Cys Gln Leu Met Gly Tyr Gly Gln Ile Asn Asp Gly Leu Asn
               340                 345                 350
   tta atc acc cac cac agc gca agg acg ttg aat ttg cag gat tac ggc    1104
   Leu Ile Thr His His Ser Ala Arg Thr Leu Asn Leu Gln Asp Tyr Gly
           355                 360                 365
   att gcc gcc gga aac agc gcc aac ctg att atc ctg ccg gct gaa aat    1152
   Ile Ala Ala Gly Asn Ser Ala Asn Leu Ile Ile Leu Pro Ala Glu Asn
       370                 375                 380
   ggg ttt gat gcg ctg cgc cgt cag gtt ccg gta cgt tat tcg gta cgt    1200
   Gly Phe Asp Ala Leu Arg Arg Gln Val Pro Val Arg Tyr Ser Val Arg
   385                 390                 395                 400
   ggc ggc aag gtg att gcc agc aca caa ccg gca caa acc acc gta tat    1248
   Gly Gly Lys Val Ile Ala Ser Thr Gln Pro Ala Gln Thr Thr Val Tyr
                   405                 410                 415
   ctg gag cag cca gaa gcc atc gat tac aaa cgt tga                    1284
   Leu Glu Gln Pro Glu Ala Ile Asp Tyr Lys Arg
               420                 425
<210> 2
   <211> 427
   <212> PRT
   <213> Escherichia coli
<400> 2
   Val Ser Asn Asn Ala Leu Gln Thr Ile Ile Asn Ala Arg Leu Pro Gly
   1               5                   10                  15
   Glu Glu Gly Leu Trp Gln Ile His Leu Gln Asp Gly Lys Ile Ser Ala
               20                  25                  30
   Ile Asp Ala Gln Ser Gly Val Met Pro Ile Thr Glu Asn Ser Leu Asp
           35                  40                  45
   Ala Glu Gln Gly Leu Val Ile Pro Pro Phe Val Glu Pro His Ile His
       50                  55                  60
   Leu Asp Thr Thr Gln Thr Ala Gly Gln Pro Asn Trp Asn Gln Ser Gly
   65                  70                  75                  80
   Thr Leu Phe Glu Gly Ile Glu Arg Trp Ala Glu Arg Lys Ala Leu Leu
                   85                  90                  95
   Thr His Asp Asp Val Lys Gln Arg Ala Trp Gln Thr Leu Lys Trp Gln
               100                 105                 110
   Ile Ala Asn Gly Ile Gln His Val Arg Thr His Val Asp Val Ser Asp
           115                 120                 125
   Ala Thr Leu Thr Ala Leu Lys Ala Met Leu Glu Val Lys Gln Glu Val
       130                 135                 140
   Ala Pro Trp Ile Asp Leu Gln Ile Val Ala Phe Pro Gln Glu Gly Ile
   145                 150                 155                 160
   Leu Ser Tyr Pro Asn Gly Glu Ala Leu Leu Glu Glu Ala Leu Arg Leu
                   165                 170                 175
   Gly Ala Asp Val Val Gly Ala Ile Pro His Phe Glu Phe Thr Arg Glu
               180                 185                 190
   Tyr Gly Val Glu Ser Leu His Lys Thr Phe Ala Leu Ala Gln Lys Tyr
           195                 200                 205
   Asp Arg Leu Ile Asp Val His Cys Asp Glu Ile Asp Asp Glu Gln Ser
       210                 215                 220
   Arg Phe Val Glu Thr Val Ala Ala Leu Ala His His Glu Gly Met Gly
   225                 230                 235                 240
   Ala Arg Val Thr Ala Ser His Thr Thr Ala Met His Ser Tyr Asn Gly
                   245                 250                 255
   Ala Tyr Thr Ser Arg Leu Phe Arg Leu Leu Lys Met Ser Gly Ile Asn
               260                 265                 270
   Phe Val Ala Asn Pro Leu Val Asn Ile His Leu Gln Gly Arg Phe Asp
           275                 280                 285
   Thr Tyr Pro Lys Arg Arg Gly Ile Thr Arg Val Lys Glu Met Leu Glu
       290                 295                 300
   Ser Gly Ile Asn Val Cys Phe Gly His Asp Asp Val Phe Asp Pro Trp
   305                 310                 315                 320
   Tyr Pro Leu Gly Thr Ala Asn Met Leu Gln Val Leu His Met Gly Leu
                   325                 330                 335
   His Val Cys Gln Leu Met Gly Tyr Gly Gln Ile Asn Asp Gly Leu Asn
               340                 345                 350
   Leu Ile Thr His His Ser Ala Arg Thr Leu Asn Leu Gln Asp Tyr Gly
   355 360 365
   Ile Ala Ala Gly Asn Ser Ala Asn Leu Ile Ile Leu Pro Ala Glu Asn
   370 375 380
   Gly Phe Asp Ala Leu Arg Arg Gln Val Pro Val Arg Tyr Ser Val Arg
   385 390 395 400
   Gly Gly Lys Val Ile Ala Ser Thr Gln Pro Ala Gln Thr Thr Val Tyr
   405 410 415
   Leu Glu Gln Pro Glu Ala Ile Asp Tyr Lys Arg
   420 425
<210> 3
   <211> 1284
   <212> DNA
   <213> Künstliche Sequenz
<220>
   <223> Beschreibung der künstlichen Sequenz: coding for cytosine deaminase (codA)
<220>
   <221> misc_feature
   <222> (1). .(3)
   <223> mutation of GTG to ATG start codon for expression in eucaryotic hosts
<220>
   <221> CDS
   <222> (1)..(1281)
   <223> coding for cytosine deaminase (codA)
<400> 3
   atg tcg aat aac gct tta caa aca att att aac gcc cgg tta cca ggc    48
   Met Ser Asn Asn Ala Leu Gln Thr Ile Ile Asn Ala Arg Leu Pro Gly
    1               5                  10                  15
   gaa gag ggg ctg tgg cag att cat ctg cag gac gga aaa atc agc gcc    96
   Glu Glu Gly Leu Trp Gln Ile His Leu Gln Asp Gly Lys Ile Ser Ala
               20                  25                  30
   att gat gcg caa tcc ggc gtg atg ccc ata act gaa aac agc ctg gat   144
   Ile Asp Ala Gln Ser Gly Val Met Pro Ile Thr Glu Asn Ser Leu Asp
           35                  40                  45
   gcc gaa caa ggt tta gtt ata ccg ccg ttt gtg gag cca cat att cac   192
   Ala Glu Gln Gly Leu Val Ile Pro Pro Phe Val Glu Pro His Ile His
       50                  55                  60
   ctg gac acc acg caa acc gcc gga caa ccg aac tgg aat cag tcc ggc   240
   Leu Asp Thr Thr Gln Thr Ala Gly Gln Pro Asn Trp Asn Gln Ser Gly
   65                  70                  75                  80
   acg ctg ttt gaa ggc att gaa cgc tgg gcc gag cgc aaa gcg tta tta   288
   Thr Leu Phe Glu Gly Ile Glu Arg Trp Ala Glu Arg Lys Ala Leu Leu
                   85                  90                  95
   acc cat gac gat gtg aaa caa cgc gca tgg caa acg ctg aaa tgg cag   336
   Thr His Asp Asp Val Lys Gln Arg Ala Trp Gln Thr Leu Lys Trp Gln
               100                 105                 110
   att gcc aac ggc att cag cat gtg cgt acc cat gtc gat gtt tcg gat   384
   Ile Ala Asn Gly Ile Gln His Val Arg Thr His Val Asp Val Ser Asp
           115                 120                 125
   gca acg cta act gcg ctg aaa gca atg ctg gaa gtg aag cag gaa gtc     432
   Ala Thr Leu Thr Ala Leu Lys Ala Met Leu Glu Val Lys Gln Glu Val
       130                 135                 140
   gcg ccg tgg att gat ctg caa atc gtc gcc ttc cct cag gaa ggg att     480
   Ala Pro Trp Ile Asp Leu Gln Ile Val Ala Phe Pro Gln Glu Gly Ile
   145                 150                 155                 160
   ttg tcg tat ccc aac ggt gaa gcg ttg ctg gaa gag gcg tta cgc tta     528
   Leu Ser Tyr Pro Asn Gly Glu Ala Leu Leu Glu Glu Ala Leu Arg Leu
                   165                 170                 175
   ggg gca gat gta gtg ggg gcg att ccg cat ttt gaa ttt acc cgt gaa     576
   Gly Ala Asp Val Val Gly Ala Ile Pro His Phe Glu Phe Thr Arg Glu
               180                 185                 190
   tac ggc gtg gag tcg ctg cat aaa acc ttc gcc ctg gcg caa aaa tac     624
   Tyr Gly Val Glu Ser Leu His Lys Thr Phe Ala Leu Ala Gln Lys Tyr
           195                 200                 205
   gac cgt ctc atc gac gtt cac tgt gat gag atc gat gac gag cag tcg     672
   Asp Arg Leu Ile Asp Val His Cys Asp Glu Ile Asp Asp Glu Gln Ser
       210                 215                 220
   cgc ttt gtc gaa acc gtt gct gcc ctg gcg cac cat gaa ggc atg ggc     720
   Arg Phe Val Glu Thr Val Ala Ala Leu Ala His His Glu Gly Met Gly
   225                 230                 235                 240
   gcg cga gtc acc gcc agc cac acc acg gca atg cac tcc tat aac ggg     768
   Ala Arg Val Thr Ala Ser His Thr Thr Ala Met His Ser Tyr Asn Gly
                   245                 250                 255
   gcg tat acc tca cgc ctg ttc cgc ttg ctg aaa atg tcc ggt att aac     816
   Ala Tyr Thr Ser Arg Leu Phe Arg Leu Leu Lys Met Ser Gly Ile Asn
               260                 265                 270
   ttt gtc gcc aac ccg ctg gtc aat att cat ctg caa gga cgt ttc gat     864
   Phe Val Ala Asn Pro Leu Val Asn Ile His Leu Gln Gly Arg Phe Asp
           275                 280                 285
   acg tat cca aaa cgt cgc ggc atc acg cgc gtt aaa gag atg ctg gag     912
   Thr Tyr Pro Lys Arg Arg Gly Ile Thr Arg Val Lys Glu Met Leu Glu
       290                 295                 300
   tcc ggc att aac gtc tgc ttt ggt cac gat gat gtc ttc gat ccg tgg     960
   Ser Gly Ile Asn Val Cys Phe Gly His Asp Asp Val Phe Asp Pro Trp
   305                 310                 315                 320
   tat ccg ctg gga acg gcg aat atg ctg caa gtg ctg cat atg ggg ctg    1008
   Tyr Pro Leu Gly Thr Ala Asn Met Leu Gln Val Leu His Met Gly Leu
                   325                 330                 335
   cat gtt tgc cag ttg atg ggc tac ggg cag att aac gat ggc ctg aat    1056
   His Val Cys Gln Leu Met Gly Tyr Gly Gln Ile Asn Asp Gly Leu Asn
               340                 345                 350
   tta atc acc cac cac agc gca agg acg ttg aat ttg cag gat tac ggc    1104
   Leu Ile Thr His His Ser Ala Arg Thr Leu Asn Leu Gln Asp Tyr Gly
           355                 360                 365
   att gcc gcc gga aac agc gcc aac ctg att atc ctg ccg gct gaa aat    1152
   Ile Ala Ala Gly Asn Ser Ala Asn Leu Ile Ile Leu Pro Ala Glu Asn
       370                 375                 380
   ggg ttt gat gcg ctg cgc cgt cag gtt ccg gta cgt tat tcg gta cgt    1200
   Gly Phe Asp Ala Leu Arg Arg Gln Val Pro Val Arg Tyr Ser Val Arg
   385                 390                 395                 400
   ggc ggc aag gtg att gcc agc aca caa ccg gca caa acc acc gta tat     1248
   Gly Gly Lys Val Ile Ala Ser Thr Gln Pro Ala Gln Thr Thr Val Tyr
                   405                 410                 415
   ctg gag cag cca gaa gcc atc gat tac aaa cgt tga                     1284
   Leu Glu Gln Pro Glu Ala Ile Asp Tyr Lys Arg
               420                 425
<210> 4
   <211> 427
   <212> PRT
   <213> Künstliche Sequenz
   <223> Beschreibung der künstlichen Sequenz: coding for cytosine deaminase (codA)
<400> 4
   Met Ser Asn Asn Ala Leu Gln Thr Ile Ile Asn Ala Arg Leu Pro Gly
   1 5 10 15
   Glu Glu Gly Leu Trp Gln Ile His Leu Gln Asp Gly Lys Ile Ser Ala
   20 25 30
   Ile Asp Ala Gln Ser Gly Val Met Pro Ile Thr Glu Asn Ser Leu Asp
   35 40 45
   Ala Glu Gln Gly Leu Val Ile Pro Pro Phe Val Glu Pro His Ile His
   50 55 60
   Leu Asp Thr Thr Gln Thr Ala Gly Gln Pro Asn Trp Asn Gln Ser Gly
   65 70 75 80
   Thr Leu Phe Glu Gly Ile Glu Arg Trp Ala Glu Arg Lys Ala Leu Leu
   85 90 95
   Thr His Asp Asp Val Lys Gln Arg Ala Trp Gln Thr Leu Lys Trp Gln
   100 105 110
   Ile Ala Asn Gly Ile Gln His Val Arg Thr His Val Asp Val Ser Asp
   115 120 125
   Ala Thr Leu Thr Ala Leu Lys Ala Met Leu Glu Val Lys Gln Glu Val
   130 135 140
   Ala Pro Trp Ile Asp Leu Gln Ile Val Ala Phe Pro Gln Glu Gly Ile
   145 150 155 160
   Leu Ser Tyr Pro Asn Gly Glu Ala Leu Leu Glu Glu Ala Leu Arg Leu
   165 170 175
   Gly Ala Asp Val Val Gly Ala Ile Pro His Phe Glu Phe Thr Arg Glu
   180 185 190
   Tyr Gly Val Glu Ser Leu His Lys Thr Phe Ala Leu Ala Gln Lys Tyr
   195 200 205
   Asp Arg Leu Ile Asp Val His Cys Asp Glu Ile Asp Asp Glu Gln Ser
   210 215 220
   Arg Phe Val Glu Thr Val Ala Ala Leu Ala His His Glu Gly Met Gly
   225 230 235 240
   Ala Arg Val Thr Ala Ser His Thr Thr Ala Met His Ser Tyr Asn Gly
   245 250 255
   Ala Tyr Thr Ser Arg Leu Phe Arg Leu Leu Lys Met Ser Gly Ile Asn
   260 265 270
   Phe Val Ala Asn Pro Leu Val Asn Ile His Leu Gln Gly Arg Phe Asp
   275 280 285
   Thr Tyr Pro Lys Arg Arg Gly Ile Thr Arg Val Lys Glu Met Leu Glu
   290 295 300
   Ser Gly Ile Asn Val Cys Phe Gly His Asp Asp Val Phe Asp Pro Trp
   305 310 315 320
   Tyr Pro Leu Gly Thr Ala Asn Met Leu Gln Val Leu His Met Gly Leu
   325 330 335
   His Val Cys Gln Leu Met Gly Tyr Gly Gln Ile Asn Asp Gly Leu Asn
   340 345 350
   Leu Ile Thr His His Ser Ala Arg Thr Leu Asn Leu Gln Asp Tyr Gly
   355 360 365
   Ile Ala Ala Gly Asn Ser Ala Asn Leu Ile Ile Leu Pro Ala Glu Asn
   370 375 380
   Gly Phe Asp Ala Leu Arg Arg Gln Val Pro Val Arg Tyr Ser Val Arg
   385 390 395 400
   Gly Gly Lys Val Ile Ala Ser Thr Gln Pro Ala Gln Thr Thr Val Tyr
   405 410 415
   Leu Glu Gln Pro Glu Ala Ile Asp Tyr Lys Arg
   420 425
<210> 5
   <211> 1221
   <212> DNA
   <213> Streptomyces griseolus
<220>
   <221> CDS
   <222> (1)..(1218)
   <223> coding for cytochrome P450-Sul (suaC)
<400> 5
   atg acc gat acc gcc acg acg ccc cag acc acg gac gca ccc gcc ttc    48
   Met Thr Asp Thr Ala Thr Thr Pro Gln Thr Thr Asp Ala Pro Ala Phe
    1               5                  10                  15
   ccg agc aac cgg agc tgt ccc tac cag tta ccg gac ggc tac gcc cag    96
   Pro Ser Asn Arg Ser Cys Pro Tyr Gln Leu Pro Asp Gly Tyr Ala Gln
               20                  25                  30
   ctc cgg gac acc ccc ggc ccc ctg cac cgg gtg acg ctc tac gac ggc   144
   Leu Arg Asp Thr Pro Gly Pro Leu His Arg Val Thr Leu Tyr Asp Gly
           35                  40                  45
   cgt cag gcg tgg gtg gtg acc aag cac gag gcc gcg cgc aaa ctg ctc   192
   Arg Gln Ala Trp Val Val Thr Lys His Glu Ala Ala Arg Lys Leu Leu
       50                  55                  60
   ggc gac ccc cgg ctg tcc tcc aac cgg acg gac gac aac ttc ccc gcc   240
   Gly Asp Pro Arg Leu Ser Ser Asn Arg Thr Asp Asp Asn Phe Pro Ala
   65                  70                  75                  80
   acg tca ccg cgc ttc gag gcc gtc cgg gag agc ccg cag gcg ttc atc   288
   Thr Ser Pro Arg Phe Glu Ala Val Arg Glu Ser Pro Gln Ala Phe Ile
                   85                  90                  95
   ggc ctg gac ccg ccc gag cac ggc acc cgg cgg cgg atg acg atc agc   336
   Gly Leu Asp Pro Pro Glu His Gly Thr Arg Arg Arg Met Thr Ile Ser
               100                 105                 110
   gag ttc acc gtc aag cgg atc aag ggc atg cgc ccc gag gtc gag gag     384
   Glu Phe Thr Val Lys Arg Ile Lys Gly Met Arg Pro Glu Val Glu Glu
           115                 120                 125
   gtg gtg cac ggc ttc ctc gac gag atg ctg gcc gcc ggc ccg acc gcc     432
   Val Val His Gly Phe Leu Asp Glu Met Leu Ala Ala Gly Pro Thr Ala
       130                 135                 140
   gac ctg gtc agt cag ttc gcg ctg ccg gtg ccc tcc atg gtg atc tgc     480
   Asp Leu Val Ser Gln Phe Ala Leu Pro Val Pro Ser Met Val Ile Cys
   145                 150                 155                 160
   cga ctc ctc ggc gtg ccc tac gcc gac cac gag ttc ttc cag gac gcg     528
   Arg Leu Leu Gly Val Pro Tyr Ala Asp His Glu Phe Phe Gln Asp Ala
                   165                 170                 175
   agc aag cgg ctg gtg cag tcc acg gac gcg cag agc gcg ctc acc gcg     576
   Ser Lys Arg Leu Val Gln Ser Thr Asp Ala Gln Ser Ala Leu Thr Ala
               180                 185                 190
   cgg aac gac ctc gcg ggt tac ctg gac ggc ctc atc acc cag ttc cag     624
   Arg Asn Asp Leu Ala Gly Tyr Leu Asp Gly Leu Ile Thr Gln Phe Gln
           195                 200                 205
   acc gaa ccg ggc gcg ggc ctg gtg ggc gct ctg gtc gcc gac cag ctg     672
   Thr Glu Pro Gly Ala Gly Leu Val Gly Ala Leu Val Ala Asp Gln Leu
       210                 215                 220
   gcc aac ggc gag atc gac cgt gag gaa ctg atc tcc acc gcg atg ctg     720
   Ala Asn Gly Glu Ile Asp Arg Glu Glu Leu Ile Ser Thr Ala Met Leu
   225                 230                 235                 240
   ctc ctc atc gcc ggc cac gag acc acg gcc tcg atg acc tcc ctc agc     768
   Leu Leu Ile Ala Gly His Glu Thr Thr Ala Ser Met Thr Ser Leu Ser
                   245                 250                 255
   gtg atc acc ctg ctg gac cac ccc gag cag tac gcc gcc ctg cgc gcc     816
   Val Ile Thr Leu Leu Asp His Pro Glu Gln Tyr Ala Ala Leu Arg Ala
               260                 265                 270
   gac cgc agc ctc gtg ccc ggc gcg gtg gag gaa ctg ctc cgc tac ctc     864
   Asp Arg Ser Leu Val Pro Gly Ala Val Glu Glu Leu Leu Arg Tyr Leu
           275                 280                 285
   gcc atc gcc gac atc gcg ggc ggc cgc gtc gcc acg gcg gac atc gag     912
   Ala Ile Ala Asp Ile Ala Gly Gly Arg Val Ala Thr Ala Asp Ile Glu
       290                 295                 300
   gtc gag ggg cac ctc atc cgg gcc ggc gag ggc gtg atc gtc gtc aac     960
   Val Glu Gly His Leu Ile Arg Ala Gly Glu Gly Val Ile Val Val Asn
   305                 310                 315                 320
   tcg ata gcc aac cgg gac ggc acg gtg tac gag gac ccg gac gcc ctc    1008
   Ser Ile Ala Asn Arg Asp Gly Thr Val Tyr Glu Asp Pro Asp Ala Leu
                   325                 330                 335
   gac atc cac cgc tcc gcg cgc cac cac ctc gcc ttc ggc ttc ggc gtg    1056
   Asp Ile His Arg Ser Ala Arg His His Leu Ala Phe Gly Phe Gly Val
               340                 345                 350
   cac cag tgc ctg ggc cag aac ctc gcc cgg ctg gag ctg gag gtc atc    1104
   His Gln Cys Leu Gly Gln Asn Leu Ala Arg Leu Glu Leu Glu Val Ile
           355                 360                 365
   ctc aac gcc ctc atg gac cgc gtc ccg acg ctg cga ctg gcc gtc ccc    1152
   Leu Asn Ala Leu Met Asp Arg Val Pro Thr Leu Arg Leu Ala Val Pro
       370                 375                 380
   gtc gag cag ttg gtg ctg cgg ccg ggt acg acg atc cag ggc gtc aac    1200
   Val Glu Gln Leu Val Leu Arg Pro Gly Thr Thr Ile Gln Gly Val Asn
   385                 390                 395                 400
   gaa ctc ccg gtc acc tgg tga                                        1221
   Glu Leu Pro Val Thr Trp
                   405
<210> 6
   <211> 406
   <212> PRT
   <213> Streptomyces griseolus
<400> 6
   Met Thr Asp Thr Ala Thr Thr Pro Gln Thr Thr Asp Ala Pro Ala Phe
   1 5 10 15
   Pro Ser Asn Arg Ser Cys Pro Tyr Gln Leu Pro Asp Gly Tyr Ala Gln
   20 25 30
   Leu Arg Asp Thr Pro Gly Pro Leu His Arg Val Thr Leu Tyr Asp Gly
   35 40 45
   Arg Gln Ala Trp Val Val Thr Lys His Glu Ala Ala Arg Lys Leu Leu
   50 55 60
   Gly Asp Pro Arg Leu Ser Ser Asn Arg Thr Asp Asp Asn Phe Pro Ala
   65 70 75 80
   Thr Ser Pro Arg Phe Glu Ala Val Arg Glu Ser Pro Gln Ala Phe Ile
   85 90 95
   Gly Leu Asp Pro Pro Glu His Gly Thr Arg Arg Arg Met Thr Ile Ser
   100 105 110
   Glu Phe Thr Val Lys Arg Ile Lys Gly Met Arg Pro Glu Val Glu Glu
   115 120 125
   Val Val His Gly Phe Leu Asp Glu Met Leu Ala Ala Gly Pro Thr Ala
   130 135 140
   Asp Leu Val Ser Gln Phe Ala Leu Pro Val Pro Ser Met Val Ile Cys
   145 150 155 160
   Arg Leu Leu Gly Val Pro Tyr Ala Asp His Glu Phe Phe Gln Asp Ala
   165 170 175
   Ser Lys Arg Leu Val Gln Ser Thr Asp Ala Gln Ser Ala Leu Thr Ala
   180 185 190
   Arg Asn Asp Leu Ala Gly Tyr Leu Asp Gly Leu Ile Thr Gln Phe Gln
   195 200 205
   Thr Glu Pro Gly Ala Gly Leu Val Gly Ala Leu Val Ala Asp Gln Leu
   210 215 220
   Ala Asn Gly Glu Ile Asp Arg Glu Glu Leu Ile Ser Thr Ala Met Leu
   225 230 235 240
   Leu Leu Ile Ala Gly His Glu Thr Thr Ala Ser Met Thr Ser Leu Ser
   245 250 255
   Val Ile Thr Leu Leu Asp His Pro Glu Gln Tyr Ala Ala Leu Arg Ala
   260 265 270
   Asp Arg Ser Leu Val Pro Gly Ala Val Glu Glu Leu Leu Arg Tyr Leu
   275 280 285
   Ala Ile Ala Asp Ile Ala Gly Gly Arg Val Ala Thr Ala Asp Ile Glu
   290 295 300
   Val Glu Gly His Leu Ile Arg Ala Gly Glu Gly Val Ile Val Val Asn
   305 310 315 320
   Ser Ile Ala Asn Arg Asp Gly Thr Val Tyr Glu Asp Pro Asp Ala Leu
   325 330 335
   Asp Ile His Arg Ser Ala Arg His His Leu Ala Phe Gly Phe Gly Val
   340 345 350
   His Gln Cys Leu Gly Gln Asn Leu Ala Arg Leu Glu Leu Glu Val Ile
   355 360 365
   Leu Asn Ala Leu Met Asp Arg Val Pro Thr Leu Arg Leu Ala Val Pro
   370 375 380
   Val Glu Gln Leu Val Leu Arg Pro Gly Thr Thr Ile Gln Gly Val Asn
   385 390 395 400
   Glu Leu Pro Val Thr Trp
   405
<210> 7
   <211> 1404
   <212> DNA
   <213> Agrobacterium tumefaciens
<220>
   <221> CDS
   <222> (1)..(1401)
   <223> coding for indole acetamide hydrolase (tms2)
<400> 7
   atg gtg ccc att acc tcg tta gca caa acc cta gaa cgc ctg aga cgg    48
   Met Val Pro Ile Thr Ser Leu Ala Gln Thr Leu Glu Arg Leu Arg Arg
    1               5                  10                  15
   aaa gac tac tcc tgc tta gaa cta gta gaa act ctg ata gcg cgt tgc    96
   Lys Asp Tyr Ser Cys Leu Glu Leu Val Glu Thr Leu Ile Ala Arg Cys
               20                  25                  30
   caa gct gca aaa cca tta aat gcc ctt ctg gct aca gac tgg gat ggc   144
   Gln Ala Ala Lys Pro Leu Asn Ala Leu Leu Ala Thr Asp Trp Asp Gly
           35                  40                  45
   ttg cgg cga agc gcc aaa aaa att gat cgt cat gga aac gcc gga tta   192
   Leu Arg Arg Ser Ala Lys Lys Ile Asp Arg His Gly Asn Ala Gly Leu
       50                  55                  60
   ggt ctt tgc ggc att cca ctc tgt ttt aag gcg aac atc gcg acc ggc   240
   Gly Leu Cys Gly Ile Pro Leu Cys Phe Lys Ala Asn Ile Ala Thr Gly
   65                  70                  75                  80
   ata ttt cct aca agc gct gct act ccg gcg ctg ata aac cac ttg cca   288
   Ile Phe Pro Thr Ser Ala Ala Thr Pro Ala Leu Ile Asn His Leu Pro
                   85                  90                  95
   aag ata cca tcc cgc gtc gca gaa aga ctt ttt tca gct gga gca ctg   336
   Lys Ile Pro Ser Arg Val Ala Glu Arg Leu Phe Ser Ala Gly Ala Leu
               100                 105                 110
   ccg ggt gcc tcg gga aac atg cat gag tta tcg ttt gga att acg agc   384
   Pro Gly Ala Ser Gly Asn Met His Glu Leu Ser Phe Gly Ile Thr Ser
           115                 120                 125
   aac aac tat gcc acc ggt gcg gtg cgg aac ccg tgg aat cca agt ctg   432
   Asn Asn Tyr Ala Thr Gly Ala Val Arg Asn Pro Trp Asn Pro Ser Leu
       130                 135                 140
   ata cca gga ggc tca agc ggt ggt gtg gct gct gcg gtg gca agc cga     480
   Ile Pro Gly Gly Ser Ser Gly Gly Val Ala Ala Ala Val Ala Ser Arg
   145                 150                 155                 160
   ttg atg tta ggc ggc ata ggc acc gat acc ggt gca tct gtt cgc cta     528
   Leu Met Leu Gly Gly Ile Gly Thr Asp Thr Gly Ala Ser Val Arg Leu
                   165                 170                 175
   ccc gca gcc ctg tgt ggc gta gta gga ttt cga ccg acg ctt gct cga     576
   Pro Ala Ala Leu Cys Gly Val Val Gly Phe Arg Pro Thr Leu Ala Arg
               180                 185                 190
   tat cca aga gat cgg ata ata ccg gtc agc ccc acc cgg gac acc gcc     624
   Tyr Pro Arg Asp Arg Ile Ile Pro Val Ser Pro Thr Arg Asp Thr Ala
           195                 200                 205
   gga atc ata gcg cag tgc gta gcc gat gtt ata atc ctc gac cag gtg     672
   Gly Ile Ile Ala Gln Cys Val Ala Asp Val Ile Ile Leu Asp Gln Val
       210                 215                 220
   att tcc gga cgg tcg gcg aaa att tca ccc atg ccg ctg aag ggg ctt     720
   Ile Ser Gly Arg Ser Ala Lys Ile Ser Pro Met Pro Leu Lys Gly Leu
   225                 230                 235                 240
   cgg atc ggc ctc ccc act acc tac ttt tac gat gac ctt gat gct gat     768
   Arg Ile Gly Leu Pro Thr Thr Tyr Phe Tyr Asp Asp Leu Asp Ala Asp
                   245                 250                 255
   gtg gcc ttc gca gct gaa acg acg att cgc ttg cta gcc aac aga ggc     816
   Val Ala Phe Ala Ala Glu Thr Thr Ile Arg Leu Leu Ala Asn Arg Gly
               260                 265                 270
   gta acc ttt gtt gaa gcc gac atc ccc cac cta gag gaa ctg aat agt     864
   Val Thr Phe Val Glu Ala Asp Ile Pro His Leu Glu Glu Leu Asn Ser
           275                 280                 285
   ggg gca agt ttg cca att gcg ctt tac gaa ttt cca cac gct cta aaa     912
   Gly Ala Ser Leu Pro Ile Ala Leu Tyr Glu Phe Pro His Ala Leu Lys
       290                 295                 300
   aag tat ctc gac gat ttt gtg gga aca gtt tct ttt tct gac gtt atc     960
   Lys Tyr Leu Asp Asp Phe Val Gly Thr Val Ser Phe Ser Asp Val Ile
   305                 310                 315                 320
   aaa gga att cgt agc ccc gat gta gcg aac att gtc agt gcg caa att     1008
   Lys Gly Ile Arg Ser Pro Asp Val Ala Asn Ile Val Ser Ala Gln Ile
                   325                 330                 335
   gat ggg cat caa att tcc aac gat gaa tat gaa ctg gcg cgt caa tcc     1056
   Asp Gly His Gln Ile Ser Asn Asp Glu Tyr Glu Leu Ala Arg Gln Ser
               340                 345                 350
   ttc agg cca agg ctc cag gcc act tat cgg aat tac ttc aga ctc tat     1104
   Phe Arg Pro Arg Leu Gln Ala Thr Tyr Arg Asn Tyr Phe Arg Leu Tyr
           355                 360                 365
   cag tta gat gca atc ctt ttc cca act gca ccc tta gcg gcc aaa gcc     1152
   Gln Leu Asp Ala Ile Leu Phe Pro Thr Ala Pro Leu Ala Ala Lys Ala
       370                 375                 380
   ata ggt cag gag tcg tca gtc atc cac aat ggc tca atg atg aac act     1200
   Ile Gly Gln Glu Ser Ser Val Ile His Asn Gly Ser Met Met Asn Thr
   385                 390                 395                 400
   ttc aag atc tac gtg cga aat gtg gac cca agc agc aac gca ggc cta     1248
   Phe Lys Ile Tyr Val Arg Asn Val Asp Pro Ser Ser Asn Ala Gly Leu
                   405                 410                 415
   cct ggg ttg agc ctt cct gcc tgc ctt aca cct gat cgc ttg cct gtt     1296
   Pro Gly Leu Ser Leu Pro Ala Cys Leu Thr Pro Asp Arg Leu Pro Val
               420                 425                 430
   gga atg gaa att gat gga tta gcg ggg tca gac cac cgt ctg tta gca     1344
   Gly Met Glu Ile Asp Gly Leu Ala Gly Ser Asp His Arg Leu Leu Ala
           435                 440                 445
   atc ggg gca gca tta gaa aaa gcc ata aat ttt cct tcc ttt ccc gat     1392
   Ile Gly Ala Ala Leu Glu Lys Ala Ile Asn Phe Pro Ser Phe Pro Asp
       450                 455                 460
   gct ttt aat tag                                                     1404
   Ala Phe Asn
   465
<210> 8
   <211> 467
   <212> PRT
   <213> Agrobacterium tumefaciens
<400> 8
   Met Val Pro Ile Thr Ser Leu Ala Gln Thr Leu Glu Arg Leu Arg Arg
   1 5 10 15
   Lys Asp Tyr Ser Cys Leu Glu Leu Val Glu Thr Leu Ile Ala Arg Cys
   20 25 30
   Gln Ala Ala Lys Pro Leu Asn Ala Leu Leu Ala Thr Asp Trp Asp Gly
   35 40 45
   Leu Arg Arg Ser Ala Lys Lys Ile Asp Arg His Gly Asn Ala Gly Leu
   50 55 60
   Gly Leu Cys Gly Ile Pro Leu Cys Phe Lys Ala Asn Ile Ala Thr Gly
   65 70 75 80
   Ile Phe Pro Thr Ser Ala Ala Thr Pro Ala Leu Ile Asn His Leu Pro
   85 90 95
   Lys Ile Pro Ser Arg Val Ala Glu Arg Leu Phe Ser Ala Gly Ala Leu
   100 105 110
   Pro Gly Ala Ser Gly Asn Met His Glu Leu Ser Phe Gly Ile Thr Ser
   115 120 125
   Asn Asn Tyr Ala Thr Gly Ala Val Arg Asn Pro Trp Asn Pro Ser Leu
   130 135 140
   Ile Pro Gly Gly Ser Ser Gly Gly Val Ala Ala Ala Val Ala Ser Arg
   145 150 155 160
   Leu Met Leu Gly Gly Ile Gly Thr Asp Thr Gly Ala Ser Val Arg Leu
   165 170 175
   Pro Ala Ala Leu Cys Gly Val Val Gly Phe Arg Pro Thr Leu Ala Arg
   180 185 190
   Tyr Pro Arg Asp Arg Ile Ile Pro Val Ser Pro Thr Arg Asp Thr Ala
   195 200 205
   Gly Ile Ile Ala Gln Cys Val Ala Asp Val Ile Ile Leu Asp Gln Val
   210 215 220
   Ile Ser Gly Arg Ser Ala Lys Ile Ser Pro Met Pro Leu Lys Gly Leu
   225 230 235 240
   Arg Ile Gly Leu Pro Thr Thr Tyr Phe Tyr Asp Asp Leu Asp Ala Asp
   245 250 255
   Val Ala Phe Ala Ala Glu Thr Thr Ile Arg Leu Leu Ala Asn Arg Gly
               260                 265                 270
   Val Thr Phe Val Glu Ala Asp Ile Pro His Leu Glu Glu Leu Asn Ser
           275                 280                 285
   Gly Ala Ser Leu Pro Ile Ala Leu Tyr Glu Phe Pro His Ala Leu Lys
       290                 295                 300
   Lys Tyr Leu Asp Asp Phe Val Gly Thr Val Ser Phe Ser Asp Val Ile
   305                 310                 315                 320
   Lys Gly Ile Arg Ser Pro Asp Val Ala Asn Ile Val Ser Ala Gln Ile
                   325                 330                 335
   Asp Gly His Gln Ile Ser Asn Asp Glu Tyr Glu Leu Ala Arg Gln Ser
               340                 345                 350
   Phe Arg Pro Arg Leu Gln Ala Thr Tyr Arg Asn Tyr Phe Arg Leu Tyr
           355                 360                 365
   Gln Leu Asp Ala Ile Leu Phe Pro Thr Ala Pro Leu Ala Ala Lys Ala
       370                 375                 380
   Ile Gly Gln Glu Ser Ser Val Ile His Asn Gly Ser Met Met Asn Thr
   385                 390                 395                 400
   Phe Lys Ile Tyr Val Arg Asn Val Asp Pro Ser Ser Asn Ala Gly Leu
                   405                 410                 415
   Pro Gly Leu Ser Leu Pro Ala Cys Leu Thr Pro Asp Arg Leu Pro Val
               420                 425                 430
   Gly Met Glu Ile Asp Gly Leu Ala Gly Ser Asp His Arg Leu Leu Ala
           435                 440                 445
   Ile Gly Ala Ala Leu Glu Lys Ala Ile Asn Phe Pro Ser Phe Pro Asp
       450                 455                 460
   Ala Phe Asn
   465
<210> 9
   <211> 1404
   <212> DNA
   <213> Agrobacterium tumefaciens
<220>
   <221> CDS
   <222> (1)..(1401)
   <223> coding for indole acetamide hydrolase (tms2)
<400> 9
   atg gtg ccc att acc tcg tta gca caa acc cta gaa cgc ctg aga cgg    48
   Met Val Pro Ile Thr Ser Leu Ala Gln Thr Leu Glu Arg Leu Arg Arg
    1               5                  10                  15
   aaa gac tac tcc tgc tta gaa cta gta gaa act ctg ata gcg cgt tgc    96
   Lys Asp Tyr Ser Cys Leu Glu Leu Val Glu Thr Leu Ile Ala Arg Cys
               20                  25                  30
   caa gct gca aaa cca tta aat gcc ctt ctg gct aca gac tgg gat ggc   144
   Gln Ala Ala Lys Pro Leu Asn Ala Leu Leu Ala Thr Asp Trp Asp Gly
           35                  40                  45
   ttg cgg cga agc gcc aaa aaa att gat cgt cat gga aac gcc gga tta   192
   Leu Arg Arg Ser Ala Lys Lys Ile Asp Arg His Gly Asn Ala Gly Leu
       50                  55                  60
   ggt ctt tgc ggc att cca ctc tgt ttt aag gcg aac atc gcg acc ggc      240
   Gly Leu Cys Gly Ile Pro Leu Cys Phe Lys Ala Asn Ile Ala Thr Gly
    65                 70                  75                  80
   ata ttt cct aca agc gct gct act ccg gcg ctg ata aac cac ttg cca      288
   Ile Phe Pro Thr Ser Ala Ala Thr Pro Ala Leu Ile Asn His Leu Pro
                   85                  90                  95
   aag ata cca tcc cgc gtc gca gaa aga ctt ttt tca gct gga gca ctg      336
   Lys Ile Pro Ser Arg Val Ala Glu Arg Leu Phe Ser Ala Gly Ala Leu
               100                 105                 110
   ccg ggt gcc tcg gga aac atg cat gag tta tcg ttt gga att acg agc      384
   Pro Gly Ala Ser Gly Asn Met His Glu Leu Ser Phe Gly Ile Thr Ser
           115                 120                 125
   aac aac tat gcc acc ggt gcg gtg cgg aac ccg tgg aat cca agt ctg      432
   Asn Asn Tyr Ala Thr Gly Ala Val Arg Asn Pro Trp Asn Pro Ser Leu
       130                 135                 140
   ata cca gga ggc tca agc ggt ggt gtg gct gct gcg gtg gca agc cga      480
   Ile Pro Gly Gly Ser Ser Gly Gly Val Ala Ala Ala Val Ala Ser Arg
   145                 150                 155                 160
   ttg atg tta ggc ggc ata ggc acc gat acc ggt gca tct gtt cgc cta      528
   Leu Met Leu Gly Gly Ile Gly Thr Asp Thr Gly Ala Ser Val Arg Leu
                   165                 170                 175
   ccc gca gcc ctg tgt ggc gta gta gga ttt cga ccg acg ctt gct cga      576
   Pro Ala Ala Leu Cys Gly Val Val Gly Phe Arg Pro Thr Leu Ala Arg
               180                 185                 190
   tat cca aga gat cgg ata ata ccg gtc agc ccc acc cgg gac acc gcc      624
   Tyr Pro Arg Asp Arg Ile Ile Pro Val Ser Pro Thr Arg Asp Thr Ala
           195                 200                 205
   gga atc ata gcg cag tgc gta gcc gat gtt ata atc ctc gat cag gtg      672
   Gly Ile Ile Ala Gln Cys Val Ala Asp Val Ile Ile Leu Asp Gln Val
       210                 215                 220
   att tcc gga cgg tcg gcg aaa att tca ccc atg ccg ctg aag ggg ctt      720
   Ile Ser Gly Arg Ser Ala Lys Ile Ser Pro Met Pro Leu Lys Gly Leu
   225                 230                 235                 240
   cgg atc ggc ctc ccc act acc tac ttt tac gat gac ctt gat gct gat      768
   Arg Ile Gly Leu Pro Thr Thr Tyr Phe Tyr Asp Asp Leu Asp Ala Asp
                   245                 250                 255
   gtg gcc ttc gca gct gaa acg acg att cgc ttg cta gcc aac aga ggc      816
   Val Ala Phe Ala Ala Glu Thr Thr Ile Arg Leu Leu Ala Asn Arg Gly
               260                 265                 270
   gta acc ttt gtt gaa gcc gac atc ccc cac cta gag gaa ctg aat agt      864
   Val Thr Phe Val Glu Ala Asp Ile Pro His Leu Glu Glu Leu Asn Ser
           275                 280                 285
   ggg gca agt ttg cca att gcg ctt tac gaa ttt cca cac gct cta aaa      912
   Gly Ala Ser Leu Pro Ile Ala Leu Tyr Glu Phe Pro His Ala Leu Lys
       290                 295                 300
   aag tat ctc gac gat ttt gtg gga aca gtt tct ttt tct gac gtt atc      960
   Lys Tyr Leu Asp Asp Phe Val Gly Thr Val Ser Phe Ser Asp Val Ile
   305                 310                 315                 320
   aaa gga att cgt agc ccc gat gta gcg aac att gtc agt gcg caa att     1008
   Lys Gly Ile Arg Ser Pro Asp Val Ala Asn Ile Val Ser Ala Gln Ile
                   325                 330                 335
   gat ggg cat caa att tcc aac gat gaa tat gaa ctg gcg cgt caa tcc    1056
   Asp Gly His Gln Ile Ser Asn Asp Glu Tyr Glu Leu Ala Arg Gln Ser
               340                 345                 350
   ttc agg cca agg ctc cag gcc act tat cgg aat tac ttc aga ctc tat    1104
   Phe Arg Pro Arg Leu Gln Ala Thr Tyr Arg Asn Tyr Phe Arg Leu Tyr
           355                 360                 365
   cag tta gat gca atc ctt ttc cca act gca ccc tta gcg gcc aaa gcc    1152
   Gln Leu Asp Ala Ile Leu Phe Pro Thr Ala Pro Leu Ala Ala Lys Ala
       370                 375                 380
   ata ggt cag gag tcg tca gtc atc cac aat ggc tca atg ata aac act    1200
   Ile Gly Gln Glu Ser Ser Val Ile His Asn Gly Ser Met Ile Asn Thr
   385                 390                 395                 400
   ttc aag atc tac gtg cga aat gtg gac cca agc agc aac gca ggc cta    1248
   Phe Lys Ile Tyr Val Arg Asn Val Asp Pro Ser Ser Asn Ala Gly Leu
                   405                 410                 415
   cct ggg ttg agc ctt cct gcc tgc ctt aca cct gat cgc ttg cct gtt    1296
   Pro Gly Leu Ser Leu Pro Ala Cys Leu Thr Pro Asp Arg Leu Pro Val
               420                 425                 430
   gga atg gaa att gac gga tta gcg ggg tca gac cac cgt ctg tta gca    1344
   Gly Met Glu Ile Asp Gly Leu Ala Gly Ser Asp His Arg Leu Leu Ala
           435                 440                 445
   atc ggg gca gca tta gaa aaa gcc ata aat ttt cct tcc ttt ccc gat    1392
   Ile Gly Ala Ala Leu Glu Lys Ala Ile Asn Phe Pro Ser Phe Pro Asp
       450                 455                 460
   gct ttt aat tag                                                    1404
   Ala Phe Asn
   465
<210> 10
   <211> 467
   <212> PRT
   <213> Agrobacterium tumefaciens
<400> 10
   Met Val Pro Ile Thr Ser Leu Ala Gln Thr Leu Glu Arg Leu Arg Arg
    1               5                  10                  15
   Lys Asp Tyr Ser Cys Leu Glu Leu Val Glu Thr Leu Ile Ala Arg Cys
               20                  25                  30
   Gln Ala Ala Lys Pro Leu Asn Ala Leu Leu Ala Thr Asp Trp Asp Gly
           35                  40                  45
   Leu Arg Arg Ser Ala Lys Lys Ile Asp Arg His Gly Asn Ala Gly Leu
       50                  55                  60
   Gly Leu Cys Gly Ile Pro Leu Cys Phe Lys Ala Asn Ile Ala Thr Gly
   65                  70                  75                  80
   Ile Phe Pro Thr Ser Ala Ala Thr Pro Ala Leu Ile Asn His Leu Pro
                   85                  90                  95
   Lys Ile Pro Ser Arg Val Ala Glu Arg Leu Phe Ser Ala Gly Ala Leu
               100                 105                 110
   Pro Gly Ala Ser Gly Asn Met His Glu Leu Ser Phe Gly Ile Thr Ser
           115                 120                 125
   Asn Asn Tyr Ala Thr Gly Ala Val Arg Asn Pro Trp Asn Pro Ser Leu
       130                 135                 140
   Ile Pro Gly Gly Ser Ser Gly Gly Val Ala Ala Ala Val Ala Ser Arg
   145 150 155 160
   Leu Met Leu Gly Gly Ile Gly Thr Asp Thr Gly Ala Ser Val Arg Leu
   165 170 175
   Pro Ala Ala Leu Cys Gly Val Val Gly Phe Arg Pro Thr Leu Ala Arg
   180 185 190
   Tyr Pro Arg Asp Arg Ile Ile Pro Val Ser Pro Thr Arg Asp Thr Ala
   195 200 205
   Gly Ile Ile Ala Gln Cys Val Ala Asp Val Ile Ile Leu Asp Gln Val
   210 215 220
   Ile Ser Gly Arg Ser Ala Lys Ile Ser Pro Met Pro Leu Lys Gly Leu
   225 230 235 240
   Arg Ile Gly Leu Pro Thr Thr Tyr Phe Tyr Asp Asp Leu Asp Ala Asp
   245 250 255
   Val Ala Phe Ala Ala Glu Thr Thr Ile Arg Leu Leu Ala Asn Arg Gly
   260 265 270
   Val Thr Phe Val Glu Ala Asp Ile Pro His Leu Glu Glu Leu Asn Ser
   275 280 285
   Gly Ala Ser Leu Pro Ile Ala Leu Tyr Glu Phe Pro His Ala Leu Lys
   290 295 300
   Lys Tyr Leu Asp Asp Phe Val Gly Thr Val Ser Phe Ser Asp Val Ile
   305 310 315 320
   Lys Gly Ile Arg Ser Pro Asp Val Ala Asn Ile Val Ser Ala Gln Ile
   325 330 335
   Asp Gly His Gln Ile Ser Asn Asp Glu Tyr Glu Leu Ala Arg Gln Ser
   340 345 350
   Phe Arg Pro Arg Leu Gln Ala Thr Tyr Arg Asn Tyr Phe Arg Leu Tyr
   355 360 365
   Gln Leu Asp Ala Ile Leu Phe Pro Thr Ala Pro Leu Ala Ala Lys Ala
   370 375 380
   Ile Gly Gln Glu Ser Ser Val Ile His Asn Gly Ser Met Ile Asn Thr
   385 390 395 400
   Phe Lys Ile Tyr Val Arg Asn Val Asp Pro Ser Ser Asn Ala Gly Leu
   405 410 415
   Pro Gly Leu Ser Leu Pro Ala Cys Leu Thr Pro Asp Arg Leu Pro Val
   420 425 430
   Gly Met Glu Ile Asp Gly Leu Ala Gly Ser Asp His Arg Leu Leu Ala
   435 440 445
   Ile Gly Ala Ala Leu Glu Lys Ala Ile Asn Phe Pro Ser Phe Pro Asp
   450 455 460
   Ala Phe Asn
   465
<210> 11
   <211> 609
   <212> DNA
   <213> Xanthobacter autotrophicus
<220>
   <221> CDS
   <222> (1)..(603)
   <223> coding for haloalkane dehalohenase
<400> 11
   atg tca acg ttt ttt gaa ccg gag aac gga atg aaa caa aac gcc aaa      48
   Met Ser Thr Phe Phe Glu Pro Glu Asn Gly Met Lys Gln Asn Ala Lys
    1               5                  10                  15
   acc gaa cga atc ctg gat gtc gcg ctc gaa ttg ctt gag aca gag ggt      96
   Thr Glu Arg Ile Leu Asp Val Ala Leu Glu Leu Leu Glu Thr Glu Gly
               20                  25                  30
   gag ttt ggt ttg acg atg agg cag gtg gca acg caa gcg gac atg tcc      144
   Glu Phe Gly Leu Thr Met Arg Gln Val Ala Thr Gln Ala Asp Met Ser
           35                  40                  45
   ctg agc aac gtt cag tac tat ttc aag tcc gag gac ctg ctc ctc gtg      192
   Leu Ser Asn Val Gln Tyr Tyr Phe Lys Ser Glu Asp Leu Leu Leu Val
       50                  55                  60
   gcc atg gca gac cgt tac ttt caa cgg tgc ctg aca acc atg gct gag      240
   Ala Met Ala Asp Arg Tyr Phe Gln Arg Cys Leu Thr Thr Met Ala Glu
    65                  70                  75                  80
   cat ccg ccc tta tcg gca ggg cgt gat caa cac gcc cag tta aga gcg      288
   His Pro Pro Leu Ser Ala Gly Arg Asp Gln His Ala Gln Leu Arg Ala
                   85                  90                  95
   ttg tta cga gaa ctg ctc ggt cat ggt ctt gag att tcc gag atg tgt      336
   Leu Leu Arg Glu Leu Leu Gly His Gly Leu Glu Ile Ser Glu Met Cys
               100                 105                 110
   cga ata ttc agg gag tac tgg gca atc gcc acc cgt aat gaa act gtt      384
   Arg Ile Phe Arg Glu Tyr Trp Ala Ile Ala Thr Arg Asn Glu Thr Val
           115                 120                 125
   cac ggc tat ctc aag tcg tac tat cgg gat ctc gcc gaa gtg atg gct      432
   His Gly Tyr Leu Lys Ser Tyr Tyr Arg Asp Leu Ala Glu Val Met Ala
       130                 135                 140
   gag aag ctt gcg cca ctg gcc agc agc gaa aag gcg ctg gcc gtg gcc      480
   Glu Lys Leu Ala Pro Leu Ala Ser Ser Glu Lys Ala Leu Ala Val Ala
   145                 150                 155                 160
   gta tct ttg gtt att cct tat gtt gag ggg tat tcg gta acg gcc att      528
   Val Ser Leu Val Ile Pro Tyr Val Glu Gly Tyr Ser Val Thr Ala Ile
                   165                 170                 175
   gca atg ccc gaa tcc att gat acg att tcc gag acg ctg acc aat gtg      576
   Ala Met Pro Glu Ser Ile Asp Thr Ile Ser Glu Thr Leu Thr Asn Val
               180                 185                 190
   gtg ttg gag cag ctt cgc atc agc aat tcatga                           609
   Val Leu Glu Gln Leu Arg Ile Ser Asn
           195                 200
<210> 12
   <211> 201
   <212> PRT
   <213> Xanthobacter autotrophicus
<400> 12
   Met Ser Thr Phe Phe Glu Pro Glu Asn Gly Met Lys Gln Asn Ala Lys
    1               5                  10                  15
   Thr Glu Arg Ile Leu Asp Val Ala Leu Glu Leu Leu Glu Thr Glu Gly
                   20                  25                  30
   Glu Phe Gly Leu Thr Met Arg Gln Val Ala Thr Gln Ala Asp Met Ser
               35                  40                  45
   Leu Ser Asn Val Gln Tyr Tyr Phe Lys Ser Glu Asp Leu Leu Leu Val
       50                  55                  60
   Ala Met Ala Asp Arg Tyr Phe Gln Arg Cys Leu Thr Thr Met Ala Glu
   65                  70                  75                  80
   His Pro Pro Leu Ser Ala Gly Arg Asp Gln His Ala Gln Leu Arg Ala
                   85                  90                  95
   Leu Leu Arg Glu Leu Leu Gly His Gly Leu Glu Ile Ser Glu Met Cys
               100                 105                 110
   Arg Ile Phe Arg Glu Tyr Trp Ala Ile Ala Thr Arg Asn Glu Thr Val
           115                 120                 125
   His Gly Tyr Leu Lys Ser Tyr Tyr Arg Asp Leu Ala Glu Val Met Ala
       130                 135                 140
   Glu Lys Leu Ala Pro Leu Ala Ser Ser Glu Lys Ala Leu Ala Val Ala
   145                 150                 155                 160
   Val Ser Leu Val Ile Pro Tyr Val Glu Gly Tyr Ser Val Thr Ala Ile
                   165                 170                 175
   Ala Met Pro Glu Ser Ile Asp Thr Ile Ser Glu Thr Leu Thr Asn Val
               180                 185                 190
   Val Leu Glu Gln Leu Arg Ile Ser Asn
           195                 200
<210> 13
   <211> 1131
   <212> DNA
   <213> Herpes simplex virus 1
<220>
   <221> CDS
   <222> (1) .. (1128)
   <223> coding for thymidine kinase (TK)
<400> 13
   atg gct tcg tac ccc tgc cat caa cac gcg tct gcg ttc gac cag gct   48
   Met Ala Ser Tyr Pro Cys His Gln His Ala Ser Ala Phe Asp Gln Ala
    1               5                  10                  15
   gcg cgt tct cgc ggc cat agc aac cga cgt acg gcg ttg cgc cct cgc   96
   Ala Arg Ser Arg Gly His Ser Asn Arg Arg Thr Ala Leu Arg Pro Arg
               20                  25                  30
   cgg cag caa gaa gcc acg gaa gtc cgc ctg gag cag aaa atg ccc acg  144
   Arg Gln Gln Glu Ala Thr Glu Val Arg Leu Glu Gln Lys Met Pro Thr
           35                  40                  45
   cta ctg cgg gtt tat ata gac ggt cct cac ggg atg ggg aaa acc acc  192
   Leu Leu Arg Val Tyr Ile Asp Gly Pro His Gly Met Gly Lys Thr Thr
       50                  55                  60
   acc acg caa ctg ctg gtg gcc ctg ggt tcg cgc gac gat atc gtc tac  240
   Thr Thr Gln Leu Leu Val Ala Leu Gly Ser Arg Asp Asp Ile Val Tyr
   65                  70                  75                  80
   gta ccc gag ccg atg act tac tgg cag gtg ctg ggg gct tcc gag aca     288
   Val Pro Glu Pro Met Thr Tyr Trp Gln Val Leu Gly Ala Ser Glu Thr
                   85                  90                  95
   atc gcg aac atc tac acc aca caa cac cgc ctc gac cag ggt gag ata     336
   Ile Ala Asn Ile Tyr Thr Thr Gln His Arg Leu Asp Gln Gly Glu Ile
               100                 105                 110
   tcg gcc ggg gac gcg gcg gtg gta atg aca agc gcc cag ata aca atg     384
   Ser Ala Gly Asp Ala Ala Val Val Met Thr Ser Ala Gln Ile Thr Met
           115                 120                 125
   ggc atg cct tat gcc gtg acc gac gcc gtt ctg gct cct cat gtc ggg     432
   Gly Met Pro Tyr Ala Val Thr Asp Ala Val Leu Ala Pro His Val Gly
       130                 135                 140
   ggg gag gct ggg agt tca cat gcc ccg ccc ccg gcc ctc acc ctc atc     480
   Gly Glu Ala Gly Ser Ser His Ala Pro Pro Pro Ala Leu Thr Leu Ile
   145                 150                 155                 160
   ttc gac cgc cat ccc atc gcc gcc ctc ctg tgc tac ccg gcc gcg cga     528
   Phe Asp Arg His Pro Ile Ala Ala Leu Leu Cys Tyr Pro Ala Ala Arg
                   165                 170                 175
   tac ctt atg ggc agc atg acc ccc cag gcc gtg ctg gcg ttc gtg gcc     576
   Tyr Leu Met Gly Ser Met Thr Pro Gln Ala Val Leu Ala Phe Val Ala
               180                 185                 190
   ctc atc ccg ccg acc ttg ccc ggc aca aac atc gtg ttg ggg gcc ctt     624
   Leu Ile Pro Pro Thr Leu Pro Gly Thr Asn Ile Val Leu Gly Ala Leu
           195                 200                 205
   ccg gag gac aga cac atc gac cgc ctg gcc aaa cgc cag cgc ccc ggc     672
   Pro Glu Asp Arg His Ile Asp Arg Leu Ala Lys Arg Gln Arg Pro Gly
       210                 215                 220
   gag cgg ctt gac ctg gct atg ctg gcc gcg att cgc cgc gtt tac ggg     720
   Glu Arg Leu Asp Leu Ala Met Leu Ala Ala Ile Arg Arg Val Tyr Gly
   225                 230                 235                 240
   ctg ctt gcc aat acg gtg cgg tat ctg cag ggc ggc ggg tcg tgg tgg     768
   Leu Leu Ala Asn Thr Val Arg Tyr Leu Gln Gly Gly Gly Ser Trp Trp
                   245                 250                 255
   gag gat tgg gga cag ctt tcg ggg acg gcc gtg ccg ccc cag ggt gcc     816
   Glu Asp Trp Gly Gln Leu Ser Gly Thr Ala Val Pro Pro Gln Gly Ala
               260                 265                 270
   gag ccc cag agc aac gcg ggc cca cga ccc cat atc ggg gac acg tta     864
   Glu Pro Gln Ser Asn Ala Gly Pro Arg Pro His Ile Gly Asp Thr Leu
           275                 280                 285
   ttt acc ctg ttt cgg gcc ccc gag ttg ctg gcc ccc aac ggc gac ctg     912
   Phe Thr Leu Phe Arg Ala Pro Glu Leu Leu Ala Pro Asn Gly Asp Leu
       290                 295                 300
   tat aac gtg ttt gcc tgg gcc ttg gac gtc ttg gcc aaa cgc ctc cgt     960
   Tyr Asn Val Phe Ala Trp Ala Leu Asp Val Leu Ala Lys Arg Leu Arg
   305                 310                 315                 320
   ccc atg cac gtc ttt atc ctg gat tac gac caa tcg ccc gcc ggc tgc     1008
   Pro Met His Val Phe Ile Leu Asp Tyr Asp Gln Ser Pro Ala Gly Cys
                   325                 330                 335
   cgg gac gcc ctg ctg caa ctt acc tcc ggg atg gtc cag acc cac gtc     1056
   Arg Asp Ala Leu Leu Gln Leu Thr Ser Gly Met Val Gln Thr His Val
               340                 345                 350
   acc acc cca ggc tcc ata ccg acg atc tgc gac ctg gcg cgc acg ttt   1104
   Thr Thr Pro Gly Ser Ile Pro Thr Ile Cys Asp Leu Ala Arg Thr Phe
           355                 360                 365
   gcc cgg gag atg ggg gag gct aac tga                               1131
   Ala Arg Glu Met Gly Glu Ala Asn
       370                 375
<210> 14
   <211> 376
   <212> PRT
   <213> Herpes simplex virus 1
<400> 14
   Met Ala Ser Tyr Pro Cys His Gln His Ala Ser Ala Phe Asp Gln Ala
   1 5 10 15
   Ala Arg Ser Arg Gly His Ser Asn Arg Arg Thr Ala Leu Arg Pro Arg
   20 25 30
   Arg Gln Gln Glu Ala Thr Glu Val Arg Leu Glu Gln Lys Met Pro Thr
   35 40 45
   Leu Leu Arg Val Tyr Ile Asp Gly Pro His Gly Met Gly Lys Thr Thr
   50 55 60
   Thr Thr Gln Leu Leu Val Ala Leu Gly Ser Arg Asp Asp Ile Val Tyr
   65 70 75 80
   Val Pro Glu Pro Met Thr Tyr Trp Gln Val Leu Gly Ala Ser Glu Thr
   85 90 95
   Ile Ala Asn Ile Tyr Thr Thr Gln His Arg Leu Asp Gln Gly Glu Ile
   100 105 110
   Ser Ala Gly Asp Ala Ala Val Val Met Thr Ser Ala Gln Ile Thr Met
   115 120 125
   Gly Met Pro Tyr Ala Val Thr Asp Ala Val Leu Ala Pro His Val Gly
   130 135 140
   Gly Glu Ala Gly Ser Ser His Ala Pro Pro Pro Ala Leu Thr Leu Ile
   145 150 155 160
   Phe Asp Arg His Pro Ile Ala Ala Leu Leu Cys Tyr Pro Ala Ala Arg
   165 170 175
   Tyr Leu Met Gly Ser Met Thr Pro Gln Ala Val Leu Ala Phe Val Ala
   180 185 190
   Leu Ile Pro Pro Thr Leu Pro Gly Thr Asn Ile Val Leu Gly Ala Leu
   195 200 205
   Pro Glu Asp Arg His Ile Asp Arg Leu Ala Lys Arg Gln Arg Pro Gly
   210 215 220
   Glu Arg Leu Asp Leu Ala Met Leu Ala Ala Ile Arg Arg Val Tyr Gly
   225 230 235 240
   Leu Leu Ala Asn Thr Val Arg Tyr Leu Gln Gly Gly Gly Ser Trp Trp
   245 250 255
   Glu Asp Trp Gly Gln Leu Ser Gly Thr Ala Val Pro Pro Gln Gly Ala
   260 265 270
   Glu Pro Gln Ser Asn Ala Gly Pro Arg Pro His Ile Gly Asp Thr Leu
   275 280 285
   Phe Thr Leu Phe Arg Ala Pro Glu Leu Leu Ala Pro Asn Gly Asp Leu
   290 295 300
   Tyr Asn Val Phe Ala Trp Ala Leu Asp Val Leu Ala Lys Arg Leu Arg
   305 310 315 320
   Pro Met His Val Phe Ile Leu Asp Tyr Asp Gln Ser Pro Ala Gly Cys
   325 330 335
   Arg Asp Ala Leu Leu Gln Leu Thr Ser Gly Met Val Gln Thr His Val
   340 345 350
   Thr Thr Pro Gly Ser Ile Pro Thr Ile Cys Asp Leu Ala Arg Thr Phe
   355 360 365
   Ala Arg Glu Met Gly Glu Ala Asn
   370 375
<210> 15
   <211> 1131
   <212> DNA
   <213> Herpes simplex virus 1
<220>
   <221> CDS
   <222> (1)..(1128)
   <223> coding for thymidine kinase (TK)
<400> 15
   atg gct tcg tac ccc tgc cat caa cac gcg tct gcg ttc gac cag gct    48
   Met Ala Ser Tyr Pro Cys His Gln His Ala Ser Ala Phe Asp Gln Ala
     1               5                  10                  15
   gcg cgt tct cgc ggc cat agc aac cga cgt acg gcg ttg cgc cct cgc    96
   Ala Arg Ser Arg Gly His Ser Asn Arg Arg Thr Ala Leu Arg Pro Arg
                20                  25                  30
   cgg cag caa gaa gcc acg gaa gtc cgc ctg gag cag aaa atg ccc acg   144
   Arg Gln Gln Glu Ala Thr Glu Val Arg Leu Glu Gln Lys Met Pro Thr
            35                  40                  45
   cta ctg cgg gtt tat ata gac ggt cct cac ggg atg ggg aaa acc acc   192
   Leu Leu Arg Val Tyr Ile Asp Gly Pro His Gly Met Gly Lys Thr Thr
        50                  55                  60
   acc acg caa ctg ctg gtg gcc ctg ggt tcg cgc gac gat atc gtc tac   240
   Thr Thr Gln Leu Leu Val Ala Leu Gly Ser Arg Asp Asp Ile Val Tyr
    65                  70                  75                  80
   gta ccc gag ccg atg act tac tgg cag gtg ctg ggg gct tcc gag aca   288
   Val Pro Glu Pro Met Thr Tyr Trp Gln Val Leu Gly Ala Ser Glu Thr
                    85                  90                  95
   atc gcg aac atc tac acc aca caa cac cgc ctc gac cag ggt gag ata   336
   Ile Ala Asn Ile Tyr Thr Thr Gln His Arg Leu Asp Gln Gly Glu Ile
               100                 105                 110
   tcg gcc ggg gac gcg gcg gtg gta atg aca agc gcc cag ata aca atg   384
   Ser Ala Gly Asp Ala Ala Val Val Met Thr Ser Ala Gln Ile Thr Met
           115                 120                 125
   ggc atg cct tat gcc gtg acc gac gcc gtt ctg gct cct cat gtc ggg   432
   Gly Met Pro Tyr Ala Val Thr Asp Ala Val Leu Ala Pro His Val Gly
       130                 135                 140
   ggg gag gct ggg agt tca cat gcc ccg ccc ccg gcc ctc acc ctc atc   480
   Gly Glu Ala Gly Ser Ser His Ala Pro Pro Pro Ala Leu Thr Leu Ile
   145                 150                 155                 160
   ttc gac cgc cat ccc atc gcc gcc ctc ctg tgc tac ccg gcc gcg cga      528
   Phe Asp Arg His Pro Ile Ala Ala Leu Leu Cys Tyr Pro Ala Ala Arg
                   165                 170                 175
   tac ctt atg ggc agc atg acc ccc cag gcc gtg ctg gcg ttc gtg gcc      576
   Tyr Leu Met Gly Ser Met Thr Pro Gln Ala Val Leu Ala Phe Val Ala
               180                 185                 190
   ctc atc ccg ccg acc ttg ccc ggc aca aac atc gtg ttg ggg gcc ctt      624
   Leu Ile Pro Pro Thr Leu Pro Gly Thr Asn Ile Val Leu Gly Ala Leu
           195                 200                 205
   ccg gag gac aga cac atc gac cgc ctg gcc aaa cgc cag cgc ccc ggc      672
   Pro Glu Asp Arg His Ile Asp Arg Leu Ala Lys Arg Gln Arg Pro Gly
       210                 215                 220
   gag cgg ctt gac ctg gct atg ctg gcc gcg att cgc cgc gtt tac ggg      720
   Glu Arg Leu Asp Leu Ala Met Leu Ala Ala Ile Arg Arg Val Tyr Gly
   225                 230                 235                 240
   ctg ctt gcc aat acg gtg cgg tat ctg cag ggc ggc ggg tcg tgg tgg      768
   Leu Leu Ala Asn Thr Val Arg Tyr Leu Gln Gly Gly Gly Ser Trp Trp
                   245                 250                 255
   gag gat tgg gga cag ctt tcg ggg acg gcc gtg ccg ccc cag ggt gcc      816
   Glu Asp Trp Gly Gln Leu Ser Gly Thr Ala Val Pro Pro Gln Gly Ala
               260                 265                 270
   gag ccc cag agc aac gcg ggc cca cga ccc cat atc ggg gac acg tta      864
   Glu Pro Gln Ser Asn Ala Gly Pro Arg Pro His Ile Gly Asp Thr Leu
           275                 280                 285
   ttt acc ctg ttt cgg gcc ccc gag ttg ctg gcc ccc aac ggc gac ctg      912
   Phe Thr Leu Phe Arg Ala Pro Glu Leu Leu Ala Pro Asn Gly Asp Leu
       290                 295                 300
   tat aac gtg ttt gcc tgg gcc ttg gac gtc ttg gcc aaa cgc ctc cgt      960
   Tyr Asn Val Phe Ala Trp Ala Leu Asp Val Leu Ala Lys Arg Leu Arg
   305                 310                 315                 320
   ccc atg cac gtc ttt atc ctg gat tac gac caa tcg ccc gcc ggc tgc     1008
   Pro Met His Val Phe Ile Leu Asp Tyr Asp Gln Ser Pro Ala Gly Cys
                   325                 330                 335
   cgg gac gcc ctg ctg caa ctt acc tcc ggg atg gtc cag acc cac gtc     1056
   Arg Asp Ala Leu Leu Gln Leu Thr Ser Gly Met Val Gln Thr His Val
               340                 345                 350
   acc acc cca ggc tcc ata ccg acg atc tgc gac ctg gcg cgc acg ttt     1104
   Thr Thr Pro Gly Ser Ile Pro Thr Ile Cys Asp Leu Ala Arg Thr Phe
           355                 360                 365
   gcc cgg gag atg ggg gag gct aac tga                                 1131
   Ala Arg Glu Met Gly Glu Ala Asn
       370                 375
<210> 16
   <211> 376
   <212> PRT
   <213> Herpes simplex virus 1
<400> 16
   Met Ala Ser Tyr Pro Cys His Gln His Ala Ser Ala Phe Asp Gln Ala
    1               5                  10                  15
   Ala Arg Ser Arg Gly His Ser Asn Arg Arg Thr Ala Leu Arg Pro Arg
               20                  25                  30
   Arg Gln Gln Glu Ala Thr Glu Val Arg Leu Glu Gln Lys Met Pro Thr
   35 40 45
   Leu Leu Arg Val Tyr Ile Asp Gly Pro His Gly Met Gly Lys Thr Thr
   50 55 60
   Thr Thr Gln Leu Leu Val Ala Leu Gly Ser Arg Asp Asp Ile Val Tyr
   65 70 75 80
   Val Pro Glu Pro Met Thr Tyr Trp Gln Val Leu Gly Ala Ser Glu Thr
   85 90 95
   Ile Ala Asn Ile Tyr Thr Thr Gln His Arg Leu Asp Gln Gly Glu Ile
   100 105 110
   Ser Ala Gly Asp Ala Ala Val Val Met Thr Ser Ala Gln Ile Thr Met
   115 120 125
   Gly Met Pro Tyr Ala Val Thr Asp Ala Val Leu Ala Pro His Val Gly
   130 135 140
   Gly Glu Ala Gly Ser Ser His Ala Pro Pro Pro Ala Leu Thr Leu Ile
   145 150 155 160
   Phe Asp Arg His Pro Ile Ala Ala Leu Leu Cys Tyr Pro Ala Ala Arg
   165 170 175
   Tyr Leu Met Gly Ser Met Thr Pro Gln Ala Val Leu Ala Phe Val Ala
   180 185 190
   Leu Ile Pro Pro Thr Leu Pro Gly Thr Asn Ile Val Leu Gly Ala Leu
   195 200 205
   Pro Glu Asp Arg His Ile Asp Arg Leu Ala Lys Arg Gln Arg Pro Gly
   210 215 220
   Glu Arg Leu Asp Leu Ala Met Leu Ala Ala Ile Arg Arg Val Tyr Gly
   225 230 235 240
   Leu Leu Ala Asn Thr Val Arg Tyr Leu Gln Gly Gly Gly Ser Trp Trp
   245 250 255
   Glu Asp Trp Gly Gln Leu Ser Gly Thr Ala Val Pro Pro Gln Gly Ala
   260 265 270
   Glu Pro Gln Ser Asn Ala Gly Pro Arg Pro His Ile Gly Asp Thr Leu
   275 280 285
   Phe Thr Leu Phe Arg Ala Pro Glu Leu Leu Ala Pro Asn Gly Asp Leu
   290 295 300
   Tyr Asn Val Phe Ala Trp Ala Leu Asp Val Leu Ala Lys Arg Leu Arg
   305 310 315 320
   Pro Met His Val Phe Ile Leu Asp Tyr Asp Gln Ser Pro Ala Gly Cys
   325 330 335
   Arg Asp Ala Leu Leu Gln Leu Thr Ser Gly Met Val Gln Thr His Val
   340 345 350
   Thr Thr Pro Gly Ser Ile Pro Thr Ile Cys Asp Leu Ala Arg Thr Phe
   355 360 365
   Ala Arg Glu Met Gly Glu Ala Asn
   370 375
<210> 17
   <211> 840
   <212> DNA
   <213> Toxoplasma gondii
<220>
   <221> CDS
   <222> (1)..(837)
   <223> coding for hypoxanthine-xanthine-guanine phosphoribosyl transferase (HXGPRTase)
<400> 17
   atg gcg tcc aaa ccc att gaa gaa tcc cgg tcg caa aaa cgg agt gcc      48
   Met Ala Ser Lys Pro Ile Glu Glu Ser Arg Ser Gln Lys Arg Ser Ala
   1               5                   10                  15
   ttc tca gac atc ttc tgt tgt tgc act cct aat gaa ggg gct atc gtg      96
   Phe Ser Asp Ile Phe Cys Cys Cys Thr Pro Asn Glu Gly Ala Ile Val
               20                  25                  30
   ccc agt gac cca atg gtc tcc acc agt gct cca gca cgc acc agt gct     144
   Pro Ser Asp Pro Met Val Ser Thr Ser Ala Pro Ala Arg Thr Ser Ala
           35                  40                  45
   cca gcg cgc tcc agt gca ctt caa gac tac ggc aag ggc aag ggc cgt     192
   Pro Ala Arg Ser Ser Ala Leu Gln Asp Tyr Gly Lys Gly Lys Gly Arg
       50                  55                  60
   att gag ccc atg tat atc ccc gac aac acc ttc tac aac gct gat gac     240
   Ile Glu Pro Met Tyr Ile Pro Asp Asn Thr Phe Tyr Asn Ala Asp Asp
   65                  70                  75                  80
   ttt ctt gtg ccc ccc cac tgc aag ccc tac att gac aaa atc ctc ctc     288
   Phe Leu Val Pro Pro His Cys Lys Pro Tyr Ile Asp Lys Ile Leu Leu
                   85                  90                  95
   cct ggt gga ttg gtc aag gac aga gtt gag aag ttg gcg tat gac atc     336
   Pro Gly Gly Leu Val Lys Asp Arg Val Glu Lys Leu Ala Tyr Asp Ile
               100                 105                 110
   cac aga act tac ttc ggc gag gag ttg cac atc att tgc atc ctg aaa     384
   His Arg Thr Tyr Phe Gly Glu Glu Leu His Ile Ile Cys Ile Leu Lys
           115                 120                 125
   ggc tct cgc ggc ttc ttc aac ctt ctg atc gac tac ctt gcc acc ata     432
   Gly Ser Arg Gly Phe Phe Asn Leu Leu Ile Asp Tyr Leu Ala Thr Ile
       130                 135                 140
   cag aag tac agt ggt cgt gag tcc agc gtg ccc ccc ttc ttc gag cac     480
   Gln Lys Tyr Ser Gly Arg Glu Ser Ser Val Pro Pro Phe Phe Glu His
   145                 150                 155                 160
   tat gtc cgc ctg aag tcc tac cag aac gac aac agc aca ggc cag ctc     528
   Tyr Val Arg Leu Lys Ser Tyr Gln Asn Asp Asn Ser Thr Gly Gln Leu
                   165                 170                 175
   acc gtc ttg agc gac gac ttg tca atc ttt cgc gac aag cac gtt ctg     576
   Thr Val Leu Ser Asp Asp Leu Ser Ile Phe Arg Asp Lys His Val Leu
               180                 185                 190
   att gtt gag gac atc gtc gac acc ggt ttc acc ctc acc gag ttc ggt     624
   Ile Val Glu Asp Ile Val Asp Thr Gly Phe Thr Leu Thr Glu Phe Gly
           195                 200                 205
   gag cgc ctg aaa gcc gtc ggt ccc aag tcg atg aga atc gcc acc ctc     672
   Glu Arg Leu Lys Ala Val Gly Pro Lys Ser Met Arg Ile Ala Thr Leu
       210                 215                 220
   gtc gag aag cgc aca gat cgc tcc aac agc ttg aag ggc gac ttc gtc     720
   Val Glu Lys Arg Thr Asp Arg Ser Asn Ser Leu Lys Gly Asp Phe Val
   225                 230                 235                 240
   ggc ttc agc att gaa gac gtc tgg atc gtt ggt tgc tgc tac gac ttc     768
   Gly Phe Ser Ile Glu Asp Val Trp Ile Val Gly Cys Cys Tyr Asp Phe
                   245                 250                 255
   aac gag atg ttc cgc gac ttc gac cac gtc gcc gtc ctg agc gac gcc     816
   Asn Glu Met Phe Arg Asp Phe Asp His Val Ala Val Leu Ser Asp Ala
               260                 265                 270
   gct cgc aaa aag ttc gag aag taa                                     840
   Ala Arg Lys Lys Phe Glu Lys
           275
<210> 18
   <211> 279
   <212> PRT
   <213> Toxoplasma gondii
<400> 18
   Met Ala Ser Lys Pro Ile Glu Glu Ser Arg Ser Gln Lys Arg Ser Ala
   1               5                   10                  15
   Phe Ser Asp Ile Phe Cys Cys Cys Thr Pro Asn Glu Gly Ala Ile Val
               20                  25                  30
   Pro Ser Asp Pro Met Val Ser Thr Ser Ala Pro Ala Arg Thr Ser Ala
           35                  40                  45
   Pro Ala Arg Ser Ser Ala Leu Gln Asp Tyr Gly Lys Gly Lys Gly Arg
       50                  55                  60
   Ile Glu Pro Met Tyr Ile Pro Asp Asn Thr Phe Tyr Asn Ala Asp Asp
   65                  70                  75                  80
   Phe Leu Val Pro Pro His Cys Lys Pro Tyr Ile Asp Lys Ile Leu Leu
                   85                  90                  95
   Pro Gly Gly Leu Val Lys Asp Arg Val Glu Lys Leu Ala Tyr Asp Ile
               100                 105                 110
   His Arg Thr Tyr Phe Gly Glu Glu Leu His Ile Ile Cys Ile Leu Lys
           115                 120                 125
   Gly Ser Arg Gly Phe Phe Asn Leu Leu Ile Asp Tyr Leu Ala Thr Ile
       130                 135                 140
   Gln Lys Tyr Ser Gly Arg Glu Ser Ser Val Pro Pro Phe Phe Glu His
   145                 150                 155                 160
   Tyr Val Arg Leu Lys Ser Tyr Gln Asn Asp Asn Ser Thr Gly Gln Leu
                   165                 170                 175
   Thr Val Leu Ser Asp Asp Leu Ser Ile Phe Arg Asp Lys His Val Leu
               180                 185                 190
   Ile Val Glu Asp Ile Val Asp Thr Gly Phe Thr Leu Thr Glu Phe Gly
           195                 200                 205
   Glu Arg Leu Lys Ala Val Gly Pro Lys Ser Met Arg Ile Ala Thr Leu
       210                 215                 220
   Val Glu Lys Arg Thr Asp Arg Ser Asn Ser Leu Lys Gly Asp Phe Val
   225                 230                 235                 240
   Gly Phe Ser Ile Glu Asp Val Trp Ile Val Gly Cys Cys Tyr Asp Phe
                   245                 250                 255
   Asn Glu Met Phe Arg Asp Phe Asp His Val Ala Val Leu Ser Asp Ala
               260                 265                 270
   Ala Arg Lys Lys Phe Glu Lys
           275
<210> 19
   <211> 459
   <212> DNA
   <213> Escherichia coli
<220>
   <221> CDS
   <222> (1)..(456)
   <223> coding for xanthine-guanine phosphoribosyl transferase (gpt)
<400> 19
   atg agc gaa aaa tac atc gtc acc tgg gac atg ttg cag atc cat gca      48
   Met Ser Glu Lys Tyr Ile Val Thr Trp Asp Met Leu Gln Ile His Ala
   1               5                   10                  15
   cgt aaa ctc gca agc cga ctg atg cct tct gaa caa tgg aaa ggc att      96
   Arg Lys Leu Ala Ser Arg Leu Met Pro Ser Glu Gln Trp Lys Gly Ile
               20                  25                  30
   att gcc gta agc cgt ggc ggt ctg gta ccg ggt gcg tta ctg gcg cgt      144
   Ile Ala Val Ser Arg Gly Gly Leu Val Pro Gly Ala Leu Leu Ala Arg
           35                  40                  45
   gaa ctg ggt att cgt cat gtc gat acc gtt tgt att tcc agc tac gat      192
   Glu Leu Gly Ile Arg His Val Asp Thr Val Cys Ile Ser Ser Tyr Asp
       50                  55                  60
   cac gac aac cag cgc gag ctt aaa gtg ctg aaa cgc gca gaa ggc gat      240
   His Asp Asn Gln Arg Glu Leu Lys Val Leu Lys Arg Ala Glu Gly Asp
   65                  70                  75                  80
   ggc gaa ggc ttc atc gtt att gat gac ctg gtg gat acc ggt ggt act      288
   Gly Glu Gly Phe Ile Val Ile Asp Asp Leu Val Asp Thr Gly Gly Thr
                   85                  90                  95
   gcg gtt gcg att cgt gaa atg tat cca aaa gcg cac ttt gtc acc atc      336
   Ala Val Ala Ile Arg Glu Met Tyr Pro Lys Ala His Phe Val Thr Ile
               100                 105                 110
   ttc gca aaa ccg gct ggt cgt ccg ctg gtt gat gac tat gtt gtt gat      384
   Phe Ala Lys Pro Ala Gly Arg Pro Leu Val Asp Asp Tyr Val Val Asp
           115                 120                 125
   atc ccg caa gat acc tgg att gaa cag ccg tgg gat atg ggc gtc gta      432
   Ile Pro Gln Asp Thr Trp Ile Glu Gln Pro Trp Asp Met Gly Val Val
       130                 135                 140
   ttc gtc ccg cca atc tcc ggt cgc taa                                  459
   Phe Val Pro Pro Ile Ser Gly Arg
   145                 150
<210> 20
   <211> 152
   <212> PRT
   <213> Escherichia coli
<400> 20
   Met Ser Glu Lys Tyr Ile Val Thr Trp Asp Met Leu Gln Ile His Ala
   1               5                   10                  15
   Arg Lys Leu Ala Ser Arg Leu Met Pro Ser Glu Gln Trp Lys Gly Ile
               20                  25                  30
   Ile Ala Val Ser Arg Gly Gly Leu Val Pro Gly Ala Leu Leu Ala Arg
               35                  40                  45
   Glu Leu Gly Ile Arg His Val Asp Thr Val Cys Ile Ser Ser Tyr Asp
           50                  55                  60
   His Asp Asn Gln Arg Glu Leu Lys Val Leu Lys Arg Ala Glu Gly Asp
    65                  70                  75                  80
   Gly Glu Gly Phe Ile Val Ile Asp Asp Leu Val Asp Thr Gly Gly Thr
                   85                  90                  95
   Ala Val Ala Ile Arg Glu Met Tyr Pro Lys Ala His Phe Val Thr Ile
               100                 105                 110
   Phe Ala Lys Pro Ala Gly Arg Pro Leu Val Asp Asp Tyr Val Val Asp
           115                 120                 125
   Ile Pro Gln Asp Thr Trp Ile Glu Gln Pro Trp Asp Met Gly Val Val
       130                 135                 140
   Phe Val Pro Pro Ile Ser Gly Arg
   145                 150
<210> 21
   <211> 459
   <212> DNA
   <213> Escherichia coli
<220>
   <221> CDS
   <222> (1)..(456)
   <223> coding for xanthine-guanine phosphoribosyl transferase (gpt)
<400> 21
   atg agc gaa aaa tac atc gtc acc tgg gac atg ttg cag atc cat gca      48
   Met Ser Glu Lys Tyr Ile Val Thr Trp Asp Met Leu Gln Ile His Ala
    1               5                  10                  15
   cgt aaa ctc gca agc cga ctg atg cct tct gaa caa tgg aaa ggc att      96
   Arg Lys Leu Ala Ser Arg Leu Met Pro Ser Glu Gln Trp Lys Gly Ile
               20                  25                  30
   att gcc gta agc cgt ggc ggt ctg gta ccg ggt gcg tta ctg gcg cgt     144
   Ile Ala Val Ser Arg Gly Gly Leu Val Pro Gly Ala Leu Leu Ala Arg
           35                  40                  45
   gaa ctg ggt att cgt cat gtc gat acc gtt tgt att tcc agc tac gat     192
   Glu Leu Gly Ile Arg His Val Asp Thr Val Cys Ile Ser Ser Tyr Asp
       50                  55                  60
   cac gac aac cag cgc gag ctt aaa gtg ctg aaa cgc gca gaa ggc gat     240
   His Asp Asn Gln Arg Glu Leu Lys Val Leu Lys Arg Ala Glu Gly Asp
   65                  70                  75                  80
   ggc gaa ggc ttc atc gtt att gat gac ctg gtg gat acc ggt ggt act     288
   Gly Glu Gly Phe Ile Val Ile Asp Asp Leu Val Asp Thr Gly Gly Thr
                   85                  90                  95
   gcg gtt gcg att cgt gaa atg tat cca aaa gcg cac ttt gtc acc atc     336
   Ala Val Ala Ile Arg Glu Met Tyr Pro Lys Ala His Phe Val Thr Ile
               100                 105                 110
   ttc gca aaa ccg gct ggt cgt ccg ctg gtt gat gac tat gtt gtt gat     384
   Phe Ala Lys Pro Ala Gly Arg Pro Leu Val Asp Asp Tyr Val Val Asp
           115                 120                 125
   atc ccg caa gat acc tgg att gaa cag ccg tgg gat atg ggc gtc gta    432
   Ile Pro Gln Asp Thr Trp Ile Glu Gln Pro Trp Asp Met Gly Val Val
       130                 135                 140
   ttc gtc ccg cca atc tcc ggt cgc taa                                459
   Phe Val Pro Pro Ile Ser Gly Arg
   145                 150
<210> 22
   <211> 152
   <212> PRT
   <213> Escherichia coli
<400> 22
   Met Ser Glu Lys Tyr Ile Val Thr Trp Asp Met Leu Gln Ile His Ala
    1               5                  10                  15
   Arg Lys Leu Ala Ser Arg Leu Met Pro Ser Glu Gln Trp Lys Gly Ile
               20                  25                  30
   Ile Ala Val Ser Arg Gly Gly Leu Val Pro Gly Ala Leu Leu Ala Arg
           35                  40                  45
   Glu Leu Gly Ile Arg His Val Asp Thr Val Cys Ile Ser Ser Tyr Asp
       50                  55                  60
   His Asp Asn Gln Arg Glu Leu Lys Val Leu Lys Arg Ala Glu Gly Asp
   65                  70                  75                  80
   Gly Glu Gly Phe Ile Val Ile Asp Asp Leu Val Asp Thr Gly Gly Thr
                   85                  90                  95
   Ala Val Ala Ile Arg Glu Met Tyr Pro Lys Ala His Phe Val Thr Ile
               100                 105                 110
   Phe Ala Lys Pro Ala Gly Arg Pro Leu Val Asp Asp Tyr Val Val Asp
           115                 120                 125
   Ile Pro Gln Asp Thr Trp Ile Glu Gln Pro Trp Asp Met Gly Val Val
       130                 135                 140
   Phe Val Pro Pro Ile Ser Gly Arg
   145                 150
<210> 23
   <211> 720
   <212> DNA
   <213> Escherichia coli
<220>
   <221> CDS
   <222> (1)..(717)
   <223> coding for purine nucleoside phosphorylase (deoD)
<400> 23
   atg gct acc cca cac att aat gca gaa atg ggc gat ttc gct gac gta    48
   Met Ala Thr Pro His Ile Asn Ala Glu Met Gly Asp Phe Ala Asp Val
   1               5                   10                  15
   gtt ttg atg cca ggc gac ccg ctg cgt gcg aag tat att gct gaa act    96
   Val Leu Met Pro Gly Asp Pro Leu Arg Ala Lys Tyr Ile Ala Glu Thr
               20                  25                  30
   ttc ctt gaa gat gcc cgt gaa gtg aac aac gtt cgc ggt atg ctg ggc    144
   Phe Leu Glu Asp Ala Arg Glu Val Asn Asn Val Arg Gly Met Leu Gly
           35                  40                  45
   ttc acc ggt act tac aaa ggc cgc aaa att tcc gta atg ggt cac ggt    192
   Phe Thr Gly Thr Tyr Lys Gly Arg Lys Ile Ser Val Met Gly His Gly
       50                  55                  60
   atg ggt atc ccg tcc tgc tcc atc tac acc aaa gaa ctg atc acc gat    240
   Met Gly Ile Pro Ser Cys Ser Ile Tyr Thr Lys Glu Leu Ile Thr Asp
   65                  70                  75                  80
   ttc ggc gtg aag aaa att atc cgc gtg ggt tcc tgt ggc gca gtt ctg    288
   Phe Gly Val Lys Lys Ile Ile Arg Val Gly Ser Cys Gly Ala Val Leu
                   85                  90                  95
   ccg cac gta aaa ctg cgc gac gtc gtt atc ggt atg ggt gcc tgc acc    336
   Pro His Val Lys Leu Arg Asp Val Val Ile Gly Met Gly Ala Cys Thr
               100                 105                 110
   gat tcc aaa gtt aac cgc atc cgt ttt aaa gac cat gac ttt gcc gct    384
   Asp Ser Lys Val Asn Arg Ile Arg Phe Lys Asp His Asp Phe Ala Ala
           115                 120                 125
   atc gct gac ttc gac atg gtg cgt aac gca gta gat gca gct aaa gca    432
   Ile Ala Asp Phe Asp Met Val Arg Asn Ala Val Asp Ala Ala Lys Ala
       130                 135                 140
   ctg ggt att gat gct cgc gtg ggt aac ctg ttc tcc gct gac ctg ttc    480
   Leu Gly Ile Asp Ala Arg Val Gly Asn Leu Phe Ser Ala Asp Leu Phe
   145                 150                 155                 160
   tac tct ccg gac ggc gaa atg ttc gac gtg atg gaa aaa tac ggc att    528
   Tyr Ser Pro Asp Gly Glu Met Phe Asp Val Met Glu Lys Tyr Gly Ile
                   165                 170                 175
   ctc ggc gtg gaa atg gaa gcg gct ggt atc tac ggc gtc gct gca gaa    576
   Leu Gly Val Glu Met Glu Ala Ala Gly Ile Tyr Gly Val Ala Ala Glu
               180                 185                 190
   ttt ggc gcg aaa gcc ctg acc atc tgc acc gta tct gac cac atc cgc    624
   Phe Gly Ala Lys Ala Leu Thr Ile Cys Thr Val Ser Asp His Ile Arg
           195                 200                 205
   act cac gag cag acc act gcc gct gag cgt cag act acc ttc aac gac    672
   Thr His Glu Gln Thr Thr Ala Ala Glu Arg Gln Thr Thr Phe Asn Asp
       210                 215                 220
   atg atc aaa atc gca ctg gaa tcc gtt ctg ctg ggc gat aaa gag taa    720
   Met Ile Lys Ile Ala Leu Glu Ser Val Leu Leu Gly Asp Lys Glu
   225                 230                 235
<210> 24
   <211> 239
   <212> PRT
   <213> Escherichia coli
<400> 24
   Met Ala Thr Pro His Ile Asn Ala Glu Met Gly Asp Phe Ala Asp Val
   1               5                   10                  15
   Val Leu Met Pro Gly Asp Pro Leu Arg Ala Lys Tyr Ile Ala Glu Thr
               20                  25                  30
   Phe Leu Glu Asp Ala Arg Glu Val Asn Asn Val Arg Gly Met Leu Gly
           35                  40                  45
   Phe Thr Gly Thr Tyr Lys Gly Arg Lys Ile Ser Val Met Gly His Gly
       50                  55                  60
   Met Gly Ile Pro Ser Cys Ser Ile Tyr Thr Lys Glu Leu Ile Thr Asp
   65                  70                  75                  80
   Phe Gly Val Lys Lys Ile Ile Arg Val Gly Ser Cys Gly Ala Val Leu
                   85                  90                  95
   Pro His Val Lys Leu Arg Asp Val Val Ile Gly Met Gly Ala Cys Thr
               100                 105                 110
   Asp Ser Lys Val Asn Arg Ile Arg Phe Lys Asp His Asp Phe Ala Ala
           115                 120                 125
   Ile Ala Asp Phe Asp Met Val Arg Asn Ala Val Asp Ala Ala Lys Ala
       130                 135                 140
   Leu Gly Ile Asp Ala Arg Val Gly Asn Leu Phe Ser Ala Asp Leu Phe
   145                 150                 155                 160
   Tyr Ser Pro Asp Gly Glu Met Phe Asp Val Met Glu Lys Tyr Gly Ile
                   165                 170                 175
   Leu Gly Val Glu Met Glu Ala Ala Gly Ile Tyr Gly Val Ala Ala Glu
               180                 185                 190
   Phe Gly Ala Lys Ala Leu Thr Ile Cys Thr Val Ser Asp His Ile Arg
           195                 200                 205
   Thr His Glu Gln Thr Thr Ala Ala Glu Arg Gln Thr Thr Phe Asn Asp
       210                 215                 220
   Met Ile Lys Ile Ala Leu Glu Ser Val Leu Leu Gly Asp Lys Glu
   225                 230                 235
<210> 25
   <211> 1545
   <212> DNA
   <213> Burkholderia caryophylli
<220>
   <221> CDS
   <222> (1)..(1542)
   <223> coding for phosphonate monoester hydrolase (pehA)
<400> 25
   atg acc aga aaa aat gtc ctg ctt atc gtc gtt gat caa tgg cga gca    48
   Met Thr Arg Lys Asn Val Leu Leu Ile Val Val Asp Gln Trp Arg Ala
    1               5                  10                  15
   gat ttt atc cct cac ctg atg cgg gcg gag ggg cgc gaa cct ttc ctt    96
   Asp Phe Ile Pro His Leu Met Arg Ala Glu Gly Arg Glu Pro Phe Leu
               20                  25                  30
   aaa act ccc aat ctt gat cgt ctt tgc cgg gaa ggc ttg acc ttc cgc    144
   Lys Thr Pro Asn Leu Asp Arg Leu Cys Arg Glu Gly Leu Thr Phe Arg
           35                  40                  45
   aat cat gtc acg acg tgc gtg ccg tgt ggt ccg gca agg gca agc ctg    192
   Asn His Val Thr Thr Cys Val Pro Cys Gly Pro Ala Arg Ala Ser Leu
       50                  55                  60
   ctg acg ggc ctc tac ctg atg aac cac cgg gcg gtg cag aac act gtt    240
   Leu Thr Gly Leu Tyr Leu Met Asn His Arg Ala Val Gln Asn Thr Val
   65                  70                  75                  80
   ccg ctt gac cag cgc cat cta aac ctt ggc aag gcc ctg cgc gcc att    288
   Pro Leu Asp Gln Arg His Leu Asn Leu Gly Lys Ala Leu Arg Ala Ile
                   85                  90                  95
   ggc tac gat ccc gcg ctc att ggt tac acc acc acg aca cct gat ccg    336
   Gly Tyr Asp Pro Ala Leu Ile Gly Tyr Thr Thr Thr Thr Pro Asp Pro
               100                 105                 110
   cgc aca acc tct gca agg gat ccg cgt ttc acg gtc ctg ggc gac atc     384
   Arg Thr Thr Ser Ala Arg Asp Pro Arg Phe Thr Val Leu Gly Asp Ile
           115                 120                 125
   atg gac ggc ttt cgt tcg gtc ggc gca ttc gag ccc aat atg gag ggg     432
   Met Asp Gly Phe Arg Ser Val Gly Ala Phe Glu Pro Asn Met Glu Gly
       130                 135                 140
   tat ttt ggc tgg gtg gcg cag aac ggc ttc gaa ctg cca gag aac cgc     480
   Tyr Phe Gly Trp Val Ala Gln Asn Gly Phe Glu Leu Pro Glu Asn Arg
   145                 150                 155                 160
   gaa gat atc tgg ctg ccg gaa ggt gaa cat tcc gtt ccc ggt gct acc     528
   Glu Asp Ile Trp Leu Pro Glu Gly Glu His Ser Val Pro Gly Ala Thr
                   165                 170                 175
   gac aaa ccg tcg cgc att ccg aag gaa ttt tcg gat tcg aca ttc ttc     576
   Asp Lys Pro Ser Arg Ile Pro Lys Glu Phe Ser Asp Ser Thr Phe Phe
               180                 185                 190
   acg gag cgc gcc ctg aca tat ctg aag ggc agg gac ggc aag cct ttc     624
   Thr Glu Arg Ala Leu Thr Tyr Leu Lys Gly Arg Asp Gly Lys Pro Phe
           195                 200                 205
   ttc ctg cat ctt ggc tat tat cgc ccg cat ccg cct ttc gta gcc tcc     672
   Phe Leu His Leu Gly Tyr Tyr Arg Pro His Pro Pro Phe Val Ala Ser
       210                 215                 220
   gcg ccc tac cat gcg atg tac aaa gcc gaa gat atg cct gcg cct ata     720
   Ala Pro Tyr His Ala Met Tyr Lys Ala Glu Asp Met Pro Ala Pro Ile
   225                 230                 235                 240
   cgt gcg gag aat ccg gat gcc gaa gcg gca cag cat ccg ctc atg aag     768
   Arg Ala Glu Asn Pro Asp Ala Glu Ala Ala Gln His Pro Leu Met Lys
                   245                 250                 255
   cac tat atc gac cac atc aga cgc ggc tcg ttc ttc cat ggc gcg gaa     816
   His Tyr Ile Asp His Ile Arg Arg Gly Ser Phe Phe His Gly Ala Glu
               260                 265                 270
   ggc tcg gga gca acg ctt gat gaa ggc gaa att cgc cag atg cgc gct     864
   Gly Ser Gly Ala Thr Leu Asp Glu Gly Glu Ile Arg Gln Met Arg Ala
           275                 280                 285
   aca tat tgc gga ctg atc acc gag atc gac gat tgt ctg ggg agg gtc     912
   Thr Tyr Cys Gly Leu Ile Thr Glu Ile Asp Asp Cys Leu Gly Arg Val
       290                 295                 300
   ttt gcc tat ctc gat gaa acc ggt cag tgg gac gac acg ctg att atc     960
   Phe Ala Tyr Leu Asp Glu Thr Gly Gln Trp Asp Asp Thr Leu Ile Ile
   305                 310                 315                 320
   ttc acg agc gat cat ggc gaa caa ctg ggc gat cat cac ctg ctc ggc    1008
   Phe Thr Ser Asp His Gly Glu Gln Leu Gly Asp His His Leu Leu Gly
                   325                 330                 335
   aag atc ggt tac aat gcc gaa agc ttc cgt att ccc ttg gtc ata aag    1056
   Lys Ile Gly Tyr Asn Ala Glu Ser Phe Arg Ile Pro Leu Val Ile Lys
               340                 345                 350
   gat gcg gga cag aac cgg cac gcc ggc cag atc gaa gaa ggc ttc tcc    1104
   Asp Ala Gly Gln Asn Arg His Ala Gly Gln Ile Glu Glu Gly Phe Ser
           355                 360                 365
   gaa agc atc gac gtc atg ccg acc atc ctc gaa tgg ctg ggc ggg gaa    1152
   Glu Ser Ile Asp Val Met Pro Thr Ile Leu Glu Trp Leu Gly Gly Glu
       370                 375                 380
   acg cct cgc gcc tgc gac ggc cgt tcg ctg ttg ccg ttt ctg gct gag    1200
   Thr Pro Arg Ala Cys Asp Gly Arg Ser Leu Leu Pro Phe Leu Ala Glu
   385                 390                 395                 400
   gga aag ccc tcc gac tgg cgc acg gaa cta cat tac gag ttc gat ttt    1248
   Gly Lys Pro Ser Asp Trp Arg Thr Glu Leu His Tyr Glu Phe Asp Phe
                   405                 410                 415
   cgc gat gtc ttc tac gat cag ccg cag aac tcg gtc cag ctt tcc cag    1296
   Arg Asp Val Phe Tyr Asp Gln Pro Gln Asn Ser Val Gln Leu Ser Gln
               420                 425                 430
   gat gat tgc agc ctc tgt gtg atc gag gac gaa aac tac aag tac gtg    1344
   Asp Asp Cys Ser Leu Cys Val Ile Glu Asp Glu Asn Tyr Lys Tyr Val
           435                 440                 445
   cat ttt gcc gcc ctg ccg ccg ctg ttc ttc gat ctg aag gca gac ccg    1392
   His Phe Ala Ala Leu Pro Pro Leu Phe Phe Asp Leu Lys Ala Asp Pro
       450                 455                 460
   cat gaa ttc agc aat ctg gct ggc gat cct gct tat gcg gcc ctc gtt    1440
   His Glu Phe Ser Asn Leu Ala Gly Asp Pro Ala Tyr Ala Ala Leu Val
   465                 470                 475                 480
   cgt gac tat gcc cag aag gca ttg tcg tgg cga ctg tct cat gcc gac    1488
   Arg Asp Tyr Ala Gln Lys Ala Leu Ser Trp Arg Leu Ser His Ala Asp
                   485                 490                 495
   cgg aca ctc acc cat tac aga tcc agc ccg caa ggg ctg aca acg cgc    1536
   Arg Thr Leu Thr His Tyr Arg Ser Ser Pro Gln Gly Leu Thr Thr Arg
               500                 505                 510
   aac cat tga                                                        1545
   Asn His
<210> 26
   <211> 514
   <212> PRT
   <213> Burkholderia caryophylli
<400> 26
   Met Thr Arg Lys Asn Val Leu Leu Ile Val Val Asp Gln Trp Arg Ala
    1               5                  10                  15
   Asp Phe Ile Pro His Leu Met Arg Ala Glu Gly Arg Glu Pro Phe Leu
               20                  25                  30
   Lys Thr Pro Asn Leu Asp Arg Leu Cys Arg Glu Gly Leu Thr Phe Arg
           35                  40                  45
   Asn His Val Thr Thr Cys Val Pro Cys Gly Pro Ala Arg Ala Ser Leu
       50                  55                  60
   Leu Thr Gly Leu Tyr Leu Met Asn His Arg Ala Val Gln Asn Thr Val
   65                  70                  75                  80
   Pro Leu Asp Gln Arg His Leu Asn Leu Gly Lys Ala Leu Arg Ala Ile
                   85                  90                  95
   Gly Tyr Asp Pro Ala Leu Ile Gly Tyr Thr Thr Thr Thr Pro Asp Pro
               100                 105                 110
   Arg Thr Thr Ser Ala Arg Asp Pro Arg Phe Thr Val Leu Gly Asp Ile
           115                 120                 125
   Met Asp Gly Phe Arg Ser Val Gly Ala Phe Glu Pro Asn Met Glu Gly
       130                 135                 140
   Tyr Phe Gly Trp Val Ala Gln Asn Gly Phe Glu Leu Pro Glu Asn Arg
   145 150 155 160
   Glu Asp Ile Trp Leu Pro Glu Gly Glu His Ser Val Pro Gly Ala Thr
   165 170 175
   Asp Lys Pro Ser Arg Ile Pro Lys Glu Phe Ser Asp Ser Thr Phe Phe
   180 185 190
   Thr Glu Arg Ala Leu Thr Tyr Leu Lys Gly Arg Asp Gly Lys Pro Phe
   195 200 205
   Phe Leu His Leu Gly Tyr Tyr Arg Pro His Pro Pro Phe Val Ala Ser
   210 215 220
   Ala Pro Tyr His Ala Met Tyr Lys Ala Glu Asp Met Pro Ala Pro Ile
   225 230 235 240
   Arg Ala Glu Asn Pro Asp Ala Glu Ala Ala Gln His Pro Leu Met Lys
   245 250 255
   His Tyr Ile Asp His Ile Arg Arg Gly Ser Phe Phe His Gly Ala Glu
   260 265 270
   Gly Ser Gly Ala Thr Leu Asp Glu Gly Glu Ile Arg Gln Met Arg Ala
   275 280 285
   Thr Tyr Cys Gly Leu Ile Thr Glu Ile Asp Asp Cys Leu Gly Arg Val
   290 295 300
   Phe Ala Tyr Leu Asp Glu Thr Gly Gln Trp Asp Asp Thr Leu Ile Ile
   305 310 315 320
   Phe Thr Ser Asp His Gly Glu Gln Leu Gly Asp His His Leu Leu Gly
   325 330 335
   Lys Ile Gly Tyr Asn Ala Glu Ser Phe Arg Ile Pro Leu Val Ile Lys
   340 345 350
   Asp Ala Gly Gln Asn Arg His Ala Gly Gln Ile Glu Glu Gly Phe Ser
   355 360 365
   Glu Ser Ile Asp Val Met Pro Thr Ile Leu Glu Trp Leu Gly Gly Glu
   370 375 380
   Thr Pro Arg Ala Cys Asp Gly Arg Ser Leu Leu Pro Phe Leu Ala Glu
   385 390 395 400
   Gly Lys Pro Ser Asp Trp Arg Thr Glu Leu His Tyr Glu Phe Asp Phe
   405 410 415
   Arg Asp Val Phe Tyr Asp Gln Pro Gln Asn Ser Val Gln Leu Ser Gln
   420 425 430
   Asp Asp Cys Ser Leu Cys Val Ile Glu Asp Glu Asn Tyr Lys Tyr Val
   435 440 445
   His Phe Ala Ala Leu Pro Pro Leu Phe Phe Asp Leu Lys Ala Asp Pro
   450 455 460
   His Glu Phe Ser Asn Leu Ala Gly Asp Pro Ala Tyr Ala Ala Leu Val
   465 470 475 480
   Arg Asp Tyr Ala Gln Lys Ala Leu Ser Trp Arg Leu Ser His Ala Asp
   485 490 495
   Arg Thr Leu Thr His Tyr Arg Ser Ser Pro Gln Gly Leu Thr Thr Arg
   500 505 510
   Asn His
<210> 27
   <211> 2250
   <212> DNA
   <213> Agrobacterium rhizogenes
<220>
   <221> CDS
   <222> (1)..(2247)
   <223> coding for tryptophane oxygenase (aux1)
<400> 27
   atg gct gga tcc tcc ttc aca ttg cca tca act ggc tca gcg ccc ctt    48
   Met Ala Gly Ser Ser Phe Thr Leu Pro Ser Thr Gly Ser Ala Pro Leu
    1               5                  10                  15
   gat atg atg ctt atc gat gat tca gat ctg ctg caa ttg ggt ctc cag    96
   Asp Met Met Leu Ile Asp Asp Ser Asp Leu Leu Gln Leu Gly Leu Gln
               20                  25                  30
   cag gta ttc tcg aag cgg tac aca gag aca ccg cag tca cgc tac aaa   144
   Gln Val Phe Ser Lys Arg Tyr Thr Glu Thr Pro Gln Ser Arg Tyr Lys
           35                  40                  45
   ctg acc agg agg gct tct cca gac gtc tca tct ggc gaa ggc aat gtg   192
   Leu Thr Arg Arg Ala Ser Pro Asp Val Ser Ser Gly Glu Gly Asn Val
       50                  55                  60
   cat gcc ctt gcg ttc ata tat gtc aac gct gag acg ttg cag atg atc   240
   His Ala Leu Ala Phe Ile Tyr Val Asn Ala Glu Thr Leu Gln Met Ile
   65                  70                  75                  80
   aaa aac gct cga tcg cta acc gaa gcg aac ggc gtc aaa gat ctt gtc   288
   Lys Asn Ala Arg Ser Leu Thr Glu Ala Asn Gly Val Lys Asp Leu Val
                   85                  90                  95
   gcc atc gac gtt ccg cca ttt cga aac gac ttc tca aga gcg cta ctc   336
   Ala Ile Asp Val Pro Pro Phe Arg Asn Asp Phe Ser Arg Ala Leu Leu
               100                 105                 110
   ctt caa gtg atc aac ttg ttg gga aac aac cga aat gcc gat gac gat   384
   Leu Gln Val Ile Asn Leu Leu Gly Asn Asn Arg Asn Ala Asp Asp Asp
           115                 120                 125
   ctt agt cac ttc ata gca gtt gct ctc cca aac agc gcc cgc tct aag   432
   Leu Ser His Phe Ile Ala Val Ala Leu Pro Asn Ser Ala Arg Ser Lys
       130                 135                 140
   atc cta acc acg gca ccg ttc gaa gga agc ttg tca gaa aac ttc agg   480
   Ile Leu Thr Thr Ala Pro Phe Glu Gly Ser Leu Ser Glu Asn Phe Arg
   145                 150                 155                 160
   ggg ttc ccg atc act cgt gaa gga aat gtg gca tgt gaa gtg cta gcc   528
   Gly Phe Pro Ile Thr Arg Glu Gly Asn Val Ala Cys Glu Val Leu Ala
                   165                 170                 175
   tat ggg aat aac ttg atg ccc aag gcc tgc tcc gat tcc ttt cca acc   576
   Tyr Gly Asn Asn Leu Met Pro Lys Ala Cys Ser Asp Ser Phe Pro Thr
               180                 185                 190
   gtg gat ctt ctt tat gac tat ggc aag ttc ttc gag agt tgc gcg gcc   624
   Val Asp Leu Leu Tyr Asp Tyr Gly Lys Phe Phe Glu Ser Cys Ala Ala
           195                 200                 205
   gat gga cgt atc ggt tat ttt cct gaa ggc gtt acg aaa cct aaa gtg   672
   Asp Gly Arg Ile Gly Tyr Phe Pro Glu Gly Val Thr Lys Pro Lys Val
       210                 215                 220
   gct ata att ggc gca ggc ttt tcc ggg ctc gtt gca gcg agc gaa cta     720
   Ala Ile Ile Gly Ala Gly Phe Ser Gly Leu Val Ala Ala Ser Glu Leu
   225                 230                 235                 240
   ctt cat gca ggg gta gac gat gtt acg gtg tat gag gcg agt gat cgg     768
   Leu His Ala Gly Val Asp Asp Val Thr Val Tyr Glu Ala Ser Asp Arg
                   245                 250                 255
   ctt gga gga aag cta tgg tca cac gga ttt aag agt gct cca aat gtg     816
   Leu Gly Gly Lys Leu Trp Ser His Gly Phe Lys Ser Ala Pro Asn Val
               260                 265                 270
   ata gcc gag atg ggg gcc atg cgt ttt ccg cga agt gaa tca tgc ttg     864
   Ile Ala Glu Met Gly Ala Met Arg Phe Pro Arg Ser Glu Ser Cys Leu
           275                 280                 285
   ttc ttc tat ctc aaa aag cac gga ctg gac tcc gtt ggt ctg ttc ccg     912
   Phe Phe Tyr Leu Lys Lys His Gly Leu Asp Ser Val Gly Leu Phe Pro
       290                 295                 300
   aat ccg gga agt gtc gat acc gca ttg ttc tac agg ggc cgt caa tat     960
   Asn Pro Gly Ser Val Asp Thr Ala Leu Phe Tyr Arg Gly Arg Gln Tyr
   305                 310                 315                 320
   atc tgg aaa gcg gga gag gag cca ccg gag ctg ttt cgt cgt gtg cac     1008
   Ile Trp Lys Ala Gly Glu Glu Pro Pro Glu Leu Phe Arg Arg Val His
                   325                 330                 335
   cat gga tgg cgc gca ttt ttg caa gat ggc tat ctc cat gat gga gtc     1056
   His Gly Trp Arg Ala Phe Leu Gln Asp Gly Tyr Leu His Asp Gly Val
               340                 345                 350
   atg ttg gcg tca ccg tta gca att gtt gac gcc ttg aat tta ggg cat     1104
   Met Leu Ala Ser Pro Leu Ala Ile Val Asp Ala Leu Asn Leu Gly His
           355                 360                 365
   cta cag cag gcg cat ggc ttc tgg caa tct tgg ctc aca tat ttt gag     1152
   Leu Gln Gln Ala His Gly Phe Trp Gln Ser Trp Leu Thr Tyr Phe Glu
       370                 375                 380
   cga gag tct ttc tct tct ggc atc gaa aaa atg ttc ttg ggc aat cat     1200
   Arg Glu Ser Phe Ser Ser Gly Ile Glu Lys Met Phe Leu Gly Asn His
   385                 390                 395                 400
   cct ccg ggg ggt gaa caa tgg aat tcc cta gat gac ttg gat ctt ttc     1248
   Pro Pro Gly Gly Glu Gln Trp Asn Ser Leu Asp Asp Leu Asp Leu Phe
                   405                 410                 415
   aaa gcg ctg ggt att gga tcc ggc gga ttc ggc cct gta ttt gaa agt     1296
   Lys Ala Leu Gly Ile Gly Ser Gly Gly Phe Gly Pro Val Phe Glu Ser
               420                 425                 430
   ggg ttt atc gag atc ctt cgc tta gtc gtc aac ggg tat gag gat aac     1344
   Gly Phe Ile Glu Ile Leu Arg Leu Val Val Asn Gly Tyr Glu Asp Asn
           435                 440                 445
   gtg cgg ctg agt tac gaa gga att tct gag ctg cct cat agg atc gcc     1392
   Val Arg Leu Ser Tyr Glu Gly Ile Ser Glu Leu Pro His Arg Ile Ala
       450                 455                 460
   tca cag gta att aac ggc aga tct att cgc gag cgt aca att cac gtt     1440
   Ser Gln Val Ile Asn Gly Arg Ser Ile Arg Glu Arg Thr Ile His Val
   465                 470                 475                 480
   caa gtc gag cag att gat aga gag gag gat aaa ata aat atc aag atc     1488
   Gln Val Glu Gln Ile Asp Arg Glu Glu Asp Lys Ile Asn Ile Lys Ile
                   485                 490                 495
   aaa gga gga aag gtt gag gtc tat gat cga gta ctg gtt aca tcc ggg     1536
   Lys Gly Gly Lys Val Glu Val Tyr Asp Arg Val Leu Val Thr Ser Gly
               500                 505                 510
   ttt gcg aac atc gaa atg cgc cat ctc ctg aca tca agc aac gca ttc     1584
   Phe Ala Asn Ile Glu Met Arg His Leu Leu Thr Ser Ser Asn Ala Phe
           515                 520                 525
   ttc cat gca gat gta agc cat gca ata ggg aac agt cat atg act ggt     1632
   Phe His Ala Asp Val Ser His Ala Ile Gly Asn Ser His Met Thr Gly
       530                 535                 540
   gcg tca aaa ctg ttc ttg ctg act aac gaa aaa ttc tgg cta caa cat     1680
   Ala Ser Lys Leu Phe Leu Leu Thr Asn Glu Lys Phe Trp Leu Gln His
   545                 550                 555                 560
   cat ttg cca tcg tgc ata ctc acc acc ggc gtt gca aag gca gtt tat     1728
   His Leu Pro Ser Cys Ile Leu Thr Thr Gly Val Ala Lys Ala Val Tyr
                   565                 570                 575
   tgc tta gac tat gat ccg cga gat cca agc ggc aaa gga ctg gtg ttg     1776
   Cys Leu Asp Tyr Asp Pro Arg Asp Pro Ser Gly Lys Gly Leu Val Leu
               580                 585                 590
   ata agc tat act tgg gag gat gac tca cat aag ctc cta gcc gtc ccc     1824
   Ile Ser Tyr Thr Trp Glu Asp Asp Ser His Lys Leu Leu Ala Val Pro
           595                 600                 605
   gac aaa aga gaa agg ttc gca tcg ctg cag cgc gat att ggg agg gca     1872
   Asp Lys Arg Glu Arg Phe Ala Ser Leu Gln Arg Asp Ile Gly Arg Ala
       610                 615                 620
   ttc cca gat ttt gcc aag cac cta act cct gca gac ggg aac tat gat     1920
   Phe Pro Asp Phe Ala Lys His Leu Thr Pro Ala Asp Gly Asn Tyr Asp
   625                 630                 635                 640
   gat aat atc gtt caa cat gat tgg ctg act gat ccc cac gct ggc gga     1968
   Asp Asn Ile Val Gln His Asp Trp Leu Thr Asp Pro His Ala Gly Gly
                   645                 650                 655
   gcg ttt aaa ctg aac cgc aga ggc aac gac gta tat tca gaa agg ctt     2016
   Ala Phe Lys Leu Asn Arg Arg Gly Asn Asp Val Tyr Ser Glu Arg Leu
               660                 665                 670
   ttc ttt cag ccc ttt gac gta atg cat ccc gcg gac gat aag gga ctt     2064
   Phe Phe Gln Pro Phe Asp Val Met His Pro Ala Asp Asp Lys Gly Leu
           675                 680                 685
   tac ttg gcc ggt tgt agc tgt tcc ttc acc gga ggg tgg gtt cat ggt     2112
   Tyr Leu Ala Gly Cys Ser Cys Ser Phe Thr Gly Gly Trp Val His Gly
       690                 695                 700
   gcc att cag acc gca tgc aac gct acg tgt gcg atc att tat ggt tcc     2160
   Ala Ile Gln Thr Ala Cys Asn Ala Thr Cys Ala Ile Ile Tyr Gly Ser
   705                 710                 715                 720
   gga cac ctg caa gag cta atc cac tgg cga cac ctc aaa gaa ggt aat     2208
   Gly His Leu Gln Glu Leu Ile His Trp Arg His Leu Lys Glu Gly Asn
                   725                 730                 735
   cca ctg gcg cac gct tgg aag cgg tat agg tat caa gcg tga             2250
   Pro Leu Ala His Ala Trp Lys Arg Tyr Arg Tyr Gln Ala
               740                 745
<210> 28
   <211> 749
   <212> PRT
   <213> Agrobacterium rhizogenes
<400> 28
   Met Ala Gly Ser Ser Phe Thr Leu Pro Ser Thr Gly Ser Ala Pro Leu
   1               5                   10                  15
   Asp Met Met Leu Ile Asp Asp Ser Asp Leu Leu Gln Leu Gly Leu Gln
               20                  25                  30
   Gln Val Phe Ser Lys Arg Tyr Thr Glu Thr Pro Gln Ser Arg Tyr Lys
           35                  40                  45
   Leu Thr Arg Arg Ala Ser Pro Asp Val Ser Ser Gly Glu Gly Asn Val
       50                  55                  60
   His Ala Leu Ala Phe Ile Tyr Val Asn Ala Glu Thr Leu Gln Met Ile
   65                  70                  75                  80
   Lys Asn Ala Arg Ser Leu Thr Glu Ala Asn Gly Val Lys Asp Leu Val
                   85                  90                  95
   Ala Ile Asp Val Pro Pro Phe Arg Asn Asp Phe Ser Arg Ala Leu Leu
               100                 105                 110
   Leu Gln Val Ile Asn Leu Leu Gly Asn Asn Arg Asn Ala Asp Asp Asp
           115                 120                 125
   Leu Ser His Phe Ile Ala Val Ala Leu Pro Asn Ser Ala Arg Ser Lys
       130                 135                 140
   Ile Leu Thr Thr Ala Pro Phe Glu Gly Ser Leu Ser Glu Asn Phe Arg
   145                 150                 155                 160
   Gly Phe Pro Ile Thr Arg Glu Gly Asn Val Ala Cys Glu Val Leu Ala
                   165                 170                 175
   Tyr Gly Asn Asn Leu Met Pro Lys Ala Cys Ser Asp Ser Phe Pro Thr
               180                 185                 190
   Val Asp Leu Leu Tyr Asp Tyr Gly Lys Phe Phe Glu Ser Cys Ala Ala
           195                 200                 205
   Asp Gly Arg Ile Gly Tyr Phe Pro Glu Gly Val Thr Lys Pro Lys Val
       210                 215                 220
   Ala Ile Ile Gly Ala Gly Phe Ser Gly Leu Val Ala Ala Ser Glu Leu
   225                 230                 235                 240
   Leu His Ala Gly Val Asp Asp Val Thr Val Tyr Glu Ala Ser Asp Arg
                   245                 250                 255
   Leu Gly Gly Lys Leu Trp Ser His Gly Phe Lys Ser Ala Pro Asn Val
               260                 265                 270
   Ile Ala Glu Met Gly Ala Met Arg Phe Pro Arg Ser Glu Ser Cys Leu
           275                 280                 285
   Phe Phe Tyr Leu Lys Lys His Gly Leu Asp Ser Val Gly Leu Phe Pro
       290                 295                 300
   Asn Pro Gly Ser Val Asp Thr Ala Leu Phe Tyr Arg Gly Arg Gln Tyr
   305                 310                 315                 320
   Ile Trp Lys Ala Gly Glu Glu Pro Pro Glu Leu Phe Arg Arg Val His
                   325                 330                 335
   His Gly Trp Arg Ala Phe Leu Gln Asp Gly Tyr Leu His Asp Gly Val
               340                 345                 350
   Met Leu Ala Ser Pro Leu Ala Ile Val Asp Ala Leu Asn Leu Gly His
   355 360 365
   Leu Gln Gln Ala His Gly Phe Trp Gln Ser Trp Leu Thr Tyr Phe Glu
   370 375 380
   Arg Glu Ser Phe Ser Ser Gly Ile Glu Lys Met Phe Leu Gly Asn His
   385 390 395 400
   Pro Pro Gly Gly Glu Gln Trp Asn Ser Leu Asp Asp Leu Asp Leu Phe
   405 410 415
   Lys Ala Leu Gly Ile Gly Ser Gly Gly Phe Gly Pro Val Phe Glu Ser
   420 425 430
   Gly Phe Ile Glu Ile Leu Arg Leu Val Val Asn Gly Tyr Glu Asp Asn
   435 440 445
   Val Arg Leu Ser Tyr Glu Gly Ile Ser Glu Leu Pro His Arg Ile Ala
   450 455 460
   Ser Gln Val Ile Asn Gly Arg Ser Ile Arg Glu Arg Thr Ile His Val
   465 470 475 480
   Gln Val Glu Gln Ile Asp Arg Glu Glu Asp Lys Ile Asn Ile Lys Ile
   485 490 495
   Lys Gly Gly Lys Val Glu Val Tyr Asp Arg Val Leu Val Thr Ser Gly
   500 505 510
   Phe Ala Asn Ile Glu Met Arg His Leu Leu Thr Ser Ser Asn Ala Phe
   515 520 525
   Phe His Ala Asp Val Ser His Ala Ile Gly Asn Ser His Met Thr Gly
   530 535 540
   Ala Ser Lys Leu Phe Leu Leu Thr Asn Glu Lys Phe Trp Leu Gln His
   545 550 555 560
   His Leu Pro Ser Cys Ile Leu Thr Thr Gly Val Ala Lys Ala Val Tyr
   565 570 575
   Cys Leu Asp Tyr Asp Pro Arg Asp Pro Ser Gly Lys Gly Leu Val Leu
   580 585 590
   Ile Ser Tyr Thr Trp Glu Asp Asp Ser His Lys Leu Leu Ala Val Pro
   595 600 605
   Asp Lys Arg Glu Arg Phe Ala Ser Leu Gln Arg Asp Ile Gly Arg Ala
   610 615 620
   Phe Pro Asp Phe Ala Lys His Leu Thr Pro Ala Asp Gly Asn Tyr Asp
   625 630 635 640
   Asp Asn Ile Val Gln His Asp Trp Leu Thr Asp Pro His Ala Gly Gly
   645 650 655
   Ala Phe Lys Leu Asn Arg Arg Gly Asn Asp Val Tyr Ser Glu Arg Leu
   660 665 670
   Phe Phe Gln Pro Phe Asp Val Met His Pro Ala Asp Asp Lys Gly Leu
   675 680 685
   Tyr Leu Ala Gly Cys Ser Cys Ser Phe Thr Gly Gly Trp Val His Gly
   690 695 700
   Ala Ile Gln Thr Ala Cys Asn Ala Thr Cys Ala Ile Ile Tyr Gly Ser
   705 710 715 720
   Gly His Leu Gln Glu Leu Ile His Trp Arg His Leu Lys Glu Gly Asn
   725 730 735
   Pro Leu Ala His Ala Trp Lys Arg Tyr Arg Tyr Gln Ala
   740 745
<210> 29
   <211> 1401
   <212> DNA
   <213> Agrobacterium rhizogenes
<220>
   <221> CDS
   <222> (1)..(1398)
   <223> coding for indole acetamide hydrolase
<400> 29
   atg gtg acc ctc tcc tcg atc acc gag acg ctt aaa tgt ctc agg gaa      48
   Met Val Thr Leu Ser Ser Ile Thr Glu Thr Leu Lys Cys Leu Arg Glu
   1               5                   10                  15
   aga aaa tac tcg tgc ttt gag tta atc gaa acg ata ata gcc cgc tgt      96
   Arg Lys Tyr Ser Cys Phe Glu Leu Ile Glu Thr Ile Ile Ala Arg Cys
               20                  25                  30
   gaa gca gca aga tcc tta aac gcc ttt ctg gaa acc gac tgg gcg cac     144
   Glu Ala Ala Arg Ser Leu Asn Ala Phe Leu Glu Thr Asp Trp Ala His
           35                  40                  45
   cta cgg tgg act gcc agc aaa atc gat caa cac gga ggt gcc ggt gtt     192
   Leu Arg Trp Thr Ala Ser Lys Ile Asp Gln His Gly Gly Ala Gly Val
       50                  55                  60
   ggc cta gct ggc gtt ccc cta tgc ttt aaa gcg aat att gcg aca ggc     240
   Gly Leu Ala Gly Val Pro Leu Cys Phe Lys Ala Asn Ile Ala Thr Gly
   65                  70                  75                  80
   agg ttc gcc gcg acc gct ggt acg cca ggc tta cag aac cac aaa ccc     288
   Arg Phe Ala Ala Thr Ala Gly Thr Pro Gly Leu Gln Asn His Lys Pro
                   85                  90                  95
   aag acg cct gcc gga gtt gca cga caa ctt ctc gcg gct ggg gca ctg     336
   Lys Thr Pro Ala Gly Val Ala Arg Gln Leu Leu Ala Ala Gly Ala Leu
               100                 105                 110
   cct ggc gct tcg gga aac atg cac gaa ttg tct ttt ggg atc acg agc     384
   Pro Gly Ala Ser Gly Asn Met His Glu Leu Ser Phe Gly Ile Thr Ser
           115                 120                 125
   aac aac ttc gcc aca ggc gcc gta cga aac ccg tgg aac cct agt ctc     432
   Asn Asn Phe Ala Thr Gly Ala Val Arg Asn Pro Trp Asn Pro Ser Leu
       130                 135                 140
   atc cca ggg gga tca agt ggg ggt gtg gcc gcc gcg gtg gcc ggc cga     480
   Ile Pro Gly Gly Ser Ser Gly Gly Val Ala Ala Ala Val Ala Gly Arg
   145                 150                 155                 160
   ttg atg ctg ggc ggc gtc gga act gac acg gga gcg tcg gtc cgt tta     528
   Leu Met Leu Gly Gly Val Gly Thr Asp Thr Gly Ala Ser Val Arg Leu
                   165                 170                 175
   ccg gcc gcc ttg tgc ggc gtg gtg ggg ttt cgt cct acc gtg ggg cga     576
   Pro Ala Ala Leu Cys Gly Val Val Gly Phe Arg Pro Thr Val Gly Arg
               180                 185                 190
   tat cca acg gac gga ata gtt ccg gta agc ccc acc cgg gac acc cct     624
   Tyr Pro Thr Asp Gly Ile Val Pro Val Ser Pro Thr Arg Asp Thr Pro
           195                 200                 205
   ggc gtt atc gca cag aat gtt ccg gac gtg att ctt ctt gac ggt atc    672
   Gly Val Ile Ala Gln Asn Val Pro Asp Val Ile Leu Leu Asp Gly Ile
       210                 215                 220
   att tgc ggg aga ccg ccg gtt aat caa acg gtc cgc ctg aag ggg ctg    720
   Ile Cys Gly Arg Pro Pro Val Asn Gln Thr Val Arg Leu Lys Gly Leu
   225                 230                 235                 240
   cgt ata ggc ttg cca acc gct tac ttt tac aac gac ctg gag ccc gat    768
   Arg Ile Gly Leu Pro Thr Ala Tyr Phe Tyr Asn Asp Leu Glu Pro Asp
                   245                 250                 255
   gtc gcc tta gca gcc gag acg att atc aga gtt ctg gca cgc aaa gat    816
   Val Ala Leu Ala Ala Glu Thr Ile Ile Arg Val Leu Ala Arg Lys Asp
               260                 265                 270
   gtt act ttt gtt gaa gca gat att cct gat tta gcg cat cac aat gaa    864
   Val Thr Phe Val Glu Ala Asp Ile Pro Asp Leu Ala His His Asn Glu
           275                 280                 285
   ggg gtc agc ttt ccg act gcc atc tac gaa ttt ccg ttg tcc ctt gaa    912
   Gly Val Ser Phe Pro Thr Ala Ile Tyr Glu Phe Pro Leu Ser Leu Glu
       290                 295                 300
   cat tat att cag aac ttc gta gag ggt gtt tcc ttt tct gag gtt gtc    960
   His Tyr Ile Gln Asn Phe Val Glu Gly Val Ser Phe Ser Glu Val Val
   305                 310                 315                 320
   aga gcg att cgc agt ccg gat gtt gca agt att ctc aat gca caa ctc    1008
   Arg Ala Ile Arg Ser Pro Asp Val Ala Ser Ile Leu Asn Ala Gln Leu
                   325                 330                 335
   tcg gat aat ctt att tcc aaa agc gag tat tgt ctg gcg cga cgt ttt    1056
   Ser Asp Asn Leu Ile Ser Lys Ser Glu Tyr Cys Leu Ala Arg Arg Phe
               340                 345                 350
   ttc aga ccg aga ctc caa gcg gcc tac cac agt tac ttc aag gcg cat    1104
   Phe Arg Pro Arg Leu Gln Ala Ala Tyr His Ser Tyr Phe Lys Ala His
           355                 360                 365
   cag cta gat gca att ctt ttc cca aca gct ccg ttg aca gcc aag cca    1152
   Gln Leu Asp Ala Ile Leu Phe Pro Thr Ala Pro Leu Thr Ala Lys Pro
       370                 375                 380
   att ggc cat gat cta tcg gtg att cac aat ggc tca atg acc gat acc    1200
   Ile Gly His Asp Leu Ser Val Ile His Asn Gly Ser Met Thr Asp Thr
   385                 390                 395                 400
   ttt aaa atc ttc gtg cgg aat gta gat ccc agc agt aat gcg ggc ctg    1248
   Phe Lys Ile Phe Val Arg Asn Val Asp Pro Ser Ser Asn Ala Gly Leu
                   405                 410                 415
   ccg ggc cta agt ctt ccc gtt tct ctt agt tcc aac ggt ctg cct att    1296
   Pro Gly Leu Ser Leu Pro Val Ser Leu Ser Ser Asn Gly Leu Pro Ile
               420                 425                 430
   ggc atg gaa atc gat ggc tct gca agc tcg gat gaa cgt ctg tta gca    1344
   Gly Met Glu Ile Asp Gly Ser Ala Ser Ser Asp Glu Arg Leu Leu Ala
           435                 440                 445
   att gga cta gcg ata gaa gaa gca ata gac ttt agg cat cgt ccg act    1392
   Ile Gly Leu Ala Ile Glu Glu Ala Ile Asp Phe Arg His Arg Pro Thr
       450                 455                 460
   ctg tcg taa                                                        1401
   Leu Ser
   465
<210> 30
   <211> 466
   <212> PRT
   <213> Agrobacterium rhizogenes
<400> 30
   Met Val Thr Leu Ser Ser Ile Thr Glu Thr Leu Lys Cys Leu Arg Glu
   1               5                   10                  15
   Arg Lys Tyr Ser Cys Phe Glu Leu Ile Glu Thr Ile Ile Ala Arg Cys
               20                  25                  30
   Glu Ala Ala Arg Ser Leu Asn Ala Phe Leu Glu Thr Asp Trp Ala His
           35                  40                  45
   Leu Arg Trp Thr Ala Ser Lys Ile Asp Gln His Gly Gly Ala Gly Val
       50                  55                  60
   Gly Leu Ala Gly Val Pro Leu Cys Phe Lys Ala Asn Ile Ala Thr Gly
   65                  70                  75                  80
   Arg Phe Ala Ala Thr Ala Gly Thr Pro Gly Leu Gln Asn His Lys Pro
                   85                  90                  95
   Lys Thr Pro Ala Gly Val Ala Arg Gln Leu Leu Ala Ala Gly Ala Leu
               100                 105                 110
   Pro Gly Ala Ser Gly Asn Met His Glu Leu Ser Phe Gly Ile Thr Ser
           115                 120                 125
   Asn Asn Phe Ala Thr Gly Ala Val Arg Asn Pro Trp Asn Pro Ser Leu
       130                 135                 140
   Ile Pro Gly Gly Ser Ser Gly Gly Val Ala Ala Ala Val Ala Gly Arg
   145                 150                 155                 160
   Leu Met Leu Gly Gly Val Gly Thr Asp Thr Gly Ala Ser Val Arg Leu
                   165                 170                 175
   Pro Ala Ala Leu Cys Gly Val Val Gly Phe Arg Pro Thr Val Gly Arg
               180                 185                 190
   Tyr Pro Thr Asp Gly Ile Val Pro Val Ser Pro Thr Arg Asp Thr Pro
           195                 200                 205
   Gly Val Ile Ala Gln Asn Val Pro Asp Val Ile Leu Leu Asp Gly Ile
       210                 215                 220
   Ile Cys Gly Arg Pro Pro Val Asn Gln Thr Val Arg Leu Lys Gly Leu
   225                 230                 235                 240
   Arg Ile Gly Leu Pro Thr Ala Tyr Phe Tyr Asn Asp Leu Glu Pro Asp
                   245                 250                 255
   Val Ala Leu Ala Ala Glu Thr Ile Ile Arg Val Leu Ala Arg Lys Asp
               260                 265                 270
   Val Thr Phe Val Glu Ala Asp Ile Pro Asp Leu Ala His His Asn Glu
           275                 280                 285
   Gly Val Ser Phe Pro Thr Ala Ile Tyr Glu Phe Pro Leu Ser Leu Glu
       290                 295                 300
   His Tyr Ile Gln Asn Phe Val Glu Gly Val Ser Phe Ser Glu Val Val
   305                 310                 315                 320
   Arg Ala Ile Arg Ser Pro Asp Val Ala Ser Ile Leu Asn Ala Gln Leu
                   325                 330                 335
   Ser Asp Asn Leu Ile Ser Lys Ser Glu Tyr Cys Leu Ala Arg Arg Phe
               340                 345                 350
   Phe Arg Pro Arg Leu Gln Ala Ala Tyr His Ser Tyr Phe Lys Ala His
   355 360 365
   Gln Leu Asp Ala Ile Leu Phe Pro Thr Ala Pro Leu Thr Ala Lys Pro
   370 375 380
   Ile Gly His Asp Leu Ser Val Ile His Asn Gly Ser Met Thr Asp Thr
   385 390 395 400
   Phe Lys Ile Phe Val Arg Asn Val Asp Pro Ser Ser Asn Ala Gly Leu
   405 410 415
   Pro Gly Leu Ser Leu Pro Val Ser Leu Ser Ser Asn Gly Leu Pro Ile
   420 425 430
   Gly Met Glu Ile Asp Gly Ser Ala Ser Ser Asp Glu Arg Leu Leu Ala
   435 440 445
   Ile Gly Leu Ala Ile Glu Glu Ala Ile Asp Phe Arg His Arg Pro Thr
   450 455 460
   Leu Ser
   465
<210> 31
   <211> 2268
   <212> DNA
   <213> Agrobacterium tumefaciens
<220>
   <221> CDS
   <222> (1)..(2265)
   <223> coding for tryptophan monooxygenase
<400> 31
   atg tca gct tca cct ctc ctt gat aac cag tgc gat cat ttc tct acc     48
   Met Ser Ala Ser Pro Leu Leu Asp Asn Gln Cys Asp His Phe Ser Thr
    1               5                  10                  15
   aaa atg gtg gat ctg ata atg gtc gat aag gct gat gaa ttg gac cgc     96
   Lys Met Val Asp Leu Ile Met Val Asp Lys Ala Asp Glu Leu Asp Arg
               20                  25                  30
   agg gtt tcc gat gcc ttc tca gaa cgt gaa gct tct agg gga agg agg    144
   Arg Val Ser Asp Ala Phe Ser Glu Arg Glu Ala Ser Arg Gly Arg Arg
           35                  40                  45
   att act caa atc tcc ggc gag tgc agc gct ggg tta gct tgc aaa agg    192
   Ile Thr Gln Ile Ser Gly Glu Cys Ser Ala Gly Leu Ala Cys Lys Arg
       50                  55                  60
   ctg gcc gac ggt cgc ttt ccc gag atc tca act ggt gag aag gta gca    240
   Leu Ala Asp Gly Arg Phe Pro Glu Ile Ser Thr Gly Glu Lys Val Ala
   65                  70                  75                  80
   gcc ctc tcc gct tac atc tat gtt ggc aag gaa att ctg ggg cgg ata    288
   Ala Leu Ser Ala Tyr Ile Tyr Val Gly Lys Glu Ile Leu Gly Arg Ile
                   85                  90                  95
   ctt gaa tcg gaa cct tgg gcg cga gca aga gtg agt ggt ctc gtt gcc    336
   Leu Glu Ser Glu Pro Trp Ala Arg Ala Arg Val Ser Gly Leu Val Ala
               100                 105                 110
   atc gac ctt gca cca ttt tgt atg gat ttc tcc gaa gca caa ctt ctc    384
   Ile Asp Leu Ala Pro Phe Cys Met Asp Phe Ser Glu Ala Gln Leu Leu
           115                 120                 125
   caa acc ctg ttt ttg ctg agc ggt aaa aga tgt gca tcc agc gat ctt     432
   Gln Thr Leu Phe Leu Leu Ser Gly Lys Arg Cys Ala Ser Ser Asp Leu
       130                 135                 140
   agt cat ttc gtg gcc att tca atc tct aag act gcc cgc tcc cga acc     480
   Ser His Phe Val Ala Ile Ser Ile Ser Lys Thr Ala Arg Ser Arg Thr
   145                 150                 155                 160
   ctg caa atg ccg ccg tac gag aaa ggc acg acg aaa cgc gtt acc ggg     528
   Leu Gln Met Pro Pro Tyr Glu Lys Gly Thr Thr Lys Arg Val Thr Gly
                   165                 170                 175
   ttt acc ctg acc ctt gaa gag gcc gta cca ttt gac atg gta gct tat     576
   Phe Thr Leu Thr Leu Glu Glu Ala Val Pro Phe Asp Met Val Ala Tyr
               180                 185                 190
   ggt cga aac ctg atg ctg aag gct tcg gca ggt tcc ttt cca aca att     624
   Gly Arg Asn Leu Met Leu Lys Ala Ser Ala Gly Ser Phe Pro Thr Ile
           195                 200                 205
   gac ttg ctc tat gac tac aga tcg ttt ttt gac caa tgt tcc gat att     672
   Asp Leu Leu Tyr Asp Tyr Arg Ser Phe Phe Asp Gln Cys Ser Asp Ile
       210                 215                 220
   gga cgg atc ggc ttc ttt ccg gaa gat gtt cct aag ccg aaa gtg gcg     720
   Gly Arg Ile Gly Phe Phe Pro Glu Asp Val Pro Lys Pro Lys Val Ala
   225                 230                 235                 240
   atc att ggc gct ggc att tcc gga ctc gtg gta gca agc gaa ctg ctt     768
   Ile Ile Gly Ala Gly Ile Ser Gly Leu Val Val Ala Ser Glu Leu Leu
                   245                 250                 255
   cat gct ggt gta gac gat gtt aca ata tat gaa gca agt gat cgg gtt     816
   His Ala Gly Val Asp Asp Val Thr Ile Tyr Glu Ala Ser Asp Arg Val
               260                 265                 270
   gga ggc aag ctt tgg tca cat gct ttc aag gat gct ccc agc gtg gtg     864
   Gly Gly Lys Leu Trp Ser His Ala Phe Lys Asp Ala Pro Ser Val Val
           275                 280                 285
   gcc gaa atg ggg gcg atg cga ttt cct cct gct gca tcg tgc ttg ttt     912
   Ala Glu Met Gly Ala Met Arg Phe Pro Pro Ala Ala Ser Cys Leu Phe
       290                 295                 300
   ttc ttc ctc gag cgg tac ggc ctg tct tcg atg agg ccg ttc cca aat     960
   Phe Phe Leu Glu Arg Tyr Gly Leu Ser Ser Met Arg Pro Phe Pro Asn
   305                 310                 315                 320
   ccc ggc aca gtc gac act aac ttg gtc tac caa ggc ctc cga tac gtg    1008
   Pro Gly Thr Val Asp Thr Asn Leu Val Tyr Gln Gly Leu Arg Tyr Val
                   325                 330                 335
   tgg aaa gcc ggg cag cag cca ccg aag ctg ttc cat cgc gtt tac agc    1056
   Trp Lys Ala Gly Gln Gln Pro Pro Lys Leu Phe His Arg Val Tyr Ser
               340                 345                 350
   ggt tgg cgt gcg ttc ttg agg gac ggt ttc cat gag gga gat att gtg    1104
   Gly Trp Arg Ala Phe Leu Arg Asp Gly Phe His Glu Gly Asp Ile Val
           355                 360                 365
   ttg gct tcg cct gtt gtt att act caa gcc ttg aaa tca gga gac att    1152
   Leu Ala Ser Pro Val Val Ile Thr Gln Ala Leu Lys Ser Gly Asp Ile
       370                 375                 380
   agg cgg gct cat gac tcc tgg caa act tgg ctg aac cgt ttc ggg agg    1200
   Arg Arg Ala His Asp Ser Trp Gln Thr Trp Leu Asn Arg Phe Gly Arg
   385                 390                 395                 400
   gag tcc ttc tct tca gcg ata gag agg atc ttt ctg ggc acg cat cct    1248
   Glu Ser Phe Ser Ser Ala Ile Glu Arg Ile Phe Leu Gly Thr His Pro
                   405                 410                 415
   cct ggt ggt gaa aca tgg agt ttc cct cat gat tgg gac cta ttc aag    1296
   Pro Gly Gly Glu Thr Trp Ser Phe Pro His Asp Trp Asp Leu Phe Lys
               420                 425                 430
   cta atg gga ata gga tct ggc ggg ttt ggt cca gtt ttt gaa agc ggg    1344
   Leu Met Gly Ile Gly Ser Gly Gly Phe Gly Pro Val Phe Glu Ser Gly
           435                 440                 445
   ttt att gag atc ctt cgc ttg gtc ata aac gga tat gaa gaa aat cag    1392
   Phe Ile Glu Ile Leu Arg Leu Val Ile Asn Gly Tyr Glu Glu Asn Gln
       450                 455                 460
   cgg atg tgc tct gaa gga atc tca gaa ctt cca cgt cga ata gcc tct    1440
   Arg Met Cys Ser Glu Gly Ile Ser Glu Leu Pro Arg Arg Ile Ala Ser
   465                 470                 475                 480
   caa gtg gtt aac ggt gtg tct gta agc cag cgt ata cgc cat gtt caa    1488
   Gln Val Val Asn Gly Val Ser Val Ser Gln Arg Ile Arg His Val Gln
                   485                 490                 495
   gtc agg gcg att gag aag gaa aag aca aaa ata aag ata agg ctt aag    1536
   Val Arg Ala Ile Glu Lys Glu Lys Thr Lys Ile Lys Ile Arg Leu Lys
               500                 505                 510
   agc ggg ata tct gaa ctt tat gat aag gtg gtg gtt aca tct gga ctc    1584
   Ser Gly Ile Ser Glu Leu Tyr Asp Lys Val Val Val Thr Ser Gly Leu
           515                 520                 525
   gca aat atc caa ctc agg cat tgt ctg aca tgc gat acc acc att ttt    1632
   Ala Asn Ile Gln Leu Arg His Cys Leu Thr Cys Asp Thr Thr Ile Phe
       530                 535                 540
   cgt gca cca gtg aac caa gcg gtt gat aac agc cat atg aca ggc tcg    1680
   Arg Ala Pro Val Asn Gln Ala Val Asp Asn Ser His Met Thr Gly Ser
   545                 550                 555                 560
   tca aaa ctc ttt ctg ctg act gaa cga aaa ttt tgg tta gac cat atc    1728
   Ser Lys Leu Phe Leu Leu Thr Glu Arg Lys Phe Trp Leu Asp His Ile
                   565                 570                 575
   ctc ccg tcc tgt gtc ctc atg gac ggg atc gca aaa gca gtg tac tgc    1776
   Leu Pro Ser Cys Val Leu Met Asp Gly Ile Ala Lys Ala Val Tyr Cys
               580                 585                 590
   ttg gac tat gag ccg cag gat ccg aat ggt aaa ggt ctg gtg ccc ccc    1824
   Leu Asp Tyr Glu Pro Gln Asp Pro Asn Gly Lys Gly Leu Val Pro Pro
           595                 600                 605
   act tat aca tgg gag gac gac tcc cac aag ctg ttg gcg gtt ccc gac    1872
   Thr Tyr Thr Trp Glu Asp Asp Ser His Lys Leu Leu Ala Val Pro Asp
       610                 615                 620
   aaa aaa gag cga ttc tgt ctg ctg cgg gac gca att tcg aga tct ttc    1920
   Lys Lys Glu Arg Phe Cys Leu Leu Arg Asp Ala Ile Ser Arg Ser Phe
   625                 630                 635                 640
   ccg gcg ttt gcc cag cat cta gtt cct gcc tgc gct gat tac gac caa    1968
   Pro Ala Phe Ala Gln His Leu Val Pro Ala Cys Ala Asp Tyr Asp Gln
                   645                 650                 655
   aat gtt gtt caa cat gat tgg ctt aca gac gag aat gcc ggg gga gct    2016
   Asn Val Val Gln His Asp Trp Leu Thr Asp Glu Asn Ala Gly Gly Ala
               660                 665                 670
   ttc aaa ctc aac cgg cgt ggc gag gat ttt tat tct gaa gaa ctt ttc     2064
   Phe Lys Leu Asn Arg Arg Gly Glu Asp Phe Tyr Ser Glu Glu Leu Phe
           675                 680                 685
   ttt caa gcg ctg gac atg cct aat gat acc gga gtt tac ttg gcg ggt     2112
   Phe Gln Ala Leu Asp Met Pro Asn Asp Thr Gly Val Tyr Leu Ala Gly
       690                 695                 700
   tgc agt tgt tcc ttc acc ggt gga tgg gtg gag ggc gct att cag acc     2160
   Cys Ser Cys Ser Phe Thr Gly Gly Trp Val Glu Gly Ala Ile Gln Thr
   705                 710                 715                 720
   gcg tgt aac gcc gtc tgt gca att atc cac aat tgt gga ggt att ttg     2208
   Ala Cys Asn Ala Val Cys Ala Ile Ile His Asn Cys Gly Gly Ile Leu
                   725                 730                 735
   gca aag gac aat cct ctc gaa cac tct tgg aag aga tat aac tac cgc     2256
   Ala Lys Asp Asn Pro Leu Glu His Ser Trp Lys Arg Tyr Asn Tyr Arg
               740                 745                 750
   aat aga aat taa                                                     2268
   Asn Arg Asn
           755
<210> 32
   <211> 755
   <212> PRT
   <213> Agrobacterium tumefaciens
<400> 32
   Met Ser Ala Ser Pro Leu Leu Asp Asn Gln Cys Asp His Phe Ser Thr
   1               5                   10                  15
   Lys Met Val Asp Leu Ile Met Val Asp Lys Ala Asp Glu Leu Asp Arg
               20                  25                  30
   Arg Val Ser Asp Ala Phe Ser Glu Arg Glu Ala Ser Arg Gly Arg Arg
           35                  40                  45
   Ile Thr Gln Ile Ser Gly Glu Cys Ser Ala Gly Leu Ala Cys Lys Arg
       50                  55                  60
   Leu Ala Asp Gly Arg Phe Pro Glu Ile Ser Thr Gly Glu Lys Val Ala
   65                  70                  75                  80
   Ala Leu Ser Ala Tyr Ile Tyr Val Gly Lys Glu Ile Leu Gly Arg Ile
                   85                  90                  95
   Leu Glu Ser Glu Pro Trp Ala Arg Ala Arg Val Ser Gly Leu Val Ala
               100                 105                 110
   Ile Asp Leu Ala Pro Phe Cys Met Asp Phe Ser Glu Ala Gln Leu Leu
           115                 120                 125
   Gln Thr Leu Phe Leu Leu Ser Gly Lys Arg Cys Ala Ser Ser Asp Leu
       130                 135                 140
   Ser His Phe Val Ala Ile Ser Ile Ser Lys Thr Ala Arg Ser Arg Thr
   145                 150                 155                 160
   Leu Gln Met Pro Pro Tyr Glu Lys Gly Thr Thr Lys Arg Val Thr Gly
                   165                 170                 175
   Phe Thr Leu Thr Leu Glu Glu Ala Val Pro Phe Asp Met Val Ala Tyr
               180                 185                 190
   Gly Arg Asn Leu Met Leu Lys Ala Ser Ala Gly Ser Phe Pro Thr Ile
           195                 200                 205
   Asp Leu Leu Tyr Asp Tyr Arg Ser Phe Phe Asp Gln Cys Ser Asp Ile
       210                 215                 220
   Gly Arg Ile Gly Phe Phe Pro Glu Asp Val Pro Lys Pro Lys Val Ala
   225                 230                 235                 240
   Ile Ile Gly Ala Gly Ile Ser Gly Leu Val Val Ala Ser Glu Leu Leu
                   245                 250                 255
   His Ala Gly Val Asp Asp Val Thr Ile Tyr Glu Ala Ser Asp Arg Val
               260                 265                 270
   Gly Gly Lys Leu Trp Ser His Ala Phe Lys Asp Ala Pro Ser Val Val
           275                 280                 285
   Ala Glu Met Gly Ala Met Arg Phe Pro Pro Ala Ala Ser Cys Leu Phe
       290                 295                 300
   Phe Phe Leu Glu Arg Tyr Gly Leu Ser Ser Met Arg Pro Phe Pro Asn
   305                 310                 315                 320
   Pro Gly Thr Val Asp Thr Asn Leu Val Tyr Gln Gly Leu Arg Tyr Val
                   325                 330                 335
   Trp Lys Ala Gly Gln Gln Pro Pro Lys Leu Phe His Arg Val Tyr Ser
               340                 345                 350
   Gly Trp Arg Ala Phe Leu Arg Asp Gly Phe His Glu Gly Asp Ile Val
           355                 360                 365
   Leu Ala Ser Pro Val Val Ile Thr Gln Ala Leu Lys Ser Gly Asp Ile
       370                 375                 380
   Arg Arg Ala His Asp Ser Trp Gln Thr Trp Leu Asn Arg Phe Gly Arg
   385                 390                 395                 400
   Glu Ser Phe Ser Ser Ala Ile Glu Arg Ile Phe Leu Gly Thr His Pro
                   405                 410                 415
   Pro Gly Gly Glu Thr Trp Ser Phe Pro His Asp Trp Asp Leu Phe Lys
               420                 425                 430
   Leu Met Gly Ile Gly Ser Gly Gly Phe Gly Pro Val Phe Glu Ser Gly
           435                 440                 445
   Phe Ile Glu Ile Leu Arg Leu Val Ile Asn Gly Tyr Glu Glu Asn Gln
       450                 455                 460
   Arg Met Cys Ser Glu Gly Ile Ser Glu Leu Pro Arg Arg Ile Ala Ser
   465                 470                 475                 480
   Gln Val Val Asn Gly Val Ser Val Ser Gln Arg Ile Arg His Val Gln
                   485                 490                 495
   Val Arg Ala Ile Glu Lys Glu Lys Thr Lys Ile Lys Ile Arg Leu Lys
               500                 505                 510
   Ser Gly Ile Ser Glu Leu Tyr Asp Lys Val Val Val Thr Ser Gly Leu
           515                 520                 525
   Ala Asn Ile Gln Leu Arg His Cys Leu Thr Cys Asp Thr Thr Ile Phe
       530                 535                 540
   Arg Ala Pro Val Asn Gln Ala Val Asp Asn Ser His Met Thr Gly Ser
   545                 550                 555                 560
   Ser Lys Leu Phe Leu Leu Thr Glu Arg Lys Phe Trp Leu Asp His Ile
                   565                 570                 575
   Leu Pro Ser Cys Val Leu Met Asp Gly Ile Ala Lys Ala Val Tyr Cys
               580                 585                 590
   Leu Asp Tyr Glu Pro Gln Asp Pro Asn Gly Lys Gly Leu Val Pro Pro
   595 600 605
   Thr Tyr Thr Trp Glu Asp Asp Ser His Lys Leu Leu Ala Val Pro Asp
   610 615 620
   Lys Lys Glu Arg Phe Cys Leu Leu Arg Asp Ala Ile Ser Arg Ser Phe
   625 630 635 640
   Pro Ala Phe Ala Gln His Leu Val Pro Ala Cys Ala Asp Tyr Asp Gln
   645 650 655
   Asn Val Val Gln His Asp Trp Leu Thr Asp Glu Asn Ala Gly Gly Ala
   660 665 670
   Phe Lys Leu Asn Arg Arg Gly Glu Asp Phe Tyr Ser Glu Glu Leu Phe
   675 680 685
   Phe Gln Ala Leu Asp Met Pro Asn Asp Thr Gly Val Tyr Leu Ala Gly
   690 695 700
   Cys Ser Cys Ser Phe Thr Gly Gly Trp Val Glu Gly Ala Ile Gln Thr
   705 710 715 720
   Ala Cys Asn Ala Val Cys Ala Ile Ile His Asn Cys Gly Gly Ile Leu
   725 730 735
   Ala Lys Asp Asn Pro Leu Glu His Ser Trp Lys Arg Tyr Asn Tyr Arg
   740 745 750
   Asn Arg Asn
   755
<210> 33
   <211> 1404
   <212> DNA
   <213> Agrobacterium tumefaciens
<220>
   <221> CDS
   <222> (1)..(1401)
   <223> coding for indole acetamide hydrolase
<400> 33
   atg gtg ccc att acc tcg tta gca caa acc cta gaa cgc ctg aga cgg   48
   Met Val Pro Ile Thr Ser Leu Ala Gln Thr Leu Glu Arg Leu Arg Arg
    1               5                  10                  15
   aaa gac tac tcc tgc tta gaa cta gta gaa act ctg ata gcg cgt tgc   96
   Lys Asp Tyr Ser Cys Leu Glu Leu Val Glu Thr Leu Ile Ala Arg Cys
               20                  25                  30
   caa gct gca aaa cca tta aat gcc ctt ctg gct aca gac tgg gat ggc  144
   Gln Ala Ala Lys Pro Leu Asn Ala Leu Leu Ala Thr Asp Trp Asp Gly
           35                  40                  45
   ttg cgg cga agc gcc aaa aaa aat gat cgt cat gga aac gcc gga tta  192
   Leu Arg Arg Ser Ala Lys Lys Asn Asp Arg His Gly Asn Ala Gly Leu
       50                  55                  60
   ggt ctt tgc ggc att cca ctc tgt ttt aag gcg aac atc gcg acc ggc  240
   Gly Leu Cys Gly Ile Pro Leu Cys Phe Lys Ala Asn Ile Ala Thr Gly
   65                  70                  75                  80
   gta ttt cct aca agc gct gct act ccg gcg ctg ata aac cac ttg cca  288
   Val Phe Pro Thr Ser Ala Ala Thr Pro Ala Leu Ile Asn His Leu Pro
                   85                  90                  95
   aag ata cca tcc cgc gtc gca gaa aga ctt ttt tca gct gga gca ctg      336
   Lys Ile Pro Ser Arg Val Ala Glu Arg Leu Phe Ser Ala Gly Ala Leu
               100                 105                 110
   ccg ggt gcc tcg gga aac atg cat gag tta tcg ttt gga att acg agc      384
   Pro Gly Ala Ser Gly Asn Met His Glu Leu Ser Phe Gly Ile Thr Ser
           115                 120                 125
   aac aac tat gcc acc ggt gcg gtg cgg aac ccg tgg aat cca agt ctg      432
   Asn Asn Tyr Ala Thr Gly Ala Val Arg Asn Pro Trp Asn Pro Ser Leu
       130                 135                 140
   ata cca ggg ggt tca agc ggt ggt gtg gct gct gcg gtg gca agc cga      480
   Ile Pro Gly Gly Ser Ser Gly Gly Val Ala Ala Ala Val Ala Ser Arg
   145                 150                 155                 160
   ttg atg tta ggc ggc ata ggc acg gat acc ggt gca tct gtt cgc cta      528
   Leu Met Leu Gly Gly Ile Gly Thr Asp Thr Gly Ala Ser Val Arg Leu
                   165                 170                 175
   ccg gca gcc ctg tgt ggc gta gta gga ttt cga ccg acg ctt ggt cga      576
   Pro Ala Ala Leu Cys Gly Val Val Gly Phe Arg Pro Thr Leu Gly Arg
               180                 185                 190
   tat cca aga gat cgg ata ata ccg ttc agc ccc acc cgg gac acc gcc      624
   Tyr Pro Arg Asp Arg Ile Ile Pro Phe Ser Pro Thr Arg Asp Thr Ala
           195                 200                 205
   gga atc ata gcg cag tgc gta gcc gat gtt ata atc ctc gac cag gtg      672
   Gly Ile Ile Ala Gln Cys Val Ala Asp Val Ile Ile Leu Asp Gln Val
       210                 215                 220
   att tcc gga cgg tcg gcg aaa att tca ccc atg ccg ctg aag ggg ctt      720
   Ile Ser Gly Arg Ser Ala Lys Ile Ser Pro Met Pro Leu Lys Gly Leu
   225                 230                 235                 240
   cgg atc ggc ctc ccc act acc tac ttt tac gat gac ctt gat gct gat      768
   Arg Ile Gly Leu Pro Thr Thr Tyr Phe Tyr Asp Asp Leu Asp Ala Asp
                   245                 250                 255
   gtg gcc ttc gca gct gaa acg acg att cgc ttg cta gcc aac aga ggc      816
   Val Ala Phe Ala Ala Glu Thr Thr Ile Arg Leu Leu Ala Asn Arg Gly
               260                 265                 270
   gta acc ttt gtt gaa gcc gac atc ccc cac cta gag gaa ttg aac agt      864
   Val Thr Phe Val Glu Ala Asp Ile Pro His Leu Glu Glu Leu Asn Ser
           275                 280                 285
   ggg gca agt ttg cca att gcg ctt tac gaa ttt cca cac gct cta aaa      912
   Gly Ala Ser Leu Pro Ile Ala Leu Tyr Glu Phe Pro His Ala Leu Lys
       290                 295                 300
   aag tat ctc gac gat ttt gtg gga aca gtt tct ttt tct gac gtt atc      960
   Lys Tyr Leu Asp Asp Phe Val Gly Thr Val Ser Phe Ser Asp Val Ile
   305                 310                 315                 320
   aaa gga att cgt agc ccc gat gta gcg aac att gtc agt gcg caa att     1008
   Lys Gly Ile Arg Ser Pro Asp Val Ala Asn Ile Val Ser Ala Gln Ile
                   325                 330                 335
   gat ggg cat caa att tcc aac gat gaa tat gaa ctg gcg cgt caa tcc     1056
   Asp Gly His Gln Ile Ser Asn Asp Glu Tyr Glu Leu Ala Arg Gln Ser
               340                 345                 350
   ttc agg cca agg ctc cag gcc act tat cgg aat tac ttc aga ctc tat     1104
   Phe Arg Pro Arg Leu Gln Ala Thr Tyr Arg Asn Tyr Phe Arg Leu Tyr
           355                 360                 365
   cag tta gat gca atc ctt ttc cca act gca ccc tta gcg gcc aaa gcc      1152
   Gln Leu Asp Ala Ile Leu Phe Pro Thr Ala Pro Leu Ala Ala Lys Ala
       370                 375                 380
   ata ggt cag gag tcg tca gtc atc cac aat ggc tca atg atg aac act      1200
   Ile Gly Gln Glu Ser Ser Val Ile His Asn Gly Ser Met Met Asn Thr
   385                 390                 395                 400
   ttc aag atc tac gtg cga aat gtg gac cca agc agc aac gca ggc cta      1248
   Phe Lys Ile Tyr Val Arg Asn Val Asp Pro Ser Ser Asn Ala Gly Leu
                   405                 410                 415
   cct ggg ttg agc ctt cct gcc tgc ctt aca cct gat cgc ttg cct gtt      1296
   Pro Gly Leu Ser Leu Pro Ala Cys Leu Thr Pro Asp Arg Leu Pro Val
               420                 425                 430
   gga atg gaa att gat gga tta gcg ggg tca gac cac cgt ctg tta gca      1344
   Gly Met Glu Ile Asp Gly Leu Ala Gly Ser Asp His Arg Leu Leu Ala
           435                 440                 445
   atc ggg gca gca tta gaa aaa gct ata aat ttt tct tcc ttt ccc gat      1392
   Ile Gly Ala Ala Leu Glu Lys Ala Ile Asn Phe Ser Ser Phe Pro Asp
       450                 455                 460
   gct ttt aat tag                                                      1404
   Ala Phe Asn
   465
<210> 34
   <211> 467
   <212> PRT
   <213> Agrobacterium tumefaciens
<400> 34
   Met Val Pro Ile Thr Ser Leu Ala Gln Thr Leu Glu Arg Leu Arg Arg
   1 5 10 15
   Lys Asp Tyr Ser Cys Leu Glu Leu Val Glu Thr Leu Ile Ala Arg Cys
   20 25 30
   Gln Ala Ala Lys Pro Leu Asn Ala Leu Leu Ala Thr Asp Trp Asp Gly
   35 40 45
   Leu Arg Arg Ser Ala Lys Lys Asn Asp Arg His Gly Asn Ala Gly Leu
   50 55 60
   Gly Leu Cys Gly Ile Pro Leu Cys Phe Lys Ala Asn Ile Ala Thr Gly
   65 70 75 80
   Val Phe Pro Thr Ser Ala Ala Thr Pro Ala Leu Ile Asn His Leu Pro
   85 90 95
   Lys Ile Pro Ser Arg Val Ala Glu Arg Leu Phe Ser Ala Gly Ala Leu
   100 105 110
   Pro Gly Ala Ser Gly Asn Met His Glu Leu Ser Phe Gly Ile Thr Ser
   115 120 125
   Asn Asn Tyr Ala Thr Gly Ala Val Arg Asn Pro Trp Asn Pro Ser Leu
   130 135 140
   Ile Pro Gly Gly Ser Ser Gly Gly Val Ala Ala Ala Val Ala Ser Arg
   145 150 155 160
   Leu Met Leu Gly Gly Ile Gly Thr Asp Thr Gly Ala Ser Val Arg Leu
   165 170 175
   Pro Ala Ala Leu Cys Gly Val Val Gly Phe Arg Pro Thr Leu Gly Arg
   180 185 190
   Tyr Pro Arg Asp Arg Ile Ile Pro Phe Ser Pro Thr Arg Asp Thr Ala
   195 200 205
   Gly Ile Ile Ala Gln Cys Val Ala Asp Val Ile Ile Leu Asp Gln Val
   210 215 220
   Ile Ser Gly Arg Ser Ala Lys Ile Ser Pro Met Pro Leu Lys Gly Leu
   225 230 235 240
   Arg Ile Gly Leu Pro Thr Thr Tyr Phe Tyr Asp Asp Leu Asp Ala Asp
   245 250 255
   Val Ala Phe Ala Ala Glu Thr Thr Ile Arg Leu Leu Ala Asn Arg Gly
   260 265 270
   Val Thr Phe Val Glu Ala Asp Ile Pro His Leu Glu Glu Leu Asn Ser
   275 280 285
   Gly Ala Ser Leu Pro Ile Ala Leu Tyr Glu Phe Pro His Ala Leu Lys
   290 295 300
   Lys Tyr Leu Asp Asp Phe Val Gly Thr Val Ser Phe Ser Asp Val Ile
   305 310 315 320
   Lys Gly Ile Arg Ser Pro Asp Val Ala Asn Ile Val Ser Ala Gln Ile
   325 330 335
   Asp Gly His Gln Ile Ser Asn Asp Glu Tyr Glu Leu Ala Arg Gln Ser
   340 345 350
   Phe Arg Pro Arg Leu Gln Ala Thr Tyr Arg Asn Tyr Phe Arg Leu Tyr
   355 360 365
   Gln Leu Asp Ala Ile Leu Phe Pro Thr Ala Pro Leu Ala Ala Lys Ala
   370 375 380
   Ile Gly Gln Glu Ser Ser Val Ile His Asn Gly Ser Met Met Asn Thr
   385 390 395 400
   Phe Lys Ile Tyr Val Arg Asn Val Asp Pro Ser Ser Asn Ala Gly Leu
   405 410 415
   Pro Gly Leu Ser Leu Pro Ala Cys Leu Thr Pro Asp Arg Leu Pro Val
   420 425 430
   Gly Met Glu Ile Asp Gly Leu Ala Gly Ser Asp His Arg Leu Leu Ala
   435 440 445
   Ile Gly Ala Ala Leu Glu Lys Ala Ile Asn Phe Ser Ser Phe Pro Asp
   450 455 460
   Ala Phe Asn
   465
<210> 35
   <211> 1419
   <212> DNA
   <213> Agrobacterium vitis
<220>
   <221> CDS
   <222> (1)..(1416)
   <223> coding for indole acetamide hydrolase
<400> 35
   atg gtg acc cta ggt tca atc aag gaa acc ctg gaa tgt ctc agg ctg    48
   Met Val Thr Leu Gly Ser Ile Lys Glu Thr Leu Glu Cys Leu Arg Leu
    1               5                  10                  15
   aaa aaa tac tcc tgt tcc gaa ctg gct gaa acc ata ata gcc cgt tgc    96
   Lys Lys Tyr Ser Cys Ser Glu Leu Ala Glu Thr Ile Ile Ala Arg Cys
               20                  25                  30
   gaa gcc gcg aaa tct ctc aat gct ctt ctg gcg act gac tgg gat tac   144
   Glu Ala Ala Lys Ser Leu Asn Ala Leu Leu Ala Thr Asp Trp Asp Tyr
           35                  40                  45
   ctg cgg cgt aat gcc aag aaa gta gat gaa gat gga agc gcc ggc gag   192
   Leu Arg Arg Asn Ala Lys Lys Val Asp Glu Asp Gly Ser Ala Gly Glu
       50                  55                  60
   ggt ctt gcc ggc atc ccg ctg tgt tct aaa gcg aac att gca aca ggc   240
   Gly Leu Ala Gly Ile Pro Leu Cys Ser Lys Ala Asn Ile Ala Thr Gly
   65                  70                  75                  80
   ata ttc cca gca agc gcg gcc acg ccg gcg ctt gat gaa cat tta cct   288
   Ile Phe Pro Ala Ser Ala Ala Thr Pro Ala Leu Asp Glu His Leu Pro
                   85                  90                  95
   aca aca cca gcc ggc gtc cgt aaa ccg ctt cta gac gct ggg gca ctg   336
   Thr Thr Pro Ala Gly Val Arg Lys Pro Leu Leu Asp Ala Gly Ala Leu
               100                 105                 110
   ata ggc gct tcg gga aac atg cat gag tta tcg ttt ggc att acc agt   384
   Ile Gly Ala Ser Gly Asn Met His Glu Leu Ser Phe Gly Ile Thr Ser
           115                 120                 125
   aac aac cac gcc act ggt gcg gtg aga aac ccc tgg aat ccc agc tta   432
   Asn Asn His Ala Thr Gly Ala Val Arg Asn Pro Trp Asn Pro Ser Leu
       130                 135                 140
   ata cca gga ggc tcg agc ggc ggc gtg gct gct gct gta gca tca cgg   480
   Ile Pro Gly Gly Ser Ser Gly Gly Val Ala Ala Ala Val Ala Ser Arg
   145                 150                 155                 160
   tta atg ctc ggc gga att ggc acc gac acg ggg gct tcg gtc cgc cta   528
   Leu Met Leu Gly Gly Ile Gly Thr Asp Thr Gly Ala Ser Val Arg Leu
                   165                 170                 175
   cct gca tcc cta tgt ggc gta gtg gga ttc cgc ccg acg atc ggc aga   576
   Pro Ala Ser Leu Cys Gly Val Val Gly Phe Arg Pro Thr Ile Gly Arg
               180                 185                 190
   tat cct gga gac cga att gtg ccg gtt agc ccc acc cgc gat aca gcc   624
   Tyr Pro Gly Asp Arg Ile Val Pro Val Ser Pro Thr Arg Asp Thr Ala
           195                 200                 205
   gga att atc gca cag agc gtt cct gat gtg ata ctc ctt gac caa atc   672
   Gly Ile Ile Ala Gln Ser Val Pro Asp Val Ile Leu Leu Asp Gln Ile
       210                 215                 220
   att tgc ggg aag ctc acg acc cac caa cct gta ccc ctg gag gga tta   720
   Ile Cys Gly Lys Leu Thr Thr His Gln Pro Val Pro Leu Glu Gly Leu
   225                 230                 235                 240
   cgt atc ggc ttg cca acc act tac ttt tac gat gac ctt gat gct gat   768
   Arg Ile Gly Leu Pro Thr Thr Tyr Phe Tyr Asp Asp Leu Asp Ala Asp
                   245                 250                 255
   gtg gcc ttc gca gct gaa aac ctt atc acg ctg ctg gcc agc aag ggt   816
   Val Ala Phe Ala Ala Glu Asn Leu Ile Thr Leu Leu Ala Ser Lys Gly
               260                 265                 270
   gta acc ttt gtt aag gcc gag att cca gat ctg cag cgt ctg aac atc   864
   Val Thr Phe Val Lys Ala Glu Ile Pro Asp Leu Gln Arg Leu Asn Ile
           275                 280                 285
   ggg gtt agc ttt cct att gcc ctg tac gag ttt ccg ttc gcc cta caa      912
   Gly Val Ser Phe Pro Ile Ala Leu Tyr Glu Phe Pro Phe Ala Leu Gln
       290                 295                 300
   aag tat atc gat gac ttt gtg aag gat gtg tct ttt tct gac gtc atc      960
   Lys Tyr Ile Asp Asp Phe Val Lys Asp Val Ser Phe Ser Asp Val Ile
   305                 310                 315                 320
   aaa gga att cgt agc cct gat gta gcc aac att gcc aat gct caa att     1008
   Lys Gly Ile Arg Ser Pro Asp Val Ala Asn Ile Ala Asn Ala Gln Ile
                   325                 330                 335
   gat gga cat caa att tcc aaa gct tca tat gaa ctg gcg cga caa tct     1056
   Asp Gly His Gln Ile Ser Lys Ala Ser Tyr Glu Leu Ala Arg Gln Ser
               340                 345                 350
   ttc aga cca aag ctg caa gcc gcc tac cat gat tac ttc aag ctg cac     1104
   Phe Arg Pro Lys Leu Gln Ala Ala Tyr His Asp Tyr Phe Lys Leu His
           355                 360                 365
   cag cta gac gcg atc ctt ttc ccg aca gct ccc ctg aca gcc aaa ccg     1152
   Gln Leu Asp Ala Ile Leu Phe Pro Thr Ala Pro Leu Thr Ala Lys Pro
       370                 375                 380
   atc ggc caa gat tta tcg gtg atg cac aat ggc gta atg gcc gac acg     1200
   Ile Gly Gln Asp Leu Ser Val Met His Asn Gly Val Met Ala Asp Thr
   385                 390                 395                 400
   ttt aaa atc ttc gtg cga aat gtg gat ccg ggg agc aac gca ggc ctg     1248
   Phe Lys Ile Phe Val Arg Asn Val Asp Pro Gly Ser Asn Ala Gly Leu
                   405                 410                 415
   cca gga tta agc ctt ccc gtt tct ctt act tca aag ggt ttg cct att     1296
   Pro Gly Leu Ser Leu Pro Val Ser Leu Thr Ser Lys Gly Leu Pro Ile
               420                 425                 430
   gga atg gaa atc gat gga tta gcg ggc atg gac gac cgt ttg cta gca     1344
   Gly Met Glu Ile Asp Gly Leu Ala Gly Met Asp Asp Arg Leu Leu Ala
           435                 440                 445
   atc gga gcg gca cta gag gaa gcg ata gct ttt cat aat tta cct gac     1392
   Ile Gly Ala Ala Leu Glu Glu Ala Ile Ala Phe His Asn Leu Pro Asp
       450                 455                 460
   ttc ccg aaa gtc gag aca aac tac tga                                 1419
   Phe Pro Lys Val Glu Thr Asn Tyr
   465                 470
<210> 36
   <211> 472
   <212> PRT
   <213> Agrobacterium vitis
<400> 36
   Met Val Thr Leu Gly Ser Ile Lys Glu Thr Leu Glu Cys Leu Arg Leu
   1 5 10 15
   Lys Lys Tyr Ser Cys Ser Glu Leu Ala Glu Thr Ile Ile Ala Arg Cys
   20 25 30
   Glu Ala Ala Lys Ser Leu Asn Ala Leu Leu Ala Thr Asp Trp Asp Tyr
   35 40 45
   Leu Arg Arg Asn Ala Lys Lys Val Asp Glu Asp Gly Ser Ala Gly Glu
   50 55 60
   Gly Leu Ala Gly Ile Pro Leu Cys Ser Lys Ala Asn Ile Ala Thr Gly
   65 70 75 80
   Ile Phe Pro Ala Ser Ala Ala Thr Pro Ala Leu Asp Glu His Leu Pro
   85 90 95
   Thr Thr Pro Ala Gly Val Arg Lys Pro Leu Leu Asp Ala Gly Ala Leu
   100 105 110
   Ile Gly Ala Ser Gly Asn Met His Glu Leu Ser Phe Gly Ile Thr Ser
   115 120 125
   Asn Asn His Ala Thr Gly Ala Val Arg Asn Pro Trp Asn Pro Ser Leu
   130 135 140
   Ile Pro Gly Gly Ser Ser Gly Gly Val Ala Ala Ala Val Ala Ser Arg
   145 150 155 160
   Leu Met Leu Gly Gly Ile Gly Thr Asp Thr Gly Ala Ser Val Arg Leu
   165 170 175
   Pro Ala Ser Leu Cys Gly Val Val Gly Phe Arg Pro Thr Ile Gly Arg
   180 185 190
   Tyr Pro Gly Asp Arg Ile Val Pro Val Ser Pro Thr Arg Asp Thr Ala
   195 200 205
   Gly Ile Ile Ala Gln Ser Val Pro Asp Val Ile Leu Leu Asp Gln Ile
   210 215 220
   Ile Cys Gly Lys Leu Thr Thr His Gln Pro Val Pro Leu Glu Gly Leu
   225 230 235 240
   Arg Ile Gly Leu Pro Thr Thr Tyr Phe Tyr Asp Asp Leu Asp Ala Asp
   245 250 255
   Val Ala Phe Ala Ala Glu Asn Leu Ile Thr Leu Leu Ala Ser Lys Gly
   260 265 270
   Val Thr Phe Val Lys Ala Glu Ile Pro Asp Leu Gln Arg Leu Asn Ile
   275 280 285
   Gly Val Ser Phe Pro Ile Ala Leu Tyr Glu Phe Pro Phe Ala Leu Gln
   290 295 300
   Lys Tyr Ile Asp Asp Phe Val Lys Asp Val Ser Phe Ser Asp Val Ile
   305 310 315 320
   Lys Gly Ile Arg Ser Pro Asp Val Ala Asn Ile Ala Asn Ala Gln Ile
   325 330 335
   Asp Gly His Gln Ile Ser Lys Ala Ser Tyr Glu Leu Ala Arg Gln Ser
   340 345 350
   Phe Arg Pro Lys Leu Gln Ala Ala Tyr His Asp Tyr Phe Lys Leu His
   355 360 365
   Gln Leu Asp Ala Ile Leu Phe Pro Thr Ala Pro Leu Thr Ala Lys Pro
   370 375 380
   Ile Gly Gln Asp Leu Ser Val Met His Asn Gly Val Met Ala Asp Thr
   385 390 395 400
   Phe Lys Ile Phe Val Arg Asn Val Asp Pro Gly Ser Asn Ala Gly Leu
   405 410 415
   Pro Gly Leu Ser Leu Pro Val Ser Leu Thr Ser Lys Gly Leu Pro Ile
   420 425 430
   Gly Met Glu Ile Asp Gly Leu Ala Gly Met Asp Asp Arg Leu Leu Ala
   435 440 445
   Ile Gly Ala Ala Leu Glu Glu Ala Ile Ala Phe His Asn Leu Pro Asp
   450 455 460
   Phe Pro Lys Val Glu Thr Asn Tyr
   465 470
<210> 37
   <211> 1263
   <212> DNA
   <213> Arabidopsis thaliana
<220>
   <221> CDS
   <222> (1)..(1260)
   <223> coding for 5-methylthioribose kinase
<400> 37
   atg tct ttt gag gag ttt acg ccg tta aac gag aag tct ctt gta gac     48
   Met Ser Phe Glu Glu Phe Thr Pro Leu Asn Glu Lys Ser Leu Val Asp
    1               5                  10                  15
   tac atc aag tca aca cct gct ctc tct tcc aag atc gga gcc gac aag     96
   Tyr Ile Lys Ser Thr Pro Ala Leu Ser Ser Lys Ile Gly Ala Asp Lys
               20                  25                  30
   tcc gat gat gat ttg gtt atc aaa gaa gtt gga gat ggc aat ctc aat    144
   Ser Asp Asp Asp Leu Val Ile Lys Glu Val Gly Asp Gly Asn Leu Asn
           35                  40                  45
   ttc gtt ttc atc gtt gtt gga tcc tct ggt tct ctt gtc atc aaa cag    192
   Phe Val Phe Ile Val Val Gly Ser Ser Gly Ser Leu Val Ile Lys Gln
       50                  55                  60
   gct ctt cca tat att cgc tgt atc ggt gaa tca tgg cca atg acg aaa    240
   Ala Leu Pro Tyr Ile Arg Cys Ile Gly Glu Ser Trp Pro Met Thr Lys
   65                  70                  75                  80
   gaa aga gct tat ttt gaa gca aca act ttg aga aag cat gga aat tta    288
   Glu Arg Ala Tyr Phe Glu Ala Thr Thr Leu Arg Lys His Gly Asn Leu
                   85                  90                  95
   tca cct gat cat gtt cct gaa gtc tac cat ttt gac aga aca atg gcg    336
   Ser Pro Asp His Val Pro Glu Val Tyr His Phe Asp Arg Thr Met Ala
               100                 105                 110
   ttg att gga atg aga tac ctt gag cct cct cat atc att ctc cgc aaa    384
   Leu Ile Gly Met Arg Tyr Leu Glu Pro Pro His Ile Ile Leu Arg Lys
           115                 120                 125
   gga ctc att gct ggg att gag tat cct ttc ctc gca gac cac atg tct    432
   Gly Leu Ile Ala Gly Ile Glu Tyr Pro Phe Leu Ala Asp His Met Ser
       130                 135                 140
   gat tac atg gcg aag act ctc ttc ttc act tct ctc ctc tat cac gat    480
   Asp Tyr Met Ala Lys Thr Leu Phe Phe Thr Ser Leu Leu Tyr His Asp
   145                 150                 155                 160
   acc aca gag cac aga aga gca gta acc gaa ttt tgt ggt aat gtg gag    528
   Thr Thr Glu His Arg Arg Ala Val Thr Glu Phe Cys Gly Asn Val Glu
                   165                 170                 175
   tta tgc cga tta acg gag caa gtt gtg ttt tcg gac cca tat aga gtt    576
   Leu Cys Arg Leu Thr Glu Gln Val Val Phe Ser Asp Pro Tyr Arg Val
               180                 185                 190
   tcc aca ttt aat cgt tgg act tca cct tat ctt gat gat gat gct aag    624
   Ser Thr Phe Asn Arg Trp Thr Ser Pro Tyr Leu Asp Asp Asp Ala Lys
           195                 200                 205
   gct gtg cgc gaa gac agt gcc ttg aag ctc gaa atc gca gag cta aaa     672
   Ala Val Arg Glu Asp Ser Ala Leu Lys Leu Glu Ile Ala Glu Leu Lys
       210                 215                 220
   tcg atg ttc tgt gaa aga gct caa gct tta ata cat ggt gat ctt cat     720
   Ser Met Phe Cys Glu Arg Ala Gln Ala Leu Ile His Gly Asp Leu His
   225                 230                 235                 240
   act ggt tct gtc atg gtt act caa gat tca acg caa gtt ata gat cca     768
   Thr Gly Ser Val Met Val Thr Gln Asp Ser Thr Gln Val Ile Asp Pro
                   245                 250                 255
   gag ttt tcg ttc tat gga ccg atg ggt ttc gat att ggc gct tat ctt     816
   Glu Phe Ser Phe Tyr Gly Pro Met Gly Phe Asp Ile Gly Ala Tyr Leu
               260                 265                 270
   ggt aac ttg ata cta gct ttc ttt gca caa gat gga cac gcc act cag     864
   Gly Asn Leu Ile Leu Ala Phe Phe Ala Gln Asp Gly His Ala Thr Gln
           275                 280                 285
   gaa aat gat cga aaa gaa tac aag cag tgg atc ttg aga acc att gag     912
   Glu Asn Asp Arg Lys Glu Tyr Lys Gln Trp Ile Leu Arg Thr Ile Glu
       290                 295                 300
   caa act tgg aat ttg ttt aac aaa agg ttc att gcg cta tgg gat caa     960
   Gln Thr Trp Asn Leu Phe Asn Lys Arg Phe Ile Ala Leu Trp Asp Gln
   305                 310                 315                 320
   aac aaa gat gga cca ggc gaa gca tac ctt gca gat atc tat aac aat    1008
   Asn Lys Asp Gly Pro Gly Glu Ala Tyr Leu Ala Asp Ile Tyr Asn Asn
                   325                 330                 335
   acc gag gtt ttg aag ttt gtt caa gaa aac tac atg agg aat ttg ttg    1056
   Thr Glu Val Leu Lys Phe Val Gln Glu Asn Tyr Met Arg Asn Leu Leu
               340                 345                 350
   cat gac tca ctc gga ttc ggc gct gca aag atg att agg aga att gtg    1104
   His Asp Ser Leu Gly Phe Gly Ala Ala Lys Met Ile Arg Arg Ile Val
           355                 360                 365
   gga gtg gca cat gtt gag gac ttt gaa tca atc gaa gaa gat aag cga    1152
   Gly Val Ala His Val Glu Asp Phe Glu Ser Ile Glu Glu Asp Lys Arg
       370                 375                 380
   aga gct att tgc gag aga agt gca ctc gag ttt gcg aag atg ctt ctc    1200
   Arg Ala Ile Cys Glu Arg Ser Ala Leu Glu Phe Ala Lys Met Leu Leu
   385                 390                 395                 400
   aag gaa agg aga aag ttt aag agt atc ggt gaa gtt gtt tca gca att    1248
   Lys Glu Arg Arg Lys Phe Lys Ser Ile Gly Glu Val Val Ser Ala Ile
                   405                 410                 415
   caa caa caa agc taa                                                1263
   Gln Gln Gln Ser
               420
<210> 38
   <211> 420
   <212> PRT
   <213> Arabidopsis thaliana
<400> 38
   Met Ser Phe Glu Glu Phe Thr Pro Leu Asn Glu Lys Ser Leu Val Asp
    1               5                  10                  15
   Tyr Ile Lys Ser Thr Pro Ala Leu Ser Ser Lys Ile Gly Ala Asp Lys
               20                  25                  30
   Ser Asp Asp Asp Leu Val Ile Lys Glu Val Gly Asp Gly Asn Leu Asn
           35                  40                  45
   Phe Val Phe Ile Val Val Gly Ser Ser Gly Ser Leu Val Ile Lys Gln
       50                  55                  60
   Ala Leu Pro Tyr Ile Arg Cys Ile Gly Glu Ser Trp Pro Met Thr Lys
   65                  70                  75                  80
   Glu Arg Ala Tyr Phe Glu Ala Thr Thr Leu Arg Lys His Gly Asn Leu
                   85                  90                  95
   Ser Pro Asp His Val Pro Glu Val Tyr His Phe Asp Arg Thr Met Ala
               100                 105                 110
   Leu Ile Gly Met Arg Tyr Leu Glu Pro Pro His Ile Ile Leu Arg Lys
           115                 120                 125
   Gly Leu Ile Ala Gly Ile Glu Tyr Pro Phe Leu Ala Asp His Met Ser
       130                 135                 140
   Asp Tyr Met Ala Lys Thr Leu Phe Phe Thr Ser Leu Leu Tyr His Asp
   145                 150                 155                 160
   Thr Thr Glu His Arg Arg Ala Val Thr Glu Phe Cys Gly Asn Val Glu
                   165                 170                 175
   Leu Cys Arg Leu Thr Glu Gln Val Val Phe Ser Asp Pro Tyr Arg Val
               180                 185                 190
   Ser Thr Phe Asn Arg Trp Thr Ser Pro Tyr Leu Asp Asp Asp Ala Lys
           195                 200                 205
   Ala Val Arg Glu Asp Ser Ala Leu Lys Leu Glu Ile Ala Glu Leu Lys
       210                 215                 220
   Ser Met Phe Cys Glu Arg Ala Gln Ala Leu Ile His Gly Asp Leu His
   225                 230                 235                 240
   Thr Gly Ser Val Met Val Thr Gln Asp Ser Thr Gln Val Ile Asp Pro
                   245                 250                 255
   Glu Phe Ser Phe Tyr Gly Pro Met Gly Phe Asp Ile Gly Ala Tyr Leu
               260                 265                 270
   Gly Asn Leu Ile Leu Ala Phe Phe Ala Gln Asp Gly His Ala Thr Gln
           275                 280                 285
   Glu Asn Asp Arg Lys Glu Tyr Lys Gln Trp Ile Leu Arg Thr Ile Glu
       290                 295                 300
   Gln Thr Trp Asn Leu Phe Asn Lys Arg Phe Ile Ala Leu Trp Asp Gln
   305                 310                 315                 320
   Asn Lys Asp Gly Pro Gly Glu Ala Tyr Leu Ala Asp Ile Tyr Asn Asn
                   325                 330                 335
   Thr Glu Val Leu Lys Phe Val Gln Glu Asn Tyr Met Arg Asn Leu Leu
               340                 345                 350
   His Asp Ser Leu Gly Phe Gly Ala Ala Lys Met Ile Arg Arg Ile Val
           355                 360                 365
   Gly Val Ala His Val Glu Asp Phe Glu Ser Ile Glu Glu Asp Lys Arg
       370                 375                 380
   Arg Ala Ile Cys Glu Arg Ser Ala Leu Glu Phe Ala Lys Met Leu Leu
   385                 390                 395                 400
   Lys Glu Arg Arg Lys Phe Lys Ser Ile Gly Glu Val Val Ser Ala Ile
                   405                 410                 415
   Gln Gln Gln Ser
   420
<210> 39
   <211> 1200
   <212> DNA
   <213> Klebsiella pneumoniae
<220>
   <221> CDS
   <222> (1)..(1197)
   <223> coding for 5-methylthioribose kinase
<400> 39
   atg tcg caa tac cat acc ttc acc gcc cac gat gcc gtg gct tac gcg    48
   Met Ser Gln Tyr His Thr Phe Thr Ala His Asp Ala Val Ala Tyr Ala
    1               5                  10                  15
   caa cag ttc gcc ggc atc gac aac cca tct gag ctg gtc agc gcg cag    96
   Gln Gln Phe Ala Gly Ile Asp Asn Pro Ser Glu Leu Val Ser Ala Gln
               20                  25                  30
   gaa gtg ggc gat ggc aac ctc aat ctg gtg ttt aaa gtg ttc gat cgt   144
   Glu Val Gly Asp Gly Asn Leu Asn Leu Val Phe Lys Val Phe Asp Arg
           35                  40                  45
   cag ggc gtc agc cgg gcg atc gtc aaa cag gcc ctg ccc tac gtg cgc   192
   Gln Gly Val Ser Arg Ala Ile Val Lys Gln Ala Leu Pro Tyr Val Arg
       50                  55                  60
   tgc gtc ggc gaa tcc tgg ccg ctg acc ctc gac cgc gcc cgt ctc gaa   240
   Cys Val Gly Glu Ser Trp Pro Leu Thr Leu Asp Arg Ala Arg Leu Glu
   65                  70                  75                  80
   gcg cag acc ctg gtc gcc cac tat cag cac agc ccg cag cac acg gta   288
   Ala Gln Thr Leu Val Ala His Tyr Gln His Ser Pro Gln His Thr Val
                   85                  90                  95
   aaa atc cat cac ttt gat ccc gag ctg gcg gtg atg gtg atg gaa gat   336
   Lys Ile His His Phe Asp Pro Glu Leu Ala Val Met Val Met Glu Asp
               100                 105                 110
   ctt tcc gac cac cgc atc tgg cgc gga gag ctt atc gct aac gtc tac   384
   Leu Ser Asp His Arg Ile Trp Arg Gly Glu Leu Ile Ala Asn Val Tyr
           115                 120                 125
   tat ccc cag gcg gcc cgc cag ctt ggc gac tat ctg gcg cag gtg ttg   432
   Tyr Pro Gln Ala Ala Arg Gln Leu Gly Asp Tyr Leu Ala Gln Val Leu
       130                 135                 140
   ttc cac acc agc gat ttc tac ctc cat ccc cac gag aaa aag gcg cag   480
   Phe His Thr Ser Asp Phe Tyr Leu His Pro His Glu Lys Lys Ala Gln
   145                 150                 155                 160
   gtg gcg cag ttt att aac ccg gcg atg tgc gag atc acc gag gat ctg   528
   Val Ala Gln Phe Ile Asn Pro Ala Met Cys Glu Ile Thr Glu Asp Leu
                   165                 170                 175
   ttc ttt aac gac ccg tat cag atc cac gag cgc aat aac tac ccg gcg   576
   Phe Phe Asn Asp Pro Tyr Gln Ile His Glu Arg Asn Asn Tyr Pro Ala
               180                 185                 190
   gag ctg gag gcc gat gtc gcc gcc ctg cgc gac gac gcc cag ctt aag   624
   Glu Leu Glu Ala Asp Val Ala Ala Leu Arg Asp Asp Ala Gln Leu Lys
           195                 200                 205
   ctg gcg gtg gcg gcg ctg aag cac cgt ttc ttt gcc cat gcg gaa gcg    672
   Leu Ala Val Ala Ala Leu Lys His Arg Phe Phe Ala His Ala Glu Ala
       210                 215                 220
   ctg ctg cac ggc gat atc cac agc ggg tcg atc ttc gtt gcc gaa ggt    720
   Leu Leu His Gly Asp Ile His Ser Gly Ser Ile Phe Val Ala Glu Gly
   225                 230                 235                 240
   agc ctg aag gcc atc gac gcc gag ttc ggc tac ttc ggc ccc atc ggc    768
   Ser Leu Lys Ala Ile Asp Ala Glu Phe Gly Tyr Phe Gly Pro Ile Gly
                   245                 250                 255
   ttc gat atc ggc acc gcc atc ggc aac ctg ctg ctg aac tac tgc ggc    816
   Phe Asp Ile Gly Thr Ala Ile Gly Asn Leu Leu Leu Asn Tyr Cys Gly
               260                 265                 270
   ctg ccg ggc cag ctc ggc att cgc gat gcc gcc gcc gcg cgc gag cag    864
   Leu Pro Gly Gln Leu Gly Ile Arg Asp Ala Ala Ala Ala Arg Glu Gln
           275                 280                 285
   cgg ctg aac gac atc cac cag ctg tgg acc acc ttc gcc gag cgc ttc    912
   Arg Leu Asn Asp Ile His Gln Leu Trp Thr Thr Phe Ala Glu Arg Phe
       290                 295                 300
   cag gcg ctg gcg gcg gag aaa acc cgc gac gcg gcg ctg gct tac ccc    960
   Gln Ala Leu Ala Ala Glu Lys Thr Arg Asp Ala Ala Leu Ala Tyr Pro
   305                 310                 315                 320
   ggc tac gcc tcc gcc ttt ctg aag aaa gtc tgg gcg gac gcg gtc ggc    1008
   Gly Tyr Ala Ser Ala Phe Leu Lys Lys Val Trp Ala Asp Ala Val Gly
                   325                 330                 335
   ttc tgc ggc agc gaa ctg atc cgc cgc agc gtc gga ctg tcg cac gtc    1056
   Phe Cys Gly Ser Glu Leu Ile Arg Arg Ser Val Gly Leu Ser His Val
               340                 345                 350
   gcg gat atc gac act atc cag gac gac gcc atg cgt cat gag tgc ctg    1104
   Ala Asp Ile Asp Thr Ile Gln Asp Asp Ala Met Arg His Glu Cys Leu
           355                 360                 365
   cgc cac gcc att acc ctg ggc aga gcg ctg atc gtg ctg gcc gag cgt    1152
   Arg His Ala Ile Thr Leu Gly Arg Ala Leu Ile Val Leu Ala Glu Arg
       370                 375                 380
   atc gac agc gtc gac gag ctg ctg gcg cgg gta cgc cag tac agc tga    1200
   Ile Asp Ser Val Asp Glu Leu Leu Ala Arg Val Arg Gln Tyr Ser
   385                 390                 395
<210> 40
   <211> 399
   <212> PRT
   <213> Klebsiella pneumoniae
<400> 40
   Met Ser Gln Tyr His Thr Phe Thr Ala His Asp Ala Val Ala Tyr Ala
   1 5 10 15
   Gln Gln Phe Ala Gly Ile Asp Asn Pro Ser Glu Leu Val Ser Ala Gln
   20 25 30
   Glu Val Gly Asp Gly Asn Leu Asn Leu Val Phe Lys Val Phe Asp Arg
   35 40 45
   Gln Gly Val Ser Arg Ala Ile Val Lys Gln Ala Leu Pro Tyr Val Arg
   50 55 60
   Cys Val Gly Glu Ser Trp Pro Leu Thr Leu Asp Arg Ala Arg Leu Glu
   65 70 75 80
   Ala Gln Thr Leu Val Ala His Tyr Gln His Ser Pro Gln His Thr Val
   85 90 95
   Lys Ile His His Phe Asp Pro Glu Leu Ala Val Met Val Met Glu Asp
   100 105 110
   Leu Ser Asp His Arg Ile Trp Arg Gly Glu Leu Ile Ala Asn Val Tyr
   115 120 125
   Tyr Pro Gln Ala Ala Arg Gln Leu Gly Asp Tyr Leu Ala Gln Val Leu
   130 135 140
   Phe His Thr Ser Asp Phe Tyr Leu His Pro His Glu Lys Lys Ala Gln
   145 150 155 160
   Val Ala Gln Phe Ile Asn Pro Ala Met Cys Glu Ile Thr Glu Asp Leu
   165 170 175
   Phe Phe Asn Asp Pro Tyr Gln Ile His Glu Arg Asn Asn Tyr Pro Ala
   180 185 190
   Glu Leu Glu Ala Asp Val Ala Ala Leu Arg Asp Asp Ala Gln Leu Lys
   195 200 205
   Leu Ala Val Ala Ala Leu Lys His Arg Phe Phe Ala His Ala Glu Ala
   210 215 220
   Leu Leu His Gly Asp Ile His Ser Gly Ser Ile Phe Val Ala Glu Gly
   225 230 235 240
   Ser Leu Lys Ala Ile Asp Ala Glu Phe Gly Tyr Phe Gly Pro Ile Gly
   245 250 255
   Phe Asp Ile Gly Thr Ala Ile Gly Asn Leu Leu Leu Asn Tyr Cys Gly
   260 265 270
   Leu Pro Gly Gln Leu Gly Ile Arg Asp Ala Ala Ala Ala Arg Glu Gln
   275 280 285
   Arg Leu Asn Asp Ile His Gln Leu Trp Thr Thr Phe Ala Glu Arg Phe
   290 295 300
   Gln Ala Leu Ala Ala Glu Lys Thr Arg Asp Ala Ala Leu Ala Tyr Pro
   305 310 315 320
   Gly Tyr Ala Ser Ala Phe Leu Lys Lys Val Trp Ala Asp Ala Val Gly
   325 330 335
   Phe Cys Gly Ser Glu Leu Ile Arg Arg Ser Val Gly Leu Ser His Val
   340 345 350
   Ala Asp Ile Asp Thr Ile Gln Asp Asp Ala Met Arg His Glu Cys Leu
   355 360 365
   Arg His Ala Ile Thr Leu Gly Arg Ala Leu Ile Val Leu Ala Glu Arg
   370 375 380
   Ile Asp Ser Val Asp Glu Leu Leu Ala Arg Val Arg Gln Tyr Ser
   385 390 395
<210> 41
   <211> 1140
   <212> DNA
   <213> Arabidopsis thaliana
<220>
   <221> CDS
   <222> (1)..(1137)
   <223> coding for alcohol dehydrogenase
<400> 41
   atg tct acc acc gga cag att att cga tgc aaa gct gct gtg gca tgg 48
   Met Ser Thr Thr Gly Gln Ile Ile Arg Cys Lys Ala Ala Val Ala Trp
   1 5 10 15
   gaa gcc gga aag cca ctg gtg atc gag gaa gtg gag gtt gct cca ccg 96
   Glu Ala Gly Lys Pro Leu Val Ile Glu Glu Val Glu Val Ala Pro Pro
   20 25 30
   cag aaa cac gaa gtt cgt atc aag att ctc ttc act tct ctc tgt cac 144
   Gln Lys His Glu Val Arg Ile Lys Ile Leu Phe Thr Ser Leu Cys His
   35 40 45
   acc gat gtt tac ttc tgg gaa gct aag gga caa aca ccg ttg ttt cca 192
   Thr Asp Val Tyr Phe Trp Glu Ala Lys Gly Gln Thr Pro Leu Phe Pro
   50 55 60
   cgt atc ttc ggc cat gaa gct gga ggg att gtt gag agt gtt gga gaa 240
   Arg Ile Phe Gly His Glu Ala Gly Gly Ile Val Glu Ser Val Gly Glu
   65 70 75 80
   gga gtg act gat ctt cag cca gga gat cat gtg ttg ccg atc ttt acc 288
   Gly Val Thr Asp Leu Gln Pro Gly Asp His Val Leu Pro Ile Phe Thr
   85 90 95
   gga gaa tgt gga gat tgt cgt cat tgc cag tcg gag gaa tca aac atg 336
   Gly Glu Cys Gly Asp Cys Arg His Cys Gln Ser Glu Glu Ser Asn Met
   100 105 110
   tgt gat ctt ctc agg atc aac aca gag cga gga ggt atg att cac gat 384
   Cys Asp Leu Leu Arg Ile Asn Thr Glu Arg Gly Gly Met Ile His Asp
   115 120 125
   ggt gaa tct aga ttc tcc att aat ggc aaa cca atc tac cat ttc ctt 432
   Gly Glu Ser Arg Phe Ser Ile Asn Gly Lys Pro Ile Tyr His Phe Leu
   130 135 140
   ggg acg tcc acg ttc agt gag tac act gtg gtt cac tct ggt cag gtc 480
   Gly Thr Ser Thr Phe Ser Glu Tyr Thr Val Val His Ser Gly Gln Val
   145 150 155 160
   gct aag atc aat ccg gat gct cct ctt gac aag gtc tgt att gtc agt 528
   Ala Lys Ile Asn Pro Asp Ala Pro Leu Asp Lys Val Cys Ile Val Ser
   165 170 175
   tgt ggt ttg tct act ggg tta gga gca act ttg aat gtg gct aaa ccc 576
   Cys Gly Leu Ser Thr Gly Leu Gly Ala Thr Leu Asn Val Ala Lys Pro
   180 185 190
   aag aaa ggt caa agt gtt gcc att ttt ggt ctt ggt gct gtt ggt tta 624
   Lys Lys Gly Gln Ser Val Ala Ile Phe Gly Leu Gly Ala Val Gly Leu
   195 200 205
   ggc gct gca gaa ggt gct aga atc gct ggt gct tct agg atc atc ggt 672
   Gly Ala Ala Glu Gly Ala Arg Ile Ala Gly Ala Ser Arg Ile Ile Gly
   210 215 220
   gtt gat ttt aac tct aaa aga ttc gac caa gct aag gaa ttc ggt gtg 720
   Val Asp Phe Asn Ser Lys Arg Phe Asp Gln Ala Lys Glu Phe Gly Val
   225 230 235 240
   acc gag tgt gtg aac ccg aaa gac cat gac aag cca att caa cag gtg 768
   Thr Glu Cys Val Asn Pro Lys Asp His Asp Lys Pro Ile Gln Gln Val
   245 250 255
   atc gct gag atg acg gat ggt ggg gtg gac agg agt gtg gaa tgc acc 816
   Ile Ala Glu Met Thr Asp Gly Gly Val Asp Arg Ser Val Glu Cys Thr
   260 265 270
   gga agc gtt cag gcc atg att caa gca ttt gaa tgt gtc cac gat ggc     864
   Gly Ser Val Gln Ala Met Ile Gln Ala Phe Glu Cys Val His Asp Gly
           275                 280                 285
   tgg ggt gtt gca gtg ctg gtg ggt gtg cca agc aaa gac gat gcc ttc     912
   Trp Gly Val Ala Val Leu Val Gly Val Pro Ser Lys Asp Asp Ala Phe
       290                 295                 300
   aag act cat ccg atg aat ttc ttg aat gag agg act ctt aag ggt act     960
   Lys Thr His Pro Met Asn Phe Leu Asn Glu Arg Thr Leu Lys Gly Thr
   305                 310                 315                 320
   ttc ttc ggg aac tac aaa ccc aaa act gac att ccc ggg gtt gtg gaa    1008
   Phe Phe Gly Asn Tyr Lys Pro Lys Thr Asp Ile Pro Gly Val Val Glu
                   325                 330                 335
   aag tac atg aac aag gag ctg gag ctt gag aaa ttc atc act cac aca    1056
   Lys Tyr Met Asn Lys Glu Leu Glu Leu Glu Lys Phe Ile Thr His Thr
               340                 345                 350
   gtg cca ttc tcg gaa atc aac aag gcc ttt gat tac atg ctg aag gga    1104
   Val Pro Phe Ser Glu Ile Asn Lys Ala Phe Asp Tyr Met Leu Lys Gly
           355                 360                 365
   gag agt att cgt tgc atc atc acc atg ggt gct tga                    1140
   Glu Ser Ile Arg Cys Ile Ile Thr Met Gly Ala
       370                 375
<210> 42
   <211> 379
   <212> PRT
   <213> Arabidopsis thaliana
<400> 42
   Met Ser Thr Thr Gly Gln Ile Ile Arg Cys Lys Ala Ala Val Ala Trp
    1               5                  10                  15
   Glu Ala Gly Lys Pro Leu Val Ile Glu Glu Val Glu Val Ala Pro Pro
               20                  25                  30
   Gln Lys His Glu Val Arg Ile Lys Ile Leu Phe Thr Ser Leu Cys His
           35                  40                  45
   Thr Asp Val Tyr Phe Trp Glu Ala Lys Gly Gln Thr Pro Leu Phe Pro
       50                  55                  60
   Arg Ile Phe Gly His Glu Ala Gly Gly Ile Val Glu Ser Val Gly Glu
   65                  70                  75                  80
   Gly Val Thr Asp Leu Gln Pro Gly Asp His Val Leu Pro Ile Phe Thr
                   85                  90                  95
   Gly Glu Cys Gly Asp Cys Arg His Cys Gln Ser Glu Glu Ser Asn Met
               100                 105                 110
   Cys Asp Leu Leu Arg Ile Asn Thr Glu Arg Gly Gly Met Ile His Asp
           115                 120                 125
   Gly Glu Ser Arg Phe Ser Ile Asn Gly Lys Pro Ile Tyr His Phe Leu
       130                 135                 140
   Gly Thr Ser Thr Phe Ser Glu Tyr Thr Val Val His Ser Gly Gln Val
   145                 150                 155                 160
   Ala Lys Ile Asn Pro Asp Ala Pro Leu Asp Lys Val Cys Ile Val Ser
                   165                 170                 175
   Cys Gly Leu Ser Thr Gly Leu Gly Ala Thr Leu Asn Val Ala Lys Pro
               180                 185                 190
   Lys Lys Gly Gln Ser Val Ala Ile Phe Gly Leu Gly Ala Val Gly Leu
   195 200 205
   Gly Ala Ala Glu Gly Ala Arg Ile Ala Gly Ala Ser Arg Ile Ile Gly
   210 215 220
   Val Asp Phe Asn Ser Lys Arg Phe Asp Gln Ala Lys Glu Phe Gly Val
   225 230 235 240
   Thr Glu Cys Val Asn Pro Lys Asp His Asp Lys Pro Ile Gln Gln Val
   245 250 255
   Ile Ala Glu Met Thr Asp Gly Gly Val Asp Arg Ser Val Glu Cys Thr
   260 265 270
   Gly Ser Val Gln Ala Met Ile Gln Ala Phe Glu Cys Val His Asp Gly
   275 280 285
   Trp Gly Val Ala Val Leu Val Gly Val Pro Ser Lys Asp Asp Ala Phe
   290 295 300
   Lys Thr His Pro Met Asn Phe Leu Asn Glu Arg Thr Leu Lys Gly Thr
   305 310 315 320
   Phe Phe Gly Asn Tyr Lys Pro Lys Thr Asp Ile Pro Gly Val Val Glu
   325 330 335
   Lys Tyr Met Asn Lys Glu Leu Glu Leu Glu Lys Phe Ile Thr His Thr
   340 345 350
   Val Pro Phe Ser Glu Ile Asn Lys Ala Phe Asp Tyr Met Leu Lys Gly
   355 360 365
   Glu Ser Ile Arg Cys Ile Ile Thr Met Gly Ala
   370 375
<210> 43
   <211> 1140
   <212> DNA
   <213> Hordeum vulgare
<220>
   <221> CDS
   <222> (1)..(1137)
   <223> coding for alcohol dehydrogenase
<400> 43
   atg gcg acg gcc ggc aag gtg atc aag tgc aaa gcc gcg gtg gcg tgg    48
   Met Ala Thr Ala Gly Lys Val Ile Lys Cys Lys Ala Ala Val Ala Trp
   1               5                   10                  15
   gag gcc ggg aag ccg ctg acc atg gag gag gtg gag gtg gcg ccg ccg    96
   Glu Ala Gly Lys Pro Leu Thr Met Glu Glu Val Glu Val Ala Pro Pro
               20                  25                  30
   cag gcc atg gag gtg cgc gtc aag atc ctc ttc acc tcc ctc tgc cac   144
   Gln Ala Met Glu Val Arg Val Lys Ile Leu Phe Thr Ser Leu Cys His
           35                  40                  45
   acc gac gtc tac ttc tgg gag gcc aag ggg cag acc ccc atg ttc cct   192
   Thr Asp Val Tyr Phe Trp Glu Ala Lys Gly Gln Thr Pro Met Phe Pro
       50                  55                  60
   cgg atc ttc ggc cat gaa gct gga ggc ata gtg gag agt gtt gga gag   240
   Arg Ile Phe Gly His Glu Ala Gly Gly Ile Val Glu Ser Val Gly Glu
   65                  70                  75                  80
   ggc gtg act gat gtt gcc cct ggt gac cac gtc ctc cct gtg ttc act     288
   Gly Val Thr Asp Val Ala Pro Gly Asp His Val Leu Pro Val Phe Thr
                   85                  90                  95
   ggg gag tgt aag gaa tgc cca cat tgc aag tct gcg gag agc aac atg     336
   Gly Glu Cys Lys Glu Cys Pro His Cys Lys Ser Ala Glu Ser Asn Met
               100                 105                 110
   tgt gat ctg ctc agg atc aac acc gac aga ggt gtg atg atc ggg gat     384
   Cys Asp Leu Leu Arg Ile Asn Thr Asp Arg Gly Val Met Ile Gly Asp
           115                 120                 125
   ggc aag tcg cgc ttc tct att ggc ggc aag ccg att tac cat ttc gta     432
   Gly Lys Ser Arg Phe Ser Ile Gly Gly Lys Pro Ile Tyr His Phe Val
       130                 135                 140
   ggg act tcc acc ttc agt gag tac act gtc atg cat gtc ggt tgt gtt     480
   Gly Thr Ser Thr Phe Ser Glu Tyr Thr Val Met His Val Gly Cys Val
   145                 150                 155                 160
   gcc aag atc aac cct gag gct ccc ctt gat aaa gtc tgt gtt ctt agc     528
   Ala Lys Ile Asn Pro Glu Ala Pro Leu Asp Lys Val Cys Val Leu Ser
                   165                 170                 175
   tgt ggt att tgc act ggt ctt ggc gcg tca att aat gtt gca aaa cca     576
   Cys Gly Ile Cys Thr Gly Leu Gly Ala Ser Ile Asn Val Ala Lys Pro
               180                 185                 190
   cca aag ggt tcc aca gtg gcg ata ttt ggg cta gga gct gtt ggc ctt     624
   Pro Lys Gly Ser Thr Val Ala Ile Phe Gly Leu Gly Ala Val Gly Leu
           195                 200                 205
   gct gct gca gaa ggt gca agg att gca ggt gca tca agg atc att ggt     672
   Ala Ala Ala Glu Gly Ala Arg Ile Ala Gly Ala Ser Arg Ile Ile Gly
       210                 215                 220
   gtt gac ctg aac gcc agc aga ttt gaa gag gct agg aag ttt ggc tgc     720
   Val Asp Leu Asn Ala Ser Arg Phe Glu Glu Ala Arg Lys Phe Gly Cys
   225                 230                 235                 240
   acg gaa ttt gtg aac ccg aaa gat cac acc aag cca gtt cag cag gtg     768
   Thr Glu Phe Val Asn Pro Lys Asp His Thr Lys Pro Val Gln Gln Val
                   245                 250                 255
   ctc gct gac atg aca aat ggc gga gtt gac cgc agt gtt gag tgc act     816
   Leu Ala Asp Met Thr Asn Gly Gly Val Asp Arg Ser Val Glu Cys Thr
               260                 265                 270
   ggc aac gtc aat gct atg ata caa gca ttt gaa tgt gtt cat gat ggc     864
   Gly Asn Val Asn Ala Met Ile Gln Ala Phe Glu Cys Val His Asp Gly
           275                 280                 285
   tgg ggt gta gct gtg ctg gtg ggt gtg cca cac aag gac gct gaa ttc     912
   Trp Gly Val Ala Val Leu Val Gly Val Pro His Lys Asp Ala Glu Phe
       290                 295                 300
   aag acc cac ccg atg aac ttc ctg aat gag agg acc ctg aag ggc acc     960
   Lys Thr His Pro Met Asn Phe Leu Asn Glu Arg Thr Leu Lys Gly Thr
   305                 310                 315                 320
   ttc ttc ggt aac ttc aag ccg cgc act gac ctg ccc aat gtc gtg gag    1008
   Phe Phe Gly Asn Phe Lys Pro Arg Thr Asp Leu Pro Asn Val Val Glu
                   325                 330                 335
   atg tac atg aag aag gag ctg gag gtg gag aag ttc atc aca cac agc    1056
   Met Tyr Met Lys Lys Glu Leu Glu Val Glu Lys Phe Ile Thr His Ser
               340                 345                 350
   gtg ccg ttc tcg gag ata aac aag gcc ttc gac ctt atg gcg aag ggg    1104
   Val Pro Phe Ser Glu Ile Asn Lys Ala Phe Asp Leu Met Ala Lys Gly
           355                 360                 365
   gag ggc atc cgt tgc atc atc cgc atg gac aac tag                    1140
   Glu Gly Ile Arg Cys Ile Ile Arg Met Asp Asn
       370                 375
<210> 44
   <211> 379
   <212> PRT
   <213> Hordeum vulgare
<400> 44
   Met Ala Thr Ala Gly Lys Val Ile Lys Cys Lys Ala Ala Val Ala Trp
   1               5                   10                  15
   Glu Ala Gly Lys Pro Leu Thr Met Glu Glu Val Glu Val Ala Pro Pro
               20                  25                  30
   Gln Ala Met Glu Val Arg Val Lys Ile Leu Phe Thr Ser Leu Cys His
           35                  40                  45
   Thr Asp Val Tyr Phe Trp Glu Ala Lys Gly Gln Thr Pro Met Phe Pro
       50                  55                  60
   Arg Ile Phe Gly His Glu Ala Gly Gly Ile Val Glu Ser Val Gly Glu
   65                  70                  75                  80
   Gly Val Thr Asp Val Ala Pro Gly Asp His Val Leu Pro Val Phe Thr
                   85                  90                  95
   Gly Glu Cys Lys Glu Cys Pro His Cys Lys Ser Ala Glu Ser Asn Met
               100                 105                 110
   Cys Asp Leu Leu Arg Ile Asn Thr Asp Arg Gly Val Met Ile Gly Asp
           115                 120                 125
   Gly Lys Ser Arg Phe Ser Ile Gly Gly Lys Pro Ile Tyr His Phe Val
       130                 135                 140
   Gly Thr Ser Thr Phe Ser Glu Tyr Thr Val Met His Val Gly Cys Val
   145                 150                 155                 160
   Ala Lys Ile Asn Pro Glu Ala Pro Leu Asp Lys Val Cys Val Leu Ser
                   165                 170                 175
   Cys Gly Ile Cys Thr Gly Leu Gly Ala Ser Ile Asn Val Ala Lys Pro
               180                 185                 190
   Pro Lys Gly Ser Thr Val Ala Ile Phe Gly Leu Gly Ala Val Gly Leu
           195                 200                 205
   Ala Ala Ala Glu Gly Ala Arg Ile Ala Gly Ala Ser Arg Ile Ile Gly
       210                 215                 220
   Val Asp Leu Asn Ala Ser Arg Phe Glu Glu Ala Arg Lys Phe Gly Cys
   225                 230                 235                 240
   Thr Glu Phe Val Asn Pro Lys Asp His Thr Lys Pro Val Gln Gln Val
                   245                 250                 255
   Leu Ala Asp Met Thr Asn Gly Gly Val Asp Arg Ser Val Glu Cys Thr
               260                 265                 270
   Gly Asn Val Asn Ala Met Ile Gln Ala Phe Glu Cys Val His Asp Gly
           275                 280                 285
   Trp Gly Val Ala Val Leu Val Gly Val Pro His Lys Asp Ala Glu Phe
       290                 295                 300
   Lys Thr His Pro Met Asn Phe Leu Asn Glu Arg Thr Leu Lys Gly Thr
   305                 310                 315                 320
   Phe Phe Gly Asn Phe Lys Pro Arg Thr Asp Leu Pro Asn Val Val Glu
                   325                 330                 335
   Met Tyr Met Lys Lys Glu Leu Glu Val Glu Lys Phe Ile Thr His Ser
               340                 345                 350
   Val Pro Phe Ser Glu Ile Asn Lys Ala Phe Asp Leu Met Ala Lys Gly
           355                 360                 365
   Glu Gly Ile Arg Cys Ile Ile Arg Met Asp Asn
       370                 375
<210> 45
   <211> 1140
   <212> DNA
   <213> Oryza sativa
<220>
   <221> CDS
   <222> (1)..(1137)
   <223> coding for alcohol dehydrogenase
<400> 45
   atg gcg acc gca ggg aag gtg atc aag tgc aaa gcg gcg gtg gca tgg      48
   Met Ala Thr Ala Gly Lys Val Ile Lys Cys Lys Ala Ala Val Ala Trp
     1               5                  10                  15
   gag gcc gcg aag ccg ctg gtg atc gag gag gtg gag gtg gcg ccg ccg      96
   Glu Ala Ala Lys Pro Leu Val Ile Glu Glu Val Glu Val Ala Pro Pro
                    20                  25                  30
   cag gcc atg gag gtg cgc gtc aag atc ctc ttc acc tcg ctc tgc cac     144
   Gln Ala Met Glu Val Arg Val Lys Ile Leu Phe Thr Ser Leu Cys His
            35                  40                  45
   acc gac gtc tac ttc tgg gag gcc aag gga cag act ccc gtg ttc cct     192
   Thr Asp Val Tyr Phe Trp Glu Ala Lys Gly Gln Thr Pro Val Phe Pro
        50                  55                  60
   cgg atc ttc ggc cat gaa gct gga ggt att gtg gag agt gtt gga gag     240
   Arg Ile Phe Gly His Glu Ala Gly Gly Ile Val Glu Ser Val Gly Glu
    65                  70                  75                  80
   ggt gtg act gat ctt gcc cct ggt gac cat gtt ctc cct gtg ttc act     288
   Gly Val Thr Asp Leu Ala Pro Gly Asp His Val Leu Pro Val Phe Thr
                    85                  90                  95
   ggg gag tgc aag gag tgt gcc cac tgc aag tca gca gag agc aac atg     336
   Gly Glu Cys Lys Glu Cys Ala His Cys Lys Ser Ala Glu Ser Asn Met
               100                 105                 110
   tgt gat ctg ctc agg atc aac act gac agg ggt gtg atg att ggt gat     384
   Cys Asp Leu Leu Arg Ile Asn Thr Asp Arg Gly Val Met Ile Gly Asp
           115                 120                 125
   ggc aaa tca cgc ttt tcc atc aac ggg aag ccc att tac cat ttc gtc     432
   Gly Lys Ser Arg Phe Ser Ile Asn Gly Lys Pro Ile Tyr His Phe Val
       130                 135                 140
   ggg act tcg acc ttc agc gag tac act gtc atg cat gtt ggt tgc gtt     480
   Gly Thr Ser Thr Phe Ser Glu Tyr Thr Val Met His Val Gly Cys Val
   145                 150                 155                 160
   gcg aag atc aac ccg gca gct cca ctt gat aaa gtt tgc gtt ctt agc    528
   Ala Lys Ile Asn Pro Ala Ala Pro Leu Asp Lys Val Cys Val Leu Ser
                   165                 170                 175
   tgt ggt att tct act ggt ctt ggt gct aca atc aat gtg gca aag cca    576
   Cys Gly Ile Ser Thr Gly Leu Gly Ala Thr Ile Asn Val Ala Lys Pro
               180                 185                 190
   cca aag ggt tcg acg gtg gcg ata ttt ggt cta gga gct gta ggc ctt    624
   Pro Lys Gly Ser Thr Val Ala Ile Phe Gly Leu Gly Ala Val Gly Leu
           195                 200                 205
   gct gcc gca gaa ggt gca agg att gca gga gcg tca agg atc att ggc    672
   Ala Ala Ala Glu Gly Ala Arg Ile Ala Gly Ala Ser Arg Ile Ile Gly
       210                 215                 220
   att gac ctg aac gcc aac aga ttt gaa gaa gct agg aaa ttt ggt tgc    720
   Ile Asp Leu Asn Ala Asn Arg Phe Glu Glu Ala Arg Lys Phe Gly Cys
   225                 230                 235                 240
   act gaa ttt gtg aac cca aag gac cat gac aag cca gtt cag cag gta    768
   Thr Glu Phe Val Asn Pro Lys Asp His Asp Lys Pro Val Gln Gln Val
                   245                 250                 255
   ctt gct gag atg acc aat ggc gga gtt gac cgc agc gtt gaa tgc act    816
   Leu Ala Glu Met Thr Asn Gly Gly Val Asp Arg Ser Val Glu Cys Thr
               260                 265                 270
   ggc aac atc aac gcc atg atc caa gca ttt gaa tgt gtt cat gat ggc    864
   Gly Asn Ile Asn Ala Met Ile Gln Ala Phe Glu Cys Val His Asp Gly
           275                 280                 285
   tgg ggt gtt gct gtt ttg gtc ggc gtg cca cac aag gac gcc gag ttc    912
   Trp Gly Val Ala Val Leu Val Gly Val Pro His Lys Asp Ala Glu Phe
       290                 295                 300
   aag acc cac ccg atg aac ttc ctg aac gag agg act ctc aag gga acc    960
   Lys Thr His Pro Met Asn Phe Leu Asn Glu Arg Thr Leu Lys Gly Thr
   305                 310                 315                 320
   ttc ttc ggc aac tac aag cca cgc acc gat ctg ccc aac gtc gtc gag   1008
   Phe Phe Gly Asn Tyr Lys Pro Arg Thr Asp Leu Pro Asn Val Val Glu
                   325                 330                 335
   ctc tac atg aag aag gag ctg gag gtg gag aag ttc atc aca cac agc   1056
   Leu Tyr Met Lys Lys Glu Leu Glu Val Glu Lys Phe Ile Thr His Ser
               340                 345                 350
   gtg ccg ttc tcg gag atc aac acg gcg ttc gac ctg atg cac aag ggc   1104
   Val Pro Phe Ser Glu Ile Asn Thr Ala Phe Asp Leu Met His Lys Gly
           355                 360                 365
   gag ggc atc cgc tgc atc atc cgc atg gag aac tga                   1140
   Glu Gly Ile Arg Cys Ile Ile Arg Met Glu Asn
       370                 375
<210> 46
   <211> 379
   <212> PRT
   <213> Oryza sativa
<400> 46
   Met Ala Thr Ala Gly Lys Val Ile Lys Cys Lys Ala Ala Val Ala Trp
   1               5                   10                  15
   Glu Ala Ala Lys Pro Leu Val Ile Glu Glu Val Glu Val Ala Pro Pro
               20                  25                  30
   Gln Ala Met Glu Val Arg Val Lys Ile Leu Phe Thr Ser Leu Cys His
   35 40 45
   Thr Asp Val Tyr Phe Trp Glu Ala Lys Gly Gln Thr Pro Val Phe Pro
   50 55 60
   Arg Ile Phe Gly His Glu Ala Gly Gly Ile Val Glu Ser Val Gly Glu
   65 70 75 80
   Gly Val Thr Asp Leu Ala Pro Gly Asp His Val Leu Pro Val Phe Thr
   85 90 95
   Gly Glu Cys Lys Glu Cys Ala His Cys Lys Ser Ala Glu Ser Asn Met
   100 105 110
   Cys Asp Leu Leu Arg Ile Asn Thr Asp Arg Gly Val Met Ile Gly Asp
   115 120 125
   Gly Lys Ser Arg Phe Ser Ile Asn Gly Lys Pro Ile Tyr His Phe Val
   130 135 140
   Gly Thr Ser Thr Phe Ser Glu Tyr Thr Val Met His Val Gly Cys Val
   145 150 155 160
   Ala Lys Ile Asn Pro Ala Ala Pro Leu Asp Lys Val Cys Val Leu Ser
   165 170 175
   Cys Gly Ile Ser Thr Gly Leu Gly Ala Thr Ile Asn Val Ala Lys Pro
   180 185 190
   Pro Lys Gly Ser Thr Val Ala Ile Phe Gly Leu Gly Ala Val Gly Leu
   195 200 205
   Ala Ala Ala Glu Gly Ala Arg Ile Ala Gly Ala Ser Arg Ile Ile Gly
   210 215 220
   Ile Asp Leu Asn Ala Asn Arg Phe Glu Glu Ala Arg Lys Phe Gly Cys
   225 230 235 240
   Thr Glu Phe Val Asn Pro Lys Asp His Asp Lys Pro Val Gln Gln Val
   245 250 255
   Leu Ala Glu Met Thr Asn Gly Gly Val Asp Arg Ser Val Glu Cys Thr
   260 265 270
   Gly Asn Ile Asn Ala Met Ile Gln Ala Phe Glu Cys Val His Asp Gly
   275 280 285
   Trp Gly Val Ala Val Leu Val Gly Val Pro His Lys Asp Ala Glu Phe
   290 295 300
   Lys Thr His Pro Met Asn Phe Leu Asn Glu Arg Thr Leu Lys Gly Thr
   305 310 315 320
   Phe Phe Gly Asn Tyr Lys Pro Arg Thr Asp Leu Pro Asn Val Val Glu
   325 330 335
   Leu Tyr Met Lys Lys Glu Leu Glu Val Glu Lys Phe Ile Thr His Ser
   340 345 350
   Val Pro Phe Ser Glu Ile Asn Thr Ala Phe Asp Leu Met His Lys Gly
   355 360 365
   Glu Gly Ile Arg Cys Ile Ile Arg Met Glu Asn
   370 375
<210> 47
   <211> 1140
   <212> DNA
   <213> Zea mays
<220>
   <221> CDS
   <222> (1)..(1137)
   <223> coding for alcohol dehydrogenase
<400> 47
   atg gcg acc gcg ggg aag gtg atc aag tgc aaa gct gcg gtg gca tgg      48
   Met Ala Thr Ala Gly Lys Val Ile Lys Cys Lys Ala Ala Val Ala Trp
    1               5                  10                  15
   gag gcc ggc aag cca ctg tcg atc gag gag gtg gag gta gcg cct ccg      96
   Glu Ala Gly Lys Pro Leu Ser Ile Glu Glu Val Glu Val Ala Pro Pro
               20                  25                  30
   cag gcc atg gag gtg cgc gtc aag atc ctc ttc acc tcg ctc tgc cac     144
   Gln Ala Met Glu Val Arg Val Lys Ile Leu Phe Thr Ser Leu Cys His
           35                  40                  45
   acc gac gtc tac ttc tgg gag gcc aag ggg cag act ccc gtg ttc cct     192
   Thr Asp Val Tyr Phe Trp Glu Ala Lys Gly Gln Thr Pro Val Phe Pro
       50                  55                  60
   cgg atc ttt ggc cat gag gct gga ggt atc ata gag agt gtt gga gag     240
   Arg Ile Phe Gly His Glu Ala Gly Gly Ile Ile Glu Ser Val Gly Glu
   65                  70                  75                  80
   ggt gtg act gac gta gct ccg ggc gac cat gtc ctt cct gtg ttc act     288
   Gly Val Thr Asp Val Ala Pro Gly Asp His Val Leu Pro Val Phe Thr
                   85                  90                  95
   ggg gag tgc aag gag tgc gcc cac tgc aag tcg gca gag agc aac atg     336
   Gly Glu Cys Lys Glu Cys Ala His Cys Lys Ser Ala Glu Ser Asn Met
               100                 105                 110
   tgt gat ttg ctc agg atc aac act gac cgc ggt gtg atg att ggc gat     384
   Cys Asp Leu Leu Arg Ile Asn Thr Asp Arg Gly Val Met Ile Gly Asp
           115                 120                 125
   ggc aag tcg cgg ttt tca atc aat ggg aag cct atc tac cac ttt gtt     432
   Gly Lys Ser Arg Phe Ser Ile Asn Gly Lys Pro Ile Tyr His Phe Val
       130                 135                 140
   ggg act tcc acc ttc agc gag tac acc gtc atg cat gtc ggt tgt gtt     480
   Gly Thr Ser Thr Phe Ser Glu Tyr Thr Val Met His Val Gly Cys Val
   145                 150                 155                 160
   gca aag atc aac cct cag gct ccc ctt gat aaa gtt tgc gtc ctt agc     528
   Ala Lys Ile Asn Pro Gln Ala Pro Leu Asp Lys Val Cys Val Leu Ser
                   165                 170                 175
   tgt ggt att tct act ggt ctt ggt gca tca att aat gtt gca aaa cct     576
   Cys Gly Ile Ser Thr Gly Leu Gly Ala Ser Ile Asn Val Ala Lys Pro
               180                 185                 190
   ccg aag ggt tcg aca gtg gct gtt ttc ggt tta gga gcc gtt ggt ctt     624
   Pro Lys Gly Ser Thr Val Ala Val Phe Gly Leu Gly Ala Val Gly Leu
           195                 200                 205
   gcc gct gca gaa ggt gca agg att gct gga gcg tca agg atc att ggt     672
   Ala Ala Ala Glu Gly Ala Arg Ile Ala Gly Ala Ser Arg Ile Ile Gly
       210                 215                 220
   gtc gac ctg aac ccc agc aga ttc gaa gaa gct agg aag ttc ggt tgc     720
   Val Asp Leu Asn Pro Ser Arg Phe Glu Glu Ala Arg Lys Phe Gly Cys
   225                 230                 235                 240
   act gaa ttt gtg aac cca aaa gac cac aac aag ccg gtg cag gag gta     768
   Thr Glu Phe Val Asn Pro Lys Asp His Asn Lys Pro Val Gln Glu Val
                   245                     250                 255
   ctt gct gag atg acc aac gga ggg gtc gac cgc agc gtg gaa tgc act     816
   Leu Ala Glu Met Thr Asn Gly Gly Val Asp Arg Ser Val Glu Cys Thr
               260                     265                 270
   ggc aac atc aat gct atg atc caa gct ttc gaa tgt gtt cat gat ggc     864
   Gly Asn Ile Asn Ala Met Ile Gln Ala Phe Glu Cys Val His Asp Gly
           275                     280                 285
   tgg ggt gtt gcc gtg ctg gtg ggt gtg ccg cat aag gac gct gag ttc     912
   Trp Gly Val Ala Val Leu Val Gly Val Pro His Lys Asp Ala Glu Phe
       290                     295                 300
   aag acc cac ccg atg aac ttc ctg aac gaa agg acc ctg aag ggg acc     960
   Lys Thr His Pro Met Asn Phe Leu Asn Glu Arg Thr Leu Lys Gly Thr
   305                     310                 315                 320
   ttc ttt ggc aac tat aag cca cgc act gat ctg cca aat gtg gtg gag     1008
   Phe Phe Gly Asn Tyr Lys Pro Arg Thr Asp Leu Pro Asn Val Val Glu
                   325                     330                 335
   ctg tac atg aaa aag gag ctg gag gtg gag aag ttc atc acg cac agc     1056
   Leu Tyr Met Lys Lys Glu Leu Glu Val Glu Lys Phe Ile Thr His Ser
               340                     345                 350
   gtc ccg ttc gcg gag atc aac aag gcg ttc aac ctg atg gcc aag ggg     1104
   Val Pro Phe Ala Glu Ile Asn Lys Ala Phe Asn Leu Met Ala Lys Gly
           355                     360                 365
   gag ggc atc cgc tgc atc atc cgc atg gag aac tag                     1140
   Glu Gly Ile Arg Cys Ile Ile Arg Met Glu Asn
       370                     375
<210> 48
   <211> 379
   <212> PRT
   <213> Zea mays
<400> 48
   Met Ala Thr Ala Gly Lys Val Ile Lys Cys Lys Ala Ala Val Ala Trp
   1 5 10 15
   Glu Ala Gly Lys Pro Leu Ser Ile Glu Glu Val Glu Val Ala Pro Pro
   20 25 30
   Gln Ala Met Glu Val Arg Val Lys Ile Leu Phe Thr Ser Leu Cys His
   35 40 45
   Thr Asp Val Tyr Phe Trp Glu Ala Lys Gly Gln Thr Pro Val Phe Pro
   50 55 60
   Arg Ile Phe Gly His Glu Ala Gly Gly Ile Ile Glu Ser Val Gly Glu
   65 70 75 80
   Gly Val Thr Asp Val Ala Pro Gly Asp His Val Leu Pro Val Phe Thr
   85 90 95
   Gly Glu Cys Lys Glu Cys Ala His Cys Lys Ser Ala Glu Ser Asn Met
   100 105 110
   Cys Asp Leu Leu Arg Ile Asn Thr Asp Arg Gly Val Met Ile Gly Asp
   115 120 125
   Gly Lys Ser Arg Phe Ser Ile Asn Gly Lys Pro Ile Tyr His Phe Val
   130 135 140
   Gly Thr Ser Thr Phe Ser Glu Tyr Thr Val Met His Val Gly Cys Val
   145                 150                 155                 160
   Ala Lys Ile Asn Pro Gln Ala Pro Leu Asp Lys Val Cys Val Leu Ser
                   165                 170                 175
   Cys Gly Ile Ser Thr Gly Leu Gly Ala Ser Ile Asn Val Ala Lys Pro
               180                 185                 190
   Pro Lys Gly Ser Thr Val Ala Val Phe Gly Leu Gly Ala Val Gly Leu
           195                 200                 205
   Ala Ala Ala Glu Gly Ala Arg Ile Ala Gly Ala Ser Arg Ile Ile Gly
       210                 215                 220
   Val Asp Leu Asn Pro Ser Arg Phe Glu Glu Ala Arg Lys Phe Gly Cys
   225                 230                 235                 240
   Thr Glu Phe Val Asn Pro Lys Asp His Asn Lys Pro Val Gln Glu Val
                   245                 250                 255
   Leu Ala Glu Met Thr Asn Gly Gly Val Asp Arg Ser Val Glu Cys Thr
               260                 265                 270
   Gly Asn Ile Asn Ala Met Ile Gln Ala Phe Glu Cys Val His Asp Gly
           275                 280                 285
   Trp Gly Val Ala Val Leu Val Gly Val Pro His Lys Asp Ala Glu Phe
       290                 295                 300
   Lys Thr His Pro Met Asn Phe Leu Asn Glu Arg Thr Leu Lys Gly Thr
   305                 310                 315                 320
   Phe Phe Gly Asn Tyr Lys Pro Arg Thr Asp Leu Pro Asn Val Val Glu
                   325                 330                 335
   Leu Tyr Met Lys Lys Glu Leu Glu Val Glu Lys Phe Ile Thr His Ser
               340                 345                 350
   Val Pro Phe Ala Glu Ile Asn Lys Ala Phe Asn Leu Met Ala Lys Gly
           355                 360                 365
   Glu Gly Ile Arg Cys Ile Ile Arg Met Glu Asn
       370                 375
<210> 49
   <211> 505
   <212> DNA
   <213> Künstliche Sequenz
<220>
   <223> Beschreibung der künstlichen Sequenz: coding for sense RNA-fragment of E.coli codA gene
<400> 49
   aagcttggct aacagtgtcg aataacgctt tacaaacaat tattaacgcc cggttaccag 60
   gcgaagaggg gctgtggcag attcatctgc aggacggaaa aatcagcgcc attgatgcgc 120
   aatccggcgt gatgcccata actgaaaaca gcctggatgc cgaacaaggt ttagttatac 180
   cgccgtttgt ggagccacat attcacctgg acaccacgca aaccgccgga caaccgaact 240
   ggaatcagtc cggcacgctg tttgaaggca ttgaacgctg ggccgagcgc aaagcgttat 300
   taacccatga cgatgtgaaa caacgcgcat ggcaaacgct gaaatggcag attgccaacg 360
   gcattcagca tgtgcgtacc catgtcgatg tttcggatgc aacgctaact gcgctgaaag 420
   caatgctgga agtgaagcag gaagtcgcgc cgtggattga tctgcaaatc gtcgccttcc 480
   ctcaggaagg gattttgtcg tcgac                                       505
<210> 50
   <211> 27
   <212> DNA
   <213> Künstliche Sequenz
<220>
   <223> Beschreibung der künstlichen Sequenz: oligonucleotide primer
<400> 50
   cgtgaatacg gcgtggagtc g 21
<210> 51
   <211> 26
   <212> DNA
   <213> Künstliche Sequenz
<220>
   <223> Beschreibung der künstlichen Sequenz: oligonucleotide primer
<400> 51
   cggcaggata atcaggttgg 20
<210> 52
   <211> 505
   <212> DNA
   <213> Künstliche Sequenz
<220>
   <223> Beschreibung der künstlichen Sequenz: coding for antisense RNA-fragment of E.coli codA gene
<400> 52
   gaattcggct aacagtgtcg aataacgctt tacaaacaat tattaacgcc cggttaccag 60
   gcgaagaggg gctgtggcag attcatctgc aggacggaaa aatcagcgcc attgatgcgc 120
   aatccggcgt gatgcccata actgaaaaca gcctggatgc cgaacaaggt ttagttatac 180
   cgccgtttgt ggagccacat attcacctgg acaccacgca aaccgccgga caaccgaact 240
   ggaatcagtc cggcacgctg tttgaaggca ttgaacgctg ggccgagcgc aaagcgttat 300
   taacccatga cgatgtgaaa caacgcgcat ggcaaacgct gaaatggcag attgccaacg 360
   gcattcagca tgtgcgtacc catgtcgatg tttcggatgc aacgctaact gcgctgaaag 420
   caatgctgga agtgaagcag gaagtcgcgc cgtggattga tctgcaaatc gtcgccttcc 480
   ctcaggaagg gattttgtcg gatcc 505
<210> 53
   <211> 27
   <212> DNA
   <213> Künstliche Sequenz
<220>
   <223> Beschreibung der künstlichen Sequenz: oligonucleotide primer
<400> 53
   gtcaacgtaa ccaaccctgc 20
<210> 54
   <211> 26
   <212> DNA
   <213> Künstliche Sequenz
<220>
   <223> Beschreibung der künstlichen Sequenz: oligonucleotide primer
<400> 54
   ggatccgaca aaatcccttc ctgagg 26
<210> 55
   <211> 5674
   <212> DNA
   <213> Künstliche Sequenz
<220>
   <223> Beschreibung der künstlichen Sequenz: vector construct pBluKS-nitP-STLSl-35S-T
<400> 55
   ccagcttttg ttccctttag tgagggttaa tttcgagctt ggcgtaatca tggtcatagc 60
   tgtttcctgt gtgaaattgt tatccgctca caattccaca caacatacga gccggaagca 120
   taaagtgtaa agcctggggt gcctaatgag tgagctaact cacattaatt gcgttgcgct 180
   cactgcccgc tttccagtcg ggaaacctgt cgtgccagct gcattaatga atcggccaac 240
   gcgcggggag aggcggtttg cgtattgggc gctcttccgc ttcctcgctc actgactcgc 300
   tgcgctcggt cgttcggctg cggcgagcgg tatcagctca ctcaaaggcg gtaatacggt 360
   tatccacaga atcaggggat aacgcaggaa agaacatgtg agcaaaaggc cagcaaaagg 420
   ccaggaaccg taaaaaggcc gcgttgctgg cgtttttcca taggctccgc ccccctgacg 480
   agcatcacaa aaatcgacgc tcaagtcaga ggtggcgaaa cccgacagga ctataaagat 540
   accaggcgtt tccccctgga agctccctcg tgcgctctcc tgttccgacc ctgccgctta 600
   ccggatacct gtccgccttt ctcccttcgg gaagcgtggc gctttctcat agctcacgct 660
   gtaggtatct cagttcggtg taggtcgttc gctccaagct gggctgtgtg cacgaacccc 720
   ccgttcagcc cgaccgctgc gccttatccg gtaactatcg tcttgagtcc aacccggtaa 780
   gacacgactt atcgccactg gcagcagcca ctggtaacag gattagcaga gcgaggtatg 840
   taggcggtgc tacagagttc ttgaagtggt ggcctaacta cggctacact agaaggacag 900
   tatttggtat ctgcgctctg ctgaagccag ttaccttcgg aaaaagagtt ggtagctctt 960
   gatccggcaa acaaaccacc gctggtagcg gtggtttttt tgtttgcaag cagcagatta 1020
   cgcgcagaaa aaaaggatct caagaagatc ctttgatctt ttctacgggg tctgacgctc 1080
   agtggaacga aaactcacgt taagggattt tggtcatgag attatcaaaa aggatcttca 1140
   cctagatcct tttaaattaa aaatgaagtt ttaaatcaat ctaaagtata tatgagtaaa 1200
   cttggtctga cagttaccaa tgcttaatca gtgaggcacc tatctcagcg atctgtctat 1260
   ttcgttcatc catagttgcc tgactccccg tcgtgtagat aactacgata cgggagggct 1320
   taccatctgg ccccagtgct gcaatgatac cgcgagaccc acgctcaccg gctccagatt 1380
   tatcagcaat aaaccagcca gccggaaggg ccgagcgcag aagtggtcct gcaactttat 1440
   ccgcctccat ccagtctatt aattgttgcc gggaagctag agtaagtagt tcgccagtta 1500
   atagtttgcg caacgttgtt gccattgcta caggcatcgt ggtgtcacgc tcgtcgtttg 1560
   gtatggcttc attcagctcc ggttcccaac gatcaaggcg agttacatga tcccccatgt 1620
   tgtgcaaaaa agcggttagc tccttcggtc ctccgatcgt tgtcagaagt aagttggccg 1680
   cagtgttatc actcatggtt atggcagcac tgcataattc tcttactgtc atgccatccg 1740
   taagatgctt ttctgtgact ggtgagtact caaccaagtc attctgagaa tagtgtatgc 1800
   ggcgaccgag ttgctcttgc ccggcgtcaa tacgggataa taccgcgcca catagcagaa 1860
   ctttaaaagt gctcatcatt ggaaaacgtt cttcggggcg aaaactctca aggatcttac 1920
   cgctgttgag atccagttcg atgtaaccca ctcgtgcacc caactgatct tcagcatctt 1980
   ttactttcac cagcgtttct gggtgagcaa aaacaggaag gcaaaatgcc gcaaaaaagg 2040
   gaataagggc gacacggaaa tgttgaatac tcatactctt cctttttcaa tattattgaa 2100
   gcatttatca gggttattgt ctcatgagcg gatacatatt tgaatgtatt tagaaaaata 2160
   aacaaatagg ggttccgcgc acatttcccc gaaaagtgcc acctgacgcg ccctgtagcg 2220
   gcgcattaag cgcggcgggt gtggtggtta cgcgcagcgt gaccgctaca cttgccagcg 2280
   ccctagcgcc cgctcctttc gctttcttcc cttcctttct cgccacgttc gccggctttc 2340
   cccgtcaagc tctaaatcgg gggctccctt tagggttccg atttagtgct ttacggcacc 2400
   tcgaccccaa aaaacttgat tagggtgatg gttcacgtag tgggccatcg ccctgataga 2460
   cggtttttcg ccctttgacg ttggagtcca cgttctttaa tagtggactc ttgttccaaa 2520
   ctggaacaac actcaaccct atctcggtct attcttttga tttataaggg attttgccga 2580
   tttcggccta ttggttaaaa aatgagctga tttaacaaaa atttaacgcg aattttaaca 2640
   aaatattaac gcttacaatt tccattcgcc attcaggctg cgcaactgtt gggaagggcg 2700
   atcggtgcgg gcctcttcgc tattacgcca gctggcgaaa gggggatgtg ctgcaaggcg 2760
   attaagttgg gtaacgccag ggttttccca gtcacgacgt tgtaaaacga cggccagtga 2820
   attgtaatac gactcactat agggcgaatt ggagctcgtc gagaccagat gttttacact 2880
   tgaccgtaaa tgagcacccg aagaaaccgg tcacattcat ttcgaaggtg gagaaagcgg 2940
   aagatgactc aaacaagtaa tcggttgtga ttcgtcagtt catgtcactc ctatgaagga 3000
   gtcaagttca aaatgttatg ttgagtttca aacttttatg ctaaactttt tttctttatt 3060
   ttcgttaata atggaagaga accaattctc ttgtatctaa agattatcca tctatcatcc 3120
   aatttgagtg ttcaattctg gatgttgtgt taccctacat tctacaacca tgtagccaat 3180
   tattatgaat ctggctttga tttcagttgt gttcttttct tttttttctt tgcatatttg 3240
   catttagaat gtttaataat taagttactg tatttccaca tacattagtt ccaagaatat 3300
   acatatatta atttattttt cttaaaaatg ttttggaatg actaatattg acaacgaaaa 3360
   tagaagctat gctaaaccat tacgtatatg tgacttcaca tgttgttgtt ttacattccc 3420
   tatatatatg gatggctgtc acaatcagaa acgtgatcga aaaaagacaa acagtgtttg 3480
   cataaaaaga ctatttcgtt tcattgacaa tttgtgttta tttgtaaaga aaagtggcaa 3540
   agtggaattt gagttcctgc aagtaagaaa gatgaaataa aagacttgag tgtgtgtttt 3600
   tttcttttat ctgaaagctg caatgaaata ttcctaccaa gcccgtttga ttattaattg 3660
   gggtttggtt ttcttgatgc gaactaattg gttatataag aaactataca atccatgtta 3720
   attcaaaaat tttgatttct cttgtaggaa tatgatttac tatatgagac tttcttttcg 3780
   ccaataatag taaatccaaa gatatttgac cggaccaaaa cacattgatc tattttttag 3840
   tttatttaat ccagtttctc tgagataatt cattaaggaa aacttagtat taacccatcc 3900
   taagattaaa taggagccaa actcacattt caaatattaa ataacataaa atggatttaa 3960
   aaaatctata cgtcaaattt tatttatgac atttcttatt taaatttata tttaatgaaa 4020
   tacagctaag acaaaccaaa aaaaaaatac tttctaagtg gtccaaaaca tcaattccgt 4080
   tcaatattat taggtagaat cgtacgacca aaaaaaggta ggttaatacg aattagaaac 4140
   atatctataa catagtatat attattacct attatgagga atcaaaatgc atcaaatatg 4200
   gatttaagga atccataaaa gaataaattc tacgggaaaa aaaatggaat aaattctttt 4260
   aagtttttta tttgtttttt atttggtagt tctccatttt gttttatttc gtttggattt 4320
   attgtgtcca aatactttgt aaaccaccgt tgtaattctt aaacggggtt ttcacttctt 4380
   ttttatattc agacataaag catcggctgg tttaatcaat caatagattt tatttttctt 4440
   ctcaattatt agtaggtttg atgtgaactt tacaaaaaaa acaaaaacaa atcaatgcag 4500
   agaaaagaaa ccacgtgggc tagtcccacc ttgtttcatt tccaccacag gttcgatctt 4560
   cgttaccgtc tccaatagga aaataaacgt gaccacaaaa aaaaaacaaa aaaaagtcta 4620
   tatattgctt ctctcaagtc tctgagtgtc atgaaccaaa gtaaaaaaca aagactcgac 4680
   ctgcaggcat gcaagcttat cgtcgactac gtaagtttct gcttctacct ttgatatata 4740
   tataataatt atcattaatt agtagtaata taatatttca aatatttttt tcaaaataaa 4800
   agaatgtagt atatagcaat tgcttttctg tagtttataa gtgtgtatat tttaatttat 4860
   aacttttcta atatatgacc aaaatttgtt gatgtgcagg tatcaccgga tccatcgaat 4920
   tcggtacgct gaaatcacca gtctctctct acaaatctat ctctctctat tttctccata 4980
   aataatgtgt gagtagtttc ccgataaggg gaanttaggg ttcttatagg gtttcgctca 5040
   tgtgttgagc atataagaaa cccttagtat gtatttgtat ttgtaaaata cttctatcaa 5100
   taaaatttct aattcctaaa accaaaatcc agtactaaaa tccagatctc ctaaagtccc 5160
   tatagatctt tgtcgtgaat ataaaccaga cacgagacga ctaaacctgg agcccagacg 5220
   ccgttcgaag ctagaagtac cgcttaggca ggaggccgtt agggaaaaga tgctaaggca 5280
   gggttggtta cgttgactcc cccgtaggtt tggtttaaat atgatgaagt ggacggaagg 5340
   aaggaggaag acaaggaagg ataaggttgc aggccctgtg caaggtaaga agatggaaat 5400
   ttgatagagg tacgctacta tacttatact atacgctaag ggaatgcttg tatttatacc 5460
   ctataccccc taataacccc ttatcaattt aagaaataat ccgcataagc ccccgcttaa 5520
   aaattggtat cagagccatg aataggtcta tgaccaaaac tcaagaggat aaaacctcac 5580
   caaaatacga aagagttctt aactctaaag ataaaagatc tttcaagatc aaaactagtt 5640
   ccctcacacc ggtgacgggg atcgcgatgg gtac 5674
<210> 56
   <211> 6046
   <212> DNA
   <213> Künstliche Sequenz
<220>
   <223> Beschreibung der künstlichen Sequenz: binary vector pSUN1
<400> 56
   ttccatggac atacaaatgg acgaacggat aaaccttttc acgccctttt aaatatccga 60
   ttattctaat aaacgctctt ttctcttagg tttacccgcc aatatatcct gtcaaacact 120
   gatagtttaa actgaaggcg ggaaacgaca atcagatcta gtaggaaaca gctatgacca 180
   tgattacgcc aagcttgcat gcctgcaggt cgactctaga ctagtggatc cgatatcgcc 240
   cgggctcgag gtaccgagct cgaattcact ggccgtcgtt ttacaacgac tcagctgctt 300
   ggtaataatt gtcattagat tgtttttatg catagatgca ctcgaaatca gccaatttta 360
   gacaagtatc aaacggatgt taattcagta cattaaagac gtccgcaatg tgttattaag 420
   ttgtctaagc gtcaatttgt ttacaccaca atatatcctg ccaccagcca gccaacagct 480
   ccccgaccgg cagctcggca caaaatcacc acgcgttacc accacgccgg ccggccgcat 540
   ggtgttgacc gtgttcgccg gcattgccga gttcgagcgt tccctaatca tcgaccgcac 600
   ccggagcggg cgcgaggccg ccaaggcccg aggcgtgaag tttggccccc gccctaccct 660
   caccccggca cagatcgcgc acgcccgcga gctgatcgac caggaaggcc gcaccgtgaa 720
   agaggcggct gcactgcttg gcgtgcatcg ctcgaccctg taccgcgcac ttgagcgcag 780
   cgaggaagtg acgcccaccg aggccaggcg gcgcggtgcc ttccgtgagg acgcattgac 840
   cgaggccgac gccctggcgg ccgccgagaa tgaacgccaa gaggaacaag catgaaaccg 900
   caccaggacg gccaggacga accgtttttc attaccgaag agatcgaggc ggagatgatc 960
   gcggccgggt acgtgttcga gccgcccgcg cacgtctcaa ccgtgcggct gcatgaaatc 1020
   ctggccggtt tgtctgatgc caagctggcg gcctggccgg ccagcttggc cgctgaagaa 1080
   accgagcgcc gccgtctaaa aaggtgatgt gtatttgagt aaaacagctt gcgtcatgcg 1140
   gtcgctgcgt atatgatgcg atgagtaaat aaacaaatac gcaaggggaa cgcatgaagg 1200
   ttatcgctgt acttaaccag aaaggcgggt caggcaagac gaccatcgca acccatctag 1260
   cccgcgccct gcaactcgcc ggggccgatg ttctgttagt cgattccgat ccccagggca 1320
   gtgcccgcga ttgggcggcc gtgcgggaag atcaaccgct aaccgttgtc ggcatcgacc 1380
   gcccgacgat tgaccgcgac gtgaaggcca tcggccggcg cgacttcgta gtgatcgacg 1440
   gagcgcccca ggcggcggac ttggctgtgt ccgcgatcaa ggcagccgac ttcgtgctga 1500
   ttccggtgca gccaagccct tacgacatat gggccaccgc cgacctggtg gagctggtta 1560
   agcagcgcat tgaggtcacg gatggaaggc tacaagcggc ctttgtcgtg tcgcgggcga 1620
   tcaaaggcac gcgcatcggc ggtgaggttg ccgaggcgct ggccgggtac gagctgccca 1680
   ttcttgagtc ccgtatcacg cagcgcgtga gctacccagg cactgccgcc gccggcacaa 1740
   ccgttcttga atcagaaccc gagggcgacg ctgcccgcga ggtccaggcg ctggccgctg 1800
   aaattaaatc aaaactcatt tgagttaatg aggtaaagag aaaatgagca aaagcacaaa 1860
   cacgctaagt gccggccgtc cgagcgcacg cagcagcaag gctgcaacgt tggccagcct 1920
   ggcagacacg ccagccatga agcgggtcaa ctttcagttg ccggcggagg atcacaccaa 1980
   gctgaagatg tacgcggtac gccaaggcaa gaccattacc gagctgctat ctgaatacat 2040
   cgcgcagcta ccagagtaaa tgagcaaatg aataaatgag tagatgaatt ttagcggcta 2100
   aaggaggcgg catggaaaat caagaacaac caggcaccga cgccgtggaa tgccccatgt 2160
   gtggaggaac gggcggttgg ccaggcgtaa gcggctgggt tgtctgccgg ccctgcaatg 2220
   gcactggaac ccccaagccc gaggaatcgg cgtgagcggt cgcaaaccat ccggcccggt 2280
   acaaatcggc gcggcgctgg gtgatgacct ggtggagaag ttgaaggccg cgcaggccgc 2340
   ccagcggcaa cgcatcgagg cagaagcacg ccccggtgaa tcgtggcaag cggccgctga 2400
   tcgaatccgc aaagaatccc ggcaaccgcc ggcagccggt gcgccgtcga ttaggaagcc 2460
   gcccaagggc gacgagcaac cagatttttt cgttccgatg ctctatgacg tgggcacccg 2520
   cgatagtcgc agcatcatgg acgtggccgt tttccgtctg tcgaagcgtg accgacgagc 2580
   tggcgaggtg atccgctacg agcttccaga cgggcacgta gaggtttccg cagggccggc 2640
   cggcatggcc agtgtgtggg attacgacct ggtactgatg gcggtttccc atctaaccga 2700
   atccatgaac cgataccggg aagggaaggg agacaagccc ggccgcgtgt tccgtccaca 2760
   cgttgcggac gtactcaagt tctgccggcg agccgatggc ggaaagcaga aagacgacct 2820
   ggtagaaacc tgcattcggt taaacaccac gcacgttgcc atgcagcgta cgaagaaggc 2880
   caagaacggc cgcctggtga cggtatccga gggtgaagcc ttgattagcc gctacaagat 2940
   cgtaaagagc gaaaccgggc ggccggagta catcgagatc gagctagctg attggatgta 3000
   ccgcgagatc acagaaggca agaacccgga cgtgctgacg gttcaccccg attacttttt 3060
   gatcgatccc ggcatcggcc gttttctcta ccgcctggca cgccgcgccg caggcaaggc 3120
   agaagccaga tggttgttca agacgatcta cgaacgcagt ggcagcgccg gagagttcaa 3180
   gaagttctgt ttcaccgtgc gcaagctgat cgggtcaaat gacctgccgg agtacgattt 3240
   gaaggaggag gcggggcagg ctggcccgat cctagtcatg cgctaccgca acctgatcga 3300
   gggcgaagca tccgccggtt cctaatgtac ggagcagatg ctagggcaaa ttgccctagc 3360
   aggggaaaaa ggtcgaaaag gtctctttcc tgtggatagc acgtacattg ggaacccaaa 3420
   gccgtacatt gggaaccgga acccgtacat tgggaaccca aagccgtaca ttgggaaccg 3480
   gtcacacatg taagtgactg atataaaaga gaaaaaaggc gatttttccg cctaaaactc 3540
   tttaaaactt attaaaactc ttaaaacccg cctggcctgt gcataactgt ctggccagcg 3600
   cacagccgaa gagctgcaaa aagcgcctac ccttcggtcg ctgcgctccc tacgccccgc 3660
   cgcttcgcgt cggcctatcg cggccgctgg ccgctcaaaa atggctggcc tacggccagg 3720
   caatctacca gggcgcggac aagccgcgcc gtcgccactc gaccgccggc gcccacatca 3780
   aggcaccctg cctcgcgcgt ttcggtgatg acggtgaaaa cctctgacac atgcagctcc 3840
   cggagacggt cacagcttgt ctgtaagcgg atgccgggag cagacaagcc cgtcagggcg 3900
   cgtcagcggg tgttggcggg tgtcggggcg cagccatgac ccagtcacgt agcgatagcg 3960
   gagtgtatac tggcttaact atgcggcatc agagcagatt gtactgagag tgcaccatat 4020
   gcggtgtgaa ataccgcaca gatgcgtaag gagaaaatac cgcatcaggc gctcttccgc 4080
   ttcctcgctc actgactcgc tgcgctcggt cgttcggctg cggcgagcgg tatcagctca 4140
   ctcaaaggcg gtaatacggt tatccacaga atcaggggat aacgcaggaa agaacatgtg 4200
   agcaaaaggc cagcaaaagg ccaggaaccg taaaaaggcc gcgttgctgg cgtttttcca 4260
   taggctccgc ccccctgacg agcatcacaa aaatcgacgc tcaagtcaga ggtggcgaaa 4320
   cccgacagga ctataaagat accaggcgtt tccccctgga agctccctcg tgcgctctcc 4380
   tgttccgacc ctgccgctta ccggatacct gtccgccttt ctcccttcgg gaagcgtggc 4440
   gctttctcat agctcacgct gtaggtatct cagttcggtg taggtcgttc gctccaagct 4500
   gggctgtgtg cacgaacccc ccgttcagcc cgaccgctgc gccttatccg gtaactatcg 4560
   tcttgagtcc aacccggtaa gacacgactt atcgccactg gcagcagcca ctggtaacag 4620
   gattagcaga gcgaggtatg taggcggtgc tacagagttc ttgaagtggt ggcctaacta 4680
   cggctacact agaaggacag tatttggtat ctgcgctctg ctgaagccag ttaccttcgg 4740
   aaaaagagtt ggtagctctt gatccggcaa acaaaccacc gctggtagcg gtggtttttt 4800
   tgtttgcaag cagcagatta cgcgcagaaa aaaaggatct caagaagatc ctttgatctt 4860
   ttctacgggg tctgacgctc agtggaacga aaactcacgt taagggattt tggtcatgca 4920
   tgatatatct cccaatttgt gtagggctta ttatgcacgc ttaaaaataa taaaagcaga 4980
   cttgacctga tagtttggct gtgagcaatt atgtgcttag tgcatctaac gcttgagtta 5040
   agccgcgccg cgaagcggcg tcggcttgaa cgaatttcta gctagacatt atttgccgac 5100
   taccttggtg atctcgcctt tcacgtagtg gacaaattct tccaactgat ctgcgcgcga 5160
   ggccaagcga tcttcttctt gtccaagata agcctgtcta gcttcaagta tgacgggctg 5220
   atactgggcc ggcaggcgct ccattgccca gtcggcagcg acatccttcg gcgcgatttt 5280
   gccggttact gcgctgtacc aaatgcggga caacgtaagc actacatttc gctcatcgcc 5340
   agcccagtcg ggcggcgagt tccatagcgt taaggtttca tttagcgcct caaatagatc 5400
   ctgttcagga accggatcaa agagttcctc cgccgctgga cctaccaagg caacgctatg 5460
   ttctcttgct tttgtcagca agatagccag atcaatgtcg atcgtggctg gctcgaagat 5520
   acctgcaaga atgtcattgc gctgccattc tccaaattgc agttcgcgct tagctggata 5580
   acgccacgga atgatgtcgt cgtgcacaac aatggtgact tctacagcgc ggagaatctc 5640
   gctctctcca ggggaagccg aagtttccaa aaggtcgttg atcaaagctc gccgcgttgt 5700
   ttcatcaagc cttacggtca ccgtaaccag caaatcaata tcactgtgtg gcttcaggcc 5760
   gccatccact gcggagccgt acaaatgtac ggccagcaac gtcggttcga gatggcgctc 5820
   gatgacgcca actacctctg atagttgagt cgatacttcg gcgatcaccg cttcccccat 5880
   gatgtttaac tttgttttag ggcgactgcc ctgctgcgta acatcgttgc tgctccataa 5940
   catcaaacat cgacccacgg cgtaacgcgc ttgctgcttg gatgcccgag gcatagactg 6000
   taccccaaaa aaacagtcat aacaagccat gaaaaccgcc actgcg 6046
<210> 57
   <211> 9838
   <212> DNA
   <213> Künstliche Sequenz
<220>
   <223> Beschreibung der künstlichen Sequenz: Transgenic expression vector for codA dsRNA pSUN1-codA-RNAi
<400> 57
   cgaattcact ggccgtcgtt ttacaacgac tcagctgctt ggtaataatt gtcattagat 60
   tgtttttatg catagatgca ctcgaaatca gccaatttta gacaagtatc aaacggatgt 120
   taattcagta cattaaagac gtccgcaatg tgttattaag ttgtctaagc gtcaatttgt 180
   ttacaccaca atatatcctg ccaccagcca gccaacagct ccccgaccgg cagctcggca 240
   caaaatcacc acgcgttacc accacgccgg ccggccgcat ggtgttgacc gtgttcgccg 300
   gcattgccga gttcgagcgt tccctaatca tcgaccgcac ccggagcggg cgcgaggccg 360
   ccaaggcccg aggcgtgaag tttggccccc gccctaccct caccccggca cagatcgcgc 420
   acgcccgcga gctgatcgac caggaaggcc gcaccgtgaa agaggcggct gcactgcttg 480
   gcgtgcatcg ctcgaccctg taccgcgcac ttgagcgcag cgaggaagtg acgcccaccg 540
   aggccaggcg gcgcggtgcc ttccgtgagg acgcattgac cgaggccgac gccctggcgg 600
   ccgccgagaa tgaacgccaa gaggaacaag catgaaaccg caccaggacg gccaggacga 660
   accgtttttc attaccgaag agatcgaggc ggagatgatc gcggccgggt acgtgttcga 720
   gccgcccgcg cacgtctcaa ccgtgcggct gcatgaaatc ctggccggtt tgtctgatgc 780
   caagctggcg gcctggccgg ccagcttggc cgctgaagaa accgagcgcc gccgtctaaa 840
   aaggtgatgt gtatttgagt aaaacagctt gcgtcatgcg gtcgctgcgt atatgatgcg 900
   atgagtaaat aaacaaatac gcaaggggaa cgcatgaagg ttatcgctgt acttaaccag 960
   aaaggcgggt caggcaagac gaccatcgca acccatctag cccgcgccct gcaactcgcc 1020
   ggggccgatg ttctgttagt cgattccgat ccccagggca gtgcccgcga ttgggcggcc 1080
   gtgcgggaag atcaaccgct aaccgttgtc ggcatcgacc gcccgacgat tgaccgcgac 1140
   gtgaaggcca tcggccggcg cgacttcgta gtgatcgacg gagcgcccca ggcggcggac 1200
   ttggctgtgt ccgcgatcaa ggcagccgac ttcgtgctga ttccggtgca gccaagccct 1260
   tacgacatat gggccaccgc cgacctggtg gagctggtta agcagcgcat tgaggtcacg 1320
   gatggaaggc tacaagcggc ctttgtcgtg tcgcgggcga tcaaaggcac gcgcatcggc 1380
   ggtgaggttg ccgaggcgct ggccgggtac gagctgccca ttcttgagtc ccgtatcacg 1440
   cagcgcgtga gctacccagg cactgccgcc gccggcacaa ccgttcttga atcagaaccc 1500
   gagggcgacg ctgcccgcga ggtccaggcg ctggccgctg aaattaaatc aaaactcatt 1560
   tgagttaatg aggtaaagag aaaatgagca aaagcacaaa cacgctaagt gccggccgtc 1620
   cgagcgcacg cagcagcaag gctgcaacgt tggccagcct ggcagacacg ccagccatga 1680
   agcgggtcaa ctttcagttg ccggcggagg atcacaccaa gctgaagatg tacgcggtac 1740
   gccaaggcaa gaccattacc gagctgctat ctgaatacat cgcgcagcta ccagagtaaa 1800
   tgagcaaatg aataaatgag tagatgaatt ttagcggcta aaggaggcgg catggaaaat 1860
   caagaacaac caggcaccga cgccgtggaa tgccccatgt gtggaggaac gggcggttgg 1920
   ccaggcgtaa gcggctgggt tgtctgccgg ccctgcaatg gcactggaac ccccaagccc 1980
   gaggaatcgg cgtgagcggt cgcaaaccat ccggcccggt acaaatcggc gcggcgctgg 2040
   gtgatgacct ggtggagaag ttgaaggccg cgcaggccgc ccagcggcaa cgcatcgagg 2100
   cagaagcacg ccccggtgaa tcgtggcaag cggccgctga tcgaatccgc aaagaatccc 2160
   ggcaaccgcc ggcagccggt gcgccgtcga ttaggaagcc gcccaagggc gacgagcaac 2220
   cagatttttt cgttccgatg ctctatgacg tgggcacccg cgatagtcgc agcatcatgg 2280
   acgtggccgt tttccgtctg tcgaagcgtg accgacgagc tggcgaggtg atccgctacg 2340
   agcttccaga cgggcacgta gaggtttccg cagggccggc cggcatggcc agtgtgtggg 2400
   attacgacct ggtactgatg gcggtttccc atctaaccga atccatgaac cgataccggg 2460
   aagggaaggg agacaagccc ggccgcgtgt tccgtccaca cgttgcggac gtactcaagt 2520
   tctgccggcg agccgatggc ggaaagcaga aagacgacct ggtagaaacc tgcattcggt 2580
   taaacaccac gcacgttgcc atgcagcgta cgaagaaggc caagaacggc cgcctggtga 2640
   cggtatccga gggtgaagcc ttgattagcc gctacaagat cgtaaagagc gaaaccgggc 2700
   ggccggagta catcgagatc gagctagctg attggatgta ccgcgagatc acagaaggca 2760
   agaacccgga cgtgctgacg gttcaccccg attacttttt gatcgatccc ggcatcggcc 2820
   gttttctcta ccgcctggca cgccgcgccg caggcaaggc agaagccaga tggttgttca 2880
   agacgatcta cgaacgcagt ggcagcgccg gagagttcaa gaagttctgt ttcaccgtgc 2940
   gcaagctgat cgggtcaaat gacctgccgg agtacgattt gaaggaggag gcggggcagg 3000
   ctggcccgat cctagtcatg cgctaccgca acctgatcga gggcgaagca tccgccggtt 3060
   cctaatgtac ggagcagatg ctagggcaaa ttgccctagc aggggaaaaa ggtcgaaaag 3120
   gtctctttcc tgtggatagc acgtacattg ggaacccaaa gccgtacatt gggaaccgga 3180
   acccgtacat tgggaaccca aagccgtaca ttgggaaccg gtcacacatg taagtgactg 3240
   atataaaaga gaaaaaaggc gatttttccg cctaaaactc tttaaaactt attaaaactc 3300
   ttaaaacccg cctggcctgt gcataactgt ctggccagcg cacagccgaa gagctgcaaa 3360
   aagcgcctac ccttcggtcg ctgcgctccc tacgccccgc cgcttcgcgt cggcctatcg 3420
   cggccgctgg ccgctcaaaa atggctggcc tacggccagg caatctacca gggcgcggac 3480
   aagccgcgcc gtcgccactc gaccgccggc gcccacatca aggcaccctg cctcgcgcgt 3540
   ttcggtgatg acggtgaaaa cctctgacac atgcagctcc cggagacggt cacagcttgt 3600
   ctgtaagcgg atgccgggag cagacaagcc cgtcagggcg cgtcagcggg tgttggcggg 3660
   tgtcggggcg cagccatgac ccagtcacgt agcgatagcg gagtgtatac tggcttaact 3720
   atgcggcatc agagcagatt gtactgagag tgcaccatat gcggtgtgaa ataccgcaca 3780
   gatgcgtaag gagaaaatac cgcatcaggc gctcttccgc ttcctcgctc actgactcgc 3840
   tgcgctcggt cgttcggctg cggcgagcgg tatcagctca ctcaaaggcg gtaatacggt 3900
   tatccacaga atcaggggat aacgcaggaa agaacatgtg agcaaaaggc cagcaaaagg 3960
   ccaggaaccg taaaaaggcc gcgttgctgg cgtttttcca taggctccgc ccccctgacg 4020
   agcatcacaa aaatcgacgc tcaagtcaga ggtggcgaaa cccgacagga ctataaagat 4080
   accaggcgtt tccccctgga agctccctcg tgcgctctcc tgttccgacc ctgccgctta 4140
   ccggatacct gtccgccttt ctcccttcgg gaagcgtggc gctttctcat agctcacgct 4200
   gtaggtatct cagttcggtg taggtcgttc gctccaagct gggctgtgtg cacgaacccc 4260
   ccgttcagcc cgaccgctgc gccttatccg gtaactatcg tcttgagtcc aacccggtaa 4320
   gacacgactt atcgccactg gcagcagcca ctggtaacag gattagcaga gcgaggtatg 4380
   taggcggtgc tacagagttc ttgaagtggt ggcctaacta cggctacact agaaggacag 4440
   tatttggtat ctgcgctctg ctgaagccag ttaccttcgg aaaaagagtt ggtagctctt 4500
   gatccggcaa acaaaccacc gctggtagcg gtggtttttt tgtttgcaag cagcagatta 4560
   cgcgcagaaa aaaaggatct caagaagatc ctttgatctt ttctacgggg tctgacgctc 4620
   agtggaacga aaactcacgt taagggattt tggtcatgca tgatatatct cccaatttgt 4680
   gtagggctta ttatgcacgc ttaaaaataa taaaagcaga cttgacctga tagtttggct 4740
   gtgagcaatt atgtgcttag tgcatctaac gcttgagtta agccgcgccg cgaagcggcg 4800
   tcggcttgaa cgaatttcta gctagacatt atttgccgac taccttggtg atctcgcctt 4860
   tcacgtagtg gacaaattct tccaactgat ctgcgcgcga ggccaagcga tcttcttctt 4920
   gtccaagata agcctgtcta gcttcaagta tgacgggctg atactgggcc ggcaggcgct 4980
   ccattgccca gtcggcagcg acatccttcg gcgcgatttt gccggttact gcgctgtacc 5040
   aaatgcggga caacgtaagc actacatttc gctcatcgcc agcccagtcg ggcggcgagt 5100
   tccatagcgt taaggtttca tttagcgcct caaatagatc ctgttcagga accggatcaa 5160
   agagttcctc cgccgctgga cctaccaagg caacgctatg ttctcttgct tttgtcagca 5220
   agatagccag atcaatgtcg atcgtggctg gctcgaagat acctgcaaga atgtcattgc 5280
   gctgccattc tccaaattgc agttcgcgct tagctggata acgccacgga atgatgtcgt 5340
   cgtgcacaac aatggtgact tctacagcgc ggagaatctc gctctctcca ggggaagccg 5400
   aagtttccaa aaggtcgttg atcaaagctc gccgcgttgt ttcatcaagc cttacggtca 5460
   ccgtaaccag caaatcaata tcactgtgtg gcttcaggcc gccatccact gcggagccgt 5520
   acaaatgtac ggccagcaac gtcggttcga gatggcgctc gatgacgcca actacctctg 5580
   atagttgagt cgatacttcg gcgatcaccg cttcccccat gatgtttaac tttgttttag 5640
   ggcgactgcc ctgctgcgta acatcgttgc tgctccataa catcaaacat cgacccacgg 5700
   cgtaacgcgc ttgctgcttg gatgcccgag gcatagactg taccccaaaa aaacagtcat 5760
   aacaagccat gaaaaccgcc actgcgttcc atggacatac aaatggacga acggataaac 5820
   cttttcacgc ccttttaaat atccgattat tctaataaac gctcttttct cttaggttta 5880
   cccgccaata tatcctgtca aacactgata gtttaaactg aaggcgggaa acgacaatca 5940
   gatctagtag gaaacagcta tgaccatgat tacgccaagc ttgcatgcct gcaggtcgac 6000
   tctagactag tggatccgat atcgcccggg ctcgaggtac ccatcgcgat ccccgtcacc 6060
   ggtgtgaggg aactagtttt gatcttgaaa gatcttttat ctttagagtt aagaactctt 6120
   tcgtattttg gtgaggtttt atcctcttga gttttggtca tagacctatt catggctctg 6180
   ataccaattt ttaagcgggg gcttatgcgg attatttctt aaattgataa ggggttatta 6240
   gggggtatag ggtataaata caagcattcc cttagcgtat agtataagta tagtagcgta 6300
   cctctatcaa atttccatct tcttaccttg cacagggcct gcaaccttat ccttccttgt 6360
   cttcctcctt ccttccgtcc acttcatcat atttaaacca aacctacggg ggagtcaacg 6420
   taaccaaccc tgccttagca tcttttccct aacggcctcc tgcctaagcg gtacttctag 6480
   cttcgaacgg cgtctgggct ccaggtttag tcgtctcgtg tctggtttat attcacgaca 6540
   aagatctata gggactttag gagatctgga ttttagtact ggattttggt tttaggaatt 6600
   agaaatttta ttgatagaag tattttacaa atacaaatac atactaaggg tttcttatat 6660
   gctcaacaca tgagcgaaac cctataagaa ccctaanttc cccttatcgg gaaactactc 6720
   acacattatt tatggagaaa atagagagag atagatttgt agagagagac tggtgatttc 6780
   agcgtaccga attcggctaa cagtgtcgaa taacgcttta caaacaatta ttaacgcccg 6840
   gttaccaggc gaagaggggc tgtggcagat tcatctgcag gacggaaaaa tcagcgccat 6900
   tgatgcgcaa tccggcgtga tgcccataac tgaaaacagc ctggatgccg aacaaggttt 6960
   agttataccg ccgtttgtgg agccacatat tcacctggac accacgcaaa ccgccggaca 7020
   accgaactgg aatcagtccg gcacgctgtt tgaaggcatt gaacgctggg ccgagcgcaa 7080
   agcgttatta acccatgacg atgtgaaaca acgcgcatgg caaacgctga aatggcagat 7140
   tgccaacggc attcagcatg tgcgtaccca tgtcgatgtt tcggatgcaa cgctaactgc 7200
   gctgaaagca atgctggaag tgaagcagga agtcgcgccg tggattgatc tgcaaatcgt 7260
   cgccttccct caggaaggga ttttgtcgga tccggtgata cctgcacatc aacaaatttt 7320
   ggtcatatat tagaaaagtt ataaattaaa atatacacac ttataaacta cagaaaagca 7380
   attgctatat actacattct tttattttga aaaaaatatt tgaaatatta tattactact 7440
   aattaatgat aattattata tatatatcaa aggtagaagc agaaacttac gtagtcgacg 7500
   acaaaatccc ttcctgaggg aaggcgacga tttgcagatc aatccacggc gcgacttcct 7560
   gcttcacttc cagcattgct ttcagcgcag ttagcgttgc atccgaaaca tcgacatggg 7620
   tacgcacatg ctgaatgccg ttggcaatct gccatttcag cgtttgccat gcgcgttgtt 7680
   tcacatcgtc atgggttaat aacgctttgc gctcggccca gcgttcaatg ccttcaaaca 7740
   gcgtgccgga ctgattccag ttcggttgtc cggcggtttg cgtggtgtcc aggtgaatat 7800
   gtggctccac aaacggcggt ataactaaac cttgttcggc atccaggctg ttttcagtta 7860
   tgggcatcac gccggattgc gcatcaatgg cgctgatttt tccgtcctgc agatgaatct 7920
   gccacagccc ctcttcgcct ggtaaccggg cgttaataat tgtttgtaaa gcgttattcg 7980
   acactgttag ccaagcttgc atgcctgcag gtcgagtctt tgttttttac tttggttcat 8040
   gacactcaga gacttgagag aagcaatata tagacttttt tttgtttttt ttttgtggtc 8100
   acgtttattt tcctattgga gacggtaacg aagatcgaac ctgtggtgga aatgaaacaa 8160
   ggtgggacta gcccacgtgg tttcttttct ctgcattgat ttgtttttgt tttttttgta 8220
   aagttcacat caaacctact aataattgag aagaaaaata aaatctattg attgattaaa 8280
   ccagccgatg ctttatgtct gaatataaaa aagaagtgaa aaccccgttt aagaattaca 8340
   acggtggttt acaaagtatt tggacacaat aaatccaaac gaaataaaac aaaatggaga 8400
   actaccaaat aaaaaacaaa taaaaaactt aaaagaattt attccatttt ttttcccgta 8460
   gaatttattc ttttatggat tccttaaatc catatttgat gcattttgat tcctcataat 8520
   aggtaataat atatactatg ttatagatat gtttctaatt cgtattaacc tacctttttt 8580
   tggtcgtacg attctaccta ataatattga acggaattga tgttttggac cacttagaaa 8640
   gtattttttt tttggtttgt cttagctgta tttcattaaa tataaattta aataagaaat 8700
   gtcataaata aaatttgacg tatagatttt ttaaatccat tttatgttat ttaatatttg 8760
   aaatgtgagt ttggctccta tttaatctta ggatgggtta atactaagtt ttccttaatg 8820
   aattatctca gagaaactgg attaaataaa ctaaaaaata gatcaatgtg ttttggtccg 8880
   gtcaaatatc tttggattta ctattattgg cgaaaagaaa gtctcatata gtaaatcata 8940
   ttcctacaag agaaatcaaa atttttgaat taacatggat tgtatagttt cttatataac 9000
   caattagttc gcatcaagaa aaccaaaccc caattaataa tcaaacgggc ttggtaggaa 9060
   tatttcattg cagctttcag ataaaagaaa aaaacacaca ctcaagtctt ttatttcatc 9120
   tttcttactt gcaggaactc aaattccact ttgccacttt tctttacaaa taaacacaaa 9180
   ttgtcaatga aacgaaatag tctttttatg caaacactgt ttgtcttttt tcgatcacgt 9240
   ttctgattgt gacagccatc catatatata gggaatgtaa aacaacaaca tgtgaagtca 9300
   catatacgta atggtttagc atagcttcta ttttcgttgt caatattagt cattccaaaa 9360
   catttttaag aaaaataaat taatatatgt atattcttgg aactaatgta tgtggaaata 9420
   cagtaactta attattaaac attctaaatg caaatatgca aagaaaaaaa agaaaagaac 9480
   acaactgaaa tcaaagccag attcataata attggctaca tggttgtaga atgtagggta 9540
   acacaacatc cagaattgaa cactcaaatt ggatgataga tggataatct ttagatacaa 9600
   gagaattggt tctcttccat tattaacgaa aataaagaaa aaaagtttag cataaaagtt 9660
   tgaaactcaa cataacattt tgaacttgac tccttcatag gagtgacatg aactgacgaa 9720
   tcacaaccga ttacttgttt gagtcatctt ccgctttctc caccttcgaa atgaatgtga 9780
   ccggtttctt cgggtgctca tttacggtca agtgtaaaac atctggtctc gacgagct 9838
<210> 58
   <211> 14184
   <212> DNA
   <213> Künstliche Sequenz
<220>
   <223> Beschreibung der künstlichen Sequenz: Expression vector pSUN1-codA-RNAi-At.Act.-2-At.Als-R-ocsT
<400> 58
<210> 59
   <211> 1011
   <212> DNA
   <213> Zea mays
<220>
   <221> CDS
   <222> (1)..(981)
   <223> coding for 5- methlythioribose kinase
<400> 59
   gca cga gca ctc ctc tcc tct cct ctc gcc ggc gca tcg ccc gac tgt      48
   Ala Arg Ala Leu Leu Ser Ser Pro Leu Ala Gly Ala Ser Pro Asp Cys
    1               5                  10                  15
   cag tca gcc tca gcc atg gcc gcg gag gag gag cag ggc ttc cgc ccg      96
   Gln Ser Ala Ser Ala Met Ala Ala Glu Glu Glu Gln Gly Phe Arg Pro
               20                  25                  30
   ctg gac gag tcg tcc ctg ctc gcc tac atc aag gcc acg ccg gcg ctc     144
   Leu Asp Glu Ser Ser Leu Leu Ala Tyr Ile Lys Ala Thr Pro Ala Leu
           35                  40                  45
   gcc tcc cgc ctc ggc ggc ggt ggc agt cta gac tcc atc gag atc aag     192
   Ala Ser Arg Leu Gly Gly Gly Gly Ser Leu Asp Ser Ile Glu Ile Lys
       50                  55                  60
   gag gtc ggc gac ggc aac ctc aac ttc gtc tac atc gtg cag tcc gag     240
   Glu Val Gly Asp Gly Asn Leu Asn Phe Val Tyr Ile Val Gln Ser Glu
   65                  70                  75                  80
   gcc ggc gcc atc gtc gtc aag cag gcg ctc ccg tac gtg cgc tgc gtg     288
   Ala Gly Ala Ile Val Val Lys Gln Ala Leu Pro Tyr Val Arg Cys Val
                   85                  90                  95
   ggg gat tcg tgg ccc atg acg cgg gag cgc gcc tac ttc gag gcc tcc     336
   Gly Asp Ser Trp Pro Met Thr Arg Glu Arg Ala Tyr Phe Glu Ala Ser
               100                 105                 110
   acg ctg cgg gag cac ggc cgc ctg tgc ccg gag cac acc ccc gag gtg     384
   Thr Leu Arg Glu His Gly Arg Leu Cys Pro Glu His Thr Pro Glu Val
           115                 120                 125
   tac cac ttc gac cgg acc ttg tcg ctg atg ggg atg cgc tac atc gag     432
   Tyr His Phe Asp Arg Thr Leu Ser Leu Met Gly Met Arg Tyr Ile Glu
       130                 135                 140
   ccc ccg cac atc atc ctc cgc aag ggc ctc gtc gcc ggt gtc gag tac     480
   Pro Pro His Ile Ile Leu Arg Lys Gly Leu Val Ala Gly Val Glu Tyr
   145                 150                 155                 160
   ccg ctg ctc gcc gac cac atg tcc gat tac atg gcc aag acg ctc ttc     528
   Pro Leu Leu Ala Asp His Met Ser Asp Tyr Met Ala Lys Thr Leu Phe
                   165                 170                 175
   ttc acc tcc ctc ctc tat aac aat acc acg gat cat aag aac gga gtt     576
   Phe Thr Ser Leu Leu Tyr Asn Asn Thr Thr Asp His Lys Asn Gly Val
               180                 185                 190
   gct aag tac tct gcg aac gtg gag atg tgt agg ctc acg gag caa gtt      624
   Ala Lys Tyr Ser Ala Asn Val Glu Met Cys Arg Leu Thr Glu Gln Val
           195                 200                 205
   gtg ttc tcg gac cca tac cgt gtt tcc aaa ttt aat cgg tgg acc tcg      672
   Val Phe Ser Asp Pro Tyr Arg Val Ser Lys Phe Asn Arg Trp Thr Ser
       210                 215                 220
   cct tat ctc gac aaa gat gct gag gca gtt cgc gag gat gat gag ctc      720
   Pro Tyr Leu Asp Lys Asp Ala Glu Ala Val Arg Glu Asp Asp Glu Leu
   225                 230                 235                 240
   aag ttg gaa gta gct ggg ctg aaa tcg atg ttt atc gag aga gct caa      768
   Lys Leu Glu Val Ala Gly Leu Lys Ser Met Phe Ile Glu Arg Ala Gln
                   245                 250                 255
   gct ctg att cat gga gat ctc cac act ggt tct atc atg gtg acc gaa      816
   Ala Leu Ile His Gly Asp Leu His Thr Gly Ser Ile Met Val Thr Glu
               260                 265                 270
   gtt caa ctc aag tca ttg atc cag aat ttg ggt tct atg ggg cca atg      864
   Val Gln Leu Lys Ser Leu Ile Gln Asn Leu Gly Ser Met Gly Pro Met
           275                 280                 285
   ggg ttt gat att ggg agc ctt cct tgg aaa cct gat ttt ggg cat act      912
   Gly Phe Asp Ile Gly Ser Leu Pro Trp Lys Pro Asp Phe Gly His Thr
       290                 295                 300
   atg cac aga atg ggc atg ctg atc aag cga atg atc gta agg ctt aca      960
   Met His Arg Met Gly Met Leu Ile Lys Arg Met Ile Val Arg Leu Thr
   305                 310                 315                 320
   aga atg gat ctt gaa gac aat tgaagagtcg tggaatttgt tccacaaaaa        1011
   Arg Met Asp Leu Glu Asp Asn
                   325
<210> 60
   <211> 327
   <212> PRT
   <213> Zea mays
<400> 60
   Ala Arg Ala Leu Leu Ser Ser Pro Leu Ala Gly Ala Ser Pro Asp Cys
   1               5                   10                  15
   Gln Ser Ala Ser Ala Met Ala Ala Glu Glu Glu Gln Gly Phe Arg Pro
               20                  25                  30
   Leu Asp Glu Ser Ser Leu Leu Ala Tyr Ile Lys Ala Thr Pro Ala Leu
           35                  40                  45
   Ala Ser Arg Leu Gly Gly Gly Gly Ser Leu Asp Ser Ile Glu Ile Lys
       50                  55                  60
   Glu Val Gly Asp Gly Asn Leu Asn Phe Val Tyr Ile Val Gln Ser Glu
   65                  70                  75                  80
   Ala Gly Ala Ile Val Val Lys Gln Ala Leu Pro Tyr Val Arg Cys Val
                   85                  90                  95
   Gly Asp Ser Trp Pro Met Thr Arg Glu Arg Ala Tyr Phe Glu Ala Ser
               100                 105                 110
   Thr Leu Arg Glu His Gly Arg Leu Cys Pro Glu His Thr Pro Glu Val
           115                 120                 125
   Tyr His Phe Asp Arg Thr Leu Ser Leu Met Gly Met Arg Tyr Ile Glu
       130                 135                 140
   Pro Pro His Ile Ile Leu Arg Lys Gly Leu Val Ala Gly Val Glu Tyr
   145                 150                 155                 160
   Pro Leu Leu Ala Asp His Met Ser Asp Tyr Met Ala Lys Thr Leu Phe
                   165                 170                 175
   Phe Thr Ser Leu Leu Tyr Asn Asn Thr Thr Asp His Lys Asn Gly Val
               180                 185                 190
   Ala Lys Tyr Ser Ala Asn Val Glu Met Cys Arg Leu Thr Glu Gln Val
           195                 200                 205
   Val Phe Ser Asp Pro Tyr Arg Val Ser Lys Phe Asn Arg Trp Thr Ser
       210                 215                 220
   Pro Tyr Leu Asp Lys Asp Ala Glu Ala Val Arg Glu Asp Asp Glu Leu
   225                 230                 235                 240
   Lys Leu Glu Val Ala Gly Leu Lys Ser Met Phe Ile Glu Arg Ala Gln
                   245                 250                 255
   Ala Leu Ile His Gly Asp Leu His Thr Gly Ser Ile Met Val Thr Glu
               260                 265                 270
   Val Gln Leu Lys Ser Leu Ile Gln Asn Leu Gly Ser Met Gly Pro Met
           275                 280                 285
   Gly Phe Asp Ile Gly Ser Leu Pro Trp Lys Pro Asp Phe Gly His Thr
       290                 295                 300
   Met His Arg Met Gly Met Leu Ile Lys Arg Met Ile Val Arg Leu Thr
   305                 310                 315                 320
   Arg Met Asp Leu Glu Asp Asn
                   325
<210> 61
   <211> 471
   <212> DNA
   <213> Brassica napus
<220>
   <221> CDS
   <222> (2)..(469)
   <223> coding for 5- methlythioribose kinase
<400> 61
   a ttt ccg ggt cga cga ttt cgt ggc aat ctc aac ttc gtt ttc atc gtc 49
     Phe Pro Gly Arg Arg Phe Arg Gly Asn Leu Asn Phe Val Phe Ile Val
     1               5                   10                  15
   atc gga tcc act ggc tca ctc gtc atc aaa cag gcg ctt ccg tat ata   97
   Ile Gly Ser Thr Gly Ser Leu Val Ile Lys Gln Ala Leu Pro Tyr Ile
               20                  25                  30
   cgt tgt att ggg gag tct tgg cca atg acg aaa gaa aga gct tac ttt   145
   Arg Cys Ile Gly Glu Ser Trp Pro Met Thr Lys Glu Arg Ala Tyr Phe
           35                  40                  45
   gaa gct aca act ctg aga aag cac gga gct ttg tct cct gat cat gtt   193
   Glu Ala Thr Thr Leu Arg Lys His Gly Ala Leu Ser Pro Asp His Val
       50                  55                  60
   cct gaa gtc tac cat ttt gac agg acc atg gct ttg att gga atg agg   241
   Pro Glu Val Tyr His Phe Asp Arg Thr Met Ala Leu Ile Gly Met Arg
   65                  70                  75                  80
   tat ctg gag cct cct cac atc atc ctc cgc aaa gga ctc gtt gct gga   289
   Tyr Leu Glu Pro Pro His Ile Ile Leu Arg Lys Gly Leu Val Ala Gly
                   85                  90                  95
   atc cag tac cct ttc ctt gca gaa cac atg gct gat tac atg gcc aaa   337
   Ile Gln Tyr Pro Phe Leu Ala Glu His Met Ala Asp Tyr Met Ala Lys
               100                 105                 110
   acc ctc ttc ttc act tcg ctc ctc tat cat gat acc aca gag cac aaa   385
   Thr Leu Phe Phe Thr Ser Leu Leu Tyr His Asp Thr Thr Glu His Lys
           115                 120                 125
   aga gca gta acc gag ttt tgt ggt aat gtg gag tta tgc cgg tta acg   433
   Arg Ala Val Thr Glu Phe Cys Gly Asn Val Glu Leu Cys Arg Leu Thr
       130                 135                 140
   gag caa gta gtg ttc tct gac ccg tat aga gtt tct ag                471
   Glu Gln Val Val Phe Ser Asp Pro Tyr Arg Val Ser
   145                 150                 155
<210> 62
   <211> 156
   <212> PRT
   <213> Brassica napus
<400> 62
   Phe Pro Gly Arg Arg Phe Arg Gly Asn Leu Asn Phe Val Phe Ile Val
   1               5                   10                  15
   Ile Gly Ser Thr Gly Ser Leu Val Ile Lys Gln Ala Leu Pro Tyr Ile
               20                  25                  30
   Arg Cys Ile Gly Glu Ser Trp Pro Met Thr Lys Glu Arg Ala Tyr Phe
           35                  40                  45
   Glu Ala Thr Thr Leu Arg Lys His Gly Ala Leu Ser Pro Asp His Val
       50                  55                  60
   Pro Glu Val Tyr His Phe Asp Arg Thr Met Ala Leu Ile Gly Met Arg
   65                  70                  75                  80
   Tyr Leu Glu Pro Pro His Ile Ile Leu Arg Lys Gly Leu Val Ala Gly
                   85                  90                  95
   Ile Gln Tyr Pro Phe Leu Ala Glu His Met Ala Asp Tyr Met Ala Lys
               100                 105                 110
   Thr Leu Phe Phe Thr Ser Leu Leu Tyr His Asp Thr Thr Glu His Lys
           115                 120                 125
   Arg Ala Val Thr Glu Phe Cys Gly Asn Val Glu Leu Cys Arg Leu Thr
       130                 135                 140
   Glu Gln Val Val Phe Ser Asp Pro Tyr Arg Val Ser
   145                 150                 155
<210> 63
   <211> 415
   <212> DNA
   <213> Brassica napus
<220>
   <221> CDS
   <222> (3)..(413)
   <223> coding for 5- methlythioribose kinase
<400> 63
   gg gtc gac gat ttc gtg ctg aga gca aaa gag atg tcg ttc gat gag      47
      Val Asp Asp Phe Val Leu Arg Ala Lys Glu Met Ser Phe Asp Glu
      1               5                   10                  15
   ttc aag ccg ttg aac gag aaa tct cta gta gag tac ata aag gca acg     95
   Phe Lys Pro Leu Asn Glu Lys Ser Leu Val Glu Tyr Ile Lys Ala Thr
                   20                  25                  30
   cct gcc ctc tcc tcc agg ctc gga gac aag tac gat gat ctg gtc atc     143
   Pro Ala Leu Ser Ser Arg Leu Gly Asp Lys Tyr Asp Asp Leu Val Ile
               35                  40                  45
   aag gaa gtt gga gat ggc aat ctc aac ttc gtt ttc atc gtt gtc gga     191
   Lys Glu Val Gly Asp Gly Asn Leu Asn Phe Val Phe Ile Val Val Gly
           50                  55                  60
   tcc act ggc tca ctc gtc atc aaa cag gcg ctt ccg tat ata cgt tgt     239
   Ser Thr Gly Ser Leu Val Ile Lys Gln Ala Leu Pro Tyr Ile Arg Cys
       65                  70                  75
   att gga gaa tca tgg cca atg acg aaa gaa aga gct tac ttt gaa gca     287
   Ile Gly Glu Ser Trp Pro Met Thr Lys Glu Arg Ala Tyr Phe Glu Ala
   80                  85                  90                  95
   aca act ctg aga aag cac ggt ggt ttg tct ccg gat cat gtt cct gaa     335
   Thr Thr Leu Arg Lys His Gly Gly Leu Ser Pro Asp His Val Pro Glu
                   100                 105                 110
   gtc tac cat ttt gac aga acc atg gct ttg att gga atg aga tac ctc     383
   Val Tyr His Phe Asp Arg Thr Met Ala Leu Ile Gly Met Arg Tyr Leu
               115                 120                 125
   gag cct cct cac atc atc ctc cgc aaa gga ct                          415
   Glu Pro Pro His Ile Ile Leu Arg Lys Gly
           130                 135
<210> 64
   <211> 137
   <212> PRT
   <213> Brassica napus
<400> 64
   Val Asp Asp Phe Val Leu Arg Ala Lys Glu Met Ser Phe Asp Glu Phe
   1               5                   10                  15
   Lys Pro Leu Asn Glu Lys Ser Leu Val Glu Tyr Ile Lys Ala Thr Pro
               20                  25                  30
   Ala Leu Ser Ser Arg Leu Gly Asp Lys Tyr Asp Asp Leu Val Ile Lys
           35                  40                  45
   Glu Val Gly Asp Gly Asn Leu Asn Phe Val Phe Ile Val Val Gly Ser
       50                  55                  60
   Thr Gly Ser Leu Val Ile Lys Gln Ala Leu Pro Tyr Ile Arg Cys Ile
   65                  70                  75                  80
   Gly Glu Ser Trp Pro Met Thr Lys Glu Arg Ala Tyr Phe Glu Ala Thr
                   85                  90                  95
   Thr Leu Arg Lys His Gly Gly Leu Ser Pro Asp His Val Pro Glu Val
               100                 105                 110
   Tyr His Phe Asp Arg Thr Met Ala Leu Ile Gly Met Arg Tyr Leu Glu
           115                 120                 125
   Pro Pro His Ile Ile Leu Arg Lys Gly
       130                 135
<210> 65
   <211> 424
   <212> DNA
   <213> Oryza sativa
<220>
   <221> CDS
   <222> (3)..(422)
   <223> coding for 5- methlythioribose kinase
<400> 65
   cc ctt ctc tac aac tcc acc act gat cac aag aaa gga gtt gct cag     47
      Leu Leu Tyr Asn Ser Thr Thr Asp His Lys Lys Gly Val Ala Gln
      1               5                   10                  15
   tac tgc gat aat gtg gag atg tgt agg ctc aca gag caa gtc gtg ttc    95
   Tyr Cys Asp Asn Val Glu Met Cys Arg Leu Thr Glu Gln Val Val Phe
                   20                  25                  30
   tca gac cca tac atg ctc gcc aaa tac aat cgt tgc aca tca ccc ttc   143
   Ser Asp Pro Tyr Met Leu Ala Lys Tyr Asn Arg Cys Thr Ser Pro Phe
               35                  40                  45
   cta gat aat gat gct gca gcg gtt cga gag gat gct gag ctt aaa ttg   191
   Leu Asp Asn Asp Ala Ala Ala Val Arg Glu Asp Ala Glu Leu Lys Leu
           50                  55                  60
   gag att gct gaa ttg aaa tca atg ttt att gag aga gca cag gct ctt   239
   Glu Ile Ala Glu Leu Lys Ser Met Phe Ile Glu Arg Ala Gln Ala Leu
       65                  70                  75
   ctt cat gga gat ctc cac act ggt tcc atc atg gtg aca cca gat tct   287
   Leu His Gly Asp Leu His Thr Gly Ser Ile Met Val Thr Pro Asp Ser
   80                  85                  90                  95
   act caa gtg att gat cca gaa ttt gct ttc tat ggc cca atg ggt tac   335
   Thr Gln Val Ile Asp Pro Glu Phe Ala Phe Tyr Gly Pro Met Gly Tyr
                   100                 105                 110
   gac att ggg gcc ttc ctg ggg aac ttg att ttg gca tat ttt tca caa   383
   Asp Ile Gly Ala Phe Leu Gly Asn Leu Ile Leu Ala Tyr Phe Ser Gln
               115                 120                 125
   gat gga cac gct gat caa gca aat gat cgt aag gct tac aa            424
   Asp Gly His Ala Asp Gln Ala Asn Asp Arg Lys Ala Tyr
           130                 135                 140
<210> 66
   <211> 140
   <212> PRT
   <213> Oryza sativa
<400> 66
   Leu Leu Tyr Asn Ser Thr Thr Asp His Lys Lys Gly Val Ala Gln Tyr
   1               5                   10                  15
   Cys Asp Asn Val Glu Met Cys Arg Leu Thr Glu Gln Val Val Phe Ser
               20                  25                  30
   Asp Pro Tyr Met Leu Ala Lys Tyr Asn Arg Cys Thr Ser Pro Phe Leu
           35                  40                  45
   Asp Asn Asp Ala Ala Ala Val Arg Glu Asp Ala Glu Leu Lys Leu Glu
       50                  55                  60
   Ile Ala Glu Leu Lys Ser Met Phe Ile Glu Arg Ala Gln Ala Leu Leu
   65                  70                  75                  80
   His Gly Asp Leu His Thr Gly Ser Ile Met Val Thr Pro Asp Ser Thr
                   85                  90                  95
   Gln Val Ile Asp Pro Glu Phe Ala Phe Tyr Gly Pro Met Gly Tyr Asp
               100                 105                 110
   Ile Gly Ala Phe Leu Gly Asn Leu Ile Leu Ala Tyr Phe Ser Gln Asp
           115                 120                 125
   Gly His Ala Asp Gln Ala Asn Asp Arg Lys Ala Tyr
       130                 135                 140
<210> 67
   <211> 404
   <212> DNA
   <213> Glycine max
<220>
   <221> CDS
   <222> (3)..(404)
   <223> coding for 5- methlythioribose kinase
<400> 67
   ta atc ccc gaa cat gtt cct gaa gtg tat cac ttt gac cgt acc atg      47
      Ile Pro Glu His Val Pro Glu Val Tyr His Phe Asp Arg Thr Met
      1               5                   10                  15
   tct ttg atc ggt atg cgt tac ttg gag ccc cca cat ata atc ctc ata      95
   Ser Leu Ile Gly Met Arg Tyr Leu Glu Pro Pro His Ile Ile Leu Ile
                   20                  25                  30
   aaa ggg ttg att gct ggg att gag tac cct ttt ttg gct gaa cac atg     143
   Lys Gly Leu Ile Ala Gly Ile Glu Tyr Pro Phe Leu Ala Glu His Met
               35                  40                  45
   gct gat ttc atg gcg aag aca ctc ttc ttc acg tct ctg ctt ttc cgt     191
   Ala Asp Phe Met Ala Lys Thr Leu Phe Phe Thr Ser Leu Leu Phe Arg
           50                  55                  60
   tcc act gct gac cac aaa cgg gac gtt gcc gaa ttt tgt ggg aat gtg     239
   Ser Thr Ala Asp His Lys Arg Asp Val Ala Glu Phe Cys Gly Asn Val
       65                  70                  75
   gag tta tgc agg ctc act gaa cag gtc gtt ttc tct gac cct tat aaa     287
   Glu Leu Cys Arg Leu Thr Glu Gln Val Val Phe Ser Asp Pro Tyr Lys
   80                  85                  90                  95
   gtt tct caa tat aat cgt tgg act tcc ccc tat ctt gat cgt gat gct     335
   Val Ser Gln Tyr Asn Arg Trp Thr Ser Pro Tyr Leu Asp Arg Asp Ala
               100                 105                 110
   gag gct gtt cgg gaa gac aat ctg ctg aag ctt gaa gtt gct gag ctg     383
   Glu Ala Val Arg Glu Asp Asn Leu Leu Lys Leu Glu Val Ala Glu Leu
           115                 120                 125
   aaa tcc aag ttc att gag agc                                         404
   Lys Ser Lys Phe Ile Glu Ser
           130
<210> 68
   <211> 134
   <212> PRT
   <213> Glycine max
<400> 68
   Ile Pro Glu His Val Pro Glu Val Tyr His Phe Asp Arg Thr Met Ser
   1               5                   10                  15
   Leu Ile Gly Met Arg Tyr Leu Glu Pro Pro His Ile Ile Leu Ile Lys
               20                  25                  30
   Gly Leu Ile Ala Gly Ile Glu Tyr Pro Phe Leu Ala Glu His Met Ala
           35                  40                  45
   Asp Phe Met Ala Lys Thr Leu Phe Phe Thr Ser Leu Leu Phe Arg Ser
       50                  55                  60
   Thr Ala Asp His Lys Arg Asp Val Ala Glu Phe Cys Gly Asn Val Glu
   65                  70                  75                  80
   Leu Cys Arg Leu Thr Glu Gln Val Val Phe Ser Asp Pro Tyr Lys Val
                   85                  90                  95
   Ser Gln Tyr Asn Arg Trp Thr Ser Pro Tyr Leu Asp Arg Asp Ala Glu
               100                 105                 110
   Ala Val Arg Glu Asp Asn Leu Leu Lys Leu Glu Val Ala Glu Leu Lys
           115                 120                 125
   Ser Lys Phe Ile Glu Ser
       130
<210> 69
   <211> 21
   <212> DNA
   <213> Künstliche Sequenz
<220>
   <223> Beschreibung der künstlichen Sequenz: oligonucleotide primer
<400> 69
   cgtgaatacg gcgtggagtc g 21
<210> 70
   <211> 20
   <212> DNA
   <213> Künstliche Sequenz
<220>
   <223> Beschreibung der künstlichen Sequenz: oligonucleotide primer
<400> 70
   cggcaggata atcaggttgg 20
<210> 71
   <211> 20
   <212> DNA
   <213> Künstliche Sequenz
<220>
   <223> Beschreibung der künstlichen Sequenz: oligonucleotide primer
<400> 71
   gtcaacgtaa ccaaccctgc 20

## Beschreibung

Die vorliegende Erfindung betrifft die Verwendung eines doppelaträngigem RNA-Molekuls, umfassend a) einen "sense"-RNA-Strang umfassend mindestens eine Ribonukleotidsequenz, die im wesentlichen identisch ist zu mindestens einen Teil des "sense"-RNA-Transkriptes einer Nukleinsauresequenz kodierend für ein Markerprotein das direkt oder indirekt einen toxischen Effekt bewirken kann, und b) einen "antisense"-RNA-Strang, der zu dem ENA-sense-Strang unter a) im wesentlichen - bevorzugt vollständig - komplementären ist, zur Verminderung der Expression, Menge, Aktivität und/oder Funktion besagtem Markerproteins in einer transformierten pflanzlichen zelle, die infolge der Wirkung des doppelsträngigem RNA-Moleküls gegenüber nicht-transformierten Zellen einen Wachstumsvorteil hat und so selektiert werden kann, wobei die Selektion unter Bedingungen durchgeführt wird, bei denen das Markerprotein seinen toxischen Effekt auf die nicht-transformierten Zellen ausüben kann.

Die Einführung genetischen Materiales in Zielzellen gelingt meist nur in einer sehr begrenzten Anzahl von Zellen einer Population. Dies macht die Unterscheidung und Isolierung von erfolgreich transformierten von nicht-transformierten Zellen erforderlich, ein Verfahren das als Selektion bezeichnet wird. Traditionell erfolgt die Selektion mittels einer sogenannten positiven Selektion, wobei die transformierte Zelle in die Lage versetzt wird, zu wachsen und zu uberleben, wohingegen die untransformierte Zelle im Wachstum gehemmt oder abgetötet wird (McCormick et al. (1986) Plant Cell Reports 5:81-84). Üblicherweise wird eine derartige positive Selektion durch Gene realisiert, die für eine Resistenz gegen ein Biozid kodieren (z.B. ein Herbizid wie Phosphinothricin, Glyphosat oder Bromoxynil, einen Metabolismusinhibitor wie 2-Desoxyglucose-6-phosphat (WO 98/45456) oder ein Antibiotikum wie Tetracyclin, Ampicillin, Kanamycin, G 418, Neomycin, Bleomycin oder Hygromycin). Derartige Gene werden auch als positive Selektionsmarker bezeichnet. Der positive Selektionsmarker wird gekoppelt (physikalisch oder mittels Cotransformation) mit der in das Zellgenom einzuführenden Nukleinsäurenequenz in die Zelle eingebracht, Anschließend werden die Zellen auf einem Medium unter dem entsprechenden Selektionsdruck (z.B. in Gegenwart eines entsprechenden Antibiotikums oder Herbizids) kultiviert, wodurch die transformierten Zellen aufgrund der erworbenen Resistenz gegen besagten Selektionsdruck einen Wachstums-/Überlebersvorteil haben und so selektioniert werden können. Beispielhaft als positive Selektionsmarker seien genannt:
- Phosphinothricinacetyltransferasen (PAT) (auch Bialophos^{®}-Resistenz; bar) acetylieren die freie Aminogruppe des Glutaminsynthaseinhibitors Phosphinothricin (PPT) und erreichen damit eine Detoxifizierung (de Block et al. (1987) EMBO J 6:2513-2518; Vickers JE et al. (1996) Plant Mol Biol Reporter 14:363-368; Thompson CJ et al. (1987) EMBO J 6:2519-2523).
- 5-Enolpyruvylshikimat-3-phosphatsynthasen (EPSPS) verleihen eine Resistenz gegen das unselektive Herbizid Glyphosat^{®} (N-(Phosphonomethyl)glycin; Steinrucken HC et al. (1980) Biochem Biophys Res Commun 94:1207-1212; Levin JG und Sprinson DB (1964) J Biol Chem 239:1142-1150; Cole DJ (1985) Mode of action of glyphosate; A literature analysis, p. 48-74. In: Grossbard E und Atkinson D (eds.) The herbicide glyphosate. Buttersworths, Boston.). Glyphosat-tolerante EPSPS Varianten zur Verwendung als Selektionsmarker sind beschrieben (Padgette SR et al. (1996). New weed control opportunities: development of soybeans with a Roundup Ready™ gene. In: Herbicide Resistant Crops (Duke SO, ed.), pp. 53-84. CRC Press, Boca Raton, FL; Saroha MK und Malik VS (1998) J Plant Biochemistry and Biotechnology 7:65-72; Padgette SR et al.(1995) Crop Science 35(5):1451-1461; US 5,510,471; US 5,776,760; US 5,864,425; US 5,633,435; US 5,627;061; US 5,463,175; EP-A 0 218 571).
- Neomycinphosphotransferasen verleihen eine Resistenz gegen Aminoglykosid-Antibiotika wie Neomycin, G418, Hygromycin, Paromomycin oder Kanamycin, indem sie durch eine Phosphorylierungsreaktion deren inhibierende Wirkung reduzieren (Beck et al. (1982) Gene 19:327-336).
- 2-Desoxyglukose-6-phosphatphosphatasen verleihen eine Resistenz gegen 2-Desoxyglukose (EP-A 0 807 836; Randez-Gil et al. (1995) Yeast 11:1233-1240; Sanz et al. (1994) Yeast 10:1195-1202).
- Acetolactatsynthasen verleihen eine Resistenz gegen Imidazolinon/Sulfonylharnstoff-Herbizide (z.B. Imazzamox, Imazapyr, Imazaquin, Imazethapyr, Amidosulforon, Azimsulfuron, Chlorimuronethyl, Chlorsulfuron; Sathasivan K et al. (1990) Nucleic Acids Res 18(8):2188).

Darüber hinaus sind Resistenzgene gegen die Antibiotika Hygromycin (Hygromycinphosphotransferasen), Chloramphenicol (Chloramphenicolacetyltransferase), Tetracyclin, Streptomycin, Zeocin und Ampicillin (ß-Lactamase Gen; Datta N, Richmond MH.(1966) Biochem J 98(1):204-9) beschrieben.

Gene wie die Isopentenyltransferase (ipt) aus Agrobacterium tumefaciens (strain:PO22) (Genbank Acc.-No.: AB025109) können ebenfalls als Selektionsmarker eingesetzt werden. Das ipt Gen ist ein Schlüsselenzym der Cytokin-Biosynthese, Seine Überexpression erleichtert die Regeneration von Pflanzen (z.B. Selektion auf Cytokin-freiem Medium) (Ebinuma H et al. (2000) Proc Natl Acad Sci USA 94:2117-2121; Ebinuma H et al. (2000) Selection of Marker-free transgenic plants using the oncogenes (*ipt*, *rol* A, B, C) of Agrobacterium as selectable markers, In Molecular Biology of Woody Plants. Kluwer Academic Publishers). Nachteilig ist hier zum einen, dass der Selektionsvorteil auf meist subtilen Unterschieden in der Zellproliferation beruht, zum anderen die Pflanze durch die Transformation mit einem Onkogen unerwünschte Eigenschaften (Galltumorbildung) erhält.

Verschiedene weitere positive Selektionsmarker sind in EP-A 0 601 092 beschrieben. Beispielhaft sind zu nennen: β-Glucuronidase (in Verbindung mit z.B. Cytokininglucuronid), Mannose-6-phosphat-Isomerase (in Verbindung mit Mannose), UDP-Galaktose-4-Epimerase (in Verbindung mit z.B. Galactose).

Negative Selektionsmarker werden zur Selektion von Organismen mit erfolgreich deletierten Markersequenzen eingesetzt (Koprek T et al. (1999) Plant J 19(6):719-726). In Gegenwart eines negativen Selektionsmarkers wird die entsprechende Zelle abgetötet oder erfährt einen Wachstumsnachteil. Bei der negativen Selektion wird beispielsweise durch den in die Pflanze eingebrachten negativen Selektionsmarker eine Verbindung, die ansonsten für die Pflanze keine nachteilige Wirkung hat, in eine Verbindung mit nachteiliger (d.h. toxischer) Wirkung umgesetzt. Beispiele für negative Selektionsmarker umfassen: Thymidinkinase (TK) z.B. des Herpes Simplex Virus (Wigler et al. (1977) Cell 11:223), zelluläre Adeninphosphoribosyltransferase (APRT) (Wigler et al. (1979) Proc Natl Acad Sci USA 76:1373), Hypoxanthinphosphoribosyltransferase (HPRT) (Jolly et al. (1983) Proc Natl Acad Sci USA 80:477), Diphtheria Toxin A Fragment (DT-A), die bakterielle Xanthin-Guaninphosphoribosyltransferase (gpt; Besnard et al. (1987) Mol. Cell. Biol. 7:4139; Mzoz and Moolten (1993) Human Gene Therapy 4:589-595), das codA Genprodukt kodierend für eine Cytosindeaminase (Gleave AP et al. (1999) Plant Mol Biol. 40(2):223-35; Perera RJ et al. (1993) Plant Mol Biol 23(4): 793-799; Stougaard J; (1993) Plant J 3:755-761; EP-A1 595 873), das Cytochrom P450 Gen (Koprek et al. (1999) Plant J 16:719-726), Gene kodierend für eine Haloalkandehalogenase (Naested H (1999) Plant J 18:571-576), das iaaH Gen (Sundaresan V et al. (1995) Genes & Development 9:1797-1810) oder das tms2 Gen (Fedoroff NV & Smith DL (1993) Plant J 3: 273-289). Die negativen Selektionsmarker werden meist in Kombination mit sogenannten "Prodrugs" oder "Pro-Toxinen" eingesetzt, Verwindungen die durch die Aktivität des Selektionsmarkers in Toxine ungesetzt werden.

5-Methylthioribose-(MTR)-kinase ist ein Enzym, dessen enzymatische Aktivität nicht in Säugern, wohl aber in Pflanzen, Bakterien und Protozoen beschrieben ist. Das Enzym kann ein MTR-Analog (5-(Trifluoromethyl)thioriboso) als sogenanntes "subversives Substrat" des Methionin-Ausweich-Stoffwechselwegs ("Salvage Pathway") über ein instabiles Intermediat zu der toxischen Verbindung Carbothionyldifluorid umsetzen.

Besagte Selektionssysteme haben verschiedene Nachteile. Der eingebracht Selektionsmarker (z.B. Antibiotikaresistenz) hat seine Berechtigung allein während der Transformation und Selektion stellt jedoch später ein in der Regel unnotiges und oft auch unerwinschtes Proteinprodukt dar. Dies kann aus Grunden der Verbraucherakzeptanz und/oder der Zulassung als Lebens- und/oder Futtermittel unvorteilhaft sein. Nachteilig ist in diesem Zusammenhang ferner, dass der zur Selektion verwendete Selektionsmarker in der Regel genetisch mit der in das Genom zu insertierenden Nukleinsäuresequenz gekoppelt ist und nicht durch Segregation im Rahmen der Vermehrung oder Kreuzung entkoppelt werden kann. In der Regel ist eine Deletion der Markersequenz erforderlich, was zusätzlich Arbeitsschritte erfordert. Daruberhinaus erfordern biotechnologische Arbeiten in zahlreichen Fällen eine Mehrfachtransformation mit verschiedenen Cenkonstrukten. Hier ist für jeden Transformationsschritt ein neuer Selektionsmarker erforderlich, wenn nicht der zuvor verwendete zunächst mühsam deletiert werden soll. Dies erfordert jedoch eine breite Palette gut funktionierender Selektionsmarker, die für die meisten pflanzlichen Organismen nicht zur Verfügung stehen.

Es stellte sich folglich die Aufgabe, neue Selektionsverfahren zur Selektions transformierter pflanzlicher Zellen und Organismen bereitzustellen, die möglichst die Nachteile der vorhandenen Systeme nicht mehr aufweisen. Diese Aufgabe wird durch die vorliegende Erfindung gelöst.

Ein ester Gegenstand der Erfindung betrifft die Verwendung eines doppelsträngigem RNA Moleküls, umfassend a) einen "sense"-RNA-Strang umfassend mindestens eine Ribonuklectidsequenz, die im wesentlichen identisch ist zu mindestens einem Teil des "sense"-RNA-Transkriptes einer Nukleinsäuresequenz kodierend für ein Markerprotein das direkt oder indirekt einen toxischen Effekt bewirken kann, und b) einen "antisense"-RNA-Strang, der zu dem RNA-sense-Strang unter a) im wesentlichen - bevorzugt vollstandig - komplementären ist, zur Verminderung der Expression, Menge, Aktivität und/oder Funktion besagtem Markerproteins in einer transformierten pflanzlichen Zelle, die infolge der Wirkung des doppelsträngigem RNA-Moleküls gegenüber nicht-transformierten zeilen einen Wachstumsvorteil hat und so selektiert werden kann, wobei die Selektion unter Bedingungen durchgeführt wird, bei denen das Markerprotein seinen toxischen Effekt auf die nicht-transformierten Zellen ausüben kann. Ein solches doppelsträngiges DNA Molekül wird auch als "MP-dsRNA" bezeichnet.

In einer bevorzugten Ausführungsform 1st das Markerprotein ein Protein, dass in der Lage ist, eine für besagte population pflanzlicher Zeiten nicht-toxische Substanz X in eine für besagte Population toxische Substanz Y direkt oder indirekt umzusetzen. Die erfindungsgemäße Verwendung umfasst in diesem Fall bevorzugt nachfolgende Schritte:
a) Transformation der Population pflanzlicher Zellen mit mindestens einer zu insertierenden Nukleinsäuresequenz in Kombination mit mindestens einer Verbindung befahigt zur Verminderung der Expression, Menge, Aktivität und/oder Funktion mindestens eines Markerproteins, und
b) Behandlung besagter Population pflanzlicher Zellen mit der Substanz X in einer Konzentration, die infolge der Umsetzung durch das Markerprotein einen für nicht-transformierte Zellen toxischen Effekt bedingt, und
c) Selektion von transformierten pflanzlichen Zellen, die in ihrem Genom besagte insertierte Nukleinsäuresequenz aufweisen und die infolge der Wirkung besagter Verbindung gegenüber nicht-transformierten Zellen einen Wachstumsvorteil aufweisen, aus besagter Population pflanzlicher Zellen, wobei die Selektion unter Bedingungen durchgeführt wird, bei denen das Markerprotein seinen toxischen Effekt auf die nicht transformierten Zellen ausüben kann..

Bevorzugt handelt es sich bei der nicht-toxischen Substanz X um eine Substanz, die naturlicherweise in pflanzlichen Zellen oder Organismen nicht oder nur in Konzentration vorkommt, die im wesentlichen keinen toxischen Effekt bewirken können. Bevorzugt wird die nicht-toxische Substanz X im Rahmen des erfindungsgemäßen Verfahrens exogen z.B. über das Medium oder das wachstumssubstrat appliziert.

Der Begriff "Verbindung befähigt zur Verminderung der Expression, Menge, Aktivität und/oder Funktion mindestens eines Markerproteins" ist kann breit verstanden werden und meint allgemein alle Verbindungen, die direkt oder indirekt, alleine oder in Kooperation mit anderen Faktoren eine Verminderung der Proteinmenge, RNA-Menge, Genaktivität, Proteinaktivität - oder funktion mindestens eines Markerproteins bewirken. Besagte Verwindungen sind infolge auch unter der Bezeichnung "anti-Markerprotein"-Verbindungen zusammengefasst. Erfindungsgemäß betrifft der Begriff "Verbindung befähigt zur Verminderung der Expression, Menge, Aktivität und/oder Funktion mindestens eines Markerproteins" und der Begriff "anti-Markerprotein"-Verbindung schließt-, die im Rahmen des erfindungsgemäßen Verfahrens zum Einsatz kommenden doppelsträngigen Ribonukleinsauresequenzen,

Das erfindungsgemäße doppelsträngige RNA Molekül (dsRNA-Molekül] hebt die negativ-selektive Wirkung des Markerproteins auf. Insofern wirkt eine "anti-Markerprotein"-Verbindungen indirekt (z.B. mittels der durch die Expressionskassette exprimierten Ribonukleinsäuresequenz als positiver Selektionsmarker. Das hierein beschriebene selektionssytem sei infolge als "reverses Selektionssystem" bezeichnet, da es die negativ-selektive Wirkung des Markerproteins "revertiert".

Das "reverse Selektionssystem" Verfahren bedeutet eine sprunghafte Verbreiterung des Repertoirs an positiven Selektionsverfahren zur Selektion transformierter pflanzlicher Zellen.

Vorteilhaft ist ferner, dass durch die Wirkung der doppelsträngigen RNA der Selektionseiffekt ohne Expression eines Fremdproteins realisiert werden kann (s.u.).

Verteilhaft ist darüber hinaus, dass das zur Selektion indirekt verwendete Markerprotein (z.B. der negative Selektionsmarker) nicht genetisch mit der in das Genem zu invertierenden Nukleinsäuresequenz gekoppelt ist, Im Unterschied zu den ansonsten üblichen Selektionsverfahren kann das Markerprotein - wenn es sich um ein Transgen handelt - durch eintache Segregation im Rahmen nachfolgender Vermehrung oder Kreuzung entfernt werden.

"pflanzliche Zelle" meint im Rahmen der vorliegenden Erfindung jegliche Art von Zelle, die von einem pflanzlichen Organismus abgeleitet oder in diesem vorhanden ist. Der Begriff umfasst dabei beispielhaft Protoplasten, Kallus- oder Zellkulturen, Mikrosporen, Pollen, Zellen in Form von Geweben wie Blättern, Meristamen, Blüten, Embryonen, Wurzeln usw. Insbesondere sind all solche Zellen und Zellpopulationen umfasst, die sich als Zielgeweben für eine Transformation eignen.

"Pflanzlicher Organismus" umfasst dabei jeden Organismus, der zur Photosynthese befähigt ist, sowie die von diesem abgeleitete Zellen, Gewebe, Teile oder Vermehrungsgut (wie Samen oder Früchte). Eingeschlossen sind im Rahmen der Erfindung alle Gattungen und Arten höherer und niedrigerer Pflanzen des Pflanzenreiches. Einjährige, mehrjährige, monocotyledone und dicotyledone Pflanzen sowie Gymnospermen sind bevorzugt.

"Pflanze" im Rahmen der Erfindung meint alle Gattungen und Arten höherer und niedrigere Pflanzen des Pflanzenreiches. Eingeschlosser unter dem Begriff sind die reifen Pflanzen, Saatgut, Sprossen und Keimlinge, sowie davon abgeleitete Teile, Vermehrungsgut (zum Beispiel Knollen, Samen oder Früchtel, Pflanzenorgane, Gewebe, Protoplasten, kallus und andere Kulturen, zum Beispiel Zellkulturen, sowie alle anderen Arten von Gruppierungen von Pflanzenzellen zu funktionellen oder strukturellen Einheiten. Reite Pflanzen meint Pflanzen, zu jedem beliebigen Entwicklungsstadium jenseits des Keimlings. Keimlinge meint eine junge, unreife Pflanzen in einem frühen Entwicklungsstadium. "Pflanze" umfasst alle einjährigen und mehrjahrige, monokotyledonen und dikotyledonen Pflanzen und schließt beispielhaft jedoch nicht einschränkend solche der Gattungen Cucurbita, Rosa, Vitis, Juglans, Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersicon, Nicotiana, Solarium, Petunia, Digitalis, Majorana, Cichorium, Helianthus, Lactuca, Bromus, Asparagus, Antirrhinum, Heterocallis, Nemesis, Pelargonium, Panieum, Pennisetum, Ranunculus, Senecio, Salpiglossis, Cucumis, Browaalia, Glycine, Pisum, Phaseolus, Lolium, Oryza, Zea, Avena, Hordeum, Secale, Triticum, Sorghum, Picea und Populus ein.

Bevorzugt sind Pflanzen nachfolgender Pflanzenfamilien: Amaranthaceae, Asteraceae, Brassicaceae, Carophyllaceae, Chenopodiaceae, Compositae, Cruciferae, Cucurbitaceae, Labiatae, Leguminosae, Papilionoideae, Liliaceae, Linaceae, Malvaceae, Rosaceae, Rubiaceae, Saxifragaceae, scrophulariaceae, Solanacea, Sterculiaceae, Tetragoniacea, Theaceae, Umbelliferae.

Bevorzugte monokotyle Pflanzen sind insbesondere ausgewänlt aus den monokotylen Kulturpflanzen, wie zum Beispiel der familie der Gramineae wie Reis, Mais, Weizen oder andere Getreidearten wie Gerste, Hirse, Roggen, Triticale oder Hafer somit dem Zuckerrohr sowie allen Arten von Gräsern.

Bevorzugte dikotyle Pflanzen sind insbesondere ausgewählt aus den dikotylen Kulturpflanzen, wie zum Beispiel
- Asteraceae wie Sonnenblume, Tagetes oder Calendula und andere mehr,
- Compositae, besonders die Gattung Lactuca, ganz besonders die Art saliva (Salat) und andere mehr,
- Cruciferae, besonders die Gattung Brassica, ganz besonders die Arten napus (Raps), campestris (Rube), oleracea cv Tastie (Kohl), oleracea cv Snowball Y (Blumenkohl) und oleracea cv Emperor (Broccoli) und weitere Kohlarten; und der Gattung Arabidopsis, ganz besonders die Art thaliana sowie Kresse oder Canola und andere mehr,
- Cucurbitaceae wie Melone, Kürbis oder Zucchini und andere mehr,
- Leguminosae besonders die Gattung Glycine, ganz besonders die Art max (Sojabohne) sowie Alfalfa, Erbse, Bohnengewächsen oder Erdnuss und andere mehr
- Rubiaceae, bevorzugt der Unterklasse Lamiidae wie beispielsweise Coffea arabica oder Coffea liberica (Kaffestrauch) und andere mehr,
- Solanaceae besonders die Gattung Lycopersicon, ganz besonders die Art esculentum (Tomate), die Gattung Solanum, ganz besonders die Art tuberosum (Kartoffel) und melongena (Aubergine) und die Gattung Capsicum, ganz besonders die Art annum (Pfeffer) sowie Tabak und andere mehr,
- Sterculiaceae, bevorzugt der Unterklasse Dilleniidae wie beispielsweise Theobroma cacao (Kakaostrauch) und andere mehr,
- Theaceae, bevorzugt der Unterklasse Dilleniidae wie beispielsweise Camellia sinensis oder Thea sinensis (Teestrauch) und andere mehr,
- Umbelliferae, besonders die Gattung Daucus (ganz besonders die Art carota (Karrotte)) und Apium (ganz besonders die Art graveolens dulce (Sellerie)) und andere mehr;
sowie Lein, Baumwolle, Hanf, Flachs, Gurke, Spinat, Möhre, Zuckerrübe und den verschiedenen Baum-, Nuss- und Weinarten, insbesondere Banane und Kiwi.

Pflanzliche Organismen im Sinne der Erfindung sind weiterhin weitere photosynthetisch aktive befähigte Organismen, wie zum Beispiel Algen, Cyanobakterien sowie Moose. Bevorzugte Algen sind Grünalgen, wie beispielsweise Algen der Gattung Haematococcus, Phaedactylum tricornatum, Volvox oder Dunaliella. Insbesondere bevorzugt ist Synechocystis.

Besonders bevorzugt ist die Gruppe der Pflanzen bestehend aus Weizen, Hafer, Hirse, Gerste, Roggen, Mais, Reis, Buchweizen, Sorghum, Triticale, Dinkel, Leinsamen, Zuckerrohr, Raps, Kresse, Arabidopsis, Kohlarten, Soja, Alfalfa, Erbse, Bohnengewächsen, Erdnuss, Kartoffel, Tabak, Tomate, Aubergine, Paprika, Sonnenblume, Tagetes, Salat, Calendula, Melone, Kürbis und Zucchini.

Am meisten bevorzugt sind
a) Pflanzen, die zur Ölproduktion geeignet sind, wie beispielsweise Raps, Sonnenblume, Sesam, Färberdistel (Carthamus tinctorius), Ölbaum, Soja, Mais, Erdnuss, Rizinus, Ölpalme, Weizen, Kakaostrauch oder verschiedene Nussarten wie beispielsweise Walnuss, Kokosnuss oder Mandel. Unter diesen wieder besonders bevorzugt sind dikotyledonen Pflanzen, insbesondere Raps, Soja und Sonnenblume.
b) Pflanzen, die der Stärkeproduktion dienen, wie beispielsweise Mais, Weizen oder Kartoffel.
c) Pflanzen, die als Nahrungs- und/oder Futtermittel und/oder Nutzpflanze genutzt werden und bei denen eine Resistenz gg. Pathogene vorteilhaft wäre, wie beispielsweise Gerste, Roggen, Reis, Kartoffel, Baumwolle, Flachs, Lein.
d) Pflanzen, die zur Produktion von Feinchemikalien wie beispielsweise Vitaminen und/oder Carotinoiden dienen können, wie beispielsweise Raps.

"Population pflanzlicher Zellen" meint jegliche Gruppe von pflanzlichen Zellen die im Rahmen der vorliegenden Erfindung einer Transformation unterworfen werden kann und von der nach dem erfindungsgemäßen Verfahren transformierte transgene pflanzliche Zellen erhalten und isoliert werden können. Besagte Population kann dabei beispielsweise auch ein pflanzliches Gewebe, Organ oder eine Zellkultur usw. sein. Beispielhaft jedoch nicht einschränkend, kann besagte Population eine isolierte Zygote, ein isolierter unreifer Embryo, embryogener Kallus, Pflänzlichen oder auch verschiedene Blütengewebe (sowohl in vitro als auch in vivo) umfassen.

"Genom" meint die Gesamtheit der Erbinformation einer pflanzlichen Zelle und umfasst sowohl die genetische Information des Zellkerns als auch die der Plastiden (z.B. Chloroplasten) und Mitochondrien. Bevorzugt meint Genom jedoch die genetische Information des Zellkerns (beispielsweise der nukleären Chromosomen).

"Selektion" meint das Identifizieren und/oder Isolieren von erfolgreich transformieren pflanzlichen Zellen aus einer Population nicht-transformierter Zellen unter Einsatz des erfindungsgemäßen Verfahrens. Dabei ist es nicht zwingend erforderlich, dass die Selektion unmittelbar nach der Transformation direkt mit den transformierten Zellen erfolgt. Es ist auch möglich, die Selektion erst zu einem späteren Zeitpunkt, ja sogar bei einer späteren Generation der aus der Transformation resultierenden pflanzlichen Organismen (bzw. von diesen abgeleiteten Zellen, Geweben, Organen oder Vermehrungsgut) vorzunehmen. So kennen beispielsweise Arabidopsispflanzen direkt mit z.B. der Vakuuminfiltrationsmethode transformiert werden (Clough S & Bent A (1998) Plant J 16(6):735-43; Bechtold N et al. (1993) CR Acad Sci Paris 1144(2):204-212) und ergeben infolge transgene Samen, welche anschließend der Selektion ausgesetzt werden konnen.

Die Tatsache, dass die zu invertierende Nukleinsäuresequenz "in Kombination mit"' der "anti-Markerprotein"-Verbindung transformiert wird, ist breit zu verstehen und meint, dass mindestens eine Nukleinsäure codierend für eine MP-dsRNA zu insertierende Nukleinsäuresequenz und mindestens eine "anti-Markerprotein"-Verbindung miteinander funktionell gekoppelt sind, so dass das Vorliegen der "anti-Markerprotein"-Verbindung in der pflanzlichen Zelle - und des damit verbundenen Selektionsvorteils - das parallele Vorliegen der insertierten Nukleinsäuresequenz als wahrscheinlich anzeigt. Die zu insertierende Nukleinsäuresequenz und die Nukleinsäure codierend für eine MP-dsRNA "anti-Markerprotein"-Verbindung können dabei bevorzugt, jedoch nicht zwingand, Teil eines einzigen Nukleinsäurekonstruktes (z.B. eines Transformationskonstruktes oder Transformationsvektors) sein, also physikatisch chemisch durch eine kovalente Bindung gekoppelt vorlieger. Sie können jedoch auch getrennt, beispielsweise im Rahmen einer Co-Transformation, gemeinsam eingeführt werden und auch so ihre Funktion im Rahmen des erfindungsgemäßen Verfahrens wahrnehmen. "In Kombination" kann auch solche Ausführunsformen umfassen, bei der die besagte RNA und die von der in das Genom insertierten Nukleinsäuresequenz exprimierte RNA einen RNA-Strang ausbilden.
"Nicht toxische Substanz X" meint allgemein Substanzen, die im Vergleich zu ihrem Umsetzungsprodukt Y - unter ansonsten gleichen Bedingungen - eine verminderte, bevorzugt eine im wesentlichen fehlende biologische Aktivität - bevorzugt Toxizität - aufweisen. Dabei ist die Toxizität der Substanz Y mindestens doppelt so hoch wie die der Substanz X, bevorzugt mindestens fünffach so noch, besondere bevorzugt, mindestens zehnfach son hoch, ganz besonders bevorzugt mindestens zwanzigfach so hoch, am meistens bevorzugt mindestens einhundertfach so hoch. "Gleiche Bedingungen" meint dabei, dass alle Bedingungen abgesehen von den unterschiedlichen Substanzen X bzw. Y gleich gehalten werden. Es werden demnach gleiche molare Konzentrationen von X bzw. Y, bei gleichem Medium, Temperatur, Organismenart und -dichte etc. eingesetzt. Die Umwandlungen der Substanz X in die Substanz Y kann auf verschiedene Weise z.B. durch Hydrolyse, Deaminierung, Verseifung, Dephosphorylierung, Phosphorylierung, Oxidation oder eine andere Art der Aktivierung, Metabolisierung oder Umsetzung realisiert werden. Beispielhaft - jedoch nicht einschränkend - kann die Substanz X die inaktive Vorstufe oder Derivat eines Pflanzenwachstumsregulators oder Herbizids sein.

"Toxizität" oder "toxischer Effekt" meint einen messbaren, negativen Einfluss auf die Physiologie der Pflanze oder der pflanzlichen Zelle und kann dabei Symptome wie beispielsweise - jedoch nicht einschränkend - ein vermindertes oder gestörtes Wachstum, eine verminderte oder gestörte Photosyntheserate, eine verminderte oder gestörte Zellteilung, eine verminderte oder gestörte Regeneration einer vollständigen Pflanze aus Zellkultur oder Kallus usw. umfassen.

Die mittels des erfindungsgemässen Verfahrens erfolgreich transformierten pflanzlichen Zellen weisen - anders ausgedrückt - gegenüber den nicht-transformierten Zellen der gleichen Ausgangspopulation einen Wachstumsvorteil oder Selektionsvorteil unter Einwirkung der Substanz "X" auf. Wachstums- oder Selektionsvorteil ist dabei breit zu verstehen und meint beispielsweise die Tatsache, dass besagte transformierte pflanzliche Zellen in der Lage sind, Schösslinge auszubilden und/oder zu vollständigen Pflanzen regenerierbar sind, wohingegen die nicht-transformierten Zellen dies nicht oder nur mit deutlicher Verzögerung realisieren können.

Der Begriff des "Markerproteins" ist breit zu verstehen und meint allgemein all solche Proteine, die befähigt sind,
i) per se einen toxischen Effekt auf die Pflanze oder pflanzliche Zelle auszuüben, oder
ii) eine nicht toxische Substanz X in eine für die Pflanze oder pflanzliche Zelle toxische Substanz Y direkt oder indirekt umzusetzen.

Dabei kann es sich bei dem Markerprotein um ein pflanzeneigenes, endogenes Gen oder aber auch um ein Transgen aus einem anderen Organismus handeln. Bevorzugt hat das Markerprotein selber keine essentielle Funktion für den das Markerprotein umfassenden Organismus. Übt das Markerprotein per se einen toxischen Effekt aus, so wird es bevorzugt nicht konstitutiv sondern beispielsweise unter einem induzierbaren Promotor exprimiert.

Bevorzugt setzt jedoch das Markerprotein eine nicht toxische Substanz X in eine für die Pflanze oder pflanzliche Zelle toxische Substanz Y direkt oder indirekt um. Insbesondere bevorzugt sind als Markerprotein die sogenannten "negativen Selektionsmarker", wie sie beispielsweise im Rahmen von gezielten Deletionen aus dem Genom eingesetzt werden.

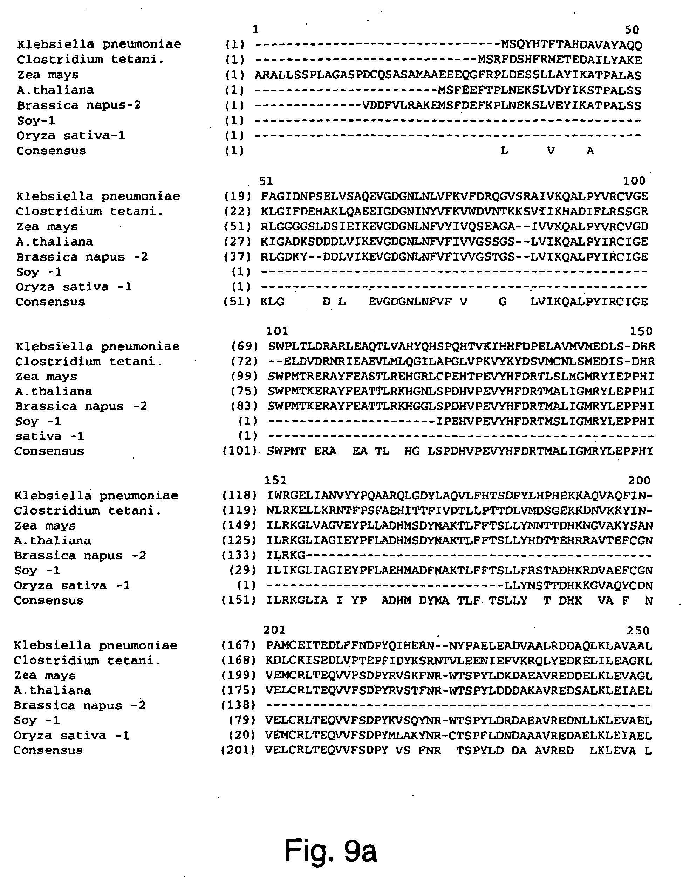

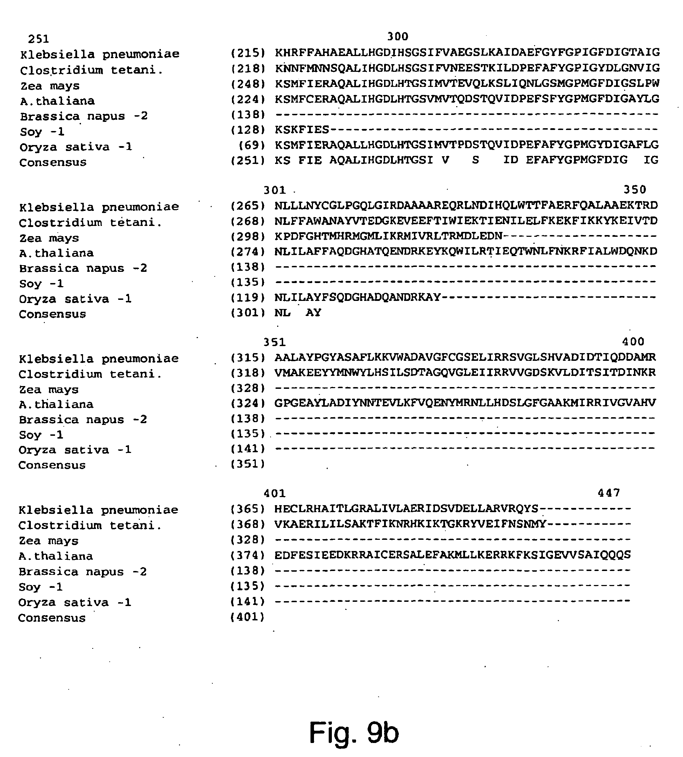

Beispielhaft jedoch nicht einschränkend sind für Markerproteine zu nennen:
(a) Cytosindeaminasen (CodA oder CDase), wobei bevorzugt Substanzen wie 5-Fluorocytosin (5-FC) als nicht toxische Substanz X eingesetzt werden. Cytosindeaminasen katalysieren die Deaminierung von Cytosin zu Uracil (Kilstrup M et al. (1989) J Bacteriol 171:2124-2127; Anderson L et al. (1989) Arch Microbiol 152:115-118). Bakterien und Pilze, die CDase-Aktivität aufweisen, konvertieren 5-FC zu dem toxischen Metaboliten ("Y") 5-Fluorouracil (5-FU) (Polak A & Scholer HJ (1975) Chemotherapy (Basel) 21:113-130). 5-FC selbst ist von geringer Toxizität (Bennett JE, in Goodman and Gilman: the Pharmacological Basis of Therapeutics. 8th ed., eds. Gilman AG et al. (Pergamon Press, New York) pp. 1165-1181) . 5-FU jedoch hat einen stark zytotoxischen Effekt da es infolge zu Fluoro-UTP (FUTP) und Fluoro-dUMP (FdUMP) metabolisiert wird und so die RNA- und DNA-Synthese inhibiert (Calabrisi P & Chabner BA in Goodman and Gilman: the Pharmacological Basis of Therapeutics. 8th ed., eds. Gilman AG et al. (Pergamon Press, New York) pp. 1209-1263); Damon LE et al. (1989) Pharmac Ther 43:155-189).
   Zellen höherer Pflanzen und Säugerzellen haben keine signifikante CDase-Aktivität und können 5-FC nicht deaminieren (Polak A et al. (1976) Chemotherapy 22:137-153; Koechlin BA et al. (1966) Biochemical Pharmacology 15:434-446). Insofern wird im Rahmen des erfindungsgemäßen Verfahrens die CDase als Transgen (z.B. in Form einer transgenen Expressionskassette) in pflanzliche Organismen eingebracht. Entsprechende transgene pflanzliche Zellen oder Organismen werden dann als Masterpflanzen als Ausgangsmaterial eingesetzt. Entsprechende CDase Sequenzen, transgene pflanzliche Organismen und die Durchführung negativer Selektionsverfahren unter Einsatz von z.B. 5-FC als nicht toxische Substanz X sind dem Fachmann bekannt (WO 93/01281; US 5,358,866; Gleave AP et al. (1999) Plant Mol Biol 40(2):223-35; Perera RJ et al. (1993) Plant Mol Biol 23(4):793-799; Stougaard J (1993) Plant J 3:755-761); EP-A1 595 837; Mullen CA et al. (1992) Proc Natl Acad Sci USA 89(1):33-37; Kobayashi T et al. (1995) Jpn J Genet 70(3):409-422; Schlaman HRM & Hooykaas PFF (1997) Plant J 11:1377-1385; Xiaohui Wang H et al. (2001) Gene 272(1-2): 249-255; Koprek T et al. (1999) Plant J 19(6):719-726; Gleave AP et al. (1999) Plant Mol Biol 40(2):223-235; Gallego ME (1999) Plant Mol Biol 39(1):83-93; Salomon S & Puchta H (1998) EMBO J 17(20):6086-6095; Thykjaer T et al. (1997) Plant Mol Biol 35(4):523-530; Serino G (1997) Plant J 12(3):697-701: Risseeuw E (1997) Plant J 11(4):717-728; Blanc V et al. (1996) Biochimie 78(6):511-517; Corneille S et al. (2001) Plant J 27:171-178). Cytosindeaminasen und die dafür kodierenden Gene können aus einer Vielzahl von Organismen, bevorzugt Mikroorganismen, wie beispielsweise den Pilzen Cryptococcus neoformans, Candida albicans, Torulopsis glabrata, Sporothrix schenckii, Aspergillus, Cladosporium und Phialophora (JE Bennett, Chapter 50: Antifungal Agents, in Goodman and Gilman's the Pharmacological Basis of Therapeutics 8th ed., A.G. Gilman, ed., Pergamon Press, New York, 1990) sowie den bakterien E.coli und Salmonella typhimurium (Andersen L et al. (1989) Arch Microbiol 152:115-118) erhalten werden.
   Auf die im Rahmen der genannten Publikationen offenbarten Sequenzen, Materialien und Verfahren wird hiermit ausdrücklich Bezug genommen.
   Insbesondere bevorzugt sind Sequenzen entsprechend der GenBank Acc.-No: S56903, sowie die in EP-A1 595 873 beschriebenen modifizierten codA Sequenzen, die eine Expression in Eukaryoten ermöglichen. Bevorzugt sind dabei Nukleinsäuresequenzen, die für Polypeptide gemäß SEQ ID NO: 2 oder - bevorzugt - 4 kodieren, insbesondere die Sequenzen gemäß SEQ ID NO: 1 oder - bevorzugt - 3.
(b) Cytochrom P-450 Enzyme, insbesondere das bakterielle Cytochrom P-450 SU1 Genprodukt (CYP105A1) aus Streptomyces griseolus (Stamm ATCC 11796), wobei bevorzugt Substanzen wie das Pro-Sulfonylharnstoffherbizid R7402 (2-Methylethyl-2-3-dihydro-N-[(4,6-dimethoxypyrimidin-2-yl)aminocarbonyl]-1,2-benzoisothiazol-7-sulfonamid-1,1-dioxid) als nicht toxische Substanz X eingesetzt werden. Entsprechende Sequenzen und die Durchführung negativer Selektionsverfahren unter Einsatz von z.B. R7402 als nicht toxische Substanz X sind dem Fachmann bekannt (O'Keefe DP et al. (1994) Plant Physiol 105:473-482; Tissier AF et al. (1999) Plant Cell 11:1841-1852; Koprek T et al. (1999) Plant J 19(6):719-726; O'Keefe DP (1991) Biochemistry 30(2):447-55). Auf die im Rahmen der genannten Publikationen offenbarten Sequenzen, Materialien und Verfahren wird hiermit ausdrücklich Bezug genommen.
   Insbesondere bevorzugt sind Sequenzen entsprechend der GenBank Acc.-No: M32238. Bevorzugt sind ferner Nukleinsäuresequenzen, die für das Polypeptid gemäß SEQ ID NO: 6 kodieren, insbesondere die Sequenz gemäß SEQ ID NO: 5.
(c) Indolessigsäurehydrolasen wie beispielsweise das tms2 Genprodukt aus Agrobacterium tumefaciens, wobei bevorzugt Substanzen wie Auxinamidverbindungen oder Naphthalacetamid (NAM) als nicht toxische Substanz X eingesetzt werden (wobei NAM zu Naphthalessigsäure, einer phytotoxischen Substanz, umgesetzt wird). Entsprechende Sequenzen und die Durchführung negativer Selektionsverfahren unter Einsatz von z.B. NAM als nicht toxische Substanz X sind dem Fachmann bekannt (Fedoroff NV & Smith DL (1993) Plant J 3:273-289; Upadhyaya NM et al. (2000) Plant Mol Biol Rep 18:227-223; Depicker AG et al. (1988) Plant Cell rep 104:1067-1071; Karlin-Neumannn GA et al. (1991) Plant Cell 3:573-582; Sundaresan V etal. (1995) Gene Develop 9:1797-1810; Cecchini E et al. (1998) Mutat Res 401(1-2):199-206; Zubko E et al. (2000) Nat Biotechnol 18:442-445). Auf die im Rahmen der genannten Publikationen offenbarten Sequenzen, Materialien und Verfahren wird hiermit ausdrücklich Bezug genommen.
   Insbesondere bevorzugt sind Sequenzen entsprechend den GenBank Acc.-No: NC_003308 (Protein_id="NP_536128,1), AE009419, AB016260 (Protein_id="BAA87807.1) und NC002147. Bevorzugt sind ferner Nukleinsäuresequenzen, die für Polypeptide gemäß SEQ ID NO: 8 oder 10 kodieren, insbesondere die Sequenzen gemäß SEQ ID NO: 7 oder 9.
(d) Haloalkandehalogenasen (dhlA Genprodukt) z.B. aus Xanthobacter autotropicus GJ10. Die Dehalogenase hydrolisiert Dihaloalkane wie 1,2-Dichloroethan (DCE) zu halogenierten Alkoholen und anorganischen Haliden (Naested H et al. (1999) Plant J 18(5)571-576; Janssen DB et al. (1994) Annu Rev Microbiol 48: 163-191; Janssen DB (1989) J Bacteriol 171(12):6791-9). Auf die im Rahmen der genannten Publikationen offenbarten Sequenzen, Materialien und Verfahren wird hiermit ausdrücklich Bezug genommen.
   Insbesondere bevorzugt sind Sequenzen entsprechend der GenBank Acc.-No: M26950. Bevorzugt sind ferner Nukleinsäuresequenzen, die für das Polypeptid gemäß SEQ ID NO: 12 kodieren, insbesondere die Sequenz gemäß SEQ ID NO: 11.
(e) Thymidinkinasen (TK), insbesondere virale TKs aus Viren wie Herpes Simplex Virus, SV40, Cytomegalovirus, Varicella-zoster Virus, insbesondere die TK des Typ 1 Herpes Simplex Virus (TK HSV-1), wobei bevorzugt Substanzen wie Acyclovir, Ganciclovir oder 1,2-Deoxy-2-fluoro-β-D-arabinofuranosil-5-iodouracil (FIAU) als nicht toxische Substanz X eingesetzt werden. Entsprechende Sequenzen und die Durchführung negativer Selektionsverfahren unter Einsatz von z.B. Acyclovir, Ganciclovir oder FIAU als nicht toxische Substanz X sind dem Fachmann bekannt (Czako M & Marton L (1994) Plant Physiol 104:1067-1071; Wigler M et al. (1977) Cell 11(1):223-232; McKnight SL et al. (1980) Nucl Acids Res 8(24):5949-5964: McKnight SL et al. (1980) Nucl Acids Res 8(24):5931-5948; Preston et al. (1981) J Virol 38(2):593-605; Wagner et al. (1981) Proc Natl Acad Sci USA 78(3):1441-1445; St. Clair et al.(1987) Antimicrob Agents Chemother 31(6):844-849). Auf die im Rahmen der genannten Publikationen offenbarten Sequenzen, Materialien und Verfahren wird hiermit ausdrücklich Bezug genommen.
   Insbesondere bevorzugt sind Sequenzen entsprechend den GenBank Acc.-No: J02224, V00470 und V00467. Bevorzugt sind ferner Nukleinsäuresequenzen, die für Polypeptide gemäß SEQ ID NO: 14 oder 16 kodieren, insbesondere die Sequenzen gemäß SEQ ID NO: 13 oder 15.
(f) Guaninphosphoribosyltransferasen, Hypoxanthinphosphoribosyltransferasen oder Xanthinguaninphosphoribosyltransferasen, wobei bevorzugt Substanzen wie 6-Thioxanthin oder Allopurinol als nicht toxische Substanz X eingesetzt werden. Bevorzugt sind Guaninphosphoribosyltransferasen (gpt) z.B. aus E. Coli (Besnard et al. (1987) Mol Cell Biol 7:4139; Mzoz and Moolten (1993) Human Gene Therapy 4:589-595; Ono et al. (1997) Hum Gene Ther 8(17):2043-55), Hypoxanthinphosphoribosyltransferasen (HPRT; Jolly et al. (1983) Proc Natl Acad Sci USA 80:477; Fonwick "The HGPRT Systern", pp. 333-373, M. Gottesman (ed.), Molecular Cell Genetics, John Wiley and Sons, New York, 1985), Xanthinguaninphosphoribosyltransferasen z.B. aus Toxoplasma gondii (Knoll LJ et al.(1998) Mol Cell Biol 18(2):807-814; Donald RG et al. (1996) J Biol Chem 271(24):14010-14019). Auf die im Rahmen der genannten Publikationen offenbarten Sequenzen, Materialien und Verfahren wird hiermit ausdrücklich Bezug genommen.
   Insbesondere bevorzugt sind Sequenzen entsprechend den GenBank Acc.-No: U10247 (Toxoplasma gondii HXGPRT), M13422 (E. coli gpt) und X00221 (E. coli gpt). Bevorzugt sind ferner Nukleinsäuresequenzen, die für Polypeptide gemäß SEQ ID NO: 18, 20 oder 22 kodieren, insbesondere die Sequenzen gemäß SEQ ID NO: 17, 19 oder 21.
(g) Purinnukleosidphosphorylasen (PNP; DeoD Genprodukt) z.B. aus E. coli, wobei bevorzugt Substanzen wie 6-Methylpurindeoxyribonukleosid als nicht toxische Substanz X eingesetzt werden. Entsprechende Sequenzen und die Durchführung negativer Selektionsverfahren unter Einsatz von z.B. 6-Methylpurindeoxyribonukleosid als nicht toxische Substanz X sind dem Fachmann bekannt (Sorscher EJ et al. (1994) Gene Therapy 1:233-238). Auf die im Rahmen der genannten Publikationen offenbarten Sequenzen, Materialien und Verfahren wird hiermit ausdrücklich Bezug genommen.
   Insbesondere bevorzugt sind Sequenzen entsprechend der GenBank Acc.-No: M60917. Bevorzugt sind ferner Nukleinsäuresequenzen, die für das Polypeptid gemäß SEQ ID NO: 24 kodieren, insbesondere die Sequenz gemäß SEQ ID NO: 23.
h) Phosphonatmonoesterhydrolasen, welche inaktive Esterderivate des Herbizides Glyphosat (z.B. Glycerylglyphosat) zu der aktiven Form des Herbizids umsetzen. Entsprechende Sequenzen und die Durchführung negativer Selektionsverfahren unter Einsatz von z.B. Glycerylglyphosat sind dem Fachmann bekannt (US 5,254,801; Dotson SB et al. (1996) Plant J 10(2):383-392; Dotson SB et al. (1996) J Biol Chem 271(42): 25754-25761). Auf die im Rahmen der genannten Publikationen offenbarten Sequenzen, Materialien und Verfahren wird hiermit ausdrücklich Bezug genommen.
   Insbesondere bevorzugt sind Sequenzen entsprechend der GenBank Acc.-No: U44852. Bevorzugt sind ferner Nukleinsäuresequenzen, die für das Polypeptid gemäß SEQ ID NO: 26 kodieren, insbesondere die Sequenz gemäß SEQ ID NO: 25.
(i) Aux-1 und - bevorzugt - Aux-2 Genprodukte z.B. der Ti-Plasmide von Agrobacterium Stämmen wie A.rhizogenes oder A.tumefaciens (Beclin C et al. (1993) Transgenics Res 2:4855); Gaudin V, Jouanin L. (1995) Plant Mol Biol. 28(1):123-36.
   Die Aktivität der beiden Enzyme bedingt die Produktion von Indolacetamid (IAA) in der pflanzlichen Zelle. Aux-1 kodiert für eine Indolacetamidsynthase (IAMS) und setzt Tryptophan zu Indolacetamid um (VanOnckelen et al. (1986) FEBS Lett. 198: 357-360). Aux-2 kodiert für das Enzym Indolacetamidhydrolase (IAMH) und setzt Indolacetamid, eine Substanz ohne Phytohormonaktivität, zu dem aktiven Auxin Indolessigsäure um (Inze D et al. (1984) Mol Gen Genet 194:265-274; Tomashow et al. (1984) Proc Natl Acad Sci USA 81:5071-5075; Schroder et al. (1984) Eur J Biochem 138:387-391). Das Enzym IAMH kann ferner einer Reihe von Indolamid-Substraten wie beispielsweise Naphthalacetamid hydrolisieren, wobei letzteres in den Pflanzenwachstumsregulator Naphthalessigsäure (NAA) umgesetzt wird. Die Verwendung des IAMH Gens als negativer Selektionsmarker ist beispielsweise in US 5,180,873 beschrieben. Entsprechende Enzyme sind auch in A. rhizogenes, A. vitis (Canaday J et al. (1992) Mol Gen Genet 235:292-303) and Pseudomonas savastanoi (Yamada et al. (1985) Proc Natl Acad Sci USA 82:6522-6526) beschrieben. Der Einsatz als negativer Selektionsmarker zum Abtöten bestimmter Zellgewebe (z.B. Pollen; US 5,426,041) oder transgener Pflanzen (US 5,180,873) ist beschrieben. Entsprechende Sequenzen und die Durchführung negativer Selektionsverfahren unter Einsatz von z.B. Naphthalacetamid sind dem Fachmann bekannt (s.o.). Auf die im Rahmen der genannten Publikationen offenbarten Sequenzen, Materialien und Verfahren wird hiermit ausdrücklich Bezug genommen.
   Insbesondere bevorzugt sind Sequenzen entsprechend den GenBank Acc.-No: M61151, AF039169 und AB025110. Bevorzugt sind ferner Nukleinsäuresequenzen, die für Polypeptide gemäß SEQ ID NO: 28, 30, 32, 34 oder 36 kodieren, insbesondere die Sequenzen gemäß SEQ ID NO: 27, 29, 31, 33 oder 35.
(j) Adeninphosphoribosyltransferasen (APRT), wobei bevorzugt Substanzen wie 4-Aminopyrazolopyrimidin als nicht toxische Substanz X eingesetzt werden. Entsprechende Sequenzen und die Durchführung negativer Selektionsverfahren unter Einsatz sind dem Fachmann bekannt (Wigler M et al. (1979) Proc Natl Acad Sci USA 76(3):1373-6; Taylor et al. "The APRT Systern", pp., 311-332, M. Gottesman (ed.), Molecular Cell Genetics, John Wiley and Sons, New York, 1985).
k) Methoxinindehydrogenasen, wobei bevorzugt Substanzen wie 2-Amino-4-methoxy-butansäure (Methoxinin) als nicht toxische Substanz X eingesetzt werden, welches zum toxischen Methoxyvinylglycin umgesetzt wird (Margraff R et al. (1980) Experimentia 36: 846).
l) Rhizobitoxinsynthasen, wobei bevorzugt Substanzen wie 2-Amino-4-methoxy-butansäure (Methoxinin) als nicht toxische Substanz X eingesetzt werden, welches zum toxischen 2-Amino-4-[2-amino-3-hydroxypropyl]-trans-3-butansäure (Rhizobitoxin) umgesetzt wird (Owens LD et al. (1973) Weed Science 21:63-66),
m) 5-Methylthioribose (MTR) kinasen, wobei bevorzugt Substanzen wie 5-(Trifluoromethyl)thioribose (MTR-Analog, "subversives Substrat") als nicht toxische Substanz X eingesetzt wird, welches über ein instabiles Intermediat zu der toxischen Substanz (Y) Carbothionyldifluorid umgesetzt wird. Die MTR-Kinase ist ein Schlüsselenzym des Methionin-Ausweich-Stoffwechselwegs ("Salvage Pathway"). Entsprechende Enzymaktivitäten wurden nicht in Säugern, wohl aber in Pflanzen, Bakterien und Protozoen beschrieben. MTR Kinasen aus verschiedenen Arten wurden aufgrund definierter Sequenzmotive identifiziert (Sekowska A et al. (2001) BMC Microbiol 1:15; http://www.biomedcentral.com/1471-2180/1/15). Entsprechende Sequenzen und die Durchführung negativer Selektionsverfahren unter Einsatz von z.B. 5-(Trifluoromethyl)thioribose sind dem Fachmann bekannt und leicht aus entsprechenden Sequenzdatenbank (z.B. GenBank) erhältlich (Sekowska A et al. (2001) BMC Microbiol 1:15; Cornell KA et al. (1996) 317:285-290). Auf die im Rahmen der genannten Publikationen offenbarten Sequenzen, Materialien und Verfahren wird hiermit ausdrücklich Bezug genommen.
   Eine pflanzliche MTR-Kinase ist bislang jedoch nicht eindeutig identifiziert worden und wird im Rahmen des erfindungsgemäßen Verfahrens bereitsgestellt (SEQ ID NO: 39 bzw. 40). Darüberhinaus werden Homologe aus anderen Pflänzenarten bereitgestellt nämlich aus Mais (SEQ ID NO: 59 bzw. 60), Raps (SEQ ID NO: 61, 63 bzw. 62, 64), Reis (SEQ ID NO: 65 bzw. 66) sowie Soja (SEQ ID NO: 67 bzw. 68).
   Ein weiterer Gegenstand der Erfindung betrifft demnach Aminosäuresequenzen kodieren für eine pflanzliche 5-Methylthioribosekinase, dadurch gekennzeichnet, dass besagte Aminosänresequenz mindestens eine Sequenz enthält ausgewählt aus der Gruppe bestehend aus SEQ ID NO: 60, 62, 64, 66 oder 68. Ein weiterer Gegenstand der Erfindung betrifft demnach Nukleinsäuresequenzen kodieren für eine pflanzliche 5-Methylthioribosekinase, dadurch gekennzeichnet, dass besagte Nukleinsäuresequenz mindestens eine Sequenz enthält ausgewählt aus der Gruppe bestehend aus SEQ ID NO: 59, 61, 63, 65 oder 67. Auch wenn besagte Sequenzen teilweise nur Fragmente vollständiger cDNAs darstellen, ist ihre Länge dennoch mehr als ausreichend um eine Verwendung und Funktionalität als antsense-RNA bzw. doppelsträngige RNA zu gewährleisten. Bevorzugt wird als Markerprotein eine pflanzliche, endogene MTR-Kinase verwendet. Weitere endogene pflanzliche MTR-Kinasen können leicht mittels Durchmusterung von Daten- oder Genbanken unter Verwendung konservierter, MTK-Kinase typischer Motive identifiziert werden. Besagte Motive können beispielsweise aus Fig. 9a-b abgeleitet werden. Solche Motive können beispielhaft jedoch nicht einschränkend folgende Sequenzen umfassen:
   E(V/I)GDGN(L/I)N(L/Y/F)V(F/Y) bevorzugt EVGDGNLN(Y/F)V(F/Y)
   KQALPY(V/I)RC
   SWPMT(R/K)ERAYF
   PEVYHFDRT
   GMRY(I/L)EPPHI
   CRLTEQVVFSDPY
   HGDLH(S/T)GS
   Weitere geeignete Motive können unschwer aus FIg.9a-b abgeleitet werden.
   Insbesondere bevorzugt sind Sequenzen entsprechend den GenBank Acc.-No: AF212863 oder AC079674 (Protein_ID=AAG51775.1). Bevorzugt sind ferner Nukleinsäuresequenzen, die für Polypeptide gemäß SEQ ID NO: 38 oder 40 kodieren, insbesondere die Sequenzen gemäß SEQ ID NO: 37 oder 39.
n) Alkoholdehydrogenasen (Adh) insbesondere pflanzliche Adh-1 genprodukte, wobei bevorzugt Substanzen wie Allylalkohol als nicht toxische Substanz X eingesetzt werden, welche so zu der toxischen Substanz (Y) Acrolein umgesetzt wird. Entsprechende Sequenzen und die Durchführung negativer Selektionsverfahren unter Einsatz von z.B. Allylalkohol sind dem Fachmann bekannt und leicht aus entsprechenden Sequenzdatenbank (z.B. GenBank) erhältlich (Wisman E et al. (1991) Mol Gen Genet 226(1-2):120-8; Jacobs M et al. (1988) Biochem Genet 26(1-2):105-22; Schwartz D. (1981) Environ Health Perspect 37:75-7). Auf die im Rahmen der genannten Publikationen offenbarten Sequenzen, Materialien und Verfahren wird hiermit ausdrücklich Bezug genommen.
   Insbesondere bevorzugt sind Sequenzen entsprechend den GenBank Acc.-No: X77943, M12196, AF172282, X04049 oder AF253472. Bevorzugt sind ferner Nukleinsäuresequenzen, die für Polypeptide gemäß SEQ ID NO: 42, 44, 46 oder 48 kodieren, insbesondere die Sequenzen gemäß SEQ ID NO: 41, 43, 45 oder 47.
(o) Weiterhin sind als negativer Selektionsmarker solche Sequenzen geeignet, die per se eine toxische Wirkung auf pflanzliche Zellen ausüben, wie beispielsweise Diptheriatoxin A, Ribonukleasen.wie Barnase sowie Ribosom-inhibierende Proteine wie Ricin. Dabei werden diese Proteine bevorzugt in den pflanzlichen Zellen nicht konstitutiv, sondern induzierbar exprimiert. Bevorzugt erfolgt die Induktion chemisch, wobei beispielsweise die unten erwähnten chemisch-induzierbaren Promotoren verwendet werden können, um diese chemischinduzierte Expression zu gewährleisten.

"Verminderung" oder "vermindern" ist im Zusammenhang mit einem Markerprotein, bzw. seiner Menge, Expression, Aktivität und/oder Funktion weit auszulegen und umfasst die teilweise oder im wesentlichen vollständige, auf unterschiedliche zellbiologische Mechanismen beruhende Unterbindung oder Blockierung der Funktionalität eines Markerproteins in einer pflanzlichen Zelle, Pflanze oder einem davon abgeleiteten Teil, Gewebe, Organ, Zellen oder Samen.

Eine Verminderung im Sinne der Erfindung umfasst auch eine mengenmäßige Verringerung eines Markerproteins bis hin zu einem im wesentlichen vollständigen Fehlen des Markerproteins (d.h. fehlende Nachweisbarkeit von Markerprotein-Aktivität bzw. Markerprotein-Funktion oder fehlende immunologische Nachweisbarkeit des Markerproteins). Dabei wird die Expression eines bestimmten Markerproteins (bzw. seiner Menge, Expression, Aktivität und/oder Funktion) in einer Zelle oder einem Organismus bevorzugt um mehr als 50 %, besonders bevorzugt um mehr als 80 %, ganz besonders bevorzugt um mehr als 90%, am meisten bevorzugt um mehr als 98 % vermindert. Insbesondere meint Verminderung auch das vollständigen Fehlen des Markerproteins (bzw. seiner Menge, Expression, Aktivität und/oder Funktion). Dabei meint Aktivität und/oder Funktion bevorzugt die Eigenschaft des Markerproteins einen toxischen Effekt auf die pflanzliche Zelle oder den pflanzlichen Organismus auszuüben bzw. die Fähigkeit die Substanz X in die Substanz Y umzusetzen. Bevorzugt wird als der durch das Markerprotein bewirkte toxische Effekt um mehr als 50 %, besonders bevorzugt um mehr als 80 %, ganz besonders bevorzugt um mehr als 90%, am meisten bevorzugt um mehr als 98 % vermindert. Selbst verständliche umfasst "Verminderung" im Rahmen des erfindungsgemäßen Verfahrens auch eine vollständige, 100%ige Verminderung oder Beseitigung des Markerproteins (bzw. seiner Menge, Expression, Aktivität und/oder Funktion) (z.B. durch Deletion des Markerprotein-Gens aus dem Genom).

Erfindungsgemäß werden dsRNA-Moleküle zur Verminderung der Expression, Menge, Aktivität und/oder Funktion des Markerproteins verwendet. Der Fachmann erkennt, dass eine Reihe verschiedener erfindungsgemaßer und nichterfindungsgemäßer Methoden zur Verfügung stehen, um die Expression, Menge, Aktivität und/oder Punktion eines Markerproteins in gewünschter Weise zu beeinflussen. Beispielhaft, seien zu nennen:
a) Einbringen mindestens einer doppelsträngigen Markerprotein. Ribonukleinsäuresequenz (MP-dsRNA) oder einer deren Expression gewahrleistenden Expressionskassette oder Expressionskassetten. Umfasst sind solche Verfahren, bei denen die MP-dsRNA gegen ein Markerprotein-Gen (also genomische DNA-Sequenzen wie Promotorsequenzen) oder ein Markerprotein-Gentranskript (also mRNA-Sequenzen) gerichtet ist.
b) Einbringen mindestens einer Markerprotein antisense-Ribonukleinsäuresequenz (MP-antisenseRNA) oder einer deren Expression gewährleistenden Expressionskassette. Umfasst sind solche Verfahren, bei denen die MP-antisenseRNA gegen ein Markerprotein-Gen (also genomische DNA-Sequensen) oder ein Markerprotein-Gentranskript (also RNA-Sequenzen) gerichtet ist. Umfasst sind auch α-anomere Nukleinsäuresequenzen.
c) Einbringen mindestens einer MP-antisenseRNA kombiniert mit einem Ribozym oder einer deren Expression gewäbrleistenden Expressionskassette
d) Einbringen mindestens einer Markerprotein sense-Ribonukleinsäuresequenz (MP-senseRNA) zur Indukti.on einer Kosuppression oder einer deren Expression gewährleistenden Expressionskassette
e) Einbringen mindestens eines DNA-oder Protein-bindenden Faktors gegen ein Markerprotein-Gen, -RNA oder -Protein oder einer dessen Expression gewährleistenden Expressionskassette
f) Eindringen mindestens einer den Markerprotein RNA-Abbau bewirkenden viralen Nukleinsäuresequenz oder einer deren Expression gewährleistenden Expressionskassette
g) Einbringen mindestens eines Konstruktes zur Erzeugung eines funktionsverlustes (z.B. Generierung von Stopp-Kodons, Verschiebungen im Leseraster etc.) an einem Markerprotein-Gen beispielsweise durch Erzeugung einer Insertion, Deletion, Inversion oder Mutation in einem Markerprotein-Gen. Bevorzugt können Knockout-Mutanten mittels gezielter Insertion in besagtes Markerprotein-Gen durch homologer Rekombination oder Einbringen von sequenzspezifischen Nukleasen gegen Markerprotein-Gensequenzen generiert werden.

Dem Fachmann ist bekannt, dass auch weitere Verfahren zur Verminderung eines Markerproteins bzw. seiner Aktivität oder Funktion eingesetzt werden können. Beispielsweise kann auch - je nach Art des verwendeten Markerproteins - das Einbringen einer dominant-negativen Variante eines Markerproteins oder einer deren Expression gewährleistenden expressionskassette vorteilhaft sein. Dabei kann jedes einzelne dieser Verfahren eine Verminderung der Expression, Menge, Aktivität und/oder Funktion eines Markerproteins bewirken. Auch eine kombinierte Anwendung ist denkbar. Weitere Methoden sind dem Fachmann bekannt und können die Behinderung oder Unterbindung der Prozessierung des Markerproteins, des Transports des Markerproteins oder dessen mRNA, Hemmung der Ribosomenanlagerung, Hemmung des RNA-Spleißens, Induktion eines Markerprotein-RNA abbauenden Enzyms und/oder Hemmung der Translationselongation oder -termination umfassen.

Die einzelnen bevorzugten Verfahren seien infolge durch beispielhafte Ausführungsformen beschrieben:

### a) Einbringen einer doppelsträngigen Ribonukleinsäuresequenz eines Markerproteins (MP-dsRNA)

Das Verfahren der Genregulation mittels doppelsträngiger RNA ("double-stranded DNA interference"; dsRNAi) ist vielfach für tierische und pflanzliche Organismen beschrieben (z.B. Matzke MA et al. (2000) Plant Mol. Biol 43:401-415: Fire A. et al (1998) Nature 391:806-811; WO 99/32619; WO 99/53050; WO 00/68374;

WO 00/44914; WO 00/44895; WO 00/49035; WO 00/63364). Auf die in den angegebenen Zitaten beschriebenen Verfahren und Methoden wird hiermit ausdrücklich Bezug genommen. dsRNAi-Verfahren beruhen auf dem Phänomen, dass durch gleichzeitiges Einbringen von komplementären Strang- und Gegenstrang eines Gentranskriptes eine hocheffiziente Unterdrückung der Expression des entsprechenden Gens gewirkt wird. Der bewirkte Phänotyp kommt dem einer entsprechenden knock-out Mutanten sehr ähnlich (Waterhouse PM et al. (1998) Proc Natl Acad Sci USA 95:13959-64). Das dsRNAi-Verfahren hat sich bei der Verminderung der Markerprotein-Expression als besonders effizient und vorteilhaft erwiesen.

Doppelsträngiges RNA-Molekül meint im Bahnen der Erfindung bevorzugt eine oder mehr Ribonukleinsäuresequenzen, die aufgrund komplementarer Sequenzen theoretisch (z.B. gemäß den Basenpaarregeln von Waston und Crick) und/oder faktisch (z.B. aufgrund
von Hybridisterungsexperimenten in vitro und/oder in vivo) in der Lage sind, doppelsträngige RNA-Strukturen auszubilden. Dem Fachmann ist bewusst, dass die Ausbildung von doppelsträngigen RNA-Strukturen, einen Gleichgewichtszustand darstellt. Bevorzugt ist das Verhältnis von doppelsträngigen Molekülen zu entsprechenden dissozierten Formen mindestens 1 zu 10, bevorzugt 1:1, besonders bevorzugt 5:1, am meisten bevorzugt 10:1.

Der Gegenstand der Erfindung bezieht sich daher Auf die Verwendung von doppelsträngige RNR-Molekülen (dsRNA-Molekule), die bei Eindringen in einen pflanzlichen Organismus (oder eine davon abgeleitete Zelle, Geweben, Organ oder Vermehrungsmaterial) die Verminderung mindestens eines Markerproteins bewirken. Das doppelsträngige RNA-Molekül zur Verminderung der Expression eines Markerproteins (MP-dsRNA) umfasst dabei bevorzugt
a) einen "sense"-RNA-Strang umfassend mindestens eine Ribonukleotidsequenz, die im wesentlichen identisch ist zu mindestens einem Teil des "sense"-RNA-Transkriptes einer Nukleinsäuresequenz kodierend für ein Markerproteins, und
b) einen "antisense"-RNA-Strang, der zu dem RNA-"sense"-Strang unter a) im wesentlichen - bevorzugt vollständig - komplementären ist.

In Bezug auf die dsRNA-Molekule meint Markerprotein-Nukleinsäuresequenz bevorzugt eine Sequenz gemäß SEQ ID NO: 1, 3, 5, 7, 9, 11, 13, 15, 17, 19, 21, 23, 25, 27, 29, 31, 33, 35, 37, 39, 41, 43, 45 oder 42 oder ein funktionelles Äquivalent derselben.

"Im wesentlichen identisch" meint, dass die dsRNA Sequenz auch Insertionen, Deletionen sowie einzelne Punktmutationen im Vergleich zu der Markerprotein Zielsequenz aufweisen kann und dennoch eine effizient Verminderung der Expression bewirkt. Bevorzugt beträgt die Homologie (nach weiter unten folgender Definition) mindestens 75 %, bevorzugt mindestens 80 %, ganz besonders bevorzugt mindestens 90 % am meisten bevorzugt 100 % zwischen dem "sense"-Strang einer inhibitorischen dsRNA und mindestens einem Teil des "sense"-RNA-Transkriptes einer Nukleinsäuresequenz kodierend für ein Markerprotein (bzw, zwischen dem "antisense"-Strang dem komplementären Strang einer Nukleinsäuresequenz kodierend für ein Markerprotein).

Eine 100%ige Sequenzidentität zwischen dsRNA und einem Markerprotein Gentranskript ist nicht zwingend erforderlich, um eine effiziente Verminderung der Markerprotein Expression zu bewirken. Demzufolge besteht der Vorteil, dass das Verfahren tolerant ist gegenüber Sequenzabweichungen, wie sie infolge genetischer Mutationen, Polymorphismen oder evolutionärer Divergenzen vorliegen können. So ist es beispielsweise möglich mit der dsRNA, die ausgehend von der Markerprotein Sequenz des einen Organismus generiert wurde, die Markerprotein Expression in einem anderen Organismus zu unterdrücken. Dies ist besonders dann vorteilhaft, wenn als Markerprotein ein pflanzeneigenes, endogenes Markerprotein verwendet wird (beispielsweise eine 5-Methylthioribosekinase oder Alkoholdehydrogenase). Zu diesem Zweck umfasst die dsRNA bevorzugt Sequenzbereiche von Markerprotein-Gentranskripten, die konservierten Bereichen entsprechen. Besagte konservierte Bereiche können aus Sequenzvergleichen leicht abgeleitet werden.

Die Länge des Teilabschnittes beträgt mindestens 10 Basen, bevorzugt mindestens 25 Basen, besonders bevorzugt mindestens 50 Basen, ganz besonders bevorzugt mindestens 100 Basen, am meisten bevorzugt mindestens 200 Basen oder mindestens 300 Basen.

Alternativ, kann eine "im wesentlichen identische" dsRNA auch als Nukleinsäuresequenz definiert werden, die befähigt ist, mit einem Teil eines Markerprotein Gentranskriptes zu hybridisieren (z.B. in 400 mM NaCl, 40 mM PIPES pH 6,4, 1 mM EDTA bei 50°C oder 70°C für 12 bis 16 h).

"Im wesentlichen komplementär" meint, dass der "antisense"-RNA-Strang auch Insertionen, Deletionen sowie einzelne Punktmutationen im Vergleich zu dem Komplement des "sense"-RNA-Stranges aufweisen kann. Bevorzugt beträgt die Homologie mindestens 80 %, bevorzugt mindestens 90 %, ganz besonders bevorzugt mindestens 95 %, am meisten bevorzugt 100% zwischen dem "antisense"-RNA-Strang und dem Komplement des "sense"-RNA-Stranges.

"Teil des "sense"-RNA-Transkriptes" einer Nukleinsäuresequenz kodierend für ein Markerprotein meint Fragmente einer RNA oder mRNA transkribiert oder transkribierbar von einer für ein Markerprotein kodierenden Nukleinsäuresequenz, bevorzugt von einem Markerprotein-Gen. Dabei haben die Fragmente bevorzugt eine Sequenzlänge von mindestens 20 Basen, bevorzugt mindestens 50 Basen, besonders bevorzugt mindestens 100 Basen, ganz besonders bevorzugt mindestens 200 Basen, am meisten bevorzugt mindestens 500 Basen. Umfasst ist auch die vollständige transkribierte RNA oder mRNA. Umfasst sind auch Sequenzen wie sie unter künstlichen Bedingungen von Regionen eines Markerprotein-Gens transkribiert werden können, die ansonsten - unter natürlichen Bedingungen - nicht transkribiert werden, wie beispielsweise Promotorregionen.

Die dsRNA kann aus einem oder mehr Strängen von Polyribonukleotiden bestehen. Natürlich können, um den gleichen Zweck zu erreichen, auch mehrere individuelle dsRNA Moleküle, die jeweils einen der oben definierten Ribonukleotidsequenzabschnitte umfassen, in die Zelle oder den Organismus eingebracht werden. Die doppelsträngige dsRNA-Struktur kann ausgehend von zwei komplementären, separaten RNA-Strängen oder - bevorzugt - ausgehend von einem einzelnen, selbstkomplementären RNA-Strang gebildet werden. In diesem Fall sind "sense"-RNA-Strang und "antisense"-RNA-Strang bevorzugt kovalent in Form eines invertierten "Repeats" miteinander verbunden.

Wie z.B. in WO 99/53050 beschrieben, kann die dsRNA auch eine Haarnadelstruktur umfassen, indem "sense"- und "antisense"-Strang durch eine verbindende Sequenz ("Linker"; beispielsweise ein Intron) verbunden werden. Die selbstkomplementären dsRNA-Strukturen sind bevorzugt, da sie lediglich die Expression einer RNA-Sequenz erfordern und die komplementären RNA-Stränge stets in einem äquimolaren Verhältnis umfassen. Bevorzugt kann ist die verbindende Sequenz ein Intron (z.B. ein Intron des ST-LS1 Gens aus Kartoffel; Vancanneyt GF et al. (1990) Mol Gen Genet 220(2):245-250).

Die Nukleinsäuresequenz kodierend für eine dsRNA kann weitere Elemente beinhalten, wie beispielsweise Transkriptionsterminationssignale oder Polyadenylierungssignale.

Sollen die zwei Stränge der dsRNA in einer Zelle oder Pflanze zusammengebracht werden, so kann dies beispielhaft auf folgende Art geschehen:
a) Transformation der Zelle oder Pflanze mit einem Vektor, der beide Expressionskassetten umfasst,
b) Kotransformation der Zelle oder Pflanze mit zwei Vektoren, wobei der eine die Expressionskassetten mit dem "sense"-Strang, der andere die Expressionskassetten mit dem "antisense"-Strang umfasst.

Die Bildung der RNA Duplex kann entweder außerhalb der Zelle oder innerhalb derselben initiiert werden.

Die dsRNA kann entweder in vivo oder in vitro synthetisiert werden. Dazu kann eine DNA-Sequenz kodierend für eine dsRNA in eine Expressionskassette unter Kontrolle mindestens eines genetischen Kontrollelementes (wie beispielsweise einem Promotor) gebracht werden. Eine Polyadenylierung ist nicht erforderlich, ebenso müssen keine Elemente zur Initiierung einer Translation vorhanden sein. Bevorzugt ist die Expressionskassette für die MP-dsRNA auf dem Transformationskonstrukt oder dem Transformationsvektor enthalten. Die Expressionskassetten kodierend für den "antisense"- und/oder den "sense"-Strang einer MP-dsRNA oder für den selbstkomplementären-Strang der dsRNA, werden dazu bevorzugt in einen Transformationsvektor insertiert und mit den unten beschriebenen Verfahren in die pflanzliche Zelle eingebracht. Für das Erfindungsgemäße Verfahren kann eine stabile Insertion in das Genom vorteilhaft sein, ist aber nicht zwingend erforderlich. Da eine dsRNA einen langanhaltenden Effekt bewirkt, ist in vielen Fällen auch eine transiente Expression ausreichend. Die dsRNA kann auch Teil der von der zu insertierenden Nukleinsäuresequenz zu exprimierenden RNA sein, indem sie beispielsweise an den 3'-untranslatierten Teil besagter RNA fusioniert wird.

Die dsRNA kann in einer Menge eingeführt werden, die zumindest eine Kopie pro Zelle ermöglicht. Höhere Mengen (z.B. mindestens 5, 10, 100, 500 oder 1000 Kopien pro Zelle) können ggf. eine effizienter Verminderung bewirken.

### b) Einbringen einer antisense-Ribonukleinsäuresequenz eines Markerproteins (MP-antisenseRNA)

Verfahren zur Verminderung eines bestimmten Proteins durch die "antisense"-Technologie sind vielfach - auch in Pflanzen - beschrieben (Sheehy et al. (1988) Proc Natl Acad Sci USA 85: 8805-8809; US 4,801,340; Mol JN et al. (1990) FEBS Lett 268(2):427-430). Das antigense Nukleinsäuremolekul hybridisiert bzw. bindet mit der zellularen mPNA und/oder genomischen DNA- kodierend für das zu vermindernde Markerprotein. Dadurch wird die Transkription und/oder Translation des Markerprotein unterdrückt. Die Hybridisierung kann auf konventionelle Art über die Bildung einer stabilen Duplex oder - im Fall von genomischer DNA - durch Bindung des antisense Nukleinsäuramoleküls mit der Duplex der genomischen DNA durch spezifische Wechselwirkung in der groben Furche der DNA-Helix entstehen.

Eine MP-antisenseRNA kann unter Verwendung der für dieses Markerprotein kodierenden Nukleinsäuresequenz, beispielsweise der Nukleinsäuresequenz gemäß SEQ ID NO: 1, 3, 5, 7, 9, 11, 13, 15, 17, 19, 21, 23, 25, 27, 29, 31, 33, 35, 37, 39, 41, 43, 45 oder 47 nach den Basenpaarregeln von Watson und Crick abgeleitet werden. Die MP-antisenseRNA kann zu der gesamten transkribierten mRNA des Markerproteins komplementür sein, sich auf die kodierenden Region beschränken oder nur aus einem Oligonukleotid bestehen, das zu einem Teil der kodierenden oder nicht-kodierenden Sequenz der mRNA komplementär ist. So kann das Oligonukleotid beispielsweise komplementär zu der Region sein, die den Translationsstart für das Markerprotein umfasst. Die MP-antisenseRMA kann eine Länge von zum Beispiel 5, 10, 15, 20, 25, 30, 35, 40, 45 oder 50 Nukleotide haben, kann aber auch länger sein und mindestens 100, 200, 500, 1000, 2000 oder 5000 Nukleotide umfassen. MP-antisenseRNA kann bevorzugt rekombinant in der Zielzelle exprimiert werden.

Die MP-antisenseRNA kann auch Teil einer von der zu invertierenden Nukleinsäuresequenz zu exprimierenden RNA sein, indem sie beispielsweise an den 3'-untranslatierten Teil besagte RNA fusioniert ist.

Hierzu können transgene Expressionskassetten enthaltend eine Nukleinsäuresequenz kodierend für zumindest einen Teil eines Markerproteins, wobei besagte Nukleinsäuresequenz mit einem in pflanzlichen Organismen funktionellen Promotor in antisense-Orientierung funktionell verknüpft ist, verwendet werden. Besagte Expressioinskasselten können Teil eines Transformationskonstruktes oder Transformationsvektors sein, oder aber auch im Hahmen einer Kotransformation eingeführt werden.

In einer Variante kann die Expression eines Markerproteins durch Nukleotidsequenzen inhibiert werden, die komplementär zu der regulatorischen Region eines Markerprotein-Gens (z.B. einem Markerprotein Promoter und/oder Enhancer) sind und triple-helikale Strukturen mit der dortigen DNA-Doppelhelix ausbilden, so dass die Transkription des Markerprotein-Gens verminderte wird. Entsprechende Verfahren sind beschrieben (Helene C (1991) Anticancer Drug Res 6(6):569-64; Helene C et al. (1992) Ann NY Acad Sci 660:27-36; Maher LJ (1992) Bioassays 14(12):807-815).

In einer anderen Variante kann die MP-antisenseRNA eine α-anomers Nukleinsäure sein. Derartige α-anomere Nukleinsäuremoleküle bilden spezifische doppelstrangige Hybride mit komplementarer RNA in denen, - im Unterschied zu den konventionellen β-Nukleinsäuren - die beiden Stränge parallel zueinander verlaufen (Gautier C et al. (1987) Nucleic Acids Res 15:6625-6641).

### c) Einbringen einer MP-antisenseRNA kombiniert mit einem Ribozym

Vorteilhaft kann die oben beschriebene antisense-Strategie mit einem Ribozym Verfahrens gekoppelt werden. Katalytische RNA-Molekule oder Ribozyme können an jede beliebige Ziel-RNA angepasst werden und spalten das Phosphodiester-Gerüst an spezifischen Positionen, wodurch die Ziel-RNA funktionell deaktiviert wird (Tanner NK (1999) FEMS Microbiol Rev 23(3):257-275). Das Ribozym wird dadurch nicht selber modifiziert, sondern ist in der Lage, weitere Ziel-RNA-Moleküle analog zu spalten, wodurch es die Eigenschaften eines Enzyms erhält. Der Einbau von Ribozymsequenzen in "antisense"-RNAs verleiht eben diesen "antisense"-RNAs diese enzymahnliche, RNA-spaltende Eigenschaft und steigert so deren Effizienz bei der Inaktivierung der Ziel-RHA. Die Herstellung und Verwendung entsprechender Ribozym-"antisense"-RNA-Molecule ist beschrieben (u.a. bei Haseloff et al. (1988) Nature 334: 585-591); Haselhoff und Gerlach (1988) Nature 334:585-591; Steinecke P et al. (1992) EMBO J 11(4):1525- 1530; de Feyter R et al. (1996) Mol Gen Genet. 250(3):329-338).

Auf diese Art können Ribozyme (z.B. "Hammerhead"-Ribozyme; Haselhoff und Gerlach (1988) Nature 334:585-591) verwendet werden, um die mRNA eines zu vermindernden Markerproteins katalytisch zu spalten und so die Translation zu verhindern. Die Ribozym-Technologie kann die Effizienz einer antisense-Strategie erhöhen. Verfahren zur Expression von Ribozymen zur Verminderung bestimmter Proteine sind beschrieben in (EP 0 291 533, EP 0 321 201, EP 0 360 257). In pflanzlichen Zellen ist eine Ribozym-Expression ebenfalls beschrieben (Steinecke P et al. (1992) EMBO J 11(4):1525-1530; de Feyter R et al. (1996) Mol Gen Genet. 250 (3):329-338). Geeignete Zielsequenzen und Ribozyme können zum Beispiel wie bei "Steinecke P, Ribozymes, Methods in Cell Biology 50, Galbraith et al. ads, Academic Press, Inc. (1995), S. 449-460" beschrieben, durch Sekundarstrukturberechnungen von Ribozym- und Ziel-RNA sowie durch deren Interaktion bestimmt werden (Bayley CC et al. (1992) Plant Mol Biol. 18(2):353-361; Lloyd AM and Davis RW et al. (1994) Mol Gen Genet. 242(6):653-657). Beispielsweise können Derivate der Tetrahymena L-19 IVS RNA konstruiert werden, die komplementäre Bereiche zu der mRNA des zu supprimierenden Markerproteins aufweisen (siehe auch US 4,987,071 und US 5,116.742). Alternativ können solche Ribozyme auch über einen Selektionsprozess aus einer Bibliothek diverser Ribozyme identifiziert werden(Bartel D und Szostak JW (1993) Science 261:1411-1418).

### d) Einbringen einer sense-Ribonukleinsäuresequenz eines Markerproteins (MP-senseRNA) zur Induktion einer Kosuppression

Die Expression einer Markerprotein Ribonukleinsäuresequenz (oder eines Teils derselben) in sense-Orientierung kann zu einer Kosuppression des entsprechenden Markerproteingens führen. Die Expression von sense-RNA mit Homologie zu einem endogenen Markerproteingen kann die Expression desselben vermindern oder ausschalten, ähnlich wie es für antisense Ansätze beschrieben wurde (Jorgensen et al. (1996) Plant Mol Biol 31(5):957-973; Goring et al. (1991) Proc Natl Acad Sci USA 88:1770-1774; Smith et al. (1990) Mol Gen Genet 224:447-481; Napoli et al. (1990) Plant Cell 2:279-289; Van der Krol et al. (1990) Plant Cell 2:291-99). Dabei kann das eingeführte Konstrukt das zu vermindernde, homologe Gen ganz oder nur teilweise repräsentieren. Die Möglichkeit zur Translation ist nicht erforderlich. Die Anwendung dieser Technologie auf Pflanzen ist beschrieben (z.B. Napoli et al. (1990) Plant Cell 2:279-289; in US 5,034,323.

Bevorzugt wird die Kosuppression unter Verwendung einer Sequenz realisiert, die im wesentlichen identisch ist zu zumindest einem Teil der Nukleinsäuresequenz kodierend für ein Markerprotein, beispielsweise der Nukleinsäuresequenz gemäß SEQ ID NO: 1, 3, 5, 7, 9, 11, 13, 15, 17, 19, 21, 23, 25, 27, 29 ,31, 33, 35, 37, 39, 41, 43, 45 oder 47.

Bevorzugt ist die MP-senseRNA so gewählt, dass es nicht zu einer Translation des Markerproteins oder eines Teils desselben kommen kann. Dazu kann beispielsweise der 5'-untranslatierte oder 3'-untranslatierte Bereich gewählt oder aber das ATG-Startkodon deletiert oder mutiert werden.

### e) Einbringen von DNA-oder Protein-bindende Faktoren gegen Markerprotein Gene, -RNAs oder Proteine

Eine Verminderung einer Markerprotein Expression ist auch mit spezifischen DNA-bindenden Faktoren z.B. mit Faktoren vom Typ der Zinkfingertranskriptionsfaktoren möglich. Diese Faktoren lagern sich an die genomische Sequenz des endogenen Zielgens, bevorzugt in den regulatorischen Bereichen, an und bewirken eine Verminderung der Expression. Entsprechende Verfahren zur Herstellung entsprechender Faktoren sind beschrieben (Dreier B et al. (2001) J Biol Chem 276(31):29466-78; Dreier B et al. (2000) J Mol Biol 303(4):489-502; Beerli RR et al. (2000) Proc Natl Acad Sci USA 97 (4):1495-1500; Beerli RR et al. (2000) J Biol Chem 275(42):32617-32627; Segal DJ and Barbas CF 3rd. (2000) Curr Opin Chem Biol 4(1):34-39; Kang JS and Kim JS (2000) J Biol Chem 275(12):8742-8748: Beerli RR et al. (1998) Proc Natl Acad Sci USA 95(25):14628- 14633; Kim JS et al. (1997) Proc Natl Acad Sci USA 94(8):3616 -3620; Klug A (1999) J Mol Biol 293(2):215-218; Tsai SY et al. (1998) Adv Drug Deliv Rev 30(1-3):23-31; Mapp AK et al. (2000) Proc Natl Acad Sci USA 97(8):3930-3935; Sharrocks AD et al. (1997) Int J Biochem Cell Biol 29(12):1371-1387; Zhang L et al. (2000) J Biol Chem 275(43):33850-33860).

Die Selektion dieser Faktoren kann unter Verwendung eines beliebigen Stückes eines Markerprotein-Gens erfolgen. Bevorzugt liegt dieser Abschnitt im Bereich der Promotorregion. Für eine Genunterdrückung kann er aber auch im Bereich der kodierenden Exons oder Introns liegen.

Ferner können Faktoren in eine Zelle eingebracht werden, die das Markerprotein selber inhibieren. Diese proteinbindenden Faktoren können z.B. Aptamere (Famulok M und Mayer G (1999) Curr Top Microbiol Immunol 243:123-36) oder Antikörper bzw. Antikörperfragmente oder einzelkettige Antikörper sein. Die Gewinnung dieser Faktoren ist beschrieben (Owen M et al. (1992) Biotechnology (N Y) 10(7):790-794; Franken E et al. (1997) Curr Opin Biotechnol 8(4):411-416; Whitelam (1996) Trend Plant Sci 1:286-272).

### f) Einbringen von den Markerprotein RNA-Abbau bewirkenden viralen Nukleinsäuresequenzen und Expressionskonstrukten

Die Markerprotein Expression kann effektiv auch durch Induktion des spezifischen Markerprotein RNA-Abbaus durch die Pflanze mit Hilfe eines viralen Expressionssystems (Amplikon; Angell SM et al. (1999) Plant J 20(3):357-362) realisiert werden. Diese Systeme - auch als "VIGS" (viral induced gene silencing) bezeichnet - bringen Nukleinsäuresequenzen mit Homologie zu dem Transkript eines zu vermindernden Markerproteins mittels viraler Vektoren in die Pflanze ein. Die Transkription wird sodann - vermutlich mediiert durch pflanzliche Abwehrmechanismen gegen Viren - abgeschaltet. Entsprechende Techniken und Verfahren sind beschrieben (Ratcliff F et al. (2001) Plant J 25(2):237-45; Fagard M und Vaucheret H (2000) Plant Mol Biol 43(2-3):285-93; Anandalakshmi R et al. (1998) Proc Natl Acad Sci USA 95(22):13079-84; Ruiz MT (1998) Plant Cell 10(6):937-46).

Bevorzugt wird die VIGS-vermittelte Verminderung unter Veiwendung einer Sequenz realisiert, die im wesentlichen identisch, ist zu zumindest einem Teil der Nukleinsäuresequenz kodierend für ein Markerprotein, beispielsweise der Nukleinsäuresequenz gemäß SEQ CD NO: 1, 3, 5, 7, 9, 11, 13, 15, 17, 19, 21, 23, 25, 27, 29, 31, 33, 36, 37, 39, 41, 43, 45 oder 47.

### g) Einbringen von Konstrukten zur Erzeugung eines Funktionsverlustes oder einer Funktionsminderung an Markerprotein-Genen

Dem Fachmann sind zahlreiche Verfahrens bekannt, wie genomische Sequenzen gezielt modifiziert werden können. Dazu zählen insbesondere verfahren wie die Erzeugung von Knockout-Mutanten mittels gezielter homologen Rekombination z.B. durch Generierung von Stopp-Kodons, Verschiebungen im Leseraster etc. (hohn B und Puchta H (1999) Proc Natl Acad Sci USA 36:8321-8323) oder die gezielte Deletion oder Inversion von Sequenzen mittels z.B. sequenzspezifischer Rekombinasen oder Nukleasen (s.u.)

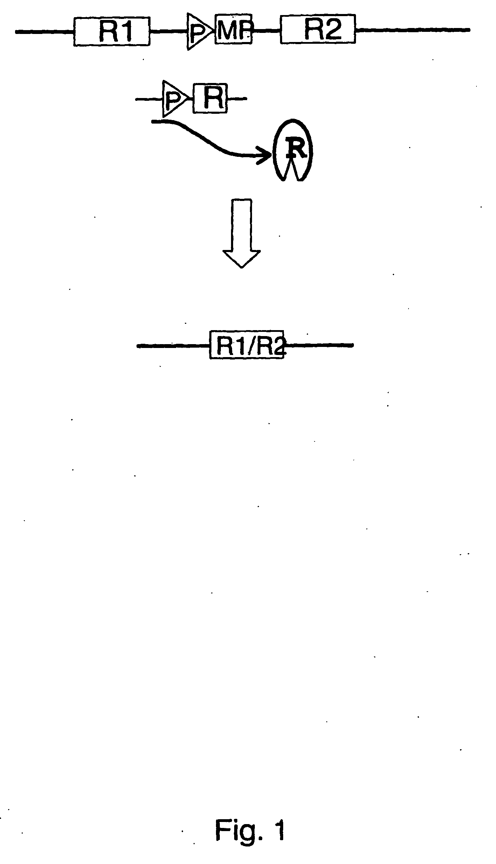

In einer Variante wird die Inaktivierung des Markerprotein-Gens durch Einbringen einer sequenzspezifischen Rekombinase realisiert. So kann beispielsweise das Markerproteingen Erkennungssequenzen für sequenzspezifische Rekombinasen umfassen oder von solchen flankiert sein, wobei dann durch Einbringen der Rekombinase bestimmte Sequenzen des Markerproteingens deletiert oder invertiert werden und so eine Inaktivierung des Markerproteinsgens erfolgt. Ein entsprechendes Vorgehen ist aschematisch in Fig. 1 dargestellt.

Entsprechende Verfahren zur Deletion/Inversion von Sequenzen mittels sequenzspezifischer Rekombinasesysteme sind dem Fachmann bekannt. Beispielhaft seien zu nennen das Cre/lox-System des Bacteriophagen P1 (Dale EC und Ow DW (1991) Proc Natl Acad Sci USA 88:10558-10562; Russell SR et al. (1992) Mol Gen Genet 234:49-59; Osborne BI et al. (1995) Plant J 7:687-701), das FLP/FRT System der Hefe (Kilby NJ et al. (1995) Plant J 8:637-652; Lyznik LA et al. (1996) Nucl Acids Res 24:3784-3789), die Gin Rekombinase des Mn Phagen, die Pin Rekombinase aus E.coli oder das R/RS System des pSR1 Plasmids (Onouchi. H et al. (1995) Mol Gen Genet 247:653- 660; Sugita K et al (2000) Plant J. 22:461-469). Bei diesen Systemen interagiert die Rekombinase (beispielsweise Cre oder FLP) spezifisch mit ihren jeweiligen Rekonbinationssequenzen (34 bp lox-Sequenz bzw. 47 bp FRT-Sequenz). Bevorzugt sind das Bacteriophagen P1 Cre/lox und das Hefe FLP/FRT System. Das FLP/FRT und cre/lox Rekombinasesystem wurde bereits in pflanzlichen Systemen angewendet (Odell et al. (1990) Mol Gen Genet 223:369-378). Das Einbringen der Rekombinase wird bevorzugt mittels rekombinanter Expression ausgehend von einer auf einem DNA-Konstrukt umfassen. Expressionskassette realisiert.

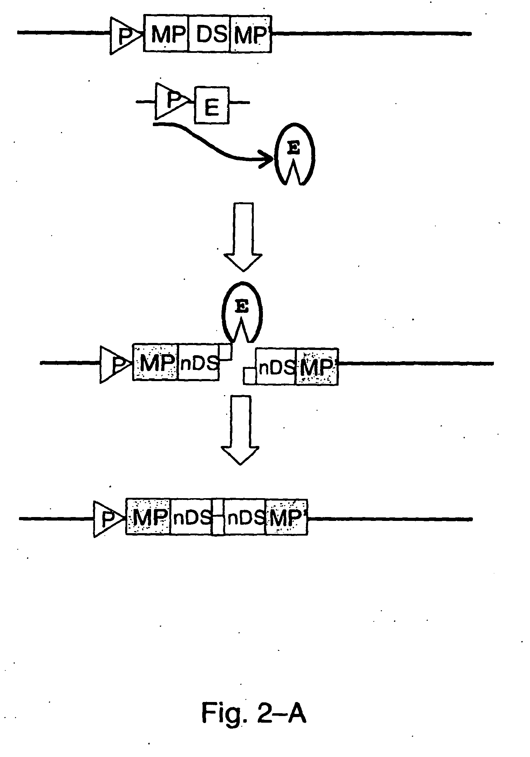

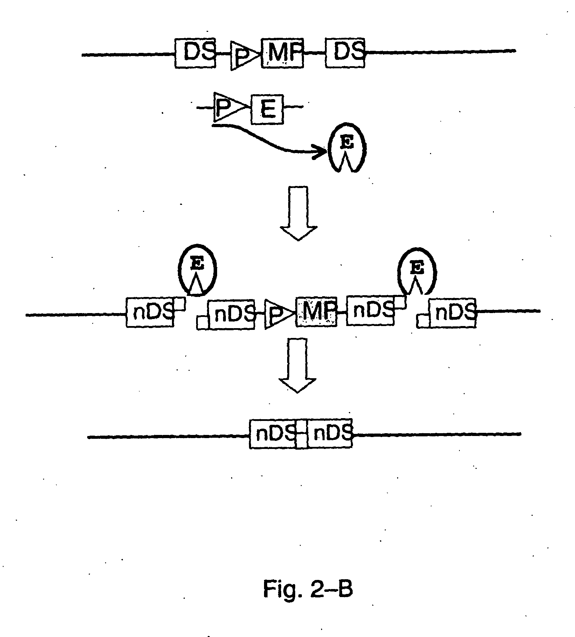

Die Verminderung der Markerprotein-Aktivität oder -Menge kann auch durch eine gezielte Deletion im Markerprotein-Gen z.B. durch sequenzspezifische Induktion von DNA-Doppelstrangbrüchen an einer Erkennungssequenz zur gezielten Induktion von DNA-Doppelatrangbrüchen in oder in der Nähe der Nukleinsäuresequenz kodierend für ein Markerprotein realisiert werden. In seiner einfachsten Ausführungsform (vgl. Fig. 2, A und B) wird herbei ein Enzym mit dem Transformationskonstrukt eingebracht, dass mindestens einen Doppelstrangbruch derart erzeugt, dass die resultierende illegitime Rekombination oder Deletion eine verminderung der Markerprotein-Aktivitat oder -Menge - beispielsweise durch Induzieren einer verschiebung im Leseraster oder Deletion essentieller Sequenzen - bewirkt.

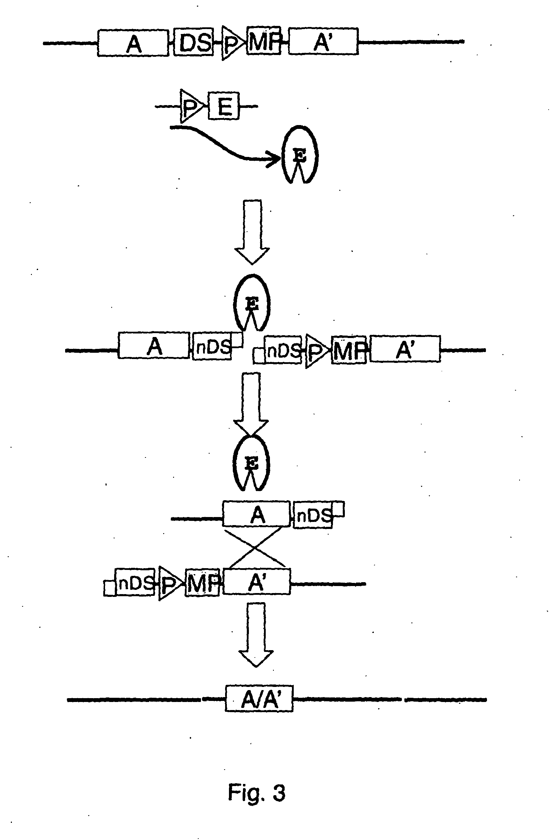

Die Effizienz dieses Ansatz kann gesteigert werden, indem die Sequenz kodierend für das Markerprotein von Sequenzen (A bzw. A') flankiert ist, die eine ausreichenden Länge und Homologie zueinander haben, um infolge des induzierten Doppelstrangbruches miteinander zu rekombinieren und so - durch eine intramolekulare homologe Rekonbination - eine Deletion der Sequenz kodierend für das Markerprotein- zu bewirken. Ein entsprechenden Vorgehen ist in einer beispielhaften Ausführungsform dieser Variante in Fig. 3 schematisch dargestellt.

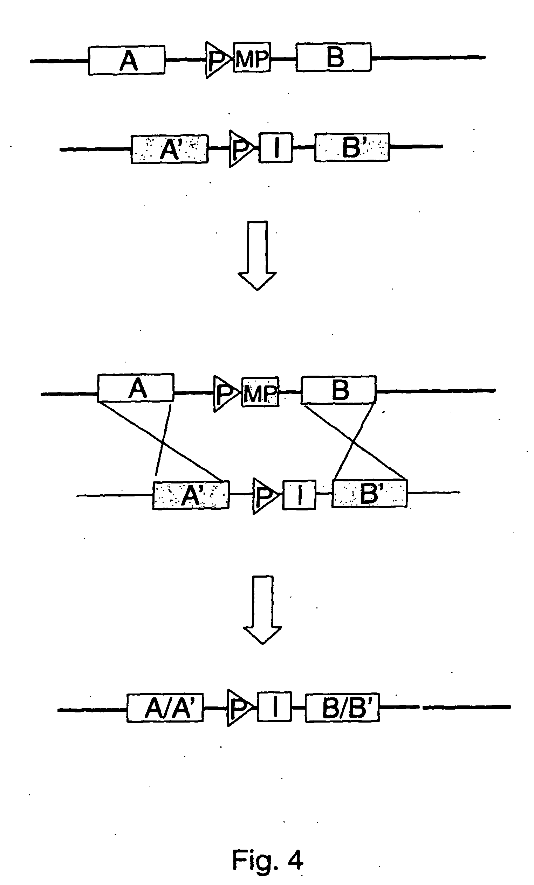

Die Verminderung der Markerprotein-Menge, -Funktion und/oder - Aktivität kann auch durch eine gezielte Insertion von Nukleinsäuresquenzen (z.B. der im Rahmen der erfindungsgemäβen Verfahrens zu insertierenden Nukleinsäuresequenz) in die Sequenz kodierend für ein Markerprotein (z.B. mittels interMolekularer homologer Rekombination) realisiert werden. Diese Variante ist besonders vorteilhaft und bevorzugt, da es neben den allgemeinen Vorteilen zudem noch eine reproduzierbare, vorhersagbare, ortsspezifische Insertion der zu invertierenden Nukleinsauresequenz in das pflanzliche Genom ermoglicht. Dadurch werden die ansonsten im Rahmen einer zufälligen, ortsunspezifischen Insertion auftretenden Positionseffekte (die sich beispielsweise in Form von unterschiedlichen Expressionshohen des Transgens oder einer unbeabsichtigen Inaktivierung endogener Gene äußern können) vermieden. Im Rahmen dieser variante verwendet man als "anti-Markerprotein"-Verbindung bevorzugt ein DNA-Konstrukt, das zumindest einen Teil der Sequenz eines Markerproteingens oder benachbarter Sequenzen umfasst, und so mit diesen in der Zielzelle gezielt rekombinieren kann, so dass durch eine Deletion, Addition oder Substitution mindestens eines Nukleotids das Markerproteingen so verändert wird, dass die Funktionalität des Markerprotein-Gens verminderte oder gänzlich aufgehoben wird. Die Veränderung kann auch die regulativen Elemente (z.B. den Promotor) des Markerprotein-Gens betreffen, so dass die kodierenden Sequenz unverändert bleibt, eine Expression (Transkription und/oder Translation) jedoch unterbleibt und vermindert wird. Bei der konventionellen homologen Rekombination ist die zu insertierende Sequenz an ihrem 5'- und/oder 3' -Ende von weiteren Nukleinsäuresequenzen (A' bzw. B') flankiert, die eine ausreichende Länge und Homologie zu entsprechenden Sequenzen des Markerprotein-Gens (A bzw. B) für die Ermoglichung der homologen Rekombination aufweisen. Die Länge liegt in der Regel in einem Bereich von mehreren hundert Basen bis zu mehreren kiloleasen (Thomas KR und Capecchi MR (1987) Cell 51:503; Strepp et al. (1998) Proc Natl Acad Sci USA 35(8):4368-4373). Für die homologe Rekombination wird die pflanzliche Zelle mit dem Rekombinationskonstrukt unter Verwendung der unten beschriebenen Verfahren transformiert und erfolgreich rekombinierte Klone basierend auf dem infolge inaktivierten Markerprotein selektioniert. Obgleich homologe Rekombination ein relativ seltenes Ereignis in pflanzlichen Organismen ist, kann durch die Rekombination in das Markerprotein-Gen hinein einem Selektionsdruck ausgewichen werden, was eine Selektion der rekombinierten Zellen und eine hinreichende effizienz des Verfahrens erlaubt. Ein entsprechendes vorgeben ist in einer beispielhaften Ausführungsform dieser Variante in Fig. 4 schematisch dargestellt.

In einer vorteilhaften Ausführungsform dar Erfindung wird jedoch die Insertion in das Markerproteingen mittels weiterer Funktionselemente erleichtert. Der Begriff ist umfassend zu verstehen und meint die Verwendung von Sequenzen bzw. von diesen abgeleiteten Transkripten oder Polypeptiden, die die Effizienz der gezielten Integration in ein Markerprotein-Gen zu steigern vermogen, Dazu stehen dem Fachmann verschiedene Verfahren zur Verfügung. Bevorzugt wird jedoch die Insertion durch Induktion eines sequenzspezifischen Doppeistrangbruches in oder in der Nahe des Markerprotein-Gens realisiert.

In einur Variante wird die Inaktivierung (d.h. die Verminderung der Menge, Expression, Aktivität oder Funktion) des Markerproteins durch Integration einer DNA-Sequenz in ein Markerprotein-Gen realisiert, wobei das Verfahren bevorzugt nachfolgende Schritte umfasst:
i) Einbringen eines Insertionskonstruktes und mindestens eines Enzyms geeignet zur Induktion von DNA-Doppelstrangbrüchen an einer Erkennungssequenz zur gezielten Induktion von DNA-Doppelstrangbrüchen in oder in der Nähe des Markerprotein-Gens, und
ii) Induktion von DNA-Doppelstrangbrüchen an den Erkennungssequenzen zur gezielten Induktion von DNA-Doppelstrangbrüchen in oder in der Nähe des Markerprotein-Gens, und
iii) Insertion des Insertionskonstruktes in das Markerprotein-Gen, wobei die Funktionalität des Markerprotein-Gens und - bevorzugt - die Funktionalität der Erkennungssequenz zur gezielten Induktion von DNA-Doppelstrangbrüchen inaktiviert wird, so dass besagte Erkennungssequenz nicht mehr durch das Enzym geeignet zur Induktion von DNA-Doppelstrangbrüchen geschnitten werden kann, und
iv) Selektion von Pflanzen oder pflanzlichen Zellen, bei denen das Insertionskonstrukt in das Markerproteingen insertiert wurde.

Das Insertionskonstrukt umfasst - bevorzugt - die in das Genom zu insertierende Nukleinsäuresequenz, kann aber auch separat von dieser eingesetzt werden.
"Enzym geeignet zur Induktion von DNA-Doppelstrangbrüchen an der Erkennungssequenz zur gezielten Induktion von DNA-Doppelstrangbrüchen" (infolge "DSBI-Enzym" für "double strand-break inducing enzyme") meint allgemein all solche Enzyme, die in der Lage sind, sequenzspezifisch Doppelstrangbrüche in doppelsträngige DNA zu erzeugen. Beispielsweise, aber nicht einschränkend, sind zu nennen:
1. Restriktionsendonukleasen, bevorzugt Typ II Restriktionsendonukleasen, besonders bevorzugt Homing-Endonukleasen wie weiter unten im Detail beschrieben.
2. Künstliche Nukleasen wie weiter unten im Detail beschrieben, wie beispielsweise chimäre Nukleasen, mutierte Restriktions- oder Homing-Endonukleasen oder RNA-Proteinpartikel abgeleitet von mobilen Introns der Gruppe II.

Sowohl natürliche als auch künstlich hergestellte DSBI-Enzyme sind geeignet. Bevorzugt sind all solche DSBI-Enzyme, deren Erkennungssequenz bekannt ist und die entweder in Form ihrer Proteine (beispielsweise durch Aufreinigung) gewonnen oder unter Verwendung ihrer Nukleinsäuresequenz exprimiert werden können.

Bevorzugte wird das DSBI-Enzym unter Kenntnis seiner spezifischen Erkennungssequenz so ausgewählte dass es zusätzlich zu der Ziel-Erkennungssequenz keine weitere funktionellen Erkennungsregionen im Genom der Zielpflanze besitzt. Ganz besonders bevorzugt sind deshalb Homing-Endonukleasen (Übersicht: Belfort M und Roberts RJ (1997) Nucleic Acids Pes 25:3379-9988; Jasin M (1996) Trends Genet 12:224-228; Roberts RJ and Macelis D (2001) Nucl Acids Res 29: 268-263). Diese erfüllen aufgrund ihrer langen Erkennungssequenzen diese Anforderung. Die für derartige Homing-Endonukleasen kodierenden Sequenzen können beispielsweise aus dem Chloroplastengenom von Chlamydomonas isoliert werden (Turmel M et al. (1993) J Mol Biol 232:446-467). Zu nennen sind beispielsweise Homing-Endonukleasen wie F-SceI, F-SceII, F-SuvI, F-TevI, F-TevII, I-AmaI, I-AniI, I-CeuI, I-CeuAIIP, I-ChuI, I-CmoeI, I-CpaI, I-CpaiI, I-CreI, I-CrepsbIP, I-GrepsbIIP, I-CrepsbIIIP, I-CrepsbIVP, I-CsmI, I-CvuI, I-CvuAIP, I-DdiII, I-DirI, I-Dmol, I-HspNIP, I-L1aI, I-MsoI, I-NaaI, I-NanI, I-Nc1IP, I-NgrIP, I-NitI, I-NjaI, I-Nsp236IP, I-PskI, I-PboIP, I-PcuIP, I-PcuAI, I-PcuVI, I-PgrIP, I-PobIP, I-PorI, I-PorIIP, I-PpbIP, I-PpoI, I-SPBetaIP, I-ScaI, I-SceI, I-SceII, I-SceIII, I-SceIV, I-SceV, I-SceVI, I-SceVII, I-SexIP, I-SneIP, I-SpomCP, I-SpomIP, I-SpomIIP, I-SquIP, I-Ssp6803T, I-SthPhiJP, I-SthPhiST3P, I-SthPhiS3bP, I-TdeIP, I-TevI, I-TevII, I-TevIII, I-UarAP_{,} I-JarHGPA1P, I-UarHGPA13P, I-VinIP, I-ZbiIP, PI-McuI, PI-MtuHIP, PI-MtuHIIP, PI-PfuI, PI-PfuII, PI-PkoI, PI-PkoII, PI-PspI, PI-Rma43812IP, PI-SPBetaIP, PI-SceI, PI-TfuI, PI-TfuII, PI-ThyI, PI-TiiI, PI-TliII. Bevorzugt sind dabei die Homing-Endonukleasen, deren Gensequenzen bereits bekannt sind, wie beilspielsweise F-SceI, I-CeuI, I-ChuI, I-DmoI, I-CpaI, I-CpaII, I-CreI, I-CsmI, F-TevI, F-TevII, I-TevI, I-TevII, I-AniI, I-CvuI, I-LlaI, I-NanI, I-MsoI, I-NitI, I-NjaI, I-PakI, I-PorI, I-PpoI, I-ScaI, I-Ssp6803I, PI-PkeI, PI-PkoII, PI-PspI, PI-TfuI, PI-TliI.

Ganz besonders bevorzugt sind
- I-CeuI (Cote MJ und Turmel M (1995) Curr Genet 27:177-183.; Gauthier A et al. (1991) Curr Genet 19:43-47; Marshall (1991) Gene 104:241-245; GenBank Acc.-No.: Z17234 Nukleotide 5102 bis 5758},
- I-ChuI (Cote V et al.(1993) Gene 129:69-76; GenBank Acc.-No.: L06107, Nukleotide 419 bis 1075),
- I-CmoeI (Drouin M et al. (2000) Nucl Acids Res 28:4566-4572),
- I-CpaI aus Chlamydomonas pallidostigmatica (GenBank Acc.-No.: L36830, Nukleotide 357 bis 815; Turmel M et al. (1995) Nucleic Acids Res 23:2519-2525; Turmel, M et al. (1995) Mol Biol Evol 12:533-545)
- I-CpaII (Turmel M et al. (1995) Mol Biol Evol 12:533-545; GenBank Acc.-No.: L39865, Nukleotide 719 bis 1423),
- I-CreI (Wang J et al. (1997) Nucleic Acids Res 25: 3767-3776; Dürrenberger, F und Rochaix JD (1991) EMBO J 10:3495-3501; GenBank Acc.-No.: X01977, Nukleotide 571 bis 1062),
- I-CsmI (Ma DP et al. (1992) Plant Mol Biol 18:1001-1004)
- I-NanI (Elde M et al. (1999) Eur J Biochem. 259:281-288; GenBank Acc.-No.: X78280, Nukleotide 418 bis 1155),
- I-NitI (GenBank Acc.-No.: X78277, Nukleotide 426 bis 1163),
- I-NjaI (GenBank Acc.-No.: X78279, Nukleotide 416 bis 1153),
- I-PpoI (Muscarella DE und Vogt VM (1989) Cell 56:443-454; Lin J und Vogt VM (1998) Mol Cell Biol 18:5809-5817; GenBank Acc.-No.: M38131, Nukleotide 86 bis 577),
- I-PspI (GenBank Acc.-No.: U00707, Nukleotide 1839 bis 3449),
- I-ScaI (Monteilhet C et al. (2000) Nucleic Acids Res 28: 1245-1251; GenBank Acc.-No.: X95974, Nukleotide 55 bis 465)
- I-SceI (WO 96/14408; US 5,962,327 dort Seq ID NO: 1),
- Endo SceI (Kawasaki et al. (1991) J Biol Chem 266:5342-5347, identisch zu F-SceI; GenBank Acc.-No.: M63839, Nukleotide 159 bis 1589),
- I-SceII (Sarguiel B et al. (1990) Nucleic Acids Res 18:5659-5665),
- I-SceIII (Sarguiel B et al. (1991) Mol Gen Genet. 255:340-341),
- I-Ssp6803I (GenBank Acc.-No.: D64003, Nukleotide 35372 bis 35824),
- I-TevI (Chu et al. (1990) Proc Natl Acad Sci USA 87:3574-3578; Bell-Pedersen et al. (1990) Nucleic Acids Res18:3763-3770; GenBank Acc.-No.: AF158101, Nukleotide 144431 bis 143694),
- I-TevII (Bell-Pedersen et al. (1990) Nucleic Acids Res 18:3763-3770;GenBank Acc.-No.: AF158101, Nukleotide 45612 bis 44836),
- I-TevIII (Eddy et al. (1991) Genes Dev. 5:1032-1041),

Ganz besonders bevorzugt sind kommerziell erhältliche Homing-Endonukleasen wie I-CeuI, I-SceI, I-PpoI, PI-PspI oder PI-SceI. Am meisten bevorzugt sind I-SceI und I-PpoI. Während das Gen kodierend für I-PpoI in der natürlichen Form genutzt werden kann, besitzt das Gen kodierend für I-SceI eine Editierstelle. Da die entsprechende Editierung in höherer Pflanzen im Unterschied zu den Mitochondrien der Hefe nicht durchgeführt wird, muss eine künstliche das I-SceI Protein kodierende Sequenz zur heterologen Expression dieses Enzyms eingesetzt werden (US 5,866,361).

Die Enzyme können in der dem Fachmann geläufigen Art und Weise aus ihren Herkunftsorganismen aufgereinigt und/oder die für sie kodierende Nukleinsäuresequenz kloniert werden. Die Sequenzen verschiedener Enzyme sind in der GenBank hinterlegt (s.o.).

Als künstliche DSBI-Enzyme seien beispielhaft chimäre Nukleasen zu nennen, die sich aus einer unspezifischen Nukleasedomäne und einer sequenzspezifischen DNA-Bindungsdomäne (z.B. bestehend aus Zinkfingern) zusammensetzen (Smith J et al. (2000) Nucl Acids Res 28(17):3361-3369; Bibikova M et al. (2001) Mol Cell Biol. 21:289-297). So wurde beispielsweise die katalytische Domäne der Restriktionsendonuklease FokI an Zinkfingerbinde-Domänen fusioniert, wodurch die Spezifität der Endonuklease definiert wurde (Chandrasegaran S & Smith J (1999) Biol Chem 380:841-848; Kim YG & Chandrasegaran S (1994) Proc Natl Acad Sci USA 91:883-887; Kim YG et al. (1996) Proc Natl Acad Sci USA 93:1156-1160). Auch die katalytische Domäne der Ho-Endonuklease aus Hefe konnte bereits mit der beschriebenen Technik eine vordefinierte Spezifität verliehen werden, indem diese an die Zinkfingerdomäne von Transkriptionsfaktoren fusioniert wurde (Nahon E & Raveh D (1998) Nucl Acids Res 26:1233-1239). Durch geeignete Mutations- und Selektionsverfahren kann man bestehende Homing-Endonukleasen an jede gewünschte Erkennungssequenz anpassen.

Wie erwähnt, eignen sich insbesondere Zinkfingerproteine als DNA-Bindungsdomäne im Rahmen von chimären Nukleasen. Diese DNA-bindenden Zinkfingerdomänen können an jede beliebige DNA-Sequenz angepasst werden. Entsprechende Verfahren zur Herstellung entsprechender Zinkfingerdomänen sind beschrieben und dem Fachmann bekannt (Beerli RR et al. (2000) Proc Natl Acad Sci 97(4):1495-1500; Beerli RR et al.(2000) J Biol Chem 275(42):32617-32627; Segal DJ and Barbas CF 3rd. (2000) Curr Opin Chem Biol 4(1):34-39; Kang JS and Kim JS (2000) J Biol Chem 275(12):8742-8748; Beerli RR et al. (1998) Proc Natl Acad Sci USA 95(25):14628-14633; Kim JS et al. (1997) Proc Natl Acad Sci USA 94(8):3616-3620; Klug A (1999) J Mol Biol 293(2):215-218; Tsai SY et al. (1998) Adv Drug Deliv Rev 30(1-3):23-31; Mapp AK et al. (2000) Proc Natl Acad Sci USA 97(8):3930-3935; Sharrocks AD et al. (1997) Int J Biochem Cell Biol 29(12):1371-1387; Zhang L et al. (2000) J Biol Chem 275(43):33850-33860). Verfahren zur Herstellung und Selektion von Zink-Finger DNA-Bindedomänen mit hoher Sequenzspezifität sind beschrieben (WO 96/06166, WO 98/53059, WO 98/53057). Durch Fusion einer so erhaltenen DNA-Bindedomäne an die katalytische Domäne einer Endonuklease (wie beispielsweise der FokI oder Ho-Endonuklease) kann man chimäre Nukleasen mit jeder beliebigen Spezifität herstellen, die als DSBI-Enzyme im Rahmen der vorliegenden Erfindung vorteilhaft eingesetzt werden können.

Künstliche DSBI-Enzyme mit veränderter Sequenzspezifität können auch durch Mutagenese bereits bekannter Restriktionsendonukleasen oder Homing Endonukleasen durch dem Fachmann geläufige Methoden erzeugt werden. Insbesondere von Interesse ist neben der Mutagenese von Homing-Endonukleasen mit dem Ziel, eine veränderte Substratspezifität zu erzielen, auch die Mutagenese von Maturasen. Maturasen haben häufig viele Gemeinsamkeiten mit Homing-Endonukleasen und lassen sich durch wenige Mutationen ggf. in Nukleasen umwandeln. Dies wurde beispielsweise für die Maturase im bi2 Intron der Bäckerhefe gezeigt. Lediglich zwei Mutationen in dem die Maturase kodierenden offenen Leseraster (ORF) reichten aus, diesem Enzym eine Homing-Endonuklease Aktivität zu verleihen (Szczepanek & Lazowska (1996) EMBO J 15:3758-3767).

Weitere künstliche Nukleasen können mit Hilfe mobiler Gruppe II Introns und den von ihnen kodierten Proteinen oder Teile dieser Proteine erzeugt werden. Mobile Gruppe II Introns bilden mit den von ihnen kodierten Proteinen RNA-Protein-Partikel, die sequenzspezifisch DNA erkennen und schneiden können. Die SequenzSpezifität kann dabei durch Mutagenese von bestimmten Bereichen des Introns (siehe unten) den Bedürfnissen angepasst werden (WO 97/10362).

Bevorzugt wird das DSBI-Enzym als Fusionsprotein mit einer Kernlokalisationssequenz (NLS) exprimiert. Diese NLS-Sequenz ermöglicht einen erleichterten Transport in den Kern und steigert die Effizienz des Rekombinationssystems. Verschiedene NLS-Sequenzen sind dem Fachmann bekannt und unter anderem beschrieben bei Jicks GR und Raikhel NV (1995) Annu. Rev. Cell Biol. 11:155-188. Bevorzugt für pflanzliche Organismen ist beispielsweise die NLS-Sequenz des SV40 "large antigen". Ganz besonders bevorzugt sind die nachfolgenden NLS-Sequenzen:

| | |
|---|---|
| NLS1: | N-Pro-Lys-Thr-Lys-Arg-Lys-Val-C |
| NLS2: | N-Pro-Lys-Lys-Lys-Arg-Lys-Val-C |

Aufgrund der geringen Größe vieler DSBI-Enzyme (wie beispielsweise der Homing-Endonukleasen) ist jedoch eine NLS-Sequenz nicht zwingend erforderlich. Diese Enzyme können die Kernporen auch ohne die Unterstützung passieren.

"Erkennungssequenz zur gezielten Induktion von DNA-Doppelstranebrüchen" meint allgemein solche Sequenzen, die unter den Bedingungen in der jeweils verwendeten eukaryotischen Zelle oder Organismus die Erkennung und Spaltung durch das DSBI-Enzym erlauben. Beispielhaft aber nicht einschränkend seien dabei in nachfolgender Tabelle 1 die Erkennungssequenzen für die jeweiligen aufgeführten DSBI-Enzyme genannt.

**Tabelle 1: Erkennungssequenzen und Herkunftsorganismus von DSBI-Enzymen ("^" gibt innerhalb einer Erkennungssequenz die Schnittstelle des DSBI-Enzyms an)**

| DSBI-Enzym | Herkunftsorganismus | Erkennungssequenz |
|---|---|---|
| CRE | Bacteriophage P1 | |
| FLP | Saccharomyces cerevisiae | |
| R | pSR1 Plasmids | |
| P-Element Transposase | Drosophila | 5'-CTAGATGAAATAACATAAGGTGG |
| I-Anti | Aspergillus nidulans | 5'-TTGAGGAGGTT^TCTCTGTAAATAANNNNNNNNNNNNNNN |
| | | 3'-AACTCCTCCAAAGAGACATTTATTNNNNNNNNNNNNNNN^ |
| I-DdiI | Dictyostelium discoideumAX3 | 5'-TTTTTTGGTCATCCAGAAGTATAT |
| | | 3'-AAAAAACCAG^TAGGTCTTCATATA |
| I-CwI | Chlorella vulgaris | 5'-CTGGGTTCAAAACGTCGTGA^GACAGTTTGG |
| | | 3'-GACCCAAGTTTTGCAG^CACTCTGTCAAACC |
| I-CsmI | Chlamydomonas smithii | 5'-GTACTAGCATGGGGTCAAATGTCTTTCTGG |
| I-CmoeI | Chlamydomonasmoewusii | 5'-TCGTAGCAGCT^CACGGTT |
| | | 3'-AGCATCG^TCGAGTGCCAA |
| I-CreI | Chlamydomonas reinhardtii | 5'-CTGGGTTCAAAACGTCGTGA^GACAGTTTGG |
| | | 3'-GACCCAAGTTTTGCAG^CACTCTGTCAAACC |
| I-ChuI | Chlamydomonas humicola | 5'-GAAGGTTTGGCACCTCG^ATGTCGGCTCATC |
| | | 3'-CTTCCAAACCGTG^GAGCTACAGCCGAGTAG |
| I-CpaI | Chlamydomonas pallidostigmatica | 5'-CGATCCTAAGGTAGCGAA^ATTCA |
| | | 3'-GCTAGGATTCCATC^GCTTTAAGT |
| I-CpaII | Chlamydomonas pallidostigmatica | 5'-CCCGGCTAACTC^TGTGCCAG |
| | | 3'-GGGCCGAT^TGAGACACGGTC |
| I-CeuI | Chlamydomonas eugametos | 5'-CGTAACTATAACGGTCCTAA^GGTAGCGAA |
| | | 3'-GCATTGATATTGCCAG^GATTCCATCGCTT |
| I-DmoI | Desulfurococcus mobilis | 5'-ATGCCTTGCCGGGTAA^GTTCCGGCGCGCAT |
| | | 3'-TACGGAACGGCC^CATTCAAGGCCGCGCGTA |
| I-Scel | S.cerevisiae | 5'-AGTTACGCTAGGGATAA^CAGGGTAATATAG |
| | | 3'-TCAATGCGATCCC^TATTGTCCCATTATATC |
| | | 5'-TAGGGATAA^CAGGGTAAT |
| | | 3'-ATCCC^TATTGTCCCATTA ("Core"-Sequenz) |
| I-SceII | S.cerevisiäe | 5'-TTTTGATTCTTTGGTCACCC^TGAAGTATA |
| | | 3'-AAAACTAAGAAACCAG^TGGGACTTCATAT |
| I-SceIII | S.cerevisiae | 5'-ATTGGAGGTTTTGGTAAC^TATTTATTACC |
| | | 3'-TAACCTCCAAAACC^ATTGATAAATAATGG |
| I-SceIV | S.cerevisiae | 5'-TCTTTTCTCTTGATTA^GCCCTAATCTACG |
| | | 3'-AGAAAAGAGAAC^TAATCGGGATTAGATGC |
| I-SceV | S.cerevisiae | 5'-AATAATTTTCT^TCTTAGTAATGCC |
| | | 3'-TTATTAAAAGAAGAATCATTA^CGG |
| I-SceVI | S.cerevisiae | 5'-GTTATTTAATG^TTTTAGTAGTTGG |
| | | 3'-CAATAAATTACAAAATCATCA^ACC |
| I-SceVII | S.cerevisiae | 5'-TGTCACATTGAGGTGCACTAGTTATTAC |
| PI-SceI | S.cerevisiae | 5'-ATCTATGTCGGGTGC^GGAGAAAGAGGTAAT |
| | | 3'-TAGATACAGCC^CACGCCTCTTTCTCCATTA |
| F-SceI | S.cerevisiae | 5'-GATGCTGTAGGC^ATAGGCTTGGTT |
| | | 3'-CTACGACA^TCCGTATCCGAACCAA |
| F-SceII | S.cerevisiae | 5'-CTTTCCGCAACA^GTAAAATT |
| | | 3'-GAAAGGCG^TTGTCATTTTAA |
| I-HmuI | Bacillus subtilis bacteriophage SPO1 | 5'-AGTAATGAGCCTAACGCTCAGCAA |
| | | 3'-TCATTACTCGGATTGC^GAGTCGTT |
| I-HmuII | Bacillus subtilis bacteriophage SP82 | |
| I-LlaI | Lactococcus lactis | 5'-CACATCCATAAC^CATATCATTTTT |
| | | 3'-GTGTAGGTATTGGTATAGTAA^AAA |
| I-MsoI | Monomastix species | 5'-CTGGGTTCAAAACGTCGTGA^GACAGTTTGG |
| | | 3'-GACCCAAGTTTTGCAG^CACTCTGTCAAACC |
| I-NanI | Naegleria andersoni | 5'-AAGTCTGGTGCCA^GCACCCGC |
| | | 3'-TTCAGACC^ACGGTCGTGGGCG |
| I-NitI | Naegleria italica | 5'-AAGTCTGGTGCCA^GCACCCGC |
| | | 3'-TTCAGACC^ACGGTCGTGGGCG |
| I-NjaI | Naegleria jamiesoni | 5'-AAGTCTGGTGCCA^GCACCCGC |
| | | 3'-TTCAGACC^ACGGTCGTGGGCG |
| I-PakI | Pseudendoclonium akinetum | 5'-CTGGGTTCAAAACGTCGTGA^GACAGTTTGG |
| | | 3'-GACCCAAGTTTTGCAG^CACTCTGTCAAACC |
| I-PorI | Pyrobaculum organotrophum | 5'-GCGAGCCCGTAAGGGT^GTGTACGGG |
| | | 3'-CGCTCGGGCATT^CCCACACATGCCC |
| I-PpoI | Physarum polycephalum | 5'-TAACTATGRCTCTCTTAA^GGTAGCCAAAT |
| | | 3'-ATTGATACTGAGAG^AATTCCATCGGTTTA |
| I-ScaI | Saccharomyces capensis | 5'-TGTCACATTGAGGTGCACT^AGTTATTAC |
| | | 3'-ACAGTGTRACTCCAC^GTGATCAATAATG |
| I-Ssp6803I | Synechocystis species | 5'-GTCGGGCT^CATAACCCGAA |
| | | 3'-CAGCCCGAGTA^TTGGGCTT |
| PI-PfuI | Pyrococcus furiosus Vcl | 5'-GAAGATGGGAGGAGGG^ACCGGACTCAACTT |
| | | 3'-CTTCTACCCTCC^TCCCTGGCCTGAGT2'GAA |
| PI-PfuII | Pyrococcus furiosus Vcl | 5'-ACGAATCCATGTGGAGA^AGAGCCTCTATA |
| | | 3'-TGCTTAGGTACAC^CTCTTCTCGGAGATRT |
| PI-PkoI | Pyrococcus kodakaraensis KOD1 | 5'-GATTTTAGAT^CCCTGTACC |
| | | 3'-CTAAAA^TCTAGGGACATGG |
| PI-PkoII | Pyrococcus kodakaraensis KOD1 | 5'-CAGTACTACG^GTTAC |
| | | 3'-GTCATG^ATGCCAATG |
| PI-PspI | Pyrococcus sp. | 5'-AAAATCCTGGCAAACAGCTAT'TAT^GGGTAT |
| | | 3'-TTTTAGGACCGTTPGTCGAT^AATACCCATA |
| PI-TfuI | Thermococcus fumicolans ST557 | 5'-TAGATTTTAGGT^CGCTATATCCTTCC |
| | | 3'-ATCTAAAA^TCCAGCGATATAGGAAGG |
| PI-TfuII | Thermococcus fumicolans ST557 | 5'-TAYGCNGAYACN^GACGGYTTYT |
| | | 3'-ATRCGNCT^RTGNCTGCCRAARA |
| PI-ThyI | Thermococcus hydrothermalis | 5'-TAYGCNGAYACN^GACGGYTTYT |
| | | 3'-ATRCGNCT^RTGNCTGCCRAARA |
| PI-TliI | Thermococcus litoralis | 5'-TAYGCNGAYACNGACGG^YTTYT |
| | | 3'-ATRCGNCTRTGNC^TGCCRAARA |
| PI-TliII | Thermococcus litoralis | 5'-AAATTGCTTGCAAACAGCTATTACGGCTAT |
| I-TevI | Bacteriophage T4 | 5'-AGTGGTATCAAC^GCTCAGTAGATG |
| | | 3'-TCACCATAGT^TGCGAGTCATCTAC |
| I-TevII | Bacteriophage T4 | 5'-GCTTATGAGTATGAAGTGAACACGT^TATTC |
| | | 3'-CGAATACTCATACTTCACTTGTG^CAATAAG |
| F-TevI | Bacteriophage T4 | 5'-GAAACACAAGA^AATGTTTAGTNNNNNNNNNNNNNN |
| | | 3'-CTTTGTGTTCTTTACAAATCATTTNNNNNNNNNNNNNNN^ |
| F-TevII | Bacteriophage T4 | 5'-TTTAATCCTCGCTTC^AGATATGGCAACTG |
| | | 3'-AAATTAGGAGCGA^AGTCTATACCGTTGAC |

Dabei sind auch kleinere Abweichungen (Degenerationen) der Erkennungssequenz umfasst, die dennoch eine Erkennung und Spaltung durch das jeweilige DSBI-Enzym ermöglichen. Derartige Abweichungen - auch in Zusammenhang mit unterschiedlichen Rahmenbedingungen wie beispielsweise Calcium oder Magnesium-Konzentration - sind beschrieben (Argast GM et al. (1998) J Mol Biol 280:345-353). Ferner sind Kernsequenzen ("Core"-Sequenzen) dieser Erkennungssequenzen umfasst. Es ist bekannt, dass auch die inneren Anteile der Erkennungssequenzen für einen induzierten Doppelstrangbruch genügen und das die äußeren nicht unbedingt relevant sind, jedoch die Effizienz der Spaltung mitbestimmen können. So kann beispielsweise für I-SceI eine 18bp-"Core"-Sequenz definiert werden.

Besagte DSBI-Erkennungssequenzen können an verschiedenen Positionen in oder in der Nähe eines Markerprotein-Gens lokalisiert werden und können - beispielsweise bei der Verwendung eines Transgens als Markerproteins - bereits bei der Konstruktion der Markerprotein-Expressionskassette eingebaut werden. Verschiedene Möglichkeiten der Lokalisation sind beispielhaft in den Fig. 2-A, 2-B, 3 und 5 sowie in den Beschreibungen dazu verdeutlicht.

In einer weiteren vorteilhaften Ausführungsform umfasst die Insertionssequenz mindestens eine Homologiesequenz A, die eine ausreichende Länge und eine ausreichende Homologie zu einer Sequenz A' in dem Markerproteingen aufweist, um eine homologe Rekombination zwischen A und A' zu gewährleisten. Bevorzugt ist Insertiönssequenz von zwei Sequenzen A und B flankiert, die eine ausreichende Länge und eine ausreichende Homologie zu einer Sequenz A' bzw. B' in dem Markerproteingen aufweisen, um eine homologe Rekombination zwischen A und A' bzw. B und B' zu gewährleisten.

"Ausreichende Länge" meint in Bezug auf die Homologiesequenzen A, A' und B, B' bevorzugt Sequenzen von einer Länge von mindestens 100 Basenpaaren, bevorzugt mindestens 250 Basenpaaren, besonders bevorzugt von mindestens 500 Basenpaaren, ganz besonders bevorzugt von mindestens 1000 Basenpaaren, am meistens bevorzugt von mindestens 2500 Basenpaaren.

"Ausreichende Homologie" meint in Bezug auf die Homologiesequenzen, bevorzugt Sequenzen die eine Homologie zueinander aufweisen von mindestens 70 %, bevorzugt 80 %, vorzugsweise mindestens 90 %, besonders bevorvzugt mindestens 95 %, ganz besonders bevorzugt mindestens 99 %, am meisten bevorzugt 100% über eine Länge von von mindestens 20 Basenpaaren, bevorzugt mindestens 50 Basenpaaren, besonders bevorzugt von mindestens 100 Basenpaaren, ganz besonders bevorzugt von mindestens 250 Basenpaaren, am meisten bevorzugt von mindestens 500 Basenpaaren.

Unter Homolgie zwischen zwei Nukleinsäuren wird die Identität der Nukleinsauresequenz über die jeweils gesamte Sequenzlänge verstanden, die durch Vergleich mit Hilfe des Programmalgorithmus GAP (Wisconsin Package Version 10.0, University of Wisconsin, Genetics Computer Group (GCG), Madison, USA) unter Einstellung folgender Parameter berechnet wird:

| | |
|---|---|
| Gap Weight: 12 | Length Weight: 4 |
| Average Match: 2, 912 | Average Mismatch: 2,003 |

In einer Variante wird die Effizienz der Rekombination durch Kombination mit Verfahren, die di.e homologe Rekombination fördern, gesteigert. Solche Verfahren sind beschrieben und umfassen beispielhaft die Expression von Proteinen wie RecA oder die Behandlung mit PARP-Inhibitoren. Es konnte gezeigt werden, dass die intrachromosomale homologe Rekombination in Tabakpflanzen durch die Verwendung von PARP-Inhibitoren erhöht, werden kann (Puchta H et al. (1995) Plant J 7:203-210). Durch den Einsatz dieser Inhibitoren kann die Rate der homologen Rekombination in den Rekombinationskonstrukten nach Induktion des sequenzspezifischen DNA-Doppelstrangbruches und damit die Effizienz der Deletion der Transgensequenzen weiter erhöht werden. Verschiedene PARP Inhibitoren können dabei zum Einsatz kommen. Bevorzugt umfasst sind Inhibitoren wie 3-Aminobenzamid, 8-Hydroxy-2-methylquinazolin-4-on (NU1025), 1,11b-Dihydro-[2H]benzopyrano[4, 3, 2-de] isoquinolin-3-on (GPI 6150), 5-Aminoisoquinolinon, 3,4-Dhydro-5-[4-(1-piperidinyl)butoxy]-1 (2H)-isoquinolinon oder die in WO 00/26192, WO 00/29384, WO 00/32579, WO 00/64878, WO 03/68206, WO 00/67734, WO 01/23386 und WO 01/23390 beschriebenen Substanzen.

Weitere geeignete Methoden sind die Einführung von Nonsense-Mutationen in endogene Markerprotein Gene zum Beispiel mittels Einführung von RNA/DNA-Oligonukleotiden in die Pflanze (Zhu et al, (200) Nat Biotechnol 18(5):555-558). Punktmutationen können auch mittels DNA-RNA Hybriden erzeugt, werden, die auch als "chimeraplasty" bekannt sind (Cole-Strauss et al. (1999) Nucl Acids Res 27(5):1323-1330; Kmiec (1999). Gene therapy American Scientist 87(3):240-247).

Die Methoden der dsRNAi, der Kosuppression mittels sense-RNA und der "VIGS" ("virus induced gene silencing") werden auch als "posttranscriptional gene silencing" (PTGS) bezeichnet. PTGS-Verfahren sind besonders vorteilhaft, weil die Anforderungen an die Homologie zwischen dem zu vermindernden Markerprotein-Gen und der transgen exprimierten sense oder dsRNA-Nukleinsäuresequenz geringer sind als beispielsweise bei einem klassischen antisense-Ansatz. So kann man unter Verwendung der Markerprotein-Nukleinsäuresequenz aus einer Art auch die Expression von homologen Markerprotein-Proteinen in anderen Arten effektiv vermindern, ohne, dass die Isolierung und Strukturaufklärung der dort vorkommenden Marketprotein-Homologen zwingend erforderlich wäre. Dies erleichtert erheblich den Arbeitsaufwand.

"Einbringen" umfasst im Rahmen der Erfindung alle Verfahren, die dazu geeignet eine "anti-Markerprotein"-Verbindung, direkt oder indirekt, in eine Pflänze oder eine Zelle, Kompartiment, Gewebe, Organ oder Samen derselben einzuführen oder dort zu generieren. Direkte und indirekte Verfahren sind umfasst. Das Einbringen kann zu einer vorübergehenden (transienten) Präsenz einer "anti-Markerprotein"-Verbindung führen oder aber auch zu einer dauerhaften (stabilen).

Einführen umfasst beispielsweise Verfahren wie Transfektion, Transduktion oder Transformation.

"Anti-Markerprotein" Verbindungen umfasst somit beispielsweise auch Expressionskassetten, die eine Expression (d.h. Transkription) einer MP-dsRNA, realisieren können.

"Expressionskassette" meint im Rahmen dieser Erfindung allgemein solche Konstruktionen in denen eine zu exprimierende Nukleinsauresequenz in funktioneller Verknüpfung mit mindestens einer genetischen Kontrollsequenz - bevorzugt einer Promotorseguenz - steht. Expressionskaseetten bestehen bevorzugt aus doppelstrangiger DNA und können eine lineare oder zirkuläre Struktur haben.

Unter einer funktionellen Verknüpfung versteht man zum Beispiel die sequentielle Anordnung eines Promotors mit einer zu transkribierenden Nukleinsauresequenz (beispielsweise kodierend für eine MP-dsRNA) und ggf. weiterer regulativer Elemente wie zum Beispiel einem Terminator und/oder Polyadenylierungssignalen derart, dass jedes der regulativen Elemente seine Funktion bei der Transkription der Nukleinsauresequenz, je nach Anordnung der Nukleinsaureseqenzen erfüllen kann. Funktion kann dabei beispielsweise die kontrolle der Expression d.h. Transkription und/oder Translation der Nukleinsauresequenz (beispielsweise kodierend für ein MP-dsRNA bebeuten Kontrolle umfasst dabei beispielweise das Initiieren, Steigerung, Steuerung oder Suppression der Expression d.h. Transkription Dazu ist nicht unbedingt eine direkte Verknüpfung im chemischen Sinne erforderlich. Genetische Kontrollsequenzen wie zum Beispiel Enhancer-Sequenzen, können ihre Funktion auch von weiter entfernten Positionen oder gar von anderen DNA-Molekulen aus auf die Zielsequenz ausüben. Bevorzugt sind Anordnungen, in denen die zu transkribierende Nukleinsäureqenz hinter der als Promoter fungierenden Sequenz positioniert wird, so dass beide Sequenzen kovalant miteinander verbunden sind. Bevorzugt ist dabei der Abstand zwischen der Promotorsequenz und der transgen zu exprimierende Nukleinsäuresequenz geringer als 200 Basenpaare, besonders bevorzugt kleiner als 100 Basenpaare, ganz besonders bevorzugt kleiner als 50 Basenpaare.

Dem Fachmann sind verschiedene Wege bekannt, um zu einer der erfindungsemässen Expressionskassette zu gelangen. Die Herstellung einer erfindungsgemässen Expressionskassette erfolgt, beispielsweise bevorzugt durch direkte Fusion einer als Promoter fungierenden Nukleinsäuresequenz mit einer zu exprimierenden Nukleotidsequenz (beispielsweise kodierend für eine MP-dsRNA). Die Herstellung einer funktionellen Verknüpfung kann mittels, gängiger Rekombinations- und Klonierungstechniken realisiert werden, wie sie beispielsweise in Maniatis T, Fritsch EF und Sambrook J (1989) Molecular Cloning: A Laboratory Manual, Cold Spring Harbor Laboratory, Cold Spring Harbor, NY sowie in Silhavy TJ et al. (1984) Experiments with Gene Fusions, Cold Spring Harbor Laboratory, Cold Spring Harbor, NY und in Ausubel FM et al. (1987) Current Protocols in Mölecular Biology, Greene Publishing Assoc. and Wiley Interscience beschrieben sind.

Vorzugsweise umfassen die erfindungsgemässen Expressionskassetten 5'-stromaufwärts von der jeweiligen transgen zu exprimierenden Nukleinsäuresequenz einen Promotor und 3'-stromabwärts eine Terminatorsequenz als zusätzliche genetische Kontrollsequenz, sowie gegebenenfalls weitere übliche regulative Elemente, und zwar jeweils funktionell verknüpft mit der transgen zu exprimierenden Nukleinsäuresequenz.

Der Begriff der "genetischen Kontrollsequenzen" ist breit zu verstehen und meint all solche Sequenzen, die einen Einfluss auf das Zustandekommen oder die Funktion der erfindungsgemässen Expressionskassette haben. Genetische Kontrollsequenzen gewährleisten zum Beispiel die Transkription und gegebenenfalls Translation in prokaryotischen oder eukaryotischen Organismen. Genetische Kontrollsequenzen sind beispielsweise beschrieben bei "Goeddel; Gene Expression Technology: Methods in Enzymology 185, Academic Press, San Diego, CA (1990)" oder "Gruber and Crosby, in: Methods in Plant Molecular Biology and Biotechnolgy, CRC Press,Boca Raton, Florida, eds.:Glick and Thompson, Chapter 7, 89-108" sowie den dort aufgewiesenen Zitaten.

Genetische Kontrollsequenzen umfassen insbesondere in Pflanzen funktionelle Promotoren. Als bevorzugte Promotoren für die Expressionskassetten ist grundsätzlich jeder Promotor geeignet, der die Expression von Genen, insbesondere Fremdgenen, in Pflanzen steuern kann.

Pflanzenspezifische oder in Pflanzen bzw. pflanzlichen Zelle funktionelle Promotoren meint grundsätzlich jeden Promotor, der die Expression von Genen, insbesondere Fremdgenen, in mindestens einer Pflanze oder einem Pflanzenteil, -zelle, -gewebe, -kultur steuern kann. Dabei kann die Expression beispielsweise konstitutiv, induzierbar oder entwicklungsabhängig sein. Bevorzugt sind:

### a) Konstitutive Promotoren

"Konstitutive" Promotoren meint solche Promotoren, die eine Expression in zahlreichen, bevorzugt allen, Geweben über einen größeren Zeitraum der Pflanzenentwicklung, bevorzugt zu allen Zeitpunkten der Pflanzenentwicklung, gewährleisten (Benfey et al.(1989) EMBO J 8:2195-2202). Vorzugsweise verwendet man insbesondere einen pflanzlichen Promotor oder einen Promotor, der einem Pflanzenvirus entstammt. Insbesondere bevorzugt ist der Promotor des 35S-Transkriptes des CaMV Blumenkohlmosaikvirus (Franck et al: (1980) Cell 21:285-294; Odell et al. (1985) Nature 313:810-812; Shewmaker et al. (1985) Virology 140:281-288; Gardner et al. (1986) Plant Mol Biol 6:221- 228) oder der 19S CaMV Promotor (US 5,352,605; WO 84/02913; Benfey et al. (1989) EMBO J 8:2195-2202) sowie der Promotor des Nitrilase-1 Gens aus Arabidopsis thaliana (GenBankAcc.-No.: Y07648, Nukleotide 2456 (alternativ 2861) bis 4308 oder alternativ 4340 oder 4344. (beispielsweise bp 2456 bis 4340).

Ein weiterer geeigneter konstitutiver Promotor ist der "Rubisco small subunit (SSU)"-Promotor (US 4,962,028), der LeguminB-Promotor (GenBank Acc.-No.: X03677), der Promotor der Nopalinsynthase aus Agrobacterium, der TR-Doppelpromotor, der OCS (Octopin Synthase) Promotor aus Agrobacterium, der Ubiquitin Promotor (Holtorf S et al. (1995) Plant Mol Biol 29:637-649), der Ubiquitin 1 Promotor (Christensen et al. (1992) Plant Mol Biol 18:675-689; Bruce et al. (1989) Proc Natl Acad Sci USA 86:9692-9696), der Smas Promotor, der Cinnamylalkoholdehydrogenase-Promotor (US 5,683,439), die Promotoren der vakuolärer ATPase Untereinheiten oder der Promotor eines prolinreichen Proteins aus Weizen (WO 91/13991), sowie weitere Promotoren von Genen, deren konstitutive Expression in Pflanzen dem Fachmann-bekannt ist.

### b) Gewebespezifische Promotoren

Bevorzugt sind Promotoren mit Spezifitäten für die Antheren, Ovarien, Blüten, Blätter, Stengel, Wurzeln oder Samen.
Samenspezifische Promotoren umfassen zum Beispiel den Promotor des Phaseolins (US 5,504,200; Bustos MM et al. (1989) Plant Cell 1(9):839-53), des 2S Albumins (Joseffson LG et al. (1987) J Biol Chem 262:12196-12201), des Legumins (Shirsat A et al. (1989) Mol Gen Genet 215(2): 326-331), des USP (unknown seed protein; Bäumlein H et al. (1991) Mol Gen Genet 225(3) :459-67), des Napins (US 5,608,152; Stalberg K et al. (1996) L Planta 199:515-519), des Saccharosebindeproteins (WO 00/26388), des Legumins B4 (LeB4; Bäumlein H et al. (1991) Mol Gens Genet 225: 121-128; Baeumlein et al. (1992) Plant Journal 2(2):233-9; Fiedler U et al. (1995) Biotechnology (NY) 13(10):1090f), des Oleosins (WO 98/45461) oder des Bce4 (WO 91/13980). Weitere geeignete samenspezifische Promotoren sind die der Gene kodierend für das "High Molecular Weight Glutenin" (HMWG), Gliadin, Verzweigungsenzym, ADP Glucose Pyrophosphatase (AGPase) oder Stärkesynthase. Bevorzugt sind ferner Promotoren, die eine samenspezifische Expression in Monokotyledonen wie Mais, Gerste, Weizen, Roggen, Reis etc. erlauben. Vorteilhaft eingesetzt werden können der Promoter des 1pt2- oder Ipt1-Gens (WO 95/15389, WO 95/23230) oder die Promotoren beschrieben in WO 99/16890 (Promotoren des Hordeins, Glutelins, Oryzins, Prolamins, Gliadins, Zeins, Kasirins oder Secalins). Weitere samenspezifische Promotoren sind beschrieben in WO 89/03887.
Knollen-, Speicherwurzel- oder Wurzel-spezifische Promotoren umfassen beispielsweise den Patatin Promotor Kasse I (B33) oder den Promotor des Cathepsin D Inhibitors aus Kartoffel.
Blattspezifische Promotoren umfassen beispielsweise den Promotor der cytosolischen FBPase aus Kartoffel (WO 97/05900), den SSU Promotor (small subunit) der Rubisco (Ribulose-1,5-bisphosphatcarboxylase) oder den ST-LSI.Promotor aus Kartoffel (Stockhaus et al. (1989) EMBO J 8:2445-2451).
Blütenspezifische Promotoren umfassen beispielsweise den Phytoen Synthase Promotor (WO 92/16635) oder den Promotor des P-rr Gens (WO 98/22593).
- Antheren-spezifische Promotoren umfassen beispielsweise den 5126-Promotor (US 5,689,049, US 5,689,051), den glob-1 Promotor und den γ-Zein Promotor.

### c) Chemisch induzierbare Promotoren

Chemisch induzierbare Promotor erlauben es, die Expression abhängig von einem exogenen Stimulus zu steuern (Übersichtsartikel: Gatz et al. (1997) Ann Rev Plant Physiol Plant Mol Biol 48:89-108). Beispielhaft seien zu nennen: Der PRP1 Promotor (Ward et al. (1993) Plant Mol Biol 22:361-366),

ein durch Salicylsäure induzierbarer Promotor (WO 95/19443), ein durch Benzolsulfonamid-induzierbarer Promotor (EP-A 0 388 186), ein durch Tetrazyklin-induzierbarer Promotor (Gatz et al. (1992) Plant J 2:397-404), ein durch Abscisinsäure induzierbarer Promotor (EP 0 335 528) bzw. ein durch Ethanol- oder Cyclohexanon-induzierbarer Promotor (WO 93/21334). Ferner geeignet ist der Promotor des Glutathione-S-transferase Isoform II Gens (GST-II-27), der durch exogen applizierte Safener wie z.B. N,N-Diallyl-2,2-dichloroacetamid aktiviert werden kann (WO 93/01294) und in zahlreichen Geweben von sowohl Monokotyledonen als auch Dikotyledonen funktionell ist.

Besonders bevorzugt sind konstitutive oder induzierbare Promotoren.

Ferner sind für die gezielte Expression in den Plastiden plastiden-spezifische Promotoren bevorzugt. Geeignete Promotoren sind beispielsweise beschrieben in WO 98/55595 oder WO 97/06250. Zu nennen sind das rpo B Promotorelement, das atoB Promotorelements, das clpP Promotorelement (siehe auch WO 99/46394) oder das 16SrDNA Promotorelement. Weiterhin sind virale Promotoren geeignet (WO 95/16783).

Eine gezielte plastidäre Expression kann auch erreicht werden, wenn man zum Beispiel einen bakteriellen oder Bakteriophagen Promotor verwendet, die resultierende Expressionskassette in die plastidäre DNA einbringt und die Expression dann durch ein Fusionsprotein aus einer bakteriellen oder Bakteriophagen Polymerase und einem plastidären Transitpeptid exprimiert. Ein entsprechendes Verfahren ist in US 5,925,806 beschrieben.

Genetische Kontrollsequenzen umfassen ferner auch die 5'-untranslatierte Regionen, Introns oder nichtkodierende 3'-Region von Genen wie beispielsweise das Actin-1 Intron, oder die Adhl-S Introns 1, 2 und 6 (allgemein: The Maize Handbook, Chapter 116, Freeling and Walbot, Eds., Springer, New York (1994)). Es ist gezeigt worden, dass diese eine signifikante Funktionen bei der Regulation der Genexpression spielen können. So wurde gezeigt, dass 5'-untranslatierte Sequenzen die transiente Expression heterologer Gene verstärken können. Sie können ferner die Gewebsspezifität fördern (Rouster J et al.(1998) Plant J. 15:435-440). Beispielhaft für Translationsverstärker sei die 5'-Leadersequenz aus dem Tabak-Mosaik-Virus zu nennen (Gallie et al. (1987) Nucl Acids Res 15:8693-8711).

Als Kontrollsequenzen geeignete Polyadenylierungssignale sind insbesondere Polyadenylierungssignale pflanzlicher Gene sowie T-DNA Polyadenylierungssignale aus *Agrobakterium tumefaciens*. Beispiele für besonders geeignete Terminatorsequenzen sind der OCS (Octopin-Synthase)-Terminator und der NOS (NopalinSynthase)-Terminator (Depicker A et al (1982) J Mol Appl Genet 1:561-573), als auch die Terminatoren von Sojabohnen Actin, RUBISCO oder alpha-Amylase aus Weizen (Baulcombe DC et al (1987) Mol Gen Genet 209:33-40).

Die Expressionskassette kann vorteilhafterweise eine oder mehrere sogenannte "enhancer Sequenzen" funktionell verknüpft mit dem Promoter enthalten, die eine erhöhte transgene Expression der Nukleinsäuresequenz ermöglichen.

Genetische Kontrollsequenzen meint ferner Sequenzen, die für_{.} Fusionsproteine bestehend aus einer Signalpeptidsequenz kodieren. Die Expression eines zielgenes ist in jedem gewünschten Zellkompartiment, wie z.B. dem Endomembransystem, der Vakuole und den Chloroplasten möglich. Durch Nutzung des sekretorischen Weges sind gewünschte Glykosylierungsreaktionen, besondere Faltungen u.ä. möglich. Auch die Sekretion des Zielproteins zur Zelloberfläche bzw, die Sezernierung ins Kulturmedium, beispielsweise bei Nutzung suspensionskultivierter Zellen oder Protoplasten ist möglich. Die dafür notwendigen Targetsequenzen können sowohl in einzelnen Vektorvariationen berücksichtigt werden als auch durch Verwendung einer geeigneten Klonierungsstrategie gemeinsam mit dem zu klonierenden Zielgen in den Vektor mit eingebracht werden. Als Targetsequenzen können sowohl Gen-eigene, sofern vorhanden, oder heterologe Sequenzen genutzt werden. Zusätzliche, heterologe zur funktionellen Verknüpfung bevorzugte aber nicht darauf beschränkte Sequenzen sind weitere Targeting-Sequenzen zur Gewährleistung der subzellulären Lokalisation im Apoplasten, in der Vakuole, in Plastiden, im Mitochondrium, im Endoplasmatischen Retikulum (ER), im Zellkern, in Ölkörperchen oder anderen Kompartimenten; sowie Translationsverstärker wie die 5'-Leadersequenz aus dem Tabak-Mosaik-Virus (Gallie et al. (1987) Nucl Acids Res 15: 8693-8711) und dergleichen. Das Verfahren, an sich nicht in den Plastiden lokalisierte Proteine, gezielt in die Plastiden zu transportieren ist beschrieben (Klosgen RB und Weil JH (1991) Mol Gen Genet 225(2):297-304; Van Breusegem F et al. (1998) Plant Mol Biol 38(3):491-496).

Als Kontrollsequenzen sind weiterhin solche zu verstehen, die eine homologe Rekombination bzw. Insertion in das Genom eines Wirtsorganismus ermöglichen oder die Entfernung aus dem Genom erlauben. Methoden wie die cre/lox-Technologie erlauben eine gewebsspezifische, unter Umständen induzierbare Entfernung der Expressionskassette aus dem Genom des Wirtsorganismus (Sauer B. Methods. 1998; 14(4):381-92). Hier werden bestimmte flankierende Sequenzen dem Zielgen angefügt (lox-Sequenzen), die später eine Entfernung mittels der cre-Rekombinase ermöglichen.

Bevorzugt kann die Expressionskassette, bestehend aus einer Verknüpfung von Promoter und zu transkribierender Nukleinsäuresequenz, integriert in einem Vektor vorliegen und durch zum Beispiel Transformation - nach einem der unten beschriebenen Verfahren - in die pflanzliche Zelle oder Organismus eingebracht werden.

"Transgen" meint bevorzugt - beispielsweise in Bezug auf eine transgene Expressionskassette, einen transgenen Expressionsvektor, einen transgenen Organismus oder Verfahren zur transgenen Expression von Nukleinsäuren - alle solche durch gentechnische Methoden zustande gekommene Konstruktionen oder Verfahren unter Verwendung derselben, in denen entweder
a) die zu exprimierenden Nukleinsäuresequenz, oder
b) der mit der zu exprimierenden Nukleinsäuresequenz gemäß a) funktionell verknüpfte Promotor, oder
c) (a) und (b)
sich nicht in ihrer natürlichen, genetischen Umgebung (d.h. an ihrem natürlichen chromosomalen Locus) befinden oder durch gentechnische Methoden modifiziert wurden, wobei die Modifikation beispielhaft eine Substitution, Addition, Deletion, Inversion oder Insertion eines oder mehrerer Nukleotidreste sein kann. Natürliche genetische Umgebung meint den natürlichen chromosomalen Locus in dem Herkunftsorganismus oder das Vorliegen in einer genomischen Bibliothek.

"Transgen" meint in Bezug auf eine Expression ("transgene Expression") bevorzugt all solche unter Einsatz einer transgenen Expressionskassette, transgenen Expressionsvektor oder transgenen Organismus - entsprechend dem oben gegebenen Definitionen - realisierten Expressionen.

Die im Rahmen des erfindungsgemässen Verfahren zum Einsatz kommenden DNA-Konstrukte und die von ihnen.abgeleiteten Vektoren können weitere Funktionselemente enthalten. Der Begriff Funktionselement ist breit zu verstehen und meint all solche Elemente, die einen Einfluss auf Herstellung, Vermehrung oder Funktion der DNA-Konstrukte oder von diesen abgeleitete Vektoren oder Organismen haben. Beispielhaft aber nicht einschränkend seien zu nennen: .

### 1. Selektionsmarker

Selektionsmarker umfassen beispielsweise solche Nukleinsäure- oder Proteinsequenzen, deren Expression einer Zelle, Gewebe oder Organismus einen Vorteil (positiver Selektionsmarker) oder Nachteil (negativer Selektionsmarker) gegenüber Zellen vermittelt, die diese Nukleinsäure oder Protein nicht exprimieren. Positive Selektionsmarker wirken beispielsweise dadurch, dass eine auf die Zelle inhibitorisch wirkende Substanz detoxifiziert wird (Bsp. Antibiotika-/Herbizidresistenz), oder eine Substanz gebildet wird, welche der Pflanze unter den gewählten Bedingungen verbesserte Regeneration oder erhöhtes Wachstum ermöglicht (zum Beispiel nutritive Marker, hormonproduzierender Marker wie ipt; s.u.). Eine andere Form positiver Selektionsmarker umfasst mutierte Proteine oder RNAs, die gegenüber einem selektiven Agenz nicht empfindlich sind (beispielsweise 16S rRNA Mutanten, die unempfindlich gegenüber Spectinomycin sind). Negative Selektionsmarker wirken beispielsweise dadurch, dass sie die Bildung einer toxischen Substanz in den transformierten Zellen katalysieren (zum Beispiel das codA Gen).

### 1.1 Positive Selektionsmarker:

Die DNA-Koristrukte können zur weiteren Steigerung der Effizienz zusätzliche positive Selektionsmarker umfassen. In einer bevorzugten Ausführungsform kann so das erfinduhgsgemäße Verfahren im Form einer doppelten Selektion realisiert werden, wobei zusammen mit der zu insertierenden Nukleinsäuresequenz ein Sequenz kodierend für eine Resistenz gegen mindestens ein Toxin, Antibiotikum oder Herbizid eingebracht wird, und die Selektion zusätzlich unter Einsatz des Toxins, Antibiotikums oder Herbizids erfolgt.

Entsprechende Proteine und Sequenzen von positiven Selektionsmarkern sowie Selektionsverfahren sind dem Fachmann geläufig. Der Selektionsmarker verleiht den erfolgreich transformierten Zellen eine Resistenz gegen ein Biozid (zum Beispiel ein Herbizid wie Phosphinothricin, Glyphosat oder Bromoxynil), einen Metabolismusinhibitor wie 2-Desoxyglucose-6-phosphat (WO 98/45456) oder ein Antibiotikum, wie zum Beispiel Tetracycline, Ampicillin, Kanamycin, G 418, Neomycin, Bleomycin oder Hygromycin. Beispielhaft als Selektionsmarker seien genannt:
- Phosphinothricinacetyltransferasen (PAT), welche die freie Aminogruppe des Glutaminsynthaseinhibitors Phosphinothricin (PRT) acetylieren und damit eine Detoxifizierung des PPT erreichen (de Block et al. (1987) EMBO J 6:2513-2518) (auch Bialophos^{®} Resistenzgen (bar) genannt). Entsprechende Sequenzen sind dem Fachmann bekannt (aus Streptomyces hygroscopicus GenBank Acc.-No.: X17220 und X05822., aus Streptomyces viridochromogenes GenBank Acc.-No.: M 22827 und X65195; US 5,489,520). Ferner sind synthetische Gene für die Expression in Plastiden beschrieben. Ein synthetisches PAT-Gen ist beschrieben in Becker et al. (1994) Plant J 5:299-307. Die Gene verleihen Resistenz gegen das Herbizid Bialaphos oder Glufosinat und sind vielbenutzer Marker in transgenen Pflanzen (Vickers JE et al. (1996) Plant Mol Miol Reporter 14:363-368; Thompson CJ et al. (1987) EMBO J 6:2519-2523).

- 5-Enolpyruvylshikimat-3-phosphatsynthasen (EPSPS), die eine Resistenz gegen Glyphosat (N-(phosphonomethyl)glycin) verleihen. Das unselektive Herbizid Glyphosat hat die 5-Enolpyruvyl-3-phosphoshikimatsynthase (EPSPS) als molekulares Target. Diese hat eine Schlüsselfunktion in der Biosynthese aromatischer Aminosäuren in Mikroben und Pflanzen, jedoch nicht in Säugern (Steinrucken HC et al. (1980) Biochem Biophys Res Commun 94:1207-1212; Levin JG und. Sprinson DB (1964) J Biol Chem 239:1142-1150; Cole DJ (1985) Mode of action of glyphosate a literature analysis, p. 48-74. In: Grossbard E und Atkinson D (eds.). The herbicide glyphosate. Butteraworths, Boston.). Glyphosat-tolerante EPSPS Varianten werden bevorzugt als Selektionsmarker verwendet (Padgette SR et al. (1996). New weed control opportunities: development of soybeans with a Roundup Ready™ gene. In: Herbicide Resistant Crops (Duke, S.O., ed.). pp. 53-84. CRC Press, Boca Raton, FL; Saroha MK und Malik VS (1998) J Plant Biochemistry and Biotechnology 7:65-72). Das EPSPS Gen des Agrobakterium sp. strain CP4 hat eine natürliche Toleranz gegen Glyphosat, die auf entsprechende transgene Pflanzen transferiert werden kann. Das CP4 EPSPS Gen wurde aus Agrobakterium sp. strain CP4 kloniert (Padgette SR et al. (1995) Crop Science 35(5):1451-1461). Sequenzen von EPSPS-Enzymen, die Glyphosattolerant sind, sind beschrieben (u.a. in US 5,510,471; US 5,776,760: US 5,864,425; US 5,633,435; US 5,627;061; US 5,463,175; EP 0 218 571). Weitere Sequenzen sind beschrieben unter GenBank Acc.-No: X63374 oder M10947.
- Glyphosat^{®} degradierende Enzyme (gox Gen; Glyphosatoxidoreduktase). GOX (beispielsweise die Glyphosatoxidoreductase aus Achromobacter sp.) katalysiert die Spaltung einer C-N Bindung im Glyphosat, welches so zu Aminomethylphosphonsäure (AMPA) und Glyoxylat umgesetzt wird. GOX kann dadurch eine Resistenz gegen Glyphosat vermitteln (Padgette SR et al. (1996) J Nutr 126(3):702-16; Shah D et al. (1986) Science 233:478-481).
- Das deh Gen kodiert für eine Dehalogenase, die Dalapon^{®} inaktiviert (GenBank Acc.-No.: AX022822, AX022820 sowie WO 99/27116)
- Die bxn Gene kodieren für Bromoxynil_{.}degradierende Nitrilaseenzyme (Genbank Acc.-No: E01313 und J03196).
- Neomycinphosphotransferasen verleihen eine Resistenz gegen Antibiotika (Aminoglykoside) wie Neomycin, G418, Hygromycin, Paromomycin oder Kanamycin, indem sie durch eine Phosphorylierungsreaktion deren inhibierende Wirkung reduzieren. Besonders bevorzugt ist das nptII Gen. Sequenzen können aus der GenBank erhalten werden (AF080390; AF080389). Zudem ist das Gen bereits Bestandteil zahlreicher Expressionsvektoren und kann unter Verwendung von dem Fachmann geläufigen Verfahren aus diesen isoliert werden (AF234316; AF234315; AF234314). Das NPTII Gen kodiert für eine Amino.glycosid-3'O-phosphotransferase aus E.coli, Tn5 (GenBank Acc.-No: U00004 Position 1401-2300; Beck et al. (1982) Gene 19 327-336).
- Das DOG^{R}1-Gen wurde aus der Hefe Saccharomyces cerevisiae isoliert (EP-A 0 807 836) und kodiert für eine 2-Desoxyglukose-6-phosphat Phosphatase, die eine Resistenz gegenüber 2-DOG verleiht (Randez-Gil et al. (1995) Yeast 11:1233-1240; Sanz et al. (1994) Yeast 10:1195-1202, GenBank Acc.-No.: NC001140; Position 194799-194056).
- Acetolactatsynthasen, die eine Resistenz gegen Imidazolinon/Sulfonylurea-Herbizide verleihen (GenBank Acc-No.: X51514; Sathasivan K et al. (1990) Nucleic Acids Res. 18(8):2188); AB049823; AF094326;, X07645.; X07644; A19547; A19546; A19545; I05376: I05373; AL133315)
- Hygromycinphosphotransferasen (z.B. GenBank Acc-No.: X74325) die eine Resistenz gegen das Antibiotikum Hygromycin verleihen. Das Gen ist Bestandteil zahlreicher Expressionsvektoren und kann unter Verwendung von dem Fachmann geläufigen Verfahren (wie beispielsweise Polymerasekettenreaktion) aus diesen isoliert werden (GenBank Acc-No.: AF294981; AF234301; AF234300; AF234299; AF234298; AF354046; AF354045)
- Resistenzgene gegen
   a) Chloramphenicol (Chloramphenicolacetyltransferase).
   b) Tetracyclin (u.a. GenBank Acc-No.: X65876; X51366). Zudem ist das Gen bereits Bestandteil zahlreicher Expressionsvektoren und kann unter Verwendung von dem Fachmann geläufigen Verfahren (wie beispielsweise Polymerasekettenreaktion) aus diesen isoliert werden
   c) Streptomycin (u.a. GenBank Acc.-No.: AJ278607).
   d) Zeocin, das entsprechende Resistenzgen ist Bestandteil zahlreicher Klonierungsvektoren (z.B. GenBank Acc.-No.: L36849) und kann unter Verwendung von dem Fachmann geläufigen Verfahren (wie beispielsweise Polymerasekettenreaktion) aus diesen isoliert werden.
   e) Ampicillin (B-Lactamase Gen; Datta N, Richmond MH (1966) Biochem J 98(1):204-9: Heffron F et al (1975) J. Bacteriol 122: 250-256; Bolivar F et al. (1977.) Gene 2:95-114). Die Sequenz ist Bestandteil zahlreicher Klonierungsvektoren und kann unter Verwendung von dem Fachmann geläufigen Verfahren (wie beispielsweise Polymerasekettenreaktion) aus diesen isoliert werden.

Gene wie die Isopentenyltransferase aus Agrobakterium tumefaciens (strain: PO22) (Genbank Acc.-No.: AB025109) können auch als Selektionsmarker eingesetzt werden. Das ipt Gen ist ein Schlüsselenzym der Cytokinin-Biosynthese. Seine Überexpression erleichtert die Regeneration von Pflanzen (z.B. Selektion auf Cytokinin-freiem Medium). Das Verfahren zur Nutzung des ipt Gens ist beschrieben (Ebinuma H et al. (2000) Proc Natl Acad Sci USA 94:2117-2121; Ebinuma H et al. (2000) Selection of Marker-free transgenic plants using the oncogenes (*ipt, rol* A, B, C) of Agrobakterium as selectable markers, In Molecular Biology of Woody Plants. Kluwer Academic Publishers).

Verschiedene weitere positive Selektionsmarker, die den transformierten Pflanzen einen Wachstumsvorteil gegenüber nicht-transformierten verleihen, sowie Verfahren zu ihrer Verwendung sind u.a. beschrieben in EP-A 0 601 092. Beispielhaft sind zu nennen β-Glucuronidase (in Verbindung mit z.B. Cytokininglucuronid), Mannose-6-phosphat-Isomerase (in Verbindung mit Mannose), UDP-Galaktose-4-Epimerase (in Verbindung mit z.B. Galactose).

Für einen in Plastiden funktionellen Selektionsmarker sind insbesondere solche bevorzugt, die eine Resistenz gegen Spectinomycin, Streptomycin, Kanamycin, Lincomycin, Gentamycin, Hygromycin, Methotrexat, Bleomycin, Phleomycin, Blasticidin, Sulfonamid, Phosphinotricin, Chlorsulfuron, Bromoxymil, Glyphosat, 2,4-Datrazin, 4-methyltryptophan, Nitrat, S-aminoethyl-L-cysteine, Lysin/Threonin, Aminoethyl-Cystein oder Betainaldehyd verleihen. Besonders bevorzugt sind die Gene aadA, nptII, BADH, FLARE-S (eine Fusion aus aadA und GFP, beschrieben bei Khan MS & Maliga P (1999) Nature Biotech 17:910-915). Geeignet ist vor allem das aadA Gen (Svab Z und Maliga P (1993) Proc Natl Acad Sci USA 90:913-917). Ferner beschrieben sind modifizierte 16S rDNA sowie die Betainealdehyddehydrogenase (BADH) aus Spinat (Daniell H et al. (2001) Trends Plant Science 6:237-239; Daniell H et al. (2001) Curr Genet 39:109-116; WO 01/64023; WO 01/64024; WO 01/64850). Auch lethal wirkende Agenzen wie beispielsweise Glyphosat können in Verbindung mit entsprechend detoxifizierenden oder resistenten Enzymen genutzt werden (WO 01/81605).

Die jeweils für die Selektion verwendeten Konzentrationen der Antibiotika, Herbizide, Biozide oder Toxine müssen an die jeweiligen Testbedingungen bzw. Organismen angepasst werden. Beispielhaft seien für Pflanzen zu nennen Kanamycin (Km) 50 mg/L, Hygromycin B 40 mg/L, Phosphinothricin (Ppt) 6 mg/L, Sepctino= mycin (Spec) 500 mg/L.

### 2. Reportergene

Reportergene kodieren für leicht quantifizierbare Proteine und gewährleisten so über Eigenfarbe oder Enzymaktivität eine Bewertung der Transformationseffizienz, des Expressionsortes oder -zeitpunktes. Ganz besonders bevorzugt sind dabei Gene kodierend für Reporter-Proteine (siehe auch Schenborn E, Groskreutz D (1999) Mol Biotechnol 13(1):29-44) wie
- "green fluorescence protein" (GFP) (Chui WL et al. (1996) Curr Biol 6:325-330; Leffel SM et al. (1997) Biotechniques 23(5):912-8; Sheen et al. (1995) Plant J 8(5):777-784; Haseloff et al. (1997) Proc Natl Acad Sci USA 94(6): 2122-2127; Reichel et al. (1996) Proc Natl Acad Sci USA 93(12):5888-5893; Tian et al. (1997) Plant Cell Rep 16:267-271; WO 97/41228)
- Chloramphenicoltransferase
- Luziferase (Millar et al. (1992) Plant Mol Biol Rep 10: 324-414; Ow et al. (1986) Science 234:856-859); erlaubt Bioluminescenzdetektion
- β-Galactosidase (kodiert für ein Enzym für das verschiedenen chromogene Substrate zur Verfügung stehen)
- β-Glucuronidase (GUS) (Jefferson et al. (1987) EMBO J 6: 3901-3907) oder das uidA Gen (kodieren für Enzyme für die verschiedene chromogene Substrate zur Verfügung stehen)
- R-Locus Genprodukt, das die Produktion von Anthocyaninpigmenten (rote Färbung) in pflanzlichen Gewebe reguliert und so eine direkte Analyse der Promoteraktivität ohne Zugabe zusätzlicher Hilfsstoffe oder chromogener Substrate ermöglicht (Dellaporta et al. (1988) In: Chromosome Structure and Function: Impact of New Concepts, 18th Stadler Genetics Symposium, 11:263-282)
- Tyrosinase (Katz et al. (1983) J Gen Microbiol 129:2703-2714), Enzym, das Tyrosin zu DOPA und Dopaquinon oxidiert, die infolge das leicht nachweisbare Melanin bilden.
- Aequorin (Prasher et al.(1985) Biochem Biophys Res Commun 126(3):1259-1268), kann in der Calcium-sensitiven Bioluminescenzdetektion verwendet werden.

3. Replikationsursprünge, die eine Vermehrung der erfindungsgemäßen Expressionskassetten oder Vektoren in zum Beispiel E.coli gewährleisten. Beispielhaft seien genannt ORI (origin of DNA replication), der pBR322 ori oder der P15A ori (Sambrook et al.: Molecular Cloning. A Laboratory Manual, 2nd ed. Cold Spring Harbor Laboratory Press, Cold Spring Harbor, NY, 1989).
4. Elemente zum Beispiel "Bordersequenzen", die einen Agrobakterien-vermittelte Transfer in Pflanzenzellen für die Übertragung und Integration ins Pflanzengenom ermöglichen, wie zum Beispiel die rechte oder linke Begrenzung der T-DNA oder die vir-Region.
5. Multiple Klonierungsregionen (MCS) erlauben und erleichtern die Insertion eines oder mehrerer Nukleinsäuresequenzen.

Die Einführung von Nukleinsäuresequenzen (z.B. Expressionskassetten) in einen pflanzlichen Organismus oder Zellen, Geweben, Organe, Teile bzw. Samen desselben kann vorteilhaft unter Verwendung von Vektoren realisiert werden, in denen diese Sequenzen enthalten sind. Vektoren können beispielhaft Plasmide, Cosmide, Phagen, Viren oder auch Agrobakterien sein. Die Sequenzen können in den Vektor (bevorzugt ein Plasmidvektor) über geeignete Restriktionsschnittstellen insertiert werden. Der entstandene Vektor kann zunächst in E.coli eingeführte und amplifiziert werden. Korrekt transformierte E.coli werden selektioniert, gezüchtet und der rekombinante Vektor mit dem Fachmann geläufigen Methoden gewonnen. Restriktionsanalyse und Sequenzierung können dazu dienen, den Klonierungsschritt zu überprüfen. Bevorzugt sind solche Vektoren, die eine stabile Integration in das Wirtsgenom ermöglichen.

Die Herstellung eines transformierten Organismus (bzw. einer transformierten Zelle oder Gewebes) erfordert, dass die entsprechende DNA (z.B. der Transformationsvektor) oder RNA in die entsprechende Wirtszelle eingebracht wird. Für diesen Vorgang, der als Transformation (oder Transduktion bzw. Transfektion) bezeichnet wird, steht eine Vielzahl von Methoden und Vektoren zur Verfügung (Keown et al. (1990) Methods in Enzymology 185:527-537; Plant Molecular Biology and Biotechnology (CRC Press, Boca Raton, Florida), Kapitel 6/7, S. 71-119 (1993); White FF (1993) Vectors for Gene Transfer in Higher Plants; in: Transgenic Plants, Bd. 1, Engineering and Utilization, Hrsgb.: Kung und Wu R, Academic Press, 15-38; Jenes B et al. (1993) Techniques for Gene Transfer, in: Transgenic Plants, Bd. 1, Engineering and Utilization, Hrsgb.: Kung und R. Wu, Academic Press, 5.128-143; Potrykus (1991) Annu Rev Plant Physiol Plant Molec Biol 42:205-225; Halford NG, Shewry PR (2000) Br Med Bull 56.(1) :62-73) .

Beispielsweise kann die DNA oder RNA direkt durch Mikroinjektion (WO 92/09696, WO 94/00583, EP-A 0 331 083, EP-A 0 175 966) oder durch Bombardierung mit DNA bzw. RNA-beschichteten Mikropartikeln (biolistische Verfahren mit der Genkanone "particle bombardment"; US 5,100,792; EP-A 0 444 882; EP-A 0 434 616; Fromm ME et al. (1990) Bio/Technology 8(9):833-9; Gordon-Kamm et al. (1990) Plant Cell 2:603) eingeführt werden. Auch kann die Zelle chemisch, zum Beispiel mit Polyethylenglycol, permeabilisiert werden, so dass die DNA durch Diffusion in die Zelle gelangen kann. Die DNA kann auch durch Protoplastenfusion mit anderen DNA-enthaltenden Einheiten wie Minicells, Zellen, Lysosomen oder Liposomen (Freeman et al. (1984) Plant Cell Physiol. 29:1353ff; US 4,536,475) erfolgen. Elektroporation ist eine weitere geeignete Methode zur Einführung von DNA, bei der die Zellen reversibel durch einen elektrischen Impuls permeabilisert werden (EP-A 290 395, WO 87/06614). Weitere Verfahren umfassen die Calciumphosphatvermittelte Transformation, die DEAE-Dextran-vermittelte Transformation, die Inkubation trockener Embryonen in DNA-haltiger Lösung, oder andere Methoden der direkten DNA-Einführung (DE 4 005 152, WO 90/12096, US 4,684,611). Entsprechende Verfahren sind beschrieben (beispielsweise bei Bilang et al. (1991) Gene 100:247-250; Scheid et al. (1991) Mol Gen Genet 228:104-112; Guerche et al. (1987) Plant Science 52:111-116;. Neuhause et al. (1987) Theor Appl Genet 75:30-36; Klein et al. (1987) Nature 327:70-73; Howell et al. (1980) Science 208:1265; Horsch et al. (1985) Science 227:1229- 1231; DeBlock et al. (1989) Plant Physiology 91:694-701; Methods for Plant Molecular Biology (Weissbach and Weissbach, eds.) Academic Press Inc. (1988); and Methods in Plant Molecular Biology (Schuler and Zielinski, eds.) Academic Press Inc. (1989)). Physikalische Methoden der DNA-Einführung in pflanzliche Zelle sind im Überblick dargestellt bei Oard (1991) Biotech Adv 9:1-11.

Im Falle dieser "direkten" Transformationsmethoden sind keine besonderen Anforderungen an das verwendete Plasmid gestellt. Einfache Plasmide wie die der pUC-Reihe, pBR322, M13mp Reihe, pACYC184 etc. können verwendet werden.

Neben diesen "direkten" Transformationstechniken kann eine Transformation auch durch bakterielle Infektion mittels Agrobacterium (z.B. EP 0 116 718), virale Infektion mittels viraler Vektoren (EP 0 067 553; US 4,407,956; WO 95/34668; WO 93/03161) oder mittels Pollen (EP 0 270 356; WO 85/01856; US 4,684,611) durchgeführt werden.

Bevorzugt erfolgt die Transformation mittels Agrobacterien, die "entwaffnete° (disarmed) Ti-Plasmidvektoren enthalten, wobei deren natürliche Fähigkeit zum Gentransfer auf Pflanzen genutzt wird (EP-A 0 270 355; EP-A 0 116 718). Agrobacterium-Transformation ist weit verbreitet für die Transformation von Dicotyledonen, wird aber auch zunehmend auf Monocotyledonen angewandt (Toriyama et al. (1988) Bio/Technology 6: 1072-1074; Zhang et al. (1988) Plant Cell Rep-7:379-384; Zhang et al. (1988) Theor Appl Genet 76:835-840; Shimamoto et al. (1989) Nature 338:274-276; Datta et al. (1990-) Bio/Technology 8: 736-740; Christou et al. (1991) Hio/Technology 9:957-962; Peng et al. (1991) International Rice Research Institute, Manila, Philippines 563-574; Cao et al. (1992) Plant Cell Rep 11:585-591; Li et al. (1993) Plant Cell Rep 12:250-255; Rathore et al. (1993) Plant Mol Biol 21:871-884; Fromm et al. (1990) Bio/Technology 8:833-839; Gordon-Kamm et al. (1990) Plant Cell 2:603-618; D'Halluin et al. (1992) Plant Cell 4:1495-1505; Walters et al. (1992) Plant Mol Biol 18:189-200; Koziel et al. (1993) Biotechnology 11:194-200; Vasil IK (1994) Plant Mol Biol 25:925-937; Weeks et al. (1993) Plant Physiol 102:1077-1084; Somers et al. (1992) Bio/Technology 10:1589-1594; WO 92/14828; Hiei et al. (1994) Plant J 6:271-282).

Die für die Agrobacterium-Transformation meist verwendeten Stämme Agrobacterium tumefaciens oder Agrobacterium rhizogenes enthalten ein Plasmid (Ti bzw. Ri Plasmid), das auf die Pflanze nach Agrobakterium-Infektion übertragen wird. Ein Teil dieses Plasmids, genannt T-DNA (transferred DNA), wird in das Genom der Pflanzenzelle integriert. Alternativ können durch Agrobakterium auch binäre Vektoren (Mini-Ti-Plasmide) auf Pflanzen übertragen und in deren Genom integriert werden.

Die Anwendung von Agrobakterium tumefaciens für die Transformation von Pflanzen unter Verwendung von Gewebekulturexplantaten ist beschrieben (u.a. Horsch RB et al. (1985) Science 225:1229ff; Fraley et al. (1983) Proc Natl Acad Sci USA 80: 4803-4807; Bevans et al. (1983) Nature 304:184-187)). Viele Stämme von Agrobakterium tumefaciens sind in der Lage, genetisches Material zu übertragen, wie z.B. die Stämme EHA101[pEHA101], EHA105[pEHA105], LBA4404[pAL4404], C58C1[pMP90] und C58C1[pGV2260] (Hood et al. (1993) Transgenic Res 2:208-218; Hoekema et al. (1983) Nature 303:179-181; Koncz and Schell (1986) Gen.Genet 204:383-396; Deblaere et al. (1985) Nucl Acids Res 13: 4777-4788).

Werden Agrobacterien verwendet, so ist die Expressionskassette in spezielle Plasmide zu integrieren, entweder in einen Zwischenvektor (englisch: shuttle or intermediate vector) oder einen binären Vektor. Wird ein Ti oder Ri Plasmid zur Transformation verwendet, ist zumindest die rechte Begrenzung, meistens jedoch die rechte und die linke Begrenzung der Ti oder Ri Plasmid T-DNA als flankierende Region mit der einzuführenden Expressionskassette verbunden. Bevorzugt werden binäre Vektoren verwendet. Binäre Vektoren können sowohl in E.coli als auch in Agrobacterium replizieren und enthalten die zur Übertragung in ein pflanzliches System erforderlichen Komponenten. Sie enthalten in der Regel ein Selektionsmarkergen für die Selektion transformierter Pflanzen (z.B. das nptII Gen, das eine Resistenz gegen Kanamycin verleiht) und einen Linker oder Polylinker flankiert von der rechten und linken T-DNA Begrenzungssequenz. Außerhalb der T-DNA-Begrenzungssequenz enthalten sie zudem noch einen Selektionsmarker, der eine Selektion transformierter E.coli und/oder Agrobakteria ermöglicht (z.B. das nptIII Gen, das eine Resistenz gegen Kanamycin verleiht). Entsprechende Vektoren können direkt in Agrobakterium transformiert werden (Holsters et al. (1978) Mol Gen Genet 163:181-187).

Binärvektoren basieren z.B. auf "broad host range"-Plasmiden wie pRK252 (Bevan et al. (1984) Nucl Acid Res 12,8711-8720) und pTJS75 (Watson et al. (1985) EMBO J 4(2):277- 284). Eine grosse Gruppe der verwendeten Binärvektoren leitet sich vom pBIN19 (Bevan et al. (1984) Nucl Acid Res 12:8711-8720) ab. Hajdukiewicz et al. entwickelten einen Binärvektor (pPZP), der kleiner und effizienter als die bisher üblichen ist (Hajdukiewicz et al. (1994) Plant Mol Biol 25:989-994). Verbesserte und besondere bevorzugte binäre Vektorsysteme zur Agrobakterium-vermittelten Transformation sind in WO 02/00900 beschrieben.

Die mit einem solchen Vektor transformierten Agrobakterien können dann in bekannter Weise zur Transformation von Pflanzen, insbesondere von Kulturpflanzen, wie z.B. von Raps, verwendet werden, indem beispielsweise verwundete Blätter oder Blattstücke in einer Agrobakterienlösung gebadet und anschliessend in geeigneten Medien kultiviert werden. Die Transformation von Pflanzen durch Agrobakterien ist beschrieben (White FF, Vectors for Gene Transfer in Higher Plants; in Transgenic Plants, Vol. 1, Engineering and Utilization, herausgegeben von S.D. Kung und R. Wu, Academic Press, 1993, S. 15-38; Jenes B et al.(1993) Techniques for Gene Transfer, in: Transgenic Plants, Vol. 1, Engineering and Utilization, herausgegeben von S.D. Kung und R. Wu, Academic Press, S.128-143; Potrykus (1991) Annu Rev Plant Physiol Plant Molec Biol 42:205- 225). Aus den transformierten Zellen der verwundeten Blätter bzw. Blattstücke können in bekannter Weise transgene Pflanzen regeneriert werden.

Für die Transformation können unterschiedliche Explantate, Zellkulturen, Gewebe, Organen, Embryonen, Samen, Mikrosporen oder anderen einzelzelligen oder mehrzelligen zelluläre Strukturen abgeleitet von einem pflanzlichen Organismus eingesetzt werden. Auf die jeweiligen Explantate, Kulturen oder Gewebe abgestimmte Transformationsverfahren sind dem Fachmann bekannt. Beispielhaft seien zu nennen: Sprossinternodien (Fry J et al. (1987) Plant Cell Rep. 6:321-325), Hypokotyle (Radke SE et al. (1988) Theor Appl Genet 75:685-694; Schröder M et al. (1994) Physiologia Plant 92: 37-46.; Stefanov I et al. (1994) Plant Sci. 95:175-186; Weier et al. (1997) Fett/Lipid 99:160-165), kotyledonäre Petiolen (Meloney MM et al. (1989) Plant Cell Rep 8:238-242; Weier D et al. (1998) Molecular Breeding 4:39-46), Mikrosporen und Proembryonen (Pechnan (1989) Plant Cell Rep. 8:387-390) und Blütenstiele (Boulter ME et al. (1990) Plant Sci 70:91-99; Guerche P et al. (1987) Mol Gen Genet 206:382-386). Bei einem direkten Gentransfer können Mesophyllprotoplasten (Chapel PJ & Glimelius K (1990) Plant Cell Rep 9: 105-108; Golz et al. (1990) Plant Mol Biol 15:475-483) aber auch Hypokotylprotoplasten (Bergmann P & Glimelius K (1993) Physiologia Plant 88:604-611) und Mikrosporen (Chen JL et al. (1994) Theor Appl Genet 88:187-192; Jonesvilleneuve E et al. (1995) Plant Cell Tissue and Organ Cult 40:97-100) und Sprossabschnitte (Seki M et al. (1991) Plant Mol Biol 17:259-263) erfolgreich eingesetzt werden.

Stabil transformierte Zellen d.h. solche, die die eingeführte DNA integriert in die DNA der Wirtszelle enthalten, können von untransformierten unter Einsatz des erfindungsgemäßen Selektionsverfahrens selektioniert werden. Die erhaltenen Pflanzen können in üblicher Weise gezüchtet und gekreuzt werden. Zwei oder mehr Generationen sollten vorzugsweise kultiviert werden, um sicherzustellen, dass die genomische Integration stabil und vererblich ist.

Sobald eine transformierte Pflanzenzelle hergestellt wurde, kann eine vollständige Pflanze unter Verwendung von dem Fachmann bekannten Verfahren erhalten werden. Hierbei geht man beispielhaft von Kalluskulturen, einzelnen Zellen (z.B. Protoplasten) oder Blattscheiben aus (Vasil et al. (1984) Cell Culture and Somatic Cel Genetics of Plants, Vol I, II and III, Laboratory Procedures and nTheir Applications, Academic Press; Weissbach and Weissbach (1989) Methods for Plant Molecular Biology, Academic Press). Aus diesen noch undifferenzierten Callus-Zellmassen kann die Bildung von Spross und Wurzel in bekannter Weise induziert werden. Die erhaltenen Sprösslinge können ausgepflanzt und gezüchtet werden. Entsprechende Verfahren sind beschrieben (Fennell et al. (1992) Plant Cell Rep. 11: 567-570; Stoeger et al. (1995) Plant Cell Rep. 14:273-278; Jahne et al. (1994) Theor Appl Genet 89:525-533).

Die Wirksamkeit der Expression der transgen exprimierten Nukleinsäuren kann beispielsweise *in vitro* durch Sprossmeristemvermehrung unter Verwendung einer der oben beschriebenen Selektionsmethoden ermittelt werden. Zudem kann eine in Art und Höhe veränderte Expression eines Zielgens und die Auswirkung auf den Phänptyp der Pflanze an Testpflanzen in Gewächshausversuchen getestet werden.

Bevorzugt wird das erfindungsgemäße Verfahren im Rahmen der Pflanzenbiotechnologie zur Erzeugung von Pflanzen mit vorteilhaften Eigenschaften eingesetzt. Die in das Genom der pflanzlichen Zelle oder des pflanzlichen Organismus zu "insertierende Nukleinsäuresequenz" umfasst bevorzugt mindestens eine Expressionskassette, wobei besagte Expressionskassette unter Kontrolle eines in pflanzlichen Zellen oder pflanzlichen Organismen funktionellen Promotors eine RNA und/oder ein Protein exprimieren kann, welche nicht die Verminderung der Expression, Menge, Aktivität und/oder Funktion eines Markerproteins bewirken, sondern - besonders bevorzugt - der so genetische veränderten Pflanze einen vorteilhaften Phänotyp verleihen. Dem Fachmann sind zahlreiche Gene und Proteine bekannt, die zum Erreichen eines vorteilhaften Phänotyp beispielsweise zur Qualitätssteigerung bei Nahrungsmitteln oder zur Produktion bestimmter Chemikalien oder Pharmazeutika (Dunwell JM (2000) J Exp Bot 51 Spec No:487-96) verwendet werden können.

So kann die Eignung der Pflanzen oder deren Samen als Nahrungs- oder Futtermittel verbessert werden, beispielsweise über eine Veränderung der Zusammensetzungen und/oder des Gehalt an Metaboliten, insbesondere Proteinen, Ölen, Vitaminen und/oder Stärke. Auch können Wachstumsrate, Ertrag oder die Resistenz gegen biotische oder abiotische Stressfaktoren erhöht werden. Vorteilhafte Effekte können sowohl durch transgene Expression von Nukleinsäuren oder Proteinen als auch durch gezielte Verminderung der Expression endogener Gene hinsichtlich des Phänotypes der transgenen Pflanze erzielt werden. Die in der transgenen Pflanze zu erzielenden vorteilhaften Effekte umfassen beispielsweise:
- Erhöhte Resistenz gegen Pathogene (biotischer Stress)
- Erhöhte Resistenz gegen Umwelteinflüsse wie Hitze, Kälte, Frost Trockenheit, UV-Licht, oxidativen Stress, Nässe, Salz etc. (abiotischer Stress)
- Erhöhte Ertragsleistung
- Verbesserte Qualität z.B. erhöhter Nährwert, erhöhte Lagerfähigkeit

Ein weiterer Gegenstand der Erfindung betrifft die Verwendung der nach dem erfindungsgemässen Verfahren hergestellten transgenen Pflanzen, und der von ihnen abgeleitete Zellen, Zellkulturen, Teile oder Vermehrungsgut wie Saaten oder Früchte, zur Herstellung von Nahrungs- oder Futtermitteln, Pharmazeutika oder Feinchemikalien, wie beispielsweise Enzymen, Vitaminen, Aminosäuren, Zucker, Fettsäuren, natürliche und synthetische Geschmacks-, Aroma- und Farbstoffe. Besonders bevorzugt ist die Produktion von Triaclyglyceriden, Lipiden, Ölen, Fettsäuren, Stärke, Tocopherolen und Tocotrienolen sowie Carotinoiden. Von Menschen und Tieren verzehrbare erfindungsgemäße, genetisch veränderte Pflanzen können auch beispielsweise direkt oder nach an sich bekannter Aufbereitung als Nahrungsmittel oder Futtermittel verwendet werden.

Wie bereits oben erwähnt, umfasst das erfindungsgemäße Verfahren in einer besonders vorteilhaften Ausführungsform in einem der Selektion nachgeschalteten Verfahrenschritt die Deletion der für das Markerprotein kodierenden Sequenz (z.B. durch Rekombinase vermittelt oder wie in WO03/004659 beschrieben) bzw. die Auskreuzung und/oder Segregation besagter Sequenzen. (Dem Fachmann ist klar, das dazu in den transformierten Zellen die in das Genom integrierte Nukleinsäureseuqnz und die für das Markerprotein kodierende Sequenz einen separaten chromosomalen Lokus aufweisen sollten. Dies ist jedoch bei der Mehrzahl der resultierenden Pflanzen allein aus statistischen Gründen gegeben). Diese Vorgehensweise ist insbesondere vorteilhaft, wenn es sich bei dem Markerprotein um ein Transgen handelt, das ansonsten in der zu transformierenden Pflanze nicht vorkommt. Die resultierende Pflanze kann zwar noch unter Umständen die Verbindung zur Verminderung der Expression, Menge, Aktivität und/oder Funktion des Markerproteins beinhalten, doch hätte diese kein "Gegenstück" mehr in Form des besagten Markerproteins, wäre also wirkungslos. Dies ist insbesondere dann der Fall, wenn das Markerprotein aus einem nicht-pflanzlichen Organismus stammt und/oder synthetisch ist (beispielsweise das codA Protein). Es können aber auch Pflanzliche Markperproteine aus anderen Pflanzenarten eingesetzt werden, die in der zu transformierenden Zelle ansonsten (d.h. wenn nicht als Transgen eingeführt) nicht vorkommen. Besagte Markerproteine sind im Rahmen dieser Erfindung als "nicht-endogene" Markerproteine bezeichnet.

Ganz besonders vorteilhaft ist es, wenn es sich bei der Verbindung zur Verminderung der Expression, Menge, Aktivität und/oder Funktion des Markerproteins um eine RNA handelt. Nach der Deletion bzw. Auskreuzung/Segregation würde die resultierende transgene Pflanze kein unnötiges (und ggf. unerwünschtes). Fremdprotein mehr aufweisen. Einziges Fremdprotein ist u.U. das aus der in das Genom insertierten Nukleinsäuresequenz resultierenden Protein. Aus Gründen der Produktzulassung ist diese Ausführungsform besondersvorteilhaft. Wie oben beschrieben kann diese RNA eine antisense RNA oder - besonders bevorzugt - eine doppelsträngige RNA sein. Sie kann separat von der für das Zielprotein kodierenden RNA exprimiert werden, aber - unter Umständen - auch auf einem Strang mit derselben.

Zusammenfassend umfasst die besonders vorteilhafte Ausführungsform folgende Merkmale:
Verfahren zur Herstellung transformierter pflanzlicher Zellen oder Organismen, umfassend nachfolgende Schritte:
   a) Transformation einer Population pflanzlicher Zellen, welche mindestens ein nicht-endogenes (bevorzugt nicht-pflanzliches) Markerprotein umfasst, das in der Lage ist, eine für besagte Population pflanzlicher Zellen nicht-toxische Substanz X in eine für besagte Population toxische Substanz Y direkt oder indirekt umzusetzen, mit mindestens einer zu insertierenden Nukleinsäuresequenz in Kombination mit mindestens einer Nukleinsäuresequenz kodierend für eine Ribonukleinsäuresequenz befähigt zur Verminderung der Expression, Menge, Aktivität und/oder Funktion besagten Markerproteins, und
   b) Behandlung besagter Population pflanzlicher Zellen mit der Substanz X in einer Konzentration, die infolge der Umsetzung durch das Markerprotein einen für nicht-transformierte Zellen toxischen Effekt bedingt, und
   c) Selektion von transformierten pflanzlichen Zellen (und/oder Populationen pflanzlicher Zellen wie pflanzlichen Geweben oder Pflanzen), die in ihrem Genom besagte Nukleinsäuresequenz aufweisen und die infolge der Wirkung besagter Verbindung gegenüber nicht-transformierten Zellen einen Wachstumsvorteil haben, aus besagter Population pflanzlicher Zellen, wobei die Selektion unter Bedingungen durchgeführt wird, bei denen das Markerprotein seinen toxischen Effekt auf die nicht- transformierten Zellen ausüben kann, und
   d) Regeneration von fertilen Pflanzen, und
   e) Auskreuzung der für das Markerprotein kodierenden Nukleinsäuresequenz und Isolation von fertilen Pflanzen, die in ihrem Genom besagte Nukleinsäuresequenz aber nicht mehr die für das Markerprotein kodierende Sequenz aufweisen.

### Sequenzen

| | | |
|---|---|---|
| 1. | SEQ ID NO: 1 | Nukleinsäuresequenz kodierend für Cytosindeaminase aus E.coli (codA) |
| 2. | SEQ ID NO: 2 | Aminosäuresequenz kodierend für Cytosindeaminase aus E.coli (codA) |
| 3. | SEQ ID NO: 3 | Nukleinsäuresequenz kodierend für Cytosindeaminase aus E.coli (codA) mit modifiziertem Startkodon (GTG/ATG) zur Expression in Eukaryoten |
| 4. | SEQ ID NO: 4 | Aminosäuresequenz kodierend für Cytosindeaminase aus E.coli (codA) mit modifiziertem Startkodon (GTG/ATG) zur Expression in Eukaryoten |
| 5. | SEQ ID NO: 5 | Nukleinsäuresequenz kodierend für Cytochrom P450-SU1 (suaC) aus Streptomyces griseolus |
| 6. | SEQ ID NO: 6 | Aminosäuresequenz kodierend für Cytochrom P450-SU1 (suaC) aus Streptomyces griseolus |
| 7. | SEQ ID NO: 7 | Nukleinsäuresequenz kodierend für Indolacetamidhydrolase (tms2) aus Agrobacterium tumefaciens |
| 8. | SEQ ID NO: 8 | Aminosäuresequenz kodierend für Indolacetamidhydrolase (tms2) aus Agrobacterium tumefaciens |
| 9. | SEQ ID NO: 9 | Nukleinsäuresequenz kodierend für Indolacetamidhydrolase (tms2) aus Agrobacterium tumefaciens |
| 10. | SEQ ID NO: 10 | Aminosäuresequenz kodierend für Indolacetamidhydrolase (tms2) aus Agrobacterium tumefaciens |
| 11. | SEQ ID NO: 11 | Nukleinsäuresequenz kodierend für Haloalkandehalogenase (dhlA) aus Xanthobacter autotrophicus |
| 12. | SEQ ID NO: 12 | Aminosäuresequenz kodierend für Haloalkandehalogenase (dhlA) aus Xanthobacter autotrophicus |
| 13. | SEQ ID NO: 13 | Nukleinsäuresequenz kodierend für Thymidinkinase aus Herpes simplex Virus 1 |
| 14. | SEQ ID NO: 14 | Aminosäuresequenz kodierend für Thymidinkinase aus Herpes simplex Virus 1 |
| 15. | SEQ ID NO: 15 | Nukleinsäuresequenz kodierend für Thymidinkinase aus Herpes simplex Virus 1 |
| 16. | SEQ ID NO: 16 | Aminosäuresequenz kodierend für Thymidinkinase aus Herpes simplex Virus 1 |
| 17. | SEQ ID NO: 17 | Nukleinsäuresequenz kodierend für Hypoxanthin-Xanthin-Guanin Phosphoribosyltransferase aus Toxoplasma gondii |
| 18. | SEQ ID NO: 18 | Aminosäuresequenz kodierend für Hypoxanthin-Xanthin-Guanin Phosphoribosyltransferase aus Toxoplasma gondii |
| 19. | SEQ ID NO: 19 | Nukleinsäuresequenz kodierend für Xanthin-Guanin-Phosphoribosyltransferase aus E.coli |
| 20. | SEQ ID NO: 20 | Aminosäuresequenz kodierend für Xanthin-Guanin-Phosphoribosyltransferase aus E.coli |
| 21. | SEQ ID NO: 21 | Nukleinsäuresequenz kodierend für Xanthin-Guanin-Phosphoribosyltransferase aus E.coli |
| 22. | SEQ ID NO: 22 | Aminosäuresequenz kodierend für Xanthin-Guanin-Phosphoribosyltransferase aus E.coli |
| 23. | SEQ ID NO: 23 | Nukleinsäuresequenz kodierend für Purinnukleosidphosphorylase (deoD) aus E.coli |
| 24. | SEQ ID NO: 24 | Nukleinsäuresequenz kodierend für Purinnukleosidphosphorylase (deoD) aus E.coli |
| 25. | SEQ ID NO: 25 | Nukleinsäuresequenz kodierend für Phosphonatmonoesterhydrolase (pehA) aus Burkholderia caryophylli |
| 26. | SEQ ID NO: 26 | Aminosäuresequenz kodierend für Phosphonatmonoesterhydrolase (pehA) aus Burkholderia caryophylli |
| 27. | SEQ ID NO: 27 | Nukleinsäuresequenz kodierend für Tryptophanoxygenase (aux1) aus Agrobacterium rhizogenes |
| 28. | SEQ ID NO: 28 | Aminosäuresequenz kodierend für Tryptophanoxygenase (aux1) aus Agrobacterium rhizogenes |
| 29. | SEQ ID NO: 29 | Nukleinsäuresequenz kodierend für Indolacetamidhydrolase (aux2) aus Agrobacterium rhizogenes |
| 30. | SEQ ID NO: 30 | Aminosäuresequenz kodierend für Indolacetamidhydrolase (aux2) aus Agrobacterium rhizogenes |
| 31. | SEQ ID NO: 31 | Nukleinsäuresequenz kodierend für Tryptophanoxygenase (aux1) aus Agrobacterium tumefaciens |
| 32. | SEQ ID NO: 32 | Aminosäuresequenz kodierend für Tryptophanoxygenase (aux1) aus Agrobacterium tumefaciens |
| 33. | SEQ ID NO: | 33-Nukleinsäuresequenz kodierend für Indolacetamidhydrolase (aux2) aus Agrobacterium tumefaciens |
| 34. | SEQ ID NO: 34 | Aminosäuresequenz kodierend für Indolacetamidhydrolase (aux2) aus Agrobacterium tumefaciens |
| 35. | SEQ ID NO: 35 | Nukleinsäuresequenz kodierend für Indolacetamidhydrolase (aux2) aus Agrobacterium vitis |
| 36. | SEQ ID NO: 36 | Aminosäuresequenz kodierend für Indolacetamidhydrolase (aux2) aus Agrobacterium vitis |
| 37. | SEQ ID NO: 37 | Nukleinsäuresequenz kodierend für 5-Methylthioribosekinase (mtrK) aus Arabidopsis thaliana |
| 38. | SEQ ID NO: 38 | Aminosäuresequenz kodierend für 5-Methylthioribosekinase (mtrK) aus Arabidopsis thaliana |
| 39. | SEQ ID NO: 39 | Nukleinsäuresequenz kodierend für 5-Methylthioribosekinase (mtrK) aus Klebsiella pneumoniae |
| 40. | SEQ ID NO: 40 | Aminosäuresequenz kodierend für 5-Methylthioribosekinase (mtrK) aus Klebsiella pneumoniae |
| 41. | SEQ ID NO: 41 | Nukleinsäuresequenz kodierend für Alkoholdehydrogenase (adh) aus Arabidopsis thaliana |
| 42. | SEQ ID NO: 42 | Aminosäuresequenz kodierend für Alkoholdehydrogenase (adh) aus Arabidopsis thaliana |
| 43. | SEQ ID NO: 43 | Nukleinsäuresequenz kodierend für Alkoholdehydrogenase (adh) aus Hordeum vulgare (Gerste) |
| 44. | SEQ ID NO: 44 | Aminosäuresequenz kodierend für Alkoholdehydrogenase (adh) aus Hordeum vulgare (Gerste) |
| 45. | SEQ ID NO: 45 | Nukleinsäuresequenz kodierend für Alkoholdehydrogenase (adh) aus Oryza sativa (Reis) |
| 46. | SEQ ID NO: 46 | Aminosäuresequenz kodierend für Alkoholdehydrogenase (adh) aus Oryza sativa (Reis) |
| 47. | SEQ ID NO: 47 | Nukleinsäuresequenz kodierend für Alkoholdehydrogenase (adh) aus Zea mays (Mais) |
| 48. | SEQ ID NO: 48 | Aminosäuresequenz kodierend für Alkoholdehydrogenase (adh) aus Zea mays (Mais) |
| 49. | SEQ ID NO: 49 | Nukleinsäuresequenz kodierend für ein sense-RNA Fragment der Cytosindeaminase aus E.coli (codARNAi-sense) |
| 50. | SEQ ID NO: 50 | Oligonukleotidprimer codA5'HindIII |
| | | 5'-AAGCTTGGCTAACAGTGTCGAATAACG-3' |
| 51. | SEQ ID NO: 51 | Oligonukleotidprimer codA3'SalI |
| | | 5'-GTCGACGACAAAATCCCTTCCTGAGG-3' |
| 52. | SEQ ID NO: 52 | Nukleinsäuresequenz kodierend für ein antisense-RNA Fragment der Cytosindeaminase aus E.coli (codARNAi-anti) |
| 53. | SEQ ID NO: 53 | Oligonukleotidprimer codA5'EcoRI |
| | | 5'-GAATTCGGCTAACAGTGTCGAATAACG-3' |
| 54. | SEQ ID NO: 54 | Oligonukleotidprimer codA3'BamHI |
| | | 5'-GGATCCGACAAAATCCCTTCCTGAGG- 3' |
| 55. | SEQ ID NO: 55 | Vektorkonstrukt pBluKS-nitP-STLS1-35S-T |
| 56. | SEQ ID NO: 56 | Expressionsvektor pSUN-1 |
| 57. | SEQ ID NO: 57 | Transgener Expressionsvektor pSUN-1-codA-RNAi |
| 58. | SEQ ID NO: 58 | Transgener Expressionsvektor |
| | | pSUNl-codA-RNAi-At.Act.-2-At.Als-R-ocsT |
| 59. | SEQ ID NO: 59 | Nukleinsäuresequenz kodierend für 5-Methylthioribosekinase (mtrK) aus Mais (Zea mays); Fragment |
| 60. | SEQ ID NO: 60 | Aminosäuresequenz kodierend für 5-Methylthioribosekinase (mtrk) aus Mais (Zea mays); Fragment |
| 61. | SEQ ID NO: 61 | Nukleinsäuresequenz kodierend für 5-Methylthioribosekinase (mtrK) aus Raps (Brassica napus), Fragment |
| 62. | SEQ ID NO: 62 | Aminosäuresequenz kodierend für 5-Methylthioribosekinase (mtrK) aus Raps (Brassica napus), Fragment |
| 63. | SEQ ID NO: 63 | Nukleinsäuresequenz kodierend für 5-Methylthioribosekinase (mtrK) aus Raps (Brassica napus), Fragment |
| 64. | SEQ ID NO: 64 | Aminosäuresequenz kodierend für 5-Methylthioribosekinase (mtrK) aus Raps (Brassica napus), Fragment |
| 65. | SEQ ID NO: 65 | Nukleinsäuresequenz kodierend für 5-Methylthioribosekinase (mtrK) aus Reis (Oryza sativa) Fragment |
| 66. | SEQ ID NO: 66 | Aminosäuresequenz kodierend für 5-Methylthioribosekinase (mtrK) aus Reis (Oryza sativa) Fragment |
| 67. | SEQ ID NO: 67 | Nukleinsäuresequenz kodierend für 5-Methylthioribosekinase (mtrK) aus Soja (Glycine max), Fragment |
| 68. | SEQ ID NO: 68 | Aminosäuresequenz kodierend für 5-Methylthioribosekinase (mtrK) aus Soja (Glycine max), Fragment |
| 69. | SEQ ID NO: 69 | Oligonukleotidprimer coc7A5'C-term |
| | | 5'-CGTGAATACGGCGTGGAGTCG-3' |
| 70. | SEQ ID NO: 70 | Oligonukleotidprimer codA3'C-term |
| | | 5'-CGGCAGGATAATCAGGTTGG-3' |
| 71. | SEQ ID NO: 71 | Oligonukleotidprimer 35sT 5' Primer |
| | | 5'-GTCAACGTAACCAACCCTGC-3' |

### Abbildungen

- Fig.1 :: Inaktivierung des Markerproteingens mittels Einbringen einer Rekombinase
P: Promotor
MP: Sequenz kodierend für ein Markerprotein
R1/R2: Rakombinase-Brkennungssequensen
R: Rekoinbinase bzw. Sequenz kodierend für Rekombinase. In einer Methode wird die Inaktivierung des Markerproteingens durch das Einbringen einer sequenzspezifischen Rekombinase realisiert. Bevorzugt wird die Rekombinase - wie hier dargestellt - ausgehend von einer Expressionskassette exprimiert.
Das Markerproteingen ist von Erkennungssequengen für sequenzspezifische Rekombinasen flankiert, wobei durch Einbringen der Rekombinase Sequenzen des Markerproteingens deletiert werden und so eine Inakt-vierung des Markerproteinsgens erfolgt.
- Fig.2-A:: Inaktivierung des Markerproteingens durch Einwirken einer sequenzpezifishen Nuklease
P: Promotor
DS: Erkennungssequenz zur gezielten Induk- tion von DNA-Dopppelstrangbrüchen
MP-DS-MP': Sequenz kodierend Für ein Markerprotein umfassend eine DS
nDS: Inaktivierte DS
E: Sequenzspezifisches Enzym zur gezielten Induktion von DNA-Doppelstrangbrüchen Das Markerprotein-Gen Kann durch eine gezielte Mutation oder Deletion im Markerprotein-Gen z.B. durch sequenzspezifische Induktion von DNA-Doppelstrangbrüchen an einer Erkennungssequenz zur gezielten Induktien von DNA- Doppelstrangbrüchen in oder in der Nähe des Markerprotein-Gens (P-MP) realisiert werden. Der Dopelstrangbruch kann in der kodierenden Region oder aber auch der nicht-kodierenden (wie beispielsweise dem Promoter) erfolgen, induziert eine illegitime Rekombination (nicht-homologe Verbindung von DNA- Enden; "non-homologous end- joining") und so z.B. eine Verschiebung im Leseraster des Markerproteins.
- Fig.2-B:: Inaktivierung des Markerproteingens durch Einwirken einer sequenzspezifischen Nuklease
P: Promotor
DS: Erkennungssequenz zur gezielten Induk- tion von DNA-Doppelstrangbrüchen
MP: Sequenz kodierend für ein Markerprotein
nDS: Inaktivierte DS
E: Sequenzspezifisches Enzym zur gezielten Induktion von DNA-Doppelstrangbrüchen Das Markerprotein-Gen kann durch eine gezielte Deletion durch sequenzspezifische Induktion von mehr als einem sequenzspezifischen DNA-Doppelstranbruch in oder in der Nähe des Markerprotein-Gens realisiert werden. Die Doppelstrangbrüche können in der kodierenden Region oder aber auch der nicht-kodierenden (wie beispielsweise dem Promotor) erfolgen und induzieren eine Deletion im Markerprotein-Gen. Bevorzugt ist das Markerprotein-Gen von DS Sequenzen flankiert und wird vollständig durch Einwirken des Enzyms E deletiert.
- Fig. 3:: Inaktivierung des Markerprotein-Gens durch Induktion einer intramolekularen homologen Rekombination infolge des Einwirkens einer sequenzspezifischen Nuklease
A/A': Sequenzen mit einer ausreichenden Länge und Homologie zueinander, um infolge des induzierten Doppelstrangbruches miteinander zu rekombinieren
P: Promotor
DS: Erkennungssequenz zur gezielten Induktion von DNA-Doppelstrangbrüchen
MP: Sequenz kodierend für ein Markerprotein
E: Sequenzspezifisches Enzym zur gezielten Induktion von DNA-Doppelstrangbrüchen Das Markerprotein-Gen kann durch eine Deletion mittels intramolekularen homologer Rekombination inaktiviert werden. Die homologe Rekombination kann durch sequenz- spezifische Induktion von DNA-Doppelstrangbrüchen an einer Erkennungssequenz zur gezielten Induktion von DNA- Doppelstrangbrüchen in oder in der Nähe des Marker- protein-Gens initiiert werden. Die homologe Rekombination erfolgt zwischen den Sequenzen A und A', die eine aus- reichenden Länge und Homologie zueinander haben, um infolge des induzierten Doppelstrangbruches miteinander zu rekombinieren. Die Rekombination bewirkt eine Deletion essentieller sequenzen des Markerprotein-Gens.
- Fig. 4:: Inaktivierung des Markerprotein-Gens durch intermole- kulare homologe Rekombination
- A/A':: Sequenzen mit einer ausreichenden Länge und Holomogie zueinander, um miteinander zu rekombinieren
B/B': Sequenzen mit einer ausreichenden Länge und Holomogie zueinander, um miteinander zu rekombinieren
P: Promotor
I: zu insertierende Nukleinsäuresequenz /: Gen von Interesse
MP: Sequenz kodierend für ein Markerprotein Die Inaktivierung des Markerprotein-Gens (P-MP) kann auch durch eine gezielte Insertion in das Markerprotein-Gen z.B. mittels intermolekularer homologer Rekombination realisiert werden. Dabei ist die zu insertierende Region an ihrem 5'- und 3'-Ende von Nukleinsäuresequenzen (A' bzw. B') flankiert, die eine ausreichende Länge und Homo- logie zu entsprechenden flankierenden Sequenzen des Markerproteingens (A bzw. B) aufweisen, um eine homologe Rekombination zwischen A und A' und B und B' zu ermög- lichen. Die Rekombination bewirkt eine Deletion essen- tieller sequenzen des Markerprotein-Gens.

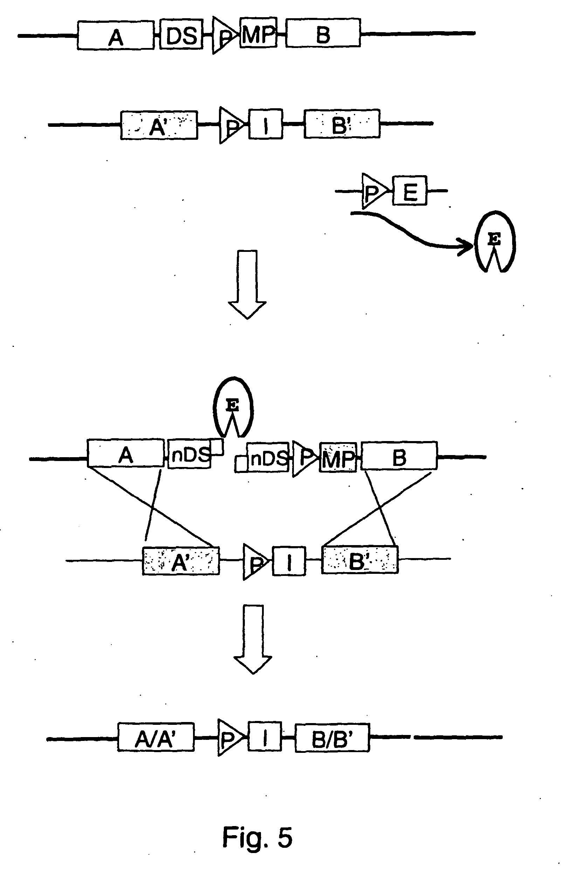

- Fig. 5:: Inaktivierung des Markerprotein-Gens durch intermole- kulare homologe Rekombination infolge des Einwirkens einer sequenzspezifischen Nuklease
A/A': Sequenzen mit einer ausreichenden Länge und Holomogie zueinander, um miteinander zu rekombinieren
B/B': Sequenzen mit einer ausreichenden Länge und Holomogie zueinander, um miteinander zu rekombinieren
P: promotor
I: zu insertierende Nukleinsäuresequenz / Gen von Interesse
MP: Sequenz kodierend für ein Markerprotein
DS: Erkennungssequenz zur gezielten Induktion von DNA-Doppelstrangbrüchen
E: Sequenzspzifisches Enzym zur gezielten Induktion von DNA-Doppelstrangbrüchen Die Inaktivierung des Markerprotein-Gens kann auch durch eine gezielte Insertion in das Markerprotein-Gen z.B. mittels intermolekularer homologer Rekombination reali- siert werden. Die homologe Rekombination kann durch sequenzspezifische Induktion von DNA-Doppelstrangbrüchen an einer Erkennungssequenz zur gezielten Induktion von DNA-Doppelstrangbrüchen in oder in der Nähe des Marker- protein-Gens initiiert werden. Dabei ist die zu inser- tierende Region an ihrem 5'- und 3'-Ende von Nuklein- säuresequenzen (A' bzw. B') flankiert, die eine aus- reichende Länge und Homologie zu entsprechenden flan- kierenden Sequenzen des Markerprotein-Gens (A bzw. B) aufweisen, um eine homologe Rekombination zwischen A und A' und B und B' zu ermöglichen. Die Rekombination bewirkt eine Deletion essentieller sequenzen des Markerprotein- Gens.

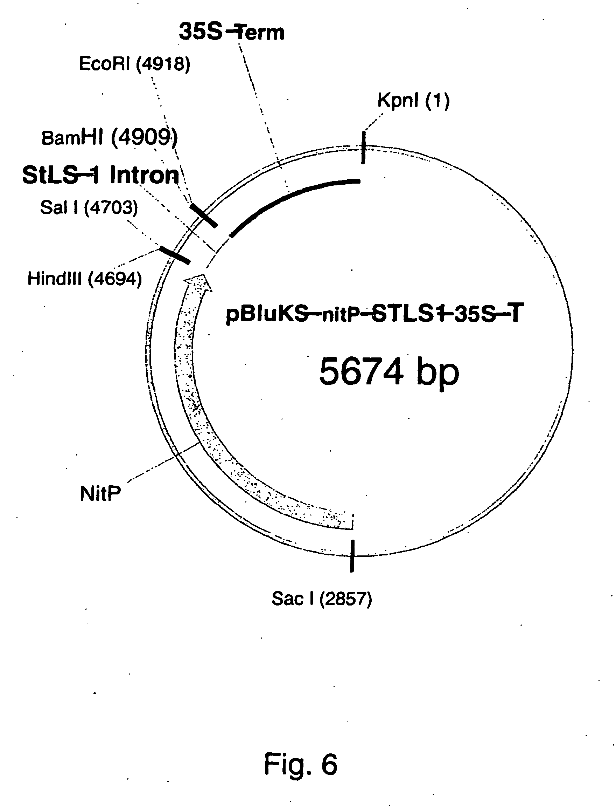

- Fig. 6:: Vektorkarte für pBluKS-nitP-STLS1-35S-T (SEQ ID NO: 55)
MitP: Promotor des NitrilaseI-Gens aus A.thaliana (Gen- Bank Acc.-No.: Y07648.2, Hillebrand et al. (1996) Gene 170:197-200)
STLS-1 Intron: Intron des ST-LS1 Gens aus Kartoffel (Van- canneyt GF et al. (1990) Mol Gen Genet 220(2):245-250).
35S-Term: Terminator des 35S CaMV Gens (Blumenkohlmosaik- virus; Franck et al. (1980) Cell 21:285-294).
Schnittstellen relevanter Restriktionsendonukleasen sind mit ihrer jeweiligen Schnittposition angegeben.

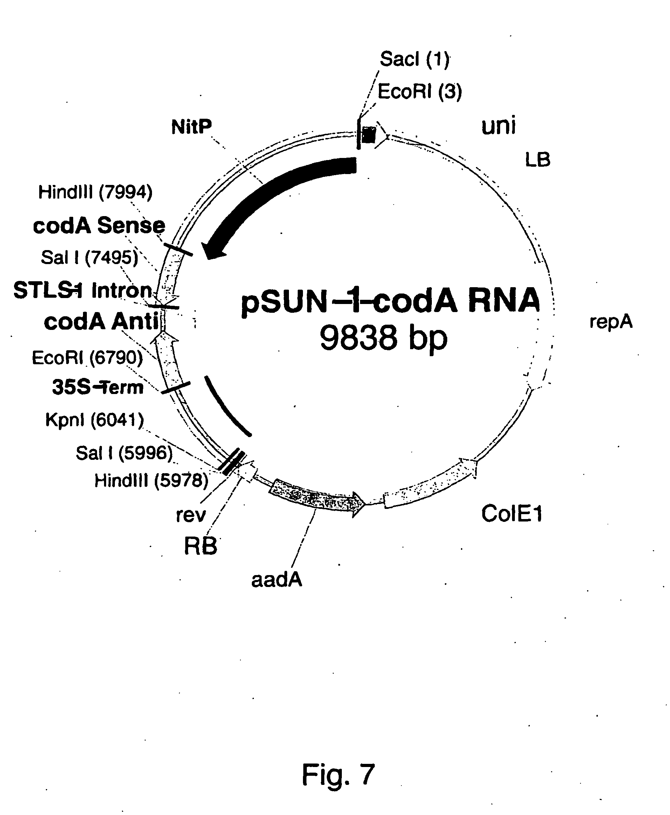

- Fig. 7:: Vektorkarte für den transgenen Expressionsvektor pSUN-1-codA-RNAi (SEQ ID NO: 57)
NitP: Promotor des NitrilaseI-Gens aus A.thaliana (Gen- Bank Acc.-No.: Y07648.2, Hillebrand et al. (1996) 170:197-200)
STLS-1 Intron: Intron des ST-LS1 Gens aus Kartoffel (Van- canneyt GF et al. (1990) Mol Gen Genet 220(2):245-250).
35S-Term: Terminator des 35S CaMV Gens (Blumenkohlmosaik- virus: Franck et al. (1980) Cell 21:285-294).
codA-sense: Nukleinsäuresequenz kodierend für ein sense- RNA Fragment der Cytosindeaminase aus E.coli (codARNAi- sense; SEQ ID NO: 49)
codA-anti: Nukleinsäuresequenz kodierend für ein anti- sense-RNA Fragment der Cytosindeaminase aus E.coli (codARNAi-anti; SEQ ID NO: 52)
LB/RB: Linke bzw. rechte Grenze der Agrobacterium T-DNA
Schnittstellen relevanter Restriktionsendonukleasen sind mit ihrer jeweiligen Schnittposition angegeben. Weitere Elemente stellen übliche Elemente eines binären Agro- bakterium-Vektors dar (aadA; ColE1; repA)

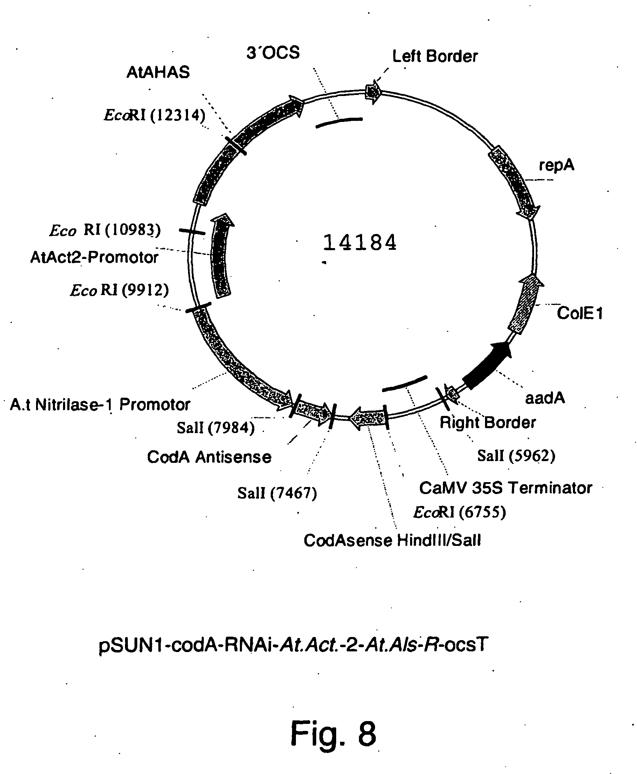

- Fig. 8:: Vektorkarte für den transgenen Expressionsvektor pSUN1-codA-RNAi-At.Act.-2-At.Als-R-ocsT (SEQ ID NO: 58)
NitP: Promotor des Nitrilase I-Gens aus A.thaliana (Gen- Bank Acc.-No.: Y07648.2, Hillebrand et al. (1996) Gene 170:197-200)
STLS-1 Intron: Intron des ST-LS1 Gens aus Kartoffel (Van- canneyt GF et al. (1990) Mol Gen Genet 220(2):245-250).
35S-Terminator: Terminator des 35S CaMV Gens (Blumenkohl- mosaikvirus; Franck et al. (1980) Cell 21:285-294).
codA-sense: Nukleinsäuresequenz kodierend für ein sense- RNA Fragment der Cytosindeaminase aus E.coli (codARNAi- sense; SEQ ID NO: 49)
codA-anti: Nukleinsäuresequenz kodierend für ein anti- sense-RNA Fragment der Cytosindeaminase aus E.coli icodARNAi-anti; SEQ ID NO: 52)
Left Border/Right Border: Linke bzw. rechte Grenze der Agrobacterium T-DNA
Schnittstellen relevanter Restriktionsendonukleasen sind mit ihrer jeweiligen Schnittposition angegeben. Weitere Elemente stellen übliche Elemente eines binären Agro- bakterium-Vektors dar (aadA; ColEl; repA)
- Fig.9a-b:: Sequenzvergleich von diversen 5-Methylthioribose (MTR) kinasen aus verschiedenen Organismen, insbesondere pflanzlichen Organismen. Gezeigt sind Sequenzen aus Klebsiella pneumoniae, Clostridium tetani, Arabidopsis thaliana (A.thaliana), Raps (Brassica napus), Sojabohne (Soy-1), Reis ( Oryza sativa-1) sowie die Konsensus- sequenz (Consensus). Homologe Bereiche können aus der Konsensussequenz leicht abgeleitet werden.

### Ausführungsbeispiele

### Allgemeine Methoden

Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die im Rahmen der vorliegenden Erfindung durchgeführten Klonierungsschritte wie z.B. Restriktionsspaltungen, Agarosegelelektrophorese, Reinigung von DNA-Fragmenten, Transfer von Nukleinsäuren auf Nitrozellulose und Nylonmembranen, Verknüpfen von DNA-Fragmenten, Transformation von E. coli Zellen, Anzucht von Bakterien, Vermehrung von Phagen und Sequenzanalyse rekombinanter DNA werden wie bei Sambrook et al. (1989) Cold Spring Harbor Laboratory Press; ISBN 0-87969-309-6 beschrieben durchgeführt. Die Sequenzierung rekombinanter DNA-Moleküle erfolgt mit einem Laserfluoreszenz-DNA-Sequerizierer der Firma ABI nach der Methode von Sanger (Sanger et al. (1977) Proc Natl Acad Sci USA 74:5463-5467).

### Beispiel 1: Herstellung der codA-Fragmente

Zunächst wird eine verkürzte und am 5' bzw. 3' Ende durch Addition von Erkennungssequenzen der Restriktionsenzyme HindIII und SalI modifizierte, Nukleinsäurevariante des codA Gens unter Verwendung der PCR-Technologie hergestellt. Dazu wird ein Teil des codA Gens (GeneBank Acc.-No.: S56903; SEQ ID NO: 1) mittels Polymerasekettenreaktion (PCR) aus dem Herkunftsorganismus E.coli unter Verwendung eines sense-spezifischen Primers (codA5'HindIII; SEQ ID NO: 50) und eines antisense-spezifischen Primers (codA3'SalI; SEQ ID NO: 51) amplifiziert.
codA5'HindIII: 5'-AAGCTTGGCTAACAGTGTCGAATAACG-3' (SEQ ID NO: 50)
codA3'SalI: 5'-GTCGACGACAAAATCCCTTCCTGAGG-3' (SEQ ID NO: 51)

Die PCR erfolgt in einem 50 µl Reaktionsansatz in dem enthalten sind:
- 2 µl (200 ng) genomische DNA von *E. coli*
- 0,2 mM dATP, dTTP, dGTP, dCTP
- 1,5 mM Mg(OAc)₂
- 5 µg Rinderserum-Albumin
- 40 pmol Primer "codA5'HindIII"
- 40 pmol Primer codA3'SalI
- 15 µl 3,3x rTth DNA Polymerase XLPuffer (PE Applied Biosystems)
- 5U rTth'DNA Polymerase XL (PE Applied Biosystems)

Die PCR wird unter folgenden Zyklus-Bedingungen durchgeführt:

| | | |
|---|---|---|
| Schritt 1: 5 Minuten | 94°C | (Denaturierung) |
| Schritt 2: 3 Sekunden | 94°C | |
| Schritt 3: 1 Minute | 60°C | (Annealing) |
| Schritt 4: 2 Minuten | 72°C | (Elongation) |

30 Wiederholungen der Schritte 2 bis 4

| |
|---|
| Schritt 5: 10 Minuten 72°C (Post-Elongation) |
| Schritt 6.: 4°C (Warteschleife) |

Das Amplifikat (codARNAi-sense; SEQ ID NO: 49) wird unter Verwendung von Standardmethoden in den PCR Klonierungsvektor pGEM-T (Promega) kloniert. Die Identität des erzeugten Amplikons wird durch Sequenzierung unter Verwendung des M13F (-40) Primers bestätigt.

Ein weiteres verkürztes und am 5'- bzw. 3'-Ende durch Addition von Erkennungssequenzen der Restriktionsenzyme EcoRI und BamHI modifiziertes Fragment des codA Gens wird mittels Polymerasekettenreaktion (PCR) aus *E.coli* unter Verwendung eines sense-spezifischen Primers (codA5'EcoRI; SEQ ID NO: 53) und eines antisense-spezifischen Primers (codA3'BamHI; SEQ ID NO: 54) amplifiziert.
codA5'EcoRI: 5'-GAATTCGGCTAACAGTGTCGAATAACG-3' (SEQ ID NO: 53)
codA3'BamHI: 5'-GGATCCGACAAAATCCCTTCCTGAGG-3' (SEQ ID NO: 54)

Die PCR erfolgt in einem 50 µl Reaktionsansatz in dem enthalten sind:
- 2 µl (200 ng)genomische DNA von *E. coli*
- 0,2 mM dATP, dTTP, dGTP, dCTP
- 1,5 mM Mg(OAc)₂
- 5 µg Rinderserum-Albumin
- 40 pmol Primer "codA5'EcoRI"
- 40 pmol Primer "codA3'BamHI"
- 15 µl 3,3x rTth DNA Polymerase XLPuffer (PE Applied Biosystems)
- 5U rTth DNA Polymerase XL (PE Applied Biosystems)

Die PCR wird unter folgenden Zyklus Bedingungen durchgeführt:

| | | |
|---|---|---|
| Schritt 1: 5 Minuten | 94°C | (Denaturierung) |
| Schritt 2: 3 Sekunden | 94°C | |
| Schritt 3: 1 Minute | 60°C | (Annealing) |
| Schritt 4: 2 Minuten | 72°C | (Elongation) |

30 Wiederholungen der Schritte 2 bis 4

| |
|---|
| Schritt 5: 10 Minuten 72°C (Post-Elongation) |
| Schritt 6: 4°C (Warteschleife) |

Das Amplifikat (codARNAi-anti; SEQ ID NO: 52) wird unter Verwendung von Staindardmethoden in den PCR Klonierungsvektor pGEM-T (Promega) kloniert. Die Identität des erzeugten Amplikons wird durch Sequenzierung unter Verwendung des M13F (-40) Primers bestätigt.

### Beispiel 2 Herstellung des transgenen Expressionsverktors zur Expression einer codA doppelsträngigen RNA

Die in Beispiel 1 generierten codA-Fragmente werden zur Herstellung eines DNA Konstruktes geeignet zur Expression einer doppelsträngigen codA-RNA verwendet (pSUN-codA-RNAi). Das Konstrukt ist geeignet zur Reduktion der RNA Fließgleichgewichtsmenge (RNA-stady state level) des codA Gens in transgenen Pflanzen und einer daraus resultierenden Unterdrückung der Expression des codA Gens unter Verwendung der "doublestrand RNA interference" (dsRNAi) Technologie. Die codA RNAi Kassette wird dazu zunächst in dem Plasmid pBluKS-nitP-STLS1-35S-T aufgebaut und anschließend in einem weiteren Klonierungsschritt vollständig in das pSUN-1 Plasmid überführt.

Der Vektor pBluKS-nitP-STLS1-35S-T (SEQ ID NO: 55) ist ein Derivat des pBluescript KS (Stratagene) und enthält den Promotor des NitrilaseI-Gens aus A. thaliana (GenBank Acc.-No.: Y07648.2, Nukleotide 2456 bis 4340, Hillebrand et al. (1996) Gene 170:197-200), das STLS-1 Intron (Vancanneyt GF et al. (1990) Mol Gen Genet 220(2):245-250), Restriktionsschnittstellen die das Intron an der 5'- bzw. 3'-Seite flankieren und eine gerichtete, Insertion von DNA Fragmenten ermöglichen, sowie den Terminator des 35S CaMV Gens (Blumenkohlmosaikvirus; Franck et al. (1980) Cell 21:285-294). Unter Verwendung dieser Restriktionsschnittstellen (HindIII, SalI, EcoRI, BamHI) werden die Fragmente codARNAi-sense (SEQ ID NO: 49) und codARNAi-anti (SEQ ID NO: 52) in diesen Vektor inseriert, wodurch die fertige codA RNAi Kassette entsteht.

Zu diesem Zweck wird zunächst das codA-sense Fragment (codARNAi-sense SEQ ID NO: 49) unter Verwendung der Enzyme HindIII und SalI aus dem pGEM-T Vektor herausgeschnitten, isoliert und in den pBluKS-nitP-STLS1-35S-T Vektor unter Standardbedingungen ligiert. Dieser Vektor wurde im Vorfeld unter Verwendung der Restriktionsenzyme HindIII und SalI geschnitten. Entsprechend positive Klone werden durch analytischen Restriktionsverdau und Sequenzierung identifiziert.

Der entstandene Vektor (pBluKS-nitP-codAsense-STLS1-35S-T) wird unter Verwendung der Restriktionsenzyme BamHI und EcoRI verdaut. Das codA-anti Fragment (codARNAi-anti; SEQ ID NO: 52) wird aus dem entsprechenden pGEM-T Vektor mit BamHI und EcoRI herausgeschnitten, isoliert und in den geschnittenen Vektor unter Standardbedingungen ligiert. Entsprechend positive Klone, welche die vollständige codA-RNAi Kassette enthalten (pBluKS-nitP-codAsense-STLS1-codAanti-35S-T), werden durch analytischen Restriktionsverdau und Sequenzierung identifiziert.

Der Transfer der codA-RNAi Kassette in den pSUN-1 Vektor (SEQ ID NO: 56) erfolgt unter Verwendung der die Kassette flankierenden Restriktionsschhitt-stellen SacI und KpnI. Der entstandene Vektor pSUN1-codA-RNAi (siehe Fig. 7: SEQ ID NO: 57) wird zur Transformation von transgenen *A. thaliana* Pflanzen verwendet, die ein aktives *codA* Gen exprimieren (s.u.). Der Pflanzenexpressions-Vektor pSUN-1 ist im Rahmen des erfindungsgemäßen Verfahrens besonders geeignet, da er keinen weiteren positiven Selektionsmarker trägt.

Der entstandene Vektor pSUN1-codA-RNAi ermöglicht die konstitutive Expression einer artifiziellen codA-dsRNA Variante, bestehend aus zwei identischen Nukleinsäureelementen, die durch ein Intron getrennt, in invertierter Form zueinander vorliegen. In Folge der Transkription dieser artifiziellen *codA*-dsRNA Variante kommt es aufgrund der Komplementarität der invertierten Nukleinsäureelemente zur Ausbildung eines doppelsträngigen RNA Moleküls. Das Vorhandensein dieses Moleküls induziert die Unterdrückung der Expression (RNA Akkumulation) des codA Gens mittels "double strand RNA interference".

### Beispiel 4: Herstellung transgener Arabidopis thaliana Pflanzen

Transgene Arabidopsis thaliana Pflanzen, die als Markerprotein das codA Gen aus E.coli transgen exprimieren ("A.thaliana-[codA]"), wurden hergestellt wie beschrieben (Kirik et al. (2000) EMBO J 19(20):5562-6).

Die A.thaliana-[codA] Pflanzen werden mit einem Agrobacterium tumefaciens Stamm (GV3101 [pMP90]) auf Grundlage einer modifizierten Vakuuminfiltrationsmethode transformiert (Clough S & Bent A (1998) Plant J 16(6):735-43; Bechtold N et al. (1993) CR Acad Sci Paris 1144(2):204-212). Die verwendeten *Agrobacterium tumefaciens* Zellen werden im Vorfeld mit dem beschriebenen DNA-Konstrukt (pSUNl-codA-RNAi) transformiert. Auf diese Art werden doppelt-transgene A.thaliana-[codA] Pflanzen erzeugt, die unter Kontrolle des konstitutiven Nitrilase1-Promotors eine artifizielle codA-doppelsträngige RNA exprimieren. Als Folge des durch die Anwesenheit dieser artifiziellen codA-dsRNA induzierten dsRNAi-Effektes wird die Expression des codA Gens unterdrückt. Diese doppelt-transgenen Pflanzen können aufgrund ihrer wiedergewonnen Fähigkeit, in Anwesenheit von 5-Fluorocytosin im Kulturmedium zu wachsen, identifiziert werden.

Samen der Primärtransformanden werden auf Grundlage der wiedergewonnenen Fähigkeit in Anwesenheit von 5-Fluorcytosin zu wachsen selektioniert. Zu diesem Zweck werden die T1 Samen der Primärtransformanden auf Selektionsmedium ausgelegt welches 200 µg/ml 5-Fluorocytosin enthält. Diese Selektionsplatten werden unter Langtagbedingungen (16 Std. Licht, 21°C/8 Std. Dunkel, 18°C) inkubiert. Keimlinge, die sich in Anwesenheit von 5-Flourocytosin normal entwickeln, werden nach 7 Tagen separiert und auf neue Selektionsplatten transferiert. Diese Platten werden bei unveränderten Bedingungen für weitere 14 inkubiert. Anschließend werden die resistenten Keimlinge in Erde pikiert und unter Kurztagbedingungen (8 Std. Licht, 21°C/16 Std. Dunkel, 18°C) kultiviert. Nach 14 Tagen werden die jungen Pflanzen in das Gewächshaus transferiert und unter Kurztagbedingungen kultiviert.

### Beispiel 5: Herstellung eines Pflanzentransformationsvektors enthaltend eine Expressions-kassette zur Expression einer doppelsträngigen codA RNA und eines pflanzlichen Selektions-markers

In den pSUN1-codA-RNAi (siehe Fig. 7; SEQ ID NO: 57) wird ein pflanzlicher Selektionsmarker bestehend aus einer mutierten Variante des A.thaliana Als-Gens kodierend für die Acetolactat-Synthase unter Kontrolle des Promotor des A.thaliana Actin-2 Gens (Meagher RB & Williamson RE (1994) The plant cytoskeleton. In The Plant Cytoskeleton (Meyerowitz, E. & Somerville, C., eds), pp. 1049-1084. Cold Spring Harbor Laboratory Press, Cold Spring Harbor, New York) und des Terminators der Octopin-Synthase (GIE-LEN J et al.(1984) EMBO J 3:835-846) eingefügt (At.Act.-2-At.Als-R-ocsT).

Dazu wird der Vektor pSUN1-codA-RNAi zunächst mit dem Restriktionsenzym Pvu II linearisiert. In diesen linearisierten Vektor erfolgt anschließend unter Standardbedingungen die Ligation eines linearen DNA Fragmentes mit stumpfen Enden, kodierend für eine mutierte Variante der Acetolactat-Synthase (Als-R-Gen). Dieses DNA Fragment wurde im Vorfeld der Ligation mit dem Restriktionsenzym KpnI verdaut und die überhängenden Enden durch eine Behandlung mit der Pwo DNA-Polymerase (Roche) gemäß der Herstellervorgaben in stumpfe Enden überführt. Diese mutierte Variante des Als Gens aus A.thaliana kann nicht durch Herbizide des Imidazolinon-Typs inhibiert werden. Durch Expression dieses mutierten A.tAls-R Gens erlangen die Pflanzen die Fähigkeit, in Anwesenheit des Herbizides Pursiut^{™} zu wachsen. Entsprechend positive Klone (pSUN1-codA-RNAi-At.Act.-2-At.Als-R-ocsT; SEQ ID NO: 57) werden durch analytischen Restriktionsverdau und Sequenzierung identifiziert.

Der entstandene Vektor ermöglicht die konstitutive Expression einer artifiziellen codA RNA Variante (bestehend aus zwei identischen Nukleinsäureelementen, die durch ein Intron getrennt, in invertierter Form zueinander vorliegen) und einer mutierten Variante des A.thaliana Als-Gens. In Folge der Transkription dieser artifiziellen codA RNA Variante kommt es aufgrund der Komplementarität der invertierten Nukleinsäureelemente zur Ausbildung eines doppelsträngigen RNA Moleküls. Das Vorhandensein dieses Moleküls induziert die Unterdrückung der Expression (RNA Akkumulation) des codA Gens mittels "double strand RNA interference". Die Expression des Als-R Gens vermittelt den Pflanzen die Fähigkeit, in Anwesenheit von Herbiziden des imidazolinon-Typs zu wachsen.

### Beispiel 6: Herstellung transgener Arabidopis thaliana Pflanzen

Transgene Arabidopsis thaliana Pflanzen, die als Markerprotein das codA Gen aus E.coli exprimieren ("A.thaliana- [codA]"), wurden wie beschrieben (Kirik et al.(2000) EMBO J 19(20) : 5562-6) hergestellt.

Die A.thaliana-[codA] Pflanzen werden mit einem Agrobacterium tumefaciens Stamm (GV3101 [pMP90]) auf Grundlage einer modifizierten Vakuuminfiltrationsmethode transformiert (Clough S & Bent A (1998) Plant J 16(6):735-43; Bechtold N et al. (1993) CR Acad Sci Paris 1144(2):204-212). Die verwendeten Agrobacterium tumefaciens Zellen werden im Vorfeld mit dem beschriebenen DNA-Konstrukt (pSUN1-codA-RNAi-At.Act.-2-At.Als-R-ocsT; SEQ ID NO: 57) transformiert. Auf diese Art werden doppelttransgene A.thaliana-[codA] Pflanzen erzeugt, die zusätzlich unter Kontrolle des konstitutiven Nitrilasel-Promotors eine artifizielle codA doppelsträngige RNA und eine Herbizid insensitive Variante des Als-Gens (Als-R) exprimieren (A.thaliana-[codA]-[codA-RNAi-At.Act.-2-At.Als-R-ocsT]). Als Folge des durch die Anwesenheit dieser artifiziellen codA-dsRNA induzierten dsRNAi-Effektes wird die Expression des codA Gens unterdrückt. Diese doppelttransgenen Pflanzen können aufgrund ihrer wiedergewonnen Fähigkeit, in Anwesenheit von 5-Flourocytosin im Kulturmedium zu wachsen, identifiziert werden. Zusätzlich können positiv transformierte Pflanzen aufgrund ihrer Fähigkeit, in Anwesenheit des Herbizides Pursuit im Kulturmedium zu wachsen, selektioniert werden.

Zum Zweck der Selektion werden daher die T1 Samen der Primärtransformanden auf Selektionsmedium ausgelegt welches 100 µg/ml 5-Fluorocytosin enthält. Diese Selektionsplatten werden unter Langtagbedingungen (16 Std. Licht, 21°C/8 Std. Dunkel, 18°C) inkubiert. Keimlinge, die sich in Anwesenheit von 5-Flourocytosin normal entwickeln, werden nach 28 Tagen separiert und auf neue Selektionsplatten transferiert. Diese Platten werden bei unveränderten Bedingungen für weitere 14 Tage inkubiert. Anschließend werden die resistenten Keimlinge in Erde pikiert und unter Kurztagbedingungen (8 std. Licht, 21°C/16 Std. Dunkel, 18°C) kultiviert. Nach weiteren 14 Tagen werden die jungen Pflanzen in das Gewächshaus transferiert und unter Kurztagbedingungen kultiviert.

Zusätzlich können Samen der Primärtransformanden - aufgrund ihrer Fähigkeit in Anwesenheit des Herbizides Pursiut^{™} zu wachsen - selektioniert werden. Es ist weiterhin möglich, eine Doppelselektion unter Verwendung des Herbizides Pursiut^{™} und 5-Fluorcytosin im Selektionsmedium durchzuführen. Zu diesem Zweck werden die T1 Samen der Primärtransformanden auf Selektionsmedium ausgelegt welches das Herbizid Pursuit^{™} in einer Konzentration von 100 nM enthält (im Falle der Doppelselektion ist ebenfalls 100 µg/ml 5-Fluorocytosin enthalten). Diese Selektionsplatten werden unter Langtagbedingungen (16 Std. Licht, 21°C/B Std. Dunkel, 18°C) inkubiert.

Keimlinge, die sich in Anwesenheit von Pursuit^{™} (Pursiut^{™} und 5-Fluorocytosin) normal entwickeln, werden nach 28 Tagen separiert und auf neue Selektionsplatten transferiert. Diese Platten werden bei unveränderten Bedingungen für weitere 14 Tage inkubiert. Anschließend werden die resistenten Keimlinge in Erde pikiert und unter Kurztagbedingungen (8 std. Licht, 21°C/16 Std. Dunkel, 18°C) kultiviert. Nach 14 Tagen werden die jungen Pflanzen in das Gewächshaus transferiert und unter Kurztagbedingungen kultiviert.

### Beispiel 7: Analyse der unter Verwendung von 5-Fluorocytosin und/oder Pursuit selektionierten doppelttransgenen A.thaliana Pflanzen (A.thaliana-[codA1]-[codA-RNAi-At.Act.-2-At.Als-R-ocsT])

Die Integration der T-DNA Region des zur Transformation verwendeten Vektors pSUN1-codA-RNAi-A.tAls-R in die genomische DNA der Ausgangspflanze (A.thaliana-[codA]) und der Verlust der codA spezifischen mRNA in diesen transgenen Pflanzen (A.thaliana-[codA]-[codA-RNAi- At.Act.-2-At.Als-R-ocsT]) kann unter Anwendung von Southern-Analysen und PCR Techniken bzw. Northern-Analysen nachgewiesen werden.

Um diese Analysen durchzuführen wird gesamt RNA und DNA (unter Verwendung des RNeasy Maxi Kit (RNA) bzw. Dneasy Plant Maxi Kit (genomische DNA) gemäß Herstellerangaben von Qiagen) aus Blattgewebe der transgenen Pflanzen und geeigneter Kontrollen isoliert.
Bei den PCR Analysen kann die genomische DNA direkt als Grundlage (Template) der PCR verwendet werden. Die gesamt-RNA wird im Vorfeld der PCR in cDNA umgeschrieben. Die cDNA Synthese erfolgt unter Verwendung der Reversen Transkriptase Superscript II (Invitrogen) gemäß der Herstellerangaben.

### Beispiel 8: Nachweis der Reduktion der codA RNA Fleißgleichgewichtsmenge in den positiv selektionierten doppelttransgenen Pflanzen (A.thaliana [codA]-[codA-RNAi-At.Act.-2-At.Als-R-ocsT]) im Vergleich zu den zur Transformation verwendeten Ausgangspflanzen (A.thaliana [codA]) durch cDNA Synthese mit anschließender PCR Amplifikation.

### PCR Amplifikation der codA spezifischen cDNA:

Die cDNA des codA Gens (ACCESSION S56903) kann unter Verwendung eines sense spezifischen Primers (codA5'C-term SEQ ID NO: 69) und eines antisense spezifischen Primers (codA3'C-term SEQ ID NO: 70) amplifiziert werden. Zu wählende µPCR Bedingungen sind die folgenden:
Die PCR erfolgte in einem 50 µl Reaktionsansatz in dem enthalten ist:
   - 2µl (200ng)cDNA aus A.thaliana -[codA] bzw. A.thaliana [codA]-[codA-RNAi-At.Act.-2-At.Als-R-ocsT]-Pflanzen
   - 0,2 mM dATP, dTTP, dGTP, dCTP
   - 1,5 mM Mg(OAc)2
   - 5 µg Rinderserum-Albumin
   - 40 pmol codA5'C-term SEQ ID NO: 69
   - 40 pmol codA3'C-term SEQ ID NO: 70
   - 15 µl 3,3x rTth DNA Polymerase XLPuffer (PE Applied Biosystems)
   - 5U rTth DNA Polymerase XL (PE Applied Biosystems)
Die PCR wurde unter folgenden Zyklus Bedingungen durchgeführt:
   Schritt 1: 5 Minuten 94°C (Denaturierung)
   Schritt 2: 3 Sekunden 94°C
   Schritt 3: 1 Minute 56°C (Annealing)
   Schritt 4: 2 Minuten 72°C (Elongation)
   30 Wiederholungen der Schritte 2 bis 4
   Schritt 5: 10 Minuten 72°C (Post-Elongation)
   Schritt 6: 4°C (Warteschleife)

In den positiv selektionierten Pflanzen ist die mRNA Fließgleichgewichtsmenge des codA Gens und die daraus resultierende Menge an CODA-Proteins derart reduziert, dass eine quantitative Umsetzung von 5-Fluorocytosin zu 5-Fluorouracil nicht mehr erfolgen kann. Die Folge ist, dass diese Pflanzen (im Gegensatz zu den nicht transformierten Pflanzen) in Anwesenheit von 5-Fluorocytosin wachsen können. Somit wird nachgewiesen, dass transgene Pflanzen aufgrund des angewendeten Prinzips der Verhinderung der Expression eines negativen Selektionsmarkers identifiziert werden können.

### Beispiel 9: Nachweis der für die codA-RNAi kodierenden DNA unter Verwendung genomischer DNA der positiv selektionierten doppelttransgenen Pflanzen (A.thaliana [codA]-[codA-RNAi- At.Act.-2-At.Als-R-ocsT])

Das codA-RNAi Transgen kann unter Verwendung eines codA spezifischen Primers (z.B codA5'HindIII SEQ ID NO: 50) und eines 35s-Terminator spezifischen Primers amplifiziert (35sT 5' Primer SEQ ID NO: 71) werden. Durch Verwendung dieser Primerkombination kann spezifisch nur die für das codA RNAi Konstrukt codierende DNA nachgewiesen werden, da das codA Gen, welches in den zur Transformation verwendeten Ausgangspflanzen (A.thaliana [codA]) bereits vorhanden war, durch den nos-Terminator flankiert wird.

Zu wählende PCR Bedingungen sind die folgenden:
Die PCR erfolgte in einem 50 µl Reaktionsansatz in dem enthalten ist:
   - 2 µl (200ng) genomische DNA aus den A.thaliana [codA]-[codA-RNAi-At.Act.-2-At.Als-R-ocsT]-Pflanzen
   - 0,2 mM dATP, dTTP, dGTP, dCTP
   - 1,5 mM Mg(OAc)2
   - 5 µg Rinderserum-Albumin
   - 40 pmol codA spezifischer sense Primer (SEQ ID NO: 50, 53 oder 69)
   - 40 pmol 35sT 5' Primer SEQ ID NO: 71
   - 15 µl 3,3x rTth DNA Polymerase XLPuffer (PE Applied Biosystems)
   - 5U rTth DNA Polymerase XL (PE Applied Biosystems)
Die PCR wurde unter folgenden Zyklus Bedingungen durchgeführt:
   Schritt 1: 5 Minuten 94°C (Denaturierung)
   Schritt 2: 3 Sekunden 94°C
   Schritt 3: 1 Minute 56°C (Annealing)
   Schritt 4: 2 Minuten 72°C (Elongation)
   30 Wiederholungen der Schritte 2-4
   Schritt 5: 10 Minuten 72°C (Post-Elongation)
   Schritt 6: 4°C (Warteschleife)

In den positiv selektionierten Pflanzen kann auf diesem Weg die Integration des codA-RNAi DNA Konstruktes in die chromosomale DNA der zur Transformation verwendeten Ausgangspflanzen nachgewiesen werden. Somit wird nachgewiesen, dass transgene Pflanzen aufgrund des angewendeten Prinzips der Verhinderung der Expression eines negativen Selektionsmarkers identifiziert werden können.

### Beispiel 10: Nachweis der Reduktion der codA RNA Fleißgleichgewichtsmenge in den positiv selektionierten doppelttransgenen Pflanzen (A.thaliana [codA]-[codA-RNAi-At.Act.-2-At.Als-R-ocsT]) im Vergleich zu den zur Transformation verwendeten Ausgangspflanzen (A. thaliana [codA]) durch Northern-Analyse.

### Gelelektrophoretische Auftrennung von RNA:

Es wird pro RNA-Agarosegel 3 g Agar in 150 ml H₂O (f.c. 1,5 % (w/v)) in der Mikrowelle gelöst und auf 60°C abgekühlt. Durch Zugabe von 20.ml 10x MEN (0,2 M MOPS, 50 mM Natriumacetat, 10 mM EDTA) und 30 ml Formaldehyd (f.c. 2,2 M) tritt weitere Abkühlung ein, so dass die gut gemischte Lösung zügig gegossen werden muss. Formaldehyd verhindert die Bildung von Sekundärstrukturen in der RNA, deshalb ist die Laufgeschwindigkeit dem Molekulargewicht annähernd proportional (LEHRBACH H et al. (1977) Biochem J 16: 4743-4751). Die RNA-Proben werden vor Auftrag auf das Gel in folgendem Ansatz denaturiert: 20 µl RNA (1-2 µg/µl), 5 µl 10x MEN-Puffer, 6 µl Formaldehyd, 20 µl Formamid.

Der Ansatz wird gemischt und 10 Minuten bei 65°C inkubiert. Nach Zugabe von 1/10 Volumen Probenpuffer und 1 µl Ethidiumbromid (10 mg/ml) wird die Probe aufgetragen. Die Gelelektrophorese erfolgt auf horizontalen Gelen in 1x MEN bei 120 V für zwei bis drei Stunden. Nach der Elektrophorese wird das Gel unter UV-Licht unter Zuhilfenahme eines Lineals zur späteren Fragmentlängenbestimmung photographiert. Es folgt der RNA-Blot auf eine Nylonmembran gemäß der Angaben in: SAMBROOK J et al. Molecular cloning: A laboratory manual. Cold Spring Harbor, New York, Cold Spring Harbor Laboratory Press. 1989.

### Radioaktive Markierung von DNA-Fragmenten und Nothern Hybridisierung

Zur Markierung des codA cDNA Fragmentes (codARNAi-sense SEQ ID No: 49) kann z.B der von Roche Diagnostics vertriebene High Prime Kit verwendet werden. Der "High prime" Kit basiert auf der von Feinberg und Vogelstein ursprünglich beschriebenen "random primed" Methode zur DNA Markierung. Zur Markierung werden ca. 25 ng DNA in 9-11 µl H20 für 10 min. bei 95°C denaturiert. Nach kurzer Inkubation auf Eis werden 4 µl High Prime Lösung (enthält eine random primer Mischung, 4 Einheiten Klenow Polymerase und jeweils 0,125 mM dATP, dTTP und dGTP in einem Reaktions-Puffer mit 50 % Glycerinanteil) und 3-5 µl [α32P]dCTP (30-50 µCi) hinzugegeben. Der Ansatz wird für mindestens 10 min bei 37°C inkubiert und das nicht eingebaute dCTP anschließend von der nunmehr radioaktiv markierten DNA durch Gelfiltration über eine Sephadex G-50-Säule getrennt. Anschließend wird das Fragment 10 min bei 95°C denaturiert und bis zur Verwendung auf Eis gehalten. Es werden folgende Hybridisierungs- und Vorinkubationspuffer benutzt:
Hypo Hybond
   250 mM Natriumphosphat-Puffer pH 7,2
   1 mM EDTA
   7 % SDS (g/v)
   250 mM NaCl
   10 µg/ml ssDNA
   5 % Polyethylenglykol (PEG) 6000
   40 % Formamid

Bei Verwendung von Hypo Hybond beträgt die Hybridisierungstemperatur 42°C, die Hybridisierungsdauer 16-24 Std. Zum Waschen der RNA-Filter werden drei verschiedene Lösungen verwendet 2 x SSC (300 mM NaCl; 30 mM NaCitrat) + 0,1% SDS, 1 x SSC + 0,1% SDS und 0,1 x SSC + 0,1 % SDS. Die Länge und Intensität des Waschens richtet sich nach der Stärke der gebundenen Aktivität. Im Anschluss an das Waschen werden die Filter in Plastikfolie eingeschweißt und ein Röntgenfilm (X-0Mat, Kodak) bei -70 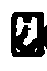 C über Nacht exponiert. Die Signalstärke auf den Röntgenfilmen ist ein Maß für die Menge der codA mRNA Moleküle in der auf den Membranen gebundenen gesamt RNA. In den positiv selektionierten Pflanzen kann somit die Reduktion der codA mRNA im Vergleich zu den zur Transformation verwendeten Ausgangspflanzen nachgewiesen werden.

In den positiv selektionierten Pflanzen ist die mRNA Fließgleichgewichtsmenge des codA Gens und die resultierende Menge an gebildeten CODA-Proteins derart reduziert, dass eine quantitative Umsetzung von 5-Fluorocytosin zu 5-Fluorouracil nicht mehr erfolgen kann. Die Folge ist, dass diese Pflanzen (im Gegensatz zu den nicht transformierten Pflanzen) in Anwesenheit von 5-Fluorocytosin wachsen können. Somit wird nachgewiesen, dass transgene Pflanzen aufgrund des angewendeten Prinzips der Verhinderung der Expression eines negativen Selektionsmarkers identifiziert werden können.

### Beispiel 11: Zusammenfassung der Ergebnisse der "Negativ-Negativ" Selektion

Die Transformation der codA-transgenen Arabidopsis Pflanzen mit dem codA-dsRNA Konstrukt (pSUN1-codA-RNAi-At.Act.-2-At.Als-R-ocsT; SEQ ID NO: 57) führt sowohl bei der Einfach-Selektion (nut mit 5-Fluorcytosin) als auch bei der Doppel-Selektion (Pursiut^{™} und 5-Fluorcytosin) zu einer signifikant erhöhten Anzahl von doppelt-transgene Pflanzen, die das RNAi-Konstrukt erfolgreich in das Genom integriert haben (jeweils im Vergleich zu nicht transformierten Pflanzen). Die Analyse mittels PCR (s.o.) bestätigt bei der Mehrzahl der so generierten Pflanzen den doppelt transgenen Status. Damit kann erfolgreich die Praktikabilität der vorliegenden Erfindung d.h. die Nutzbarkeit der Repression eines negativen Markers zur positiven Selektion (quasi eine "negativ-negativ" Selektion) gezeigt werden.

## Patentansprüche

1. Verwendung eines doppelsträngigem RNA-Moleküls umfassend
a) einen "sense"-RNA-Strang umfassend mindestens eine Ribonukleotidsequenz, die im wesentlichen identisch ist zu mindestens einem Teil des "sense"-RNA-Transkriptes einer Nukleinsäuresequenz kodierend für ein Markerprotein das direkt oder indirekt einen toxischen Effekt bewirken kann" und
b) einen "antisense"-RNA-Strang, der zu dem RNA-sense-Strang unter a) im wesentlichen - bevorzugt vollständig - komplementären ist
zur Verminderung der Expression, Menge, Aktivität und/oder Funktion besagten Markerproteins in einer transformierten pflanzlichen Zelle, die infolge der Wirkung des doppelsträngigem RNA-Moleküls gegenüber nicht-transformierten Zellen einen Wachstumsvorteil hat und so selektiert werden kann, wobei die Selektion unter Bedingungen durchgeführt wird, bei denen das Markerprotein seinen toxischen Effekt auf die nichttransformierten Zellen ausüben kann.

2. Verwendung eines doppelsträngigem RNA-Moleküls nach Anspruch 1, wobei das Markerprotein in der Lage ist, eine für besagte Population pflanzlicher Zellen nicht-toxische Substanz X in eine für besagte Population toxische Substanz Y direkt oder indirekt umzusetzen.

3. Verwendung eines doppelsträngigem RNA-Moleküls nach Anspruch 2, wobei es sich bei der nicht-toxischen Substanz X um eine Substanz handelt, die natürlicherweise in pflanzlichen Zellen oder Organismen nicht oder nur in Konzentration vorkommt, die im wesentlichen keinen toxischen Effekt bewirken können.

4. Verwendung eines doppelsträngigem RNA-Moleküls nach Anspruch 2 oder 3, wobei es sich bei der Substanz X um eine Substanz handelt ausgewählt aus der Gruppe bestehend aus Pro-Herbiziden, Pro-Antibiotika, Nukleosidanaloga, 5-Fluorocytosin, Auxinamidverbindungen, Naphthalacetamid, Dihaloalkanen, Acyclovir, Ganciclovir, 1,2-Deoxy-2- fluoro-b-D-arabinofuranosil-5-iodouracil, 6-Thioxanthin, Allopurinol, 6-Methylpurindeoxyribonukleosid, 4-Aminopyrazolopyrimidin, 2-Amino-4-methoxy-butansäure, 5-(Trifluoromethyl)thioribose und Allylalkohol.

5. Verwendung eines doppelsträngigem RNA-Moleküls nach einem der Ansprüche 1 bis 4, wobei das Markerprotein ausgewählt ist aus der Gruppe bestehend aus Cytosindeaminasen, Cytochrom P-450 Enyzmen, Indolessigsäurehydrolasen, Haloalkandehalogenasen, Thymidinkinasen, Guaninphosphoribosyltransferasen, Hypoxanthinphosphoribosyltransferasen, Xanthinguaninphosphoribosyltransferasen, Purinnukleosidphosphorylasen, Phosphonatmonoesterhydrolasen, Indolacetamidsynthasen, Indolacetamidhydrolasen, Adeninphosphoribosyltransferasen, Methoxinindehydrogenasen, Rhizobitoxinsynthasen, 5-Methylthioribosekinasen und Alkoholdehydrogenasen.

6. Verwendung eines doppelsträngigem RNA-Moleküls nach einem der Ansprüche 1 bis 5, wobei das Markerprotein kodiert wird durch eine Sequenz gemäß SEQ ID NO: 2, 4, 6, 8, 10, 12, 14, 16, 18, 20, 22, 24, 26, 28, 30, 32, 34, 36, 38, 40, 42, 44, 46 oder 48.

7. Verwendung eines doppelsträngigem RNA-Moleküls nach einem der Ansprüche 1 bis 6, wobei "sense"-RNA-Strang und "antisense"-RNA-Strang kovalent in Form eines invertierten Repeats miteinander verbunden sind.

## Claims

1. The use of a double-stranded RNA molecule comprising
a) a "sense" RNA strand comprising at least one ribonucleotide sequence which is essentially identical to at least a part of the "sense" RNA transcript of a nucleic acid sequence coding for a marker protein which may directly or indirectly cause a toxic effect, and
b) an "antisense" RNA strand which is essentially, preferably fully, complementary to the RNA sense strand under a),
for reducing the expression, amount, activity and/or function of said marker protein in a transformed plant cell which has a growth advantage over nontransformed cells, due to the action of the double-stranded RNA molecule, and can thus be selected, the selection being carried out under conditions under which the marker protein can exert its toxic effect on the nontransformed cells.

2. The use of a double-stranded RNA molecule according to claim 1, wherein the marker protein is capable of converting directly or indirectly a substance X which is nontoxic for said population of plant cells into a substance Y which is toxic for said population.

3. The use of a double-stranded RNA molecule according to claim 2, wherein the nontoxic substance X is a substance which does not naturally occur in plant cells or organisms or occurs naturally therein only at a concentration which can essentially not cause any toxic effect.

4. The use of a double-stranded RNA molecule according to claim 2 or 3, wherein the substance X is a substance selected from the group consisting of proherbicides, proantibiotics, nucleoside analogs, 5-fluorocytosine, auxinamide compounds, naphthalacetamide, dihaloalkanes, Acyclovir, Ganciclovir, 1,2-deoxy-2-fluoro-b-D-arabinofuranosil-5-iodouracil, 6-thioxanthine, allopurinol, 6-methylpurine deoxyribonucleoside, 4-aminopyrazolopyrimidine, 2-amino-4-methoxybutanoic acid, 5-(trifluoromethyl)thioribose and allyl alcohol.

5. The use of a double-stranded RNA molecule according to any of claims 1 to 4, wherein the marker protein is selected from the group consisting of cytosine deaminases, cytochrome P-450 enzymes, indoleacetic acid hydrolases, haloalkane dehalogenases, thymidine kinases, guanine phosphoribosyl transferases, hypoxanthine phosphoribosyl transferases, xanthine guanine phosphoribosyl transferases, purine nucleoside phosphorylases, phosphonate monoester hydrolases, indoleacetamide synthases, indoleacetamide hydrolases, adenine phosphoribosyl transferases, methoxinine dehydrogenases, rhizobitoxin synthases, 5-methylthioribose kinases and alcohol dehydrogenases.

6. The use of a double-stranded RNA molecule according to any of claims 1 to 5, wherein the marker protein is encoded by a sequence according to SEQ ID NO: 2, 4, 6, 8, 10, 12, 14, 16, 18, 20, 22, 24, 26, 28, 30, 32, 34, 36, 38, 40, 42, 44, 46 or 48.

7. The use of a double-stranded RNA molecule according to any of claims 1 to 6, wherein the "sense" RNA strand and the "antisense" RNA strand are covalently linked to one another in the form of an inverted repeat.

## Revendications

1. Utilisation d'une molécule d'ARN double brin, comprenant
a) un brin d'ARN "sens", comprenant au moins une séquence ribonucléotidique, qui pour l'essentiel est identique à au moins une partie du transcrit d'ARN "sens" d'une séquence d'acide nucléique codant pour une protéine marqueur qui peut avoir directement ou indirectement un effet toxique, et
b) un brin d'ARN "antisens", qui pour l'essentiel est complémentaire, et de préférence complètement complémentaire, du brin sens d'ARN de a),
pour diminuer l'expression, la quantité, l'activité et/ou la fonction de ladite protéine marqueur dans une cellule végétale transformée, qui en conséquence de l'action de la molécule d'ARN double brin vis-à-vis de cellules non transformées présente un avantage de croissance et ainsi peut être sélectionnée, la sélection étant mise en oeuvre dans des conditions dans lesquelles la protéine marqueur peut exercer son effet toxique sur les cellules non transformées.

2. Utilisation d'une molécule d'ARN double brin selon la revendication 1, la protéine marqueur étant à même de convertir directement ou indirectement une substance X, non toxique pour la population mentionnée de cellules végétales, en une substance Y toxique pour ladite population.

3. Utilisation d'une molécule d'ARN double brin selon la revendication 2, dans laquelle, pour ce qui concerne la substance non toxique X, il s'agit d'une substance qui n'apparaît pas naturellement dans des cellules végétales ou dans des organismes végétaux, ou seulement à des concentrations qui pour l'essentiel ne peuvent avoir aucun effet toxique.

4. Utilisation d'une molécule d'ARN double brin selon la revendication 2 ou 3, pour laquelle, pour ce qui concerne la substance X, il s'agit d'une substance choisie dans le groupe consistant en les proherbicides, les pro-antibiotiques, les analogues de nucléosides, la 5-fluorocytosine, les composés auxinamides, le naphtalacétamide, les dihalogénoalcanes, l'acyclovir, le ganciclovir, le 1,2-désoxy-2-fluoro-b-D-arabinofurannosyl-5-iodouracile, la 6-thioxanthine, l'allopurinol, le 6-méthylpurinedésoxyribonucléoside, la 4-aminopyrazolopyrimidine, l'acide 2-amino-4-méthoxy-butanoïque, le 5-(trifluorométhyl)thioribose et l'alcool allylique.

5. Utilisation d'une molécule d'ARN double brin selon l'une des revendications 1 à 4, pour laquelle la protéine marqueur est choisie dans le groupe consistant en les cytosinedésaminases, les enzymes cytochrome P-450, les (acide indolacétique)hydrolases, les halogènalcane-déshalogénases, les thymidinekinases, les guaninephosphoribosyltransférases, les hypoxanthinephosphoribosyltransférases, les xanthineguaninephosphoribosyltransférases, les purinenucléosidephosphorylases, les phosphonate-monoester-hydrolases, les indolacétamide-synthases, les indolacétamide-hydrolases, les adéninephosphoribosyltransférases, les méthoxinine-déshydrogénases, les rhizobitoxine-synthases, les 5-méthylthioribosekinases et les alcool-déshydrogénases.

6. Utilisation d'une molécule d'ARN double brin selon l'une des revendications 1 à 5, pour laquelle la protéine marqueur est codée par une séquence selon SEQ ID N° 2, 4, 6, 8, 10, 12, 14, 16, 18, 20, 22, 24, 26, 28, 30, 32, 34, 36, 38, 40, 42, 44, 46 ou 48.

7. Utilisation d'une molécule d'ARN double brin selon l'une des revendications 1 à 6, pour laquelle le brin d'ARN "sens" et le brin d'ARN "antisens" sont liés l'un à l'autre d'une manière covalente sous forme d'une répétition inversée.
